# EUROPEAN PATENT SPECIFICATION

(11) **EP 4 332 087 B1**
(45) Date of publication and mention of the grant of the patent: **29.07.2026**
(21) Application number: 23163495.7
(22) Date of filing: 22.03.2023
(51) Int. Cl.: C07C 229/12, A61K 9/00, C07D 207/08, C12N 15/11, C07D 211/08, C07D 211/16, C07D 223/04, C07D 265/30, A61K 9/51, C07C 235/06, C07C 271/22, C07C 275/16, C07C 327/22, C07C 329/06

(54) **LIPID NANOPARTICLES**
LIPIDNANOPARTIKEL
NANOPARTICULES LIPIDIQUES

(30) Priority: 29.04.2022 CN 202210478132; 29.04.2022 CN 202210478559; 26.10.2022 CN 202211319726; 14.11.2022 CN 202211419648
(43) Date of publication of application: 06.03.2024
(73) Proprietor: Beijing Jitai Life Sciences Ltd., Beijing 100094 (CN); Metis TechBio Co., Ltd., Beijing 102629 (CN); Hangzhou Jitai Life Sciences Ltd, Hangzhou, Zhejiang, 310051 (CN)
(72) Inventor: YANG, Liu, BEIJING, 100192 (CN); YIN, Le, BEIJING, 100192 (CN); LIU, Andong, BEIJING, 100192 (CN); ZHANG, Lin, BEIJING, 100192 (CN); LAI, Caida, ZHEJIANG, 310051 (CN); WANG, Wenshou, ZHEJIANG, 310051 (CN)
(74) Representative: Cabinet Becker et Associés

(56) References cited:
- WO-A1-2013/086322
- WO-A1-2014/089239
- US-B1- 11 246 933

## Description

### CROSS REFERENCE TO RELATED APPLICATIONS

The present application claims the priority of the Chinese Patent Application No. 202210478559.X filed on April 29, 2022, Chinese Patent Application No. 202210478132.X filed on April 29, 2022, Chinese Patent Application No. 202211319726.2 filed on October 26, 2022, and Chinese Patent Application No. 202211419648.3 filed on November 14, 2022.

### FIELD OF THE INVENTION

The present disclosure relates to a nanoparticle composition wherein the lipid ingredient comprises a new class of ionizable cationic lipid compound, or pharmaceutically acceptable salts, isotopic variants, tautomers or stereoisomers thereof. The present disclosure also relates to a pharmaceutical composition comprising the nanoparticle composition.

### BACKGROUND OF THE INVENTION

Gene therapy refers to the introduction of exogenous genes into target cells to correct or compensate for genetic defects or abnormalities within the cell, so as to achieve the purpose of treatment. In the past few decades, research related to the treatment of clinical diseases through gene therapy has received more and more attention. Especially in recent years, siRNA-related drugs and mRNA vaccines have been approved by the FDA for clinical treatment, which has further promoted research and related investment in the field of gene therapy.

Due to their unique nature, nucleic acid drugs are able to act on non-druggable targets that are difficult to be acted upon by conventional small molecule chemical drugs, proteins or antibodies, and have significant applications values in the treatment or prevention of diseases such as tumors, infectious diseases and genetic diseases.

However, nucleic acid substances are easily degraded by nucleases in organisms, and nucleic acid substances themselves are negatively charged, which makes it difficult for them to enter the cell through the cell membrane. Thus, delivery technology is a major challenge in nucleic acid drug development.

As a nucleic acid delivery material, lipid nanoparticles (LNP) are one of the most important nucleic acid delivery systems with the advantages of easy to prepare, good biodegradability, no immunogenicity and good safety. The nucleic acid vaccine developed by Modema and BioNTech uses LNP as the delivery system, and the main components of LNP include cationic lipids, cholesterol, neutral lipids and polyethylene glycol-conjugated lipids. Among them, cationic lipid molecules are the core of LNP delivery system, and their molecular structure plays a decisive role in the delivery efficiency, targeting, and formulation stability, and the like, of the entire liposome nanoparticles. US 11246933 B1 has disclosed lipid nanoparticle biodegradable compositions comprising a cationic ionizable lipid, structural lipids, neutral lipids,and PEGylated lipids.

Due to the different requirements of the delivery system for the delivery of different kinds of nucleic acid substances and the specific delivery of different targets, in order to meet the different needs of gene therapy, further development of new lipid molecules and continuous improvement of LNP technology are needed to obtain new LNP formulations with good nucleic acid delivery efficiency.

### SUMMARY OF THE INVENTION

The present disclosure develops a new class of lipid nanoparticles that can be used to deliver various biologically active substances with high delivery efficiency.

In one aspect, the present disclosure provides a nanoparticle composition, comprising a lipid ingredient, and optionally comprising a load;
wherein the lipid ingredient comprises the following components in the molar percentages:
Ionizable cationic lipids 20 mol%-85 mol%:
Structure lipids 10 mol%-75 mol%;
Neutral lipids 1.0 mol%-30 mol%;
Polymer lipids 0.25 mol%-10 mol%;
wherein the ionizable cationic lipid is the compound of formula (VI) or formula (VII), or a pharmaceutically acceptable salt, isotopic variant, tautomer or stereoisomer thereof, 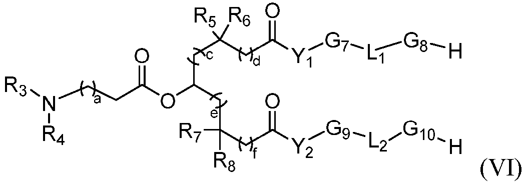 or 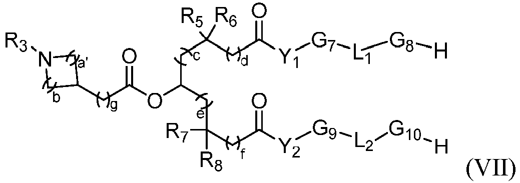 wherein,
   a, a', b and g are independently 0, 1, 2, 3, 4 or 5, a' and b are not 0 at the same time;
   a'+g=0, 1, 2, 3, 4 or 5;
   c and e are independently 3, 4, 5, 6, 7, 8 or 9;
   d and f are independently 0, 1, 2, 3 or 4;
   c+d=3, 4, 5, 6, 7, 8 or 9, e+f=3, 4, 5, 6, 7, 8 or 9;
   methylenes in 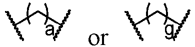 are optionally and independently substituted with 1, 2, 3, 4 or 5 C₁₋₆ alkyl;
   R₃ and R₄ are independently H, C₁₋₆ alkyl, C₁₋₆ haloalkyl, 3- to 10-membered cycloalkyl or 3- to 10-membered heterocyclyl, which is optionally substituted with 1, 2, 3, 4 or 5 R*;
   or, R₃ and R₄ are taken together with the N atom to which they are attached to form 3- to 10-membered heterocyclyl, which is optionally substituted with 1, 2, 3, 4 or 5 R*;
   R* is independently H, halogen, cyano, C₁₋₆ alkyl, C₁₋₆ haloalkyl, -L_{b}-OR_{b} or -L_{b}-NR_{b}R'_{b};
   R₅, R₆, R₇ and R₈ are independently C₁₋₃ alkyl;
   Y₁ and Y₂ are independently O, S or NRₐ;
   L₁ and L₂ are independently -(CRR')₂-, -CH=CH-, -C≡C- or -NR''-;
   G₇, G₈, G₉ and G₁₀ are independently a chemical bond or C₁₋₁₂ alkylene, which is optionally substituted with 1, 2, 3, 4, 5 or 6 R;
   G₇ and G₈ have a total length of 6, 7, 8, 9, 10, 11 or 12 carbon atoms;
   G₉ and G₁₀ have a total length of 6, 7, 8, 9, 10, 11 or 12 carbon atoms;
   R and R' are independently H, C₁₋₁₄ alkyl, -Lₐ-ORₐ or -Lₐ-NRₐR'ₐ;
   Lₐ is independently a chemical bond or C₁₋₁₄ alkylene;
   L_{b} is independently a chemical bond or C₁₋₆ alkylene;
   Rₐ and R'ₐ are independently H, C₁₋₁₄ alkyl, 3- to 10-membered cycloalkyl or 3- to 10-membered heterocyclyl;
   R_{b} and R'_{b} are independently H, C₁₋₆ alkyl, 3- to 10-membered cycloalkyl or 3- to 10-membered heterocyclyl;
   R'' is independently H or C₁₋₁₄ alkyl;
   alternatively, wherein in the compound of formula (VI) or formula (VII),
   a is 0, 1, 2, 3 or 4, alternatively 1, 2, 3 or 4, alternatively 2, 3 or 4;
   a' and b are independently 0, 1, 2, 3 or 4, alternatively 2;
   g is 0, 1 or 2, alternatively 0 or 1;
   a'+g=0, 1, 2, 3, 4 or 5, alternatively a'+g=2 or 3;
   c and e are independently 3, 4, 5 or 6;
   d and f are independently 0, 1 or 2;
   c+d=4, 5 or 6, e+f=4, 5 or 6;
   methylenes in 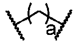 are optionally and independently substituted with 1, 2, 3, 4 or 5 C₁₋₆ alkyl;
   methylenes in 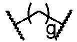 are optionally and independently substituted with 1 or 2 C₁₋₆ alkyl;
   R₃ and R₄ are independently C₁₋₆ alkyl, which is optionally substituted with 1, 2 or 3 R*;
   R* is independently H, C₁₋₆ alkyl, C₁₋₆ haloalkyl or -OR_{b};
   or, R₃ and R₄ are taken together with the N atom to which they are attached to form 3- to 7-membered heterocyclyl, alternatively 5-membered heterocyclyl, which is optionally substituted with 1, 2 or 3 R*;
   R₅, R₆, R₇ and R₈ are independently C₁₋₃ alkyl;
   Y₁ and Y₂ are independently O, S or NRₐ, alternatively O or S;
   L₁ and L₂ are independently -(CHR)₂-, -CH=CH-, -C≡C- or -NR''-, alternatively -(CHR)₂-, -CH=CH- or -C≡C-;
   G₇ and G₉ are independently a chemical bond or C₁₋₆ alkylene;
   G₈ and G₁₀ are independently C₁₋₁₀ alkylene;
   G₇ and G₈ have a total length of 6, 7, 8, 9 or 10 carbon atoms;
   G₉ and G₁₀ have a total length of 6, 7, 8, 9 or 10 carbon atoms;
   1, 2 or 3 methylenes in G₇, G₈, G₉ or G₁₀ are optionally and independently substituted with 1 R;
   R is independently H or C₁₋₈ alkyl;
   R'' is independently H or C₁₋₁₀ alkyl;
   Rₐ is independently H or C₁₋₁₀ alkyl;
   R_{b} is independently H or C₁₋₆ alkyl, alternatively H.

US11246933B1 discloses that the incorporation of the biodegradable group(s) into the tail chain of a lipid compound in a lipid nanoparticle results in faster metabolism and removal of the lipid from the body following delivery of the active agent to a target area. As a result, these lipids which contain the biodegradable groups have lower toxicity than similar lipids without the biodegradable groups. The tail chain of the cationic lipid compound of the present disclosure has biodegradable group(s), thereby has superior toxicity profile to similar lipids without biodegradable groups, such as DLin-MC3-DMA.

In another aspect, the present disclosure provides a pharmaceutical composition, comprising the nanoparticle composition of the present disclosure, and pharmaceutically acceptable excipient(s).

In another aspect, the present disclosure provides a nanoparticle composition of the present disclosure, or a pharmaceutical composition of the present disclosure, for use in treating, diagnosing, and/or preventing a disease.

In another aspect, the present disclosure provides a nanoparticle composition of the present disclosure, or a pharmaceutical composition of the present disclosure, for use in delivering a load.

In a specific embodiment, the load is selected from one or more of therapeutic agents, prophylactic agents, and diagnostic agents; alternatively, the therapeutic agent, prophylactic agent, or diagnostic agent is a nucleic acid.

In a more specific embodiment, the nucleic acid is selected from one or more of ASO, RNA and DNA.

In a more specific embodiment, the RNA is selected from one or more of interfering RNA (RNAi), small interfering RNA (siRNA), short hairpin RNA (shRNA), antisense RNA (aRNA), messenger RNA (mRNA), modified messenger RNA (mmRNA), long non-coding RNA (lncRNA), microRNA (miRNA), small activating RNA (saRNA), multimeric coding nucleic acid (MCNA), polymeric coding nucleic acid (PCNA), guide RNA (gRNA), CRISPRRNA (crRNA) and nucleases, alternatively mRNA, more alternatively modified mRNA.

### TERMINOLOGY

### Chemical terminology

Terminology of specific functional groups and chemical terms are described in more detail below.

When a range of values is listed, it is intended to encompass each value and sub-range within the range. For example, "C₁₋₆ alkyl" is intended to include C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅ and C₅₋₆ alkyl.

"C₁₋₂₈ alkyl" refers to a radical of a linear or branched, saturated hydrocarbon group having 1 to 28 carbon atoms. In some embodiments, C₄₋₂₈ alkyl, C₄₋₂₄ alkyl, C₄₋₂₀ alkyl, C₈₋₁₀ alkyl, C₂₋₈ alkyl, C₇₋₉ alkyl, C₄₋₆ alkyl, C₁₋₂₀ alkyl, C₁₋₁₄ alkyl, C₂₋₁₄ alkyl, C₁₋₁₃ alkyl, C₁₋₁₂ alkyl, C₁₋₁₀ alkyl, C₁₋₈ alkyl, C₁₋₇ alkyl, C₂₋₇ alkyl, C₁₋₆ alkyl, C₁₋₅ alkyl, C₅ alkyl, C₁₋₄ alkyl, C₂₋₄ alkyl, C₁₋₃ alkyl, C₂₋₃ alkyl, C₁₋₂ alkyl and Me are alternative. Examples of C₁₋₆ alkyl include methyl (C₁), ethyl (C₂), n-propyl (C₃), iso-propyl (C₃), n-butyl (C₄), tert-butyl (C₄), sec-butyl (C₄), iso-butyl (C₄), n-pentyl (C₅), 3-pentyl (C₅), pentyl (C₅), neopentyl (C₅), 3-methyl-2-butyl (Cs), tert-pentyl (C₅) and n-hexyl (C₆). The term "C₁₋₆ alkyl" also includes heteroalkyl, wherein one or more (e.g., 1, 2, 3 or 4) carbon atoms are substituted with heteroatoms (e.g., oxygen, sulfur, nitrogen, boron, silicon, phosphorus). Alkyl groups can be optionally substituted with one or more substituents, for example, with 1 to 5 substituents, 1 to 3 substituents or 1 substituent. Conventional abbreviations of alkyl include Me (-CH₃), Et (-CH₂CH₃), iPr (-CH(CH₃)₂), nPr (-CH₂CH₂CH₃), n-Bu (-CH₂CH₂CH₂CH₃) or i-Bu (-CH₂CH(CH₃)₂).

"C₂₋₂₀ alkenyl" refers to a radical of a linear or branched hydrocarbon group having 2 to 20 carbon atoms and at least one carbon-carbon double bond. "C₄₋₂₈ alkenyl" refers to a radical of a linear or branched hydrocarbon group having 4 to 28 carbon atoms and at least one carbon-carbon double bond. In some embodiments, C₄₋₂₀ alkenyl, C₂₋₁₃ alkenyl, C₂₋₁₀ alkenyl, C₂₋₆ alkenyl, and C₂₋₄ alkenyl is alternative. Examples of C₂₋₆ alkenyl include vinyl (C₂), 1-propenyl (C₃), 2-propenyl (C₃), 1-butenyl (C₄), 2-butenyl (C₄), butadienyl (C₄), pentenyl (C₅), pentadienyl (C₅), hexenyl (C₆), etc. The term "C₂₋₆ alkenyl" also includes heteroalkenyl, wherein one or more (e.g., 1, 2, 3 or 4) carbon atoms are replaced by heteroatoms (e.g., oxygen, sulfur, nitrogen, boron, silicon, phosphorus). The alkenyl groups can be optionally substituted with one or more substituents, for example, with 1 to 5 substituents, 1 to 3 substituents or 1 substituent.

"C₂₋₂₀ alkynyl" refers to a radical of a linear or branched hydrocarbon group having 2 to 20 carbon atoms, at least one carbon-carbon triple bond and optionally one or more carbon-carbon double bonds. "C₄₋₂₈ alkynyl" refers to a radical of a linear or branched hydrocarbon group having 4 to 28 carbon atoms, at least one carbon-carbon triple bond and optionally one or more carbon-carbon double bonds. In some embodiments, C₄₋₂₀ alkynyl, C₂₋₁₃ alkynyl, C₂₋₁₀ alkynyl, C₂₋₆ alkynyl, and C₂₋₄ alkynyl is alternative. Examples of C₂₋₆ alkynyl include, but are not limited to, ethynyl (C₂), 1-propynyl (C₃), 2-propynyl (C₃), 1-butynyl (C₄), 2-butynyl (C₄), pentynyl (C₅), hexynyl (C₆), etc. The term "C₂₋₆ alkynyl" also includes heteroalkynyl, wherein one or more (e.g., 1, 2, 3 or 4) carbon atoms are replaced by heteroatoms (e.g., oxygen, sulfur, nitrogen, boron, silicon, phosphorus). The alkynyl groups can be substituted with one or more substituents, for example, with 1 to 5 substituents, 1 to 3 substituents or 1 substituent.

"C₁₋₂₀ alkylene" refers to a divalent group formed by removing another hydrogen of the C₁₋₂₀ alkyl, and can be substituted or unsubstituted. In some embodiments, C₄₋₂₀ alkylene, C₈₋₁₀ alkylene, C₂₋₈ alkylene, C₇₋₉ alkylene, C₄₋₆ alkylene, C₁₋₂₀ alkylene, C₁₋₁₄ alkylene, C₂₋₁₄ alkylene, C₁₋₁₃ alkylene, C₁₋₁₂ alkylene, C₁₋₁₀ alkylene, C₁₋₈ alkylene, C₁₋₇ alkylene, C₂₋₇ alkylene, C₁₋₆ alkylene, C₁₋₅ alkylene, C₅ alkylene, C₁₋₄ alkylene, C₂₋₄ alkylene, C₁₋₃ alkylene, C₂₋₃ alkylene, C₁₋₂ alkylene, and methylene are alternative. The unsubstituted alkylene groups include, but are not limited to, methylene (-CH₂-), ethylene (-CH₂CH₂-), propylene (-CH₂CH₂CH₂-), butylene (-CH₂CH₂CH₂CH₂-), pentylene (-CH₂CH₂CH₂CH₂CH₂-), hexylene (-CH₂CH₂CH₂CH₂CH₂CH₂-), etc. Examples of substituted alkylene groups, such as those substituted with one or more alkyl (methyl) groups, include, but are not limited to, substituted methylene (-CH(CH₃)-, -C(CH₃)₂-), substituted ethylene (-CH(CH₃)CH₂-, -CH₂CH(CH₃)-, -C(CH₃)₂CH₂-, -CH₂C(CH₃)₂-), substituted propylene (-CH(CH₃)CH₂CH₂-, -CH₂CH(CH₃)CH₂-, -CH₂CH₂CH(CH₃)-, -C(CH₃)₂CH₂CH₂-, -CH₂C(CH₃)₂CH₂-, -CH₂CH₂C(CH₃)₂-), etc.

"C₂₋₁₃ alkenylene" refers to a C₂₋₁₃ alkenyl group wherein another hydrogen is removed to provide a divalent radical of alkenylene, and which may be substituted or unsubstituted. In some embodiments, C₂₋₁₀ alkenyl, C₂₋₆ alkenyl, and C₂₋₄ alkenylene is yet alternative. Exemplary unsubstituted alkenylene groups include, but are not limited to, ethylene (-CH=CH-) and propenylene (e.g., -CH=CHCH₂-, -CH₂-CH=CH-). Exemplary substituted alkenylene groups, e.g., substituted with one or more alkyl (methyl) groups, include but are not limited to, substituted ethylene (-C(CH₃)=CH-, -CH=C(CH₃)-), substituted propylene (e.g., -C(CH₃)=CHCH₂-, -CH=C(CH₃)CH₂-, -CH=CHCH(CH₃)-, -CH=CHC(CH₃)₂-, -CH(CH₃)-CH=CH-, -C(CH₃)₂-CH=CH-, -CH₂-C(CH₃)=CH-, -CH₂-CH=C(CH₃)-).

"C₂₋₁₃ alkynylene" refers to a C₂₋₁₃ alkynyl group wherein another hydrogen is removed to provide a divalent radical of alkynylene, and which may be substituted or unsubstituted. In some embodiments, C₂₋₁₀ alkynylene, C₂₋₆ alkynylene, and C₂₋₄ alkynylene is yet alternative. Exemplary alkynylene groups include, but are not limited to, ethynylene (-C≡C-), substituted or unsubstituted propynylene (-C≡CCH₂-).

"C₀₋₆ alkylene" refers to the chemical bond and the "C₁₋₆ alkylene" described above, "C₀₋₄ alkylene" refers to the chemical bond and the"C₁₋₄ alkylene" described above.

The term "variable A and variable B have a total length of x carbon atoms" means that the total number of carbon atoms of the main chain in the group represented by variable A and the number of carbon atoms of the main chain in the group represented by variable B is x.

"Halo" or "halogen" refers to fluorine (F), chlorine (Cl), bromine (Br) and iodine (I).

Thus, "C₁₋₁₀ haloalkyl" refers to the above "C₁₋₁₀ alkyl", which is substituted by one or more halogen. In some embodiments, C₁₋₆ haloalkyl and C₁₋₄ haloalkyl is yet alternative, and still alternatively C₁₋₂ haloalkyl. Exemplary haloalkyl groups include, but are not limited to, -CF₃, -CH₂F, -CHF₂, -CHFCH₂F, -CH₂CHF₂, -CF₂CF₃, -CCl₃, -CH₂Cl, -CHCl₂, 2,2,2-trifluoro-1,1-dimethyl-ethyl. The haloalkyl can be substituted at any available point of attachment, for example, with 1 to 5 substituents, 1 to 3 substituents or 1 substituent.

"C₃₋₁₄ cycloalkyl" or "3- to 14-membered cycloalkyl" refers to a radical of a non-aromatic cyclic hydrocarbon group having from 3 to 14 ring carbon atoms and zero heteroatoms, optionally wherein 1, 2 or 3 double or triple bonds are contained. In some embodiments, 3- to 10-membered cycloalkyl, 5- to 10-membered cycloalkyl, 3- to 8-membered cycloalkyl, 3- to 7-membered cycloalkyl, 3- to 6-membered cycloalkylare yet alternative, and still alternatively 5- to 7-membered cycloalkyl, 4- to 6-membered cycloalkyl, 5- to 6-membered cycloalkyl, 5-membered cycloalkyl, and 6-membered cycloalkyl. The cycloalkyl also includes a ring system in which the cycloalkyl ring described above is fused with one or more aryl or heteroaryl groups, wherein the point of attachment is on the cycloalkyl ring, and in such case, the number of carbon atoms continues to represent the number of carbon atoms in the cycloalkyl system. The cycloalkyl further comprises the cycloalkyl described above, in which the substituents on any non-adjacent carbon atoms are connected to form a bridged ring, together forming a polycyclic alkane sharing two or more carbon atoms. The cycloalkyl further comprises the cycloalkyl described above, in which the substituents on the same carbon atom are connected to form a ring, together forming a polycyclic alkane sharing one carbon atom. Exemplary cycloalkyl groups include, but are not limited to, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇), cycloheptadienyl (C₇), cycloheptatrienyl (C₇), etc. The cycloalkyl can be substituted with one or more substituents, for example, with 1 to 5 substituents, 1 to 3 substituents or 1 substituent.

"C₃₋₁₀ cycloalkylene" refers to a divalent radical formed by removing another hydrogen of C₃₋₁₀ cycloalkyl group and may be substituted or unsubstituted. In some embodiments, C₃₋₆ cycloalkylene and C₃₋₄ cycloalkylene groups are particularly alternative, especially alternatively cyclopropylene.

"3- to 14-membered heterocyclyl" refers to a saturated or unsaturated radical of 3- to 14-membered non-aromatic ring system having ring carbon atoms and 1 to 5 ring heteroatoms, wherein each of the heteroatoms is independently selected from nitrogen, oxygen, sulfur, boron, phosphorus and silicon, optionally wherein 1, 2 or 3 double or triple bonds are contained. In the heterocyclyl containing one or more nitrogen atoms, the point of attachment can be a carbon or nitrogen atom as long as the valence permits. In some embodiments, 3- to 10-membered heterocyclyl is alternative, which is a radical of 3- to 10-membered non-aromatic ring system having ring carbon atoms and 1 to 5 ring heteroatoms; in some embodiments, 5- to 10-membered heterocyclyl is alternative, which is a radical of 5- to 10-membered non-aromatic ring system having ring carbon atoms and 1 to 5 ring heteroatoms; in some embodiments, 3- to 8-membered heterocyclyl is alternative, which is a radical of 3- to 8-membered non-aromatic ring system having ring carbon atoms and 1 to 4 ring heteroatoms; in some embodiments, 3- to 7-membered heterocyclyl is alternative, which is a radical of 3- to 7-membered non-aromatic ring system having ring carbon atoms and 1 to 4 ring heteroatoms; 5- to 7-membered heterocyclyl is alternative, which is a radical of 5- to 7-membered non-aromatic ring system having ring carbon atoms and 1 to 3 ring heteroatoms; 3- to 6-membered heterocyclyl is alternative, which is a radical of 3- to 6-membered non-aromatic ring system having ring carbon atoms and 1 to 3 ring heteroatoms; 4- to 6-membered heterocyclyl is alternative, which is a radical of 4- to 6-membered non-aromatic ring system having ring carbon atoms and 1 to 3 ring heteroatoms; 5- to 6-membered heterocyclyl is more alternative, which is a radical of 5- to 6-membered non-aromatic ring system having ring carbon atoms and 1 to 3 ring heteroatoms; 5-membered heterocyclyl is more alternative, which is a radical of 5-membered non-aromatic ring system having ring carbon atoms and 1 to 3 ring heteroatoms; 6-membered heterocyclyl is more alternative, which is a radical of 6-membered non-aromatic ring system having ring carbon atoms and 1 to 3 ring heteroatoms. The heterocyclyl also includes a ring system wherein the heterocyclyl described above is fused with one or more cycloalkyl groups, wherein the point of attachment is on the heterocyclyl ring, or the heterocyclyl described above is fused with one or more aryl or heteroaryl groups, wherein the point of attachment is on the heterocyclyl ring; and in such cases, the number of ring members continues to represent the number of ring members in the heterocyclyl ring system. The heterocyclyl further comprises the heterocyclyl described above, in which the substituents on any non-adjacent carbon or nitrogen atoms are connected to form a bridge ring, together forming a polycyclic heteroalkane sharing two or more carbon or nitrogen atoms. The heterocyclyl further comprises the heterocyclyl described above, in which the substituents on the same carbon atom are connected to form a ring, together forming a polycyclic heteroalkane sharing one carbon atom. Exemplary 3-membered heterocyclyl groups containing one heteroatom include, but are not limited to, aziridinyl, oxiranyl and thiorenyl. Exemplary 4-membered heterocyclyl groups containing one heteroatom include, but are not limited to, azetidinyl, oxetanyl and thietanyl. Exemplary 5-membered heterocyclyl groups containing one heteroatom include, but are not limited to, tetrahydrofuranyl, dihydrofuranyl, tetrahydrothiophenyl, dihydrothienyl, pyrrolidinyl, dihydropyrrolyl and pyrrolyl-2,5-dione. Exemplary 5-membered heterocyclyl groups containing two heteroatoms include, but are not limited to, pyrazolidyl, dioxolanyl, oxasulfuranyl, disulfuranyl, and oxazolidin-2-one. Exemplary 5-membered heterocyclyl groups containing three heteroatoms include, but are not limited to, triazolinyl, oxadiazolinyl, and thiadiazolinyl. Exemplary 6-membered heterocyclyl groups containing one heteroatom include, but are not limited to, piperidyl, tetrahydropyranyl, dihydropyridyl and thianyl. Exemplary 6-membered heterocyclyl groups containing two heteroatoms include, but are not limited to, piperazinyl, morpholinyl, dithianyl and dioxanyl. Exemplary 6-membered heterocyclyl groups containing three heteroatoms include, but are not limited to, triazinanyl. Exemplary 7-membered heterocyclyl groups containing one heteroatom include, but are not limited to, azepanyl, oxepanyl and thiepanyl. Exemplary 5-membered heterocyclyl groups fused with a C₆ aryl (also referred as 5,6-bicyclic heterocyclyl herein) include, but are not limited to, indolinyl, isoindolinyl, dihydrobenzofuranyl, dihydrobenzothiophenyl, benzoxazolinonyl, etc. Exemplary 6-membered heterocyclyl groups fused with a C₆ aryl (also referred as 6,6-bicyclic heterocyclyl herein) include, but are not limited to, tetrahydroquinolinyl, tetrahydroisoquinolinyl, etc. The heterocyclyl further includes the heterocyclyl described above sharing one or two atoms with a cycloalkyl, heterocyclyl, aryl or heteroaryl to form a bridged or spiro ring, as long as the valence permits, where the shared atom may be carbon or nitrogen atoms. The heterocyclyl further includes the heterocyclyl described above, which optionally can be substituted with one or more substituents, e.g., with 1 to 5 substituents, 1 to 3 substituents or 1 substituent.

"C₆₋₁₀ aryl" refers to a radical of monocyclic or polycyclic (e.g., bicyclic) 4n+2 aromatic ring system having 6-10 ring carbon atoms and zero heteroatoms (e.g., having 6 or 10 shared π electrons in a cyclic array). In some embodiments, the aryl group has six ring carbon atoms ("C₆ aryl"; for example, phenyl). In some embodiments, the aryl group has ten ring carbon atoms ("C₁₀ aryl"; for example, naphthyl, e.g., 1-naphthyl and 2-naphthyl). The aryl group also includes a ring system in which the aryl ring described above is fused with one or more cycloalkyl or heterocyclyl groups, and the point of attachment is on the aryl ring, in which case the number of carbon atoms continues to represent the number of carbon atoms in the aryl ring system. The aryl can be substituted with one or more substituents, for example, with 1 to 5 substituents, 1 to 3 substituents or 1 substituent.

"5- to 14-membered heteroaryl" refers to a radical of 5- to 14-membered monocyclic or bicyclic 4n+2 aromatic ring system (e.g., having 6, 10 or 14 shared π electrons in a cyclic array) having ring carbon atoms and 1-4 ring heteroatoms, wherein each heteroatom is independently selected from nitrogen, oxygen and sulfur. In the heteroaryl group containing one or more nitrogen atoms, the point of attachment can be a carbon or nitrogen atom as long as the valence permits. heteroaryl bicyclic systems may include one or more heteroatoms in one or two rings. heteroaryl also includes ring systems wherein the heteroaryl ring described above is fused with one or more cycloalkyl or heterocyclyl groups, and the point of attachment is on the heteroaryl ring. In such case, the number the carbon atoms continues to represent the number of carbon atoms in the heteroaryl ring system. In some embodiments, 5-to 10-membered heteroaryl groups are alternative, which are radicals of 5- to 10-membered monocyclic or bicyclic 4n+2 aromatic ring systems having ring carbon atoms and 1-4 ring heteroatoms. In other embodiments, 5- to 6-membered heteroaryl groups are yet alternative, which are radicals of 5- to 6-membered monocyclic or bicyclic 4n+2 aromatic ring systems having ring carbon atoms and 1-4 ring heteroatoms. Exemplary 5-membered heteroaryl groups containing one heteroatom include, but are not limited to, pyrrolyl, furyl and thienyl. Exemplary 5-membered heteroaryl groups containing two heteroatoms include, but are not limited to, imidazolyl, pyrazolyl, oxazolyl, isoxazolyl, thiazolyl, and isothiazolyl. Exemplary 5-membered heteroaryl groups containing three heteroatoms include, but are not limited to, triazolyl, oxadiazolyl (such as, 1,2,4-oxadiazolyl), and thiadiazolyl. Exemplary 5-membered heteroaryl groups containing four heteroatoms include, but are not limited to, tetrazolyl. Exemplary 6-membered heteroaryl groups containing one heteroatom include, but are not limited to, pyridyl or pyridonyl. Exemplary 6-membered heteroaryl groups containing two heteroatoms include, but are not limited to, pyridazinyl, pyrimidinyl, and pyrazinyl. Exemplary 6-membered heteroaryl groups containing three or four heteroatoms include, but are not limited to, triazinyl and tetrazinyl, respectively. Exemplary 7-membered heteroaryl groups containing one heteroatom include, but are not limited to, azepinyl, oxepinyl, and thiepinyl. Exemplary 5,6-bicyclic heteroaryl groups include, but are not limited to, indolyl, isoindolyl, indazolyl, benzotriazolyl, benzothiophenyl, isobenzothiophenyl, benzofuranyl, benzoisofuranyl, benzimidazolyl, benzoxazolyl, benzoisoxazolyl, benzoxadiazolyl, benzothiazolyl, benzoisothiazolyl, benzothiadiazolyl, indolizinyl and purinyl. Exemplary 6,6-bicyclic heteroaryl groups include, but are not limited to, naphthyridinyl, pteridinyl, quinolyl, isoquinolyl, cinnolinyl, quinoxalinyl, phthalazinyl and quinazolinyl. The heteroaryl can be substituted with one or more substituents, for example, with 1 to 5 substituents, 1 to 3 substituents or 1 substituent.

"Hydroxyalkyl" refers to an alkyl group that is substituted with one or more hydroxyl groups.

"Alkoxy" refers to an oxyether form of a linear or branched-chain alkyl group, i.e., an -O-alkyl group. Similarly, "methoxy" refers to -O-CH₃.

"Optionally substituted with" means that it can be substituted with the specified substituents or unsubstituted.

The divalent groups formed by removing another hydrogen from the groups defined above such as alkyl, alkenyl, alkynyl, cycloalkyl, heterocyclyl, aryl and heteroaryl are collectively referred to as "-ylene". Ring-forming groups such as cycloalkyl, heterocyclyl, aryl and heteroaryl are collectively referred to as "cyclic groups".

alkyl, alkenyl, alkynyl, cycloalkyl, heterocyclyl, aryl and heteroaryl as defined herein are optionally substituted groups.

Exemplary substituents on carbon atoms include, but are not limited to, halogen, -CN, -NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa}, -CO₂H, -CHO, -C(OR^{cc})₂, -CO₂R^{aa}, -OC(=O)R^{aa}, -OCO₂R^{aa}, -C(=O)N(R^{bb})2, -OC(=O)N(R^{bb})₂, -NR^{bb}C(=O)R^{aa}, -NR^{bb}CO₂R^{aa}, -NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa}, -C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa}, -NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃, -C(=S)N(R^{bb})₂, -C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)₂R^{aa}, -OP(=O)₂R^{aa}, -P(=O)(R^{aa})₂, -OP(=O)(R^{aa})₂, -OP(=O)(OR^{cc})₂, -P(=O)₂N(Rbb)₂, -OP(=O)₂N(R^{bb})₂, -P(=O)(NR^{bb})₂, -OP(=O)(Nk^{bb})₂, -NR^{bb}P(=O)(OR^{cc})₂, -NR^{bb}P(=O)(NR^{bb})₂, -P(R^{cc})₂, -P(R^{cc})₃, -OP(R^{cc})₂, -OP(R^{cc})₃, -B(R^{aa})₂, -B(OR^{cc})₂, -BR^{aa}(OR^{cc}), alkyl, haloalkyl, alkenyl, alkynyl, cycloalkyl, heterocyclyl, aryl and heteroaryl, wherein each of the alkyl, alkenyl, alkynyl, cycloalkyl, heterocyclyl, aryl and heteroaryl is independently substituted with 0, 1, 2, 3, 4 or 5 R^{dd} groups;
or two geminal hydrogen on a carbon atom are replaced with =O, =S, =NN(R^{bb})₂, =NNR^{bb}C(=O)R^{aa}, =NNR^{bb}C(=O)OR^{aa}, =NNR^{bb}S(=O)2R^{aa}, =NR^{bb} or =NOR^{cc} groups;
each of the R^{aa} is independently selected from alkyl, haloalkyl, alkenyl, alkynyl, cycloalkyl, heterocyclyl, aryl and heteroaryl, or two of the R^{aa} groups are combined to form a heterocyclyl or heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups;
each of the R^{bb} is independently selected from hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{cc})OR^{aa}, -C(=NR^{cc})N(R^{cc})₂₁ -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl and heteroaryl, or two R^{bb} groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups;
each of the R^{cc} is independently selected from hydrogen, alkyl, haloalkyl, alkenyl, alkynyl, cycloalkyl, heterocyclyl, aryl and heteroaryl, or two R^{cc} groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups;
each of the R^{dd} is independently selected from halogen, -CN, -NO₂, -N₃, -SO₂H, -SO₃H, -OH, -OR^{ee}, -ON(R^{ff})₂, -N(R^{ff})₂, -N(R^{ff})₃⁺X⁻, -N(OR^{ee})Rff, -SH, -SR^{ee}, -SSR^{ee}, -C(=O)R^{ee}, -CO₂H, -CO₂R^{ee}, -OC(=O)R^{ee}, -OCO₂R^{ee}, -C(=O)N(R^{ff})₂, -OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, -OC(=NR^{ff})R^{ee}, -OC(=NR^{ff})OR^{ee}, -C(=NR^{ff})N(R^{ff})₂, -OC(=NR^{ff})N(R^{ff})₂, -NR^{ff}C(=NR^{ff})N(R^{ff})₂, -NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, -C(=S)SR^{ee}, -SC(=S)SR^{ee}, -P(=O)₂R^{ee}, -P(=O)(R^{ee})₂, -OP(=O)(R^{ee})₂, -OP(=O)(OR^{ee})₂, alkyl, haloalkyl, alkenyl, alkynyl, cycloalkyl, heterocyclyl, aryl, heteroaryl, wherein each of the alkyl, alkenyl, alkynyl, cycloalkyl, heterocyclyl, aryl and heteroaryl is independently substituted with 0, 1, 2, 3, 4 or 5 R^{gg} groups, or two geminal R^{dd} substituents can be combined to form=O or =S;
each of the R^{ee} is independently selected from alkyl, haloalkyl, alkenyl, alkynyl, cycloalkyl, aryl, heterocyclyl, and heteroaryl, wherein each of the alkyl, alkenyl, alkynyl, cycloalkyl, heterocyclyl, aryl and heteroaryl is independently substituted with 0, 1, 2, 3, 4 or 5 R^{gg} groups;
each of the R^{ff} is independently selected from hydrogen, alkyl, haloalkyl, alkenyl, alkynyl, cycloalkyl, heterocyclyl, aryl and heteroaryl, or two R^{ff} groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{gg} groups;
each of the R^{gg} is independently selected from halogen, -CN, -NO₂, -N₃, -SO₂H, -SO₃H, -OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl)⁺X⁻, -NH₃⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, -OC(=O)NH(C₁₋₆ alkyl), -NHC(=O)(C₁₋₆ alkyl), -N(C₁₋₆ alkyl)C(=O)(C₁₋₆ alkyl), -NHCO₂(C₁₋₆ alkyl), -NHC(=O)N(C₁₋₆ alkyl)₂, -NHC(=O)NH(C₁₋₆ alkyl), -NHC(=O)NH₂, -C(=NH)O(C₁-₆ alkyl), -OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, -OC(NH)NH(C₁₋₆ alkyl), -OC(NH)NH₂, -NHC(NH)N(C₁₋₆ alkyl)₂, -NHC(=NH)NH₂, -NHSO₂(C₁₋₆ alkyl), -SO₂N(C₁₋₆ alkyl)₂, -SO₂NH(C₁₋₆ alkyl), -SO₂NH₂, -SO₂C₁₋₆ alkyl, -SO₂OC₁₋₆ alkyl, -OSO₂C₁₋₆ alkyl, -SOC₁₋₆ alkyl, -Si(C₁₋₆ alkyl)₃, -OSi(C₁₋₆ alkyl)₃, -C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ haloalkyl, C₂-C₆ alkenyl, C₂-C₆ alkynyl, C₃-C₇ cycloalkyl, C₆-C₁₀ aryl, C₃-C₇ heterocyclyl, C₅-C₁₀ heteroaryl; or two geminal R^{gg} substituents may combine to form =O or =S; wherein X⁻ is a counter-ion.

Exemplary substituents on nitrogen atoms include, but are not limited to, hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{bb})R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl and heteroaryl, or two R^{cc} groups attached to a nitrogen atom combine to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups, and wherein R^{aa}, R^{bb}, R^{cc} and R^{dd} are as described herein.

"Nucleic acids" refers to single- or double-stranded deoxyribonucleic acid (DNA) or ribonucleic acid (RNA) molecules and their heterozygous molecules. Examples of nucleic acid molecules include, but are not limited to, messenger RNA (mRNA), microRNA (miRNA), small interfering RNA (siRNA), self-amplified RNA (saRNA), and antisense oligonucleotides (ASO), etc. Nucleic acids may be further chemically modified, and the chemical modifier selected from one of, or a combination of: pseudouridine, N1-methyl-pseudouridine, 5-methoxyuridine, and 5-methylcytosine. mRNA molecules contain protein coding regions and may further contain expression regulatory sequences. Typical expression regulatory sequences include, but are not limited to, 5' cap, 5' untranslated region (5' UTR), 3' untranslated region (3' UTR), polyadenylate sequence (PolyA), miRNA binding sites.

"Cationic lipids" refers to lipid molecules that is capable of being positively charged at physiological pH conditions. In some embodiments, the cationic lipid is an amino lipid.

"Neutral lipids" refers to lipid molecules that is not charged at a particular pH, such as physiological pH conditions. Examples of neutral lipids include, but are not limited to 1,2-distearoyl-sn-glycero-3-phosphocholine (DSPC), 1,2-dimyristoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC), 1-palmitoyl-2-oleoyl-sn-glycero-3-phosphocholine (POPC), 1,2-dioleoyl-sn-glycero-3-phosphoethanolamine (DOPE), 1,2-dimyristoyl-sn-glycero-3-phosphoethanolamine (DMPE), 1-palmitoyl-2-oleoyl-sn-glycero-3-phosphoethanolamine (POPE), and 1,2-dipalmitoyl-sn-glycero-3-phosphoethanolamine (DPPE).

"Structure lipids" refers to lipids that enhance the stability of nanoparticles by filling the gaps between lipids, commonly such as steroids. The steroid is a compound having a perhydrocyclopentanophenanthrene carbon framework. In an alternative embodiment, the steroid is cholesterol, sitosterol, coprosterol, fucosterol, brassicasterol, ergosterol, tomatine, ursolic acid, α-tocopherol, stigmasterol, avenasterol, ergocalciferol or campesterol.

"Polymer lipids" refers to molecules containing a polymer moiety and a lipid moiety. In some embodiments, the polymer lipid is a polyethylene glycol (PEG) lipid. Other lipids that can reduce aggregation, such as products of compounds having uncharged, hydrophilic, space-barrier moieties coupled with lipid may also be used.

"Lipid nanoparticles" refers to particles containing lipid components of nanoscale size.

"Biodegradable groups" refers to functional groups that contain biodegradable bonds, such as esters, disulfide bonds and amides, etc. Biodegradation may affect the process of removing compounds from the body. The biodegradable groups of the present disclosure are oriented from the head to the tail in ionizable lipid molecules.

### Other terminology

The term "treating" as used herein relates to reversing, alleviating or inhibiting the progression or prevention of the disorders or conditions to which the term applies, or of one or more symptoms of such disorders or conditions. The noun "treatment" as used herein relates to the action of treating, which is a verb, and the latter is as just defined.

The term "pharmaceutically acceptable salt" as used herein refers to those carboxylate and amino acid addition salts of the compounds of the present disclosure, which are suitable for the contact with patients' tissues within a reliable medical judgment, and do not produce inappropriate toxicity, irritation, allergy, etc. They are commensurate with a reasonable benefit/risk ratio, and are effective for their intended use. The term includes, if possible, the zwitterionic form of the compounds of the disclosure.

The pharmaceutically acceptable base addition salts are formed with metals or amines, such as alkali metal and alkaline earth metal hydroxides or organic amines. Examples of the metals used as cations include sodium, potassium, magnesium, calcium, etc. Examples of suitable amines are N,N'-dibenzylethylenediamine, chloroprocaine, choline, diethanolamine, ethylenediamine, N-methylglucamine and procaine.

The base addition salt of the acidic compound can be prepared by contacting the free acid form with a sufficient amount of the required base to form a salt in a conventional manner. The free acid can be regenerated by contacting the salt form with an acid in a conventional manner and then isolating the free acid. The free acid forms are somewhat different from their respective salt forms in their physical properties, such as solubility in polar solvents. But for the purposes of the present disclosure, the salts are still equivalent to their respective free acids.

The salts can be prepared from the inorganic acids, which include sulfates, pyrosulfates, bisulfates, sulfites, bisulfites, nitrates, phosphates, monohydrogen phosphates, dihydrogen phosphates, metaphosphates, pyrophosphates, chlorides, bromides and iodides. Examples of the acids include hydrochloric acid, nitric acid, sulfuric acid, hydrobromic acid, hydroiodic acid, phosphoric acid, etc. The representative salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthalate, methanesulfonate, glucoheptanate, lactobionate, lauryl sulfonate, isethionate, etc. The salts can also be prepared from the organic acids, which include aliphatic monocarboxylic and dicarboxylic acids, phenyl-substituted alkanoic acids, hydroxyalkanoic acids, alkanedioic acid, aromatic acids, aliphatic and aromatic sulfonic acids, etc. The representative salts include acetate, propionate, octanoate, isobutyrate, oxalate, malonate, succinate, suberate, sebacate, fumarate, maleate, mandelate, benzoate, chlorobenzoate, methyl benzoate, dinitrobenzoate, naphthoate, besylate, tosylate, phenylacetate, citrate, lactate, maleate, tartrate, methanesulfonate, etc. The pharmaceutically acceptable salts can include cations based on alkali metals and alkaline earth metals, such as sodium, lithium, potassium, calcium, magnesium, etc., as well as non-toxic ammonium, quaternary ammonium, and amine cations including, but not limited to, ammonium, tetramethylammonium, tetraethylammonium, methylamine, dimethylamine, trimethylamine, triethylamine, ethylamine, etc. Salts of amino acids are also included, such as arginine salts, gluconates, galacturonates, etc. (for example, see Berge S. M. et al., "Pharmaceutical Salts," J. Pharm. Sci., 1977; 66: 1- 19 for reference).

"Subjects" to which administration is contemplated include, but are not limited to, humans (e.g., males or females of any age group, e.g., paediatric subjects (e.g., infants, children, adolescents) or adult subjects (e.g., young adults, middle-aged adults or older adults) and/or non-human animals, such as mammals, e.g., primates (e.g., cynomolgus monkeys, rhesus monkeys), cattle, pigs, horses, sheep, goats, rodents, cats and/or dogs. In some embodiments, the subject is a human. In some embodiments, the subject is a non-human animal. The terms "human", "patient" and "subject" can be used interchangeably herein.

"Disease", "disorder", and "condition" can be used interchangeably herein.

Unless otherwise indicated, the term "treatment" as used herein includes the effect on a subject who is suffering from a particular disease, disorder, or condition, which reduces the severity of the disease, disorder, or condition, or delays or slows the progression of the disease, disorder or condition ("therapeutic treatment"). The term also includes the effect that occurs before the subject begins to suffer from a specific disease, disorder or condition ("prophylactic treatment").

Generally, the "effective amount" of a pharmaceutical composition refers to an amount sufficient to elicit a target biological response. As understood by those skilled in the art, the effective amount of the pharmaceutical composition of the disclosure can vary depending on the following factors, such as the desired biological endpoint, the pharmacokinetics of the pharmaceutical composition, the diseases being treated, the mode of administration, and the age, health status and symptoms of the subjects. The effective amount includes therapeutically effective amount and prophylactically effective amount.

Unless otherwise indicated, the "therapeutically effective amount" of the pharmaceutical composition as used herein is an amount sufficient to provide therapeutic benefits in the course of treating a disease, disorder or condition, or to delay or minimize one or more symptoms associated with the disease, disorder or condition. The therapeutically effective amount of a pharmaceutical composition refers to the amount of the therapeutic agent that, when used alone or in combination with other therapies, provides a therapeutic benefit in the treatment of a disease, disorder or condition. The term "therapeutically effective amount" can include an amount that improves the overall treatment, reduces or avoids the symptoms or causes of the disease or condition, or enhances the therapeutic effect of other therapeutic agents.

Unless otherwise indicated, the "prophylactically effective amount" of the pharmaceutical composition as used herein is an amount sufficient to prevent a disease, disorder or condition, or an amount sufficient to prevent one or more symptoms associated with a disease, disorder or condition, or an amount sufficient to prevent the recurrence of a disease, disorder or condition. The prophylactically effective amount of a pharmaceutical composition refers to the amount of a therapeutic agent that, when used alone or in combination with other agents, provides a prophylactic benefit in the prevention of a disease, disorder or condition. The term "prophylactically effective amount" can include an amount that improves the overall prevention, or an amount that enhances the prophylactic effect of other preventive agents.

"Combination" and related terms refer to the simultaneous or sequential administration of the pharmaceutical compositions of the present disclosure and other therapeutic agents. For example, the pharmaceutical compositions of the present disclosure can be administered simultaneously or sequentially in separate unit dosage with other therapeutic agents, or simultaneously in a single unit dosage with other therapeutic agents.

### DETAILED DESCRIPTION OF THE INVENTION

As used herein, "compounds of the present disclosure" refers to the following compounds of formula (VI), formula (VII), pharmaceutically acceptable salts, isotopic variants, tautomers or stereoisomers thereof.

In the present disclosure, compounds are named using standard nomenclature. For compounds having an asymmetric center, it should be understood, unless otherwise stated, that all optical isomers and mixtures thereof are included. Furthermore, unless otherwise specified, all isomer compounds and carbon-carbon double bonds included in the present disclosure may occur in the form of Z and E. Compounds which exist in different tautomeric forms, one of which is not limited to any particular tautomer, but is intended to cover all tautomeric forms.

In one embodiment, the present disclosure relates to a nanoparticle composition, comprising a lipid ingredient, and optionally comprising a load;
wherein the lipid ingredient comprises the following components in the molar percentages:
Ionizable cationic lipids 20 mol%-85 mol%:
Structure lipids 10 mol%-75 mol%;
Neutral lipids 1.0 mol%-30 mol%;
Polymer lipids 0.25 mol%-10 mol%;
wherein the ionizable cationic lipid is the compound of formula (VI) or formula (VII), or a pharmaceutically acceptable salt, isotopic variant, tautomer or stereoisomer thereof, 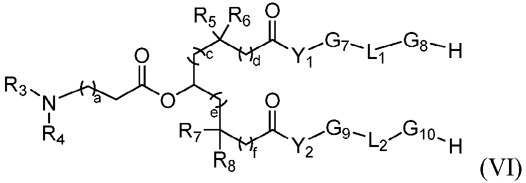 or 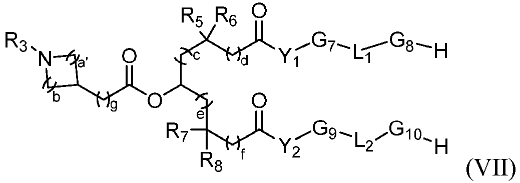 wherein,
   a, a', b and g are independently 0, 1, 2, 3, 4 or 5, a' and b are not 0 at the same time;
   a'+g=0, 1, 2, 3, 4 or 5;
   c and e are independently 3, 4, 5, 6, 7, 8 or 9;
   d and f are independently 0, 1, 2, 3 or 4;
   c+d=3, 4, 5, 6, 7, 8 or 9, e+f=3, 4, 5, 6, 7, 8 or 9;
   methylenes in 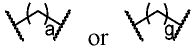 are optionally and independently substituted with 1, 2, 3, 4 or 5 C₁₋₆ alkyl;
   R₃ and R₄ are independently H, C₁₋₆ alkyl, C₁₋₆ haloalkyl, 3- to 10-membered cycloalkyl or 3- to 10-membered heterocyclyl, which is optionally substituted with 1, 2, 3, 4 or 5 R*;
   or, R₃ and R₄ are taken together with the N atom to which they are attached to form 3- to 10-membered heterocyclyl, which is optionally substituted with 1, 2, 3, 4 or 5 R*;
   R* is independently H, halogen, cyano, C₁₋₆ alkyl, C₁₋₆ haloalkyl, -L_{b}-OR_{b} or -L_{b}-NR_{b}R'_{b};
   R₅, R₆, R₇ and R₈ are independently C₁₋₃ alkyl;
   Y₁ and Y₂ are independently O, S or NRₐ;
   L₁ and L₂ are independently -(CRR')₂-, -CH=CH-, -C≡C- or -NR''-;
   G₇, G₈, G₉ and G₁₀ are independently a chemical bond or C₁₋₁₂ alkylene, which is optionally substituted with 1, 2, 3, 4, 5 or 6 R;
   G₇ and G₈ have a total length of 6, 7, 8, 9, 10, 11 or 12 carbon atoms;
   G₉ and G₁₀ have a total length of 6, 7, 8, 9, 10, 11 or 12 carbon atoms;
   R and R' are independently H, C₁₋₁₄ alkyl, -Lₐ-ORₐ or -Lₐ-NRₐR'ₐ;
   Lₐ is independently a chemical bond or C₁₋₁₄ alkylene;
   L_{b} is independently a chemical bond or C₁₋₆ alkylene;
   Rₐ and R'ₐ are independently H, C₁₋₁₄ alkyl, 3- to 10-membered cycloalkyl or 3- to 10-membered heterocyclyl;
   R_{b} and R'_{b} are independently H, C₁₋₆ alkyl, 3- to 10-membered cycloalkyl or 3- to 10-membered heterocyclyl;
   R'' is independently H or C₁₋₁₄ alkyl;
   alternatively, wherein in the compound of formula (VI) or formula (VII),
   a is 0, 1, 2, 3 or 4, alternatively 1, 2, 3 or 4, alternatively 2, 3 or 4;
   a' and b are independently 0, 1, 2, 3 or 4, alternatively 2;
   g is 0, 1 or 2, alternatively 0 or 1;
   a'+g=0, 1, 2, 3, 4 or 5, alternatively a'+g=2 or 3;
   c and e are independently 3, 4, 5 or 6;
   d and f are independently 0, 1 or 2;
   c+d=4, 5 or 6, e+f=4, 5 or 6;
   methylenes in 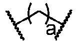 are optionally and independently substituted with 1, 2, 3, 4 or 5 C₁₋₆ alkyl;
   methylenes in 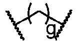 are optionally and independently substituted with 1 or 2 C₁₋₆ alkyl;
   R₃ and R₄ are independently C₁₋₆ alkyl, which is optionally substituted with 1, 2 or 3 R*;
   R* is independently H, C₁₋₆ alkyl, C₁₋₆ haloalkyl or -OR_{b};
   or, R₃ and R₄ are taken together with the N atom to which they are attached to form 3- to 7-membered heterocyclyl, alternatively 5-membered heterocyclyl, which is optionally substituted with 1, 2 or 3 R*;
   R₅, R₆, R₇ and R₈ are independently C₁₋₃ alkyl;
   Y₁ and Y₂ are independently O, S or NRₐ, alternatively O or S;
   L₁ and L₂ are independently -(CHR)₂-, -CH=CH-, -C≡C- or -NR''-, alternatively -(CHR)₂-, -CH=CH- or -C≡C-;
   G₇ and G₉ are independently a chemical bond or C₁₋₆ alkylene;
   G₈ and G₁₀ are independently C₁₋₁₀ alkylene;
   G₇ and G₈ have a total length of 6, 7, 8, 9 or 10 carbon atoms;
   G₉ and G₁₀ have a total length of 6, 7, 8, 9 or 10 carbon atoms;
   1, 2 or 3 methylenes in G₇, G₈, G₉ or G₁₀ are optionally and independently substituted with 1 R;
   R is independently H or C₁₋₈ alkyl;
   R'' is independently H or C₁₋₁₀ alkyl;
   Rₐ is independently H or C₁₋₁₀ alkyl;
   R_{b} is independently H or C₁₋₆ alkyl, alternatively H.

### R₃ and R₄

In one embodiment, R₃ is C₁₋₆ alkyl; in another embodiment, R₃ is optionally substituted with 1, 2, or 3 R*.

In one embodiment, R₄ is C₁₋₆ alkyl; in another embodiment, R₄ is optionally substituted with 1, 2, or 3 R*.

In one embodiment, R₃, R₄ are taken together with the N atom to which they are attached to form 3- to 7-membered heterocyclyl; in another embodiment, R₃, R₄ are taken together with the N atom to which they are attached to form 5- to 7-membered heterocyclyl; in another embodiment, R₃, R₄ are taken together with the N atom to which they are attached to form 4- to 6-membered heterocyclyl; in another embodiment, R₃, R₄ are taken together with the N atom to which they are attached to form 5-membered heterocyclyl; in another embodiment, R₃, R₄ are taken together with the N atom to which they are attached to form 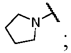 in another embodiment, R₃, R₄ are taken together with the N atom to which they are attached to form 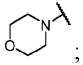 in another embodiment, R₃, R₄ are taken together with the N atom to which they are attached to form 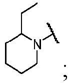 in another embodiment, R₃, R₄ are taken together with the N atom to which they are attached to form 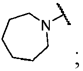 in another embodiment, the heterocyclyl formed by R₃ and R₄ taken together with the N atom to which they are attached is optionally substituted with 1, 2, or 3 R*.

In a more specific embodiment, R₃ and R₄ are independently C₁₋₆ alkyl, which is optionally substituted with 1, 2 or 3 R*;
or, R₃ and R₄ are taken together with the N atom to which they are attached to form 3- to 7-membered heterocyclyl, which is optionally substituted with 1, 2 or 3 R*;
or, R₃ and R₄ are taken together with the N atom to which they are attached to form 5- to 7-membered heterocyclyl, alternatively 4- to 6-membered heterocyclyl, more alternatively 5-membered heterocyclyl, which is optionally substituted with 1, 2, 3, 4 or 5 R*;
or, R₄ and R₉ are taken together with the atoms to which they are attached to form 6-membered heterocyclyl, which is optionally substituted with 1, 2 or 3 R*.

In a more specific embodiment, R₃ is Me, -CH₂CH₃ or -CH(CH₃)₂, more alternatively Me or -CH₂CH₃; R₄ is Me;
or, R₃ and R₄ are taken together with the N atom to which they are attached to form 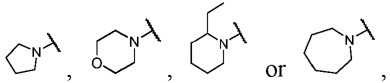 alternatively 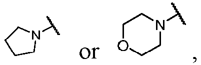 more alternatively

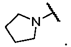

### R*

In one embodiment, R* is H; in another embodiment, R* is C₁₋₆ alkyl; in another embodiment, R* is C₁₋₆ haloalkyl; in another embodiment, R* is -OR_{b}.

In a more specific embodiment, R* is independently H, C₁₋₆ alkyl, C₁₋₆ haloalkyl or -OR_{b}; in another more specific embodiment, R* is independently H, halogen, C₁₋₆ alkyl or C₁₋₆ haloalkyl; in another more specific embodiment, R* is independently H, C₁₋₆ alkyl or C₁₋₆ haloalkyl; in another more specific embodiment, R* is H, Me or OH; in another more specific embodiment, R* is H or Me.

### R₅, R₆, R₇ and R₈

In one embodiment, R₅ is C₁₋₃ alkyl; in another embodiment, R₅ is Me.

In one embodiment, R₆ is C₁₋₃ alkyl; in another embodiment, R₆ is Me.

In one embodiment, R₇ is C₁₋₃ alkyl; in another embodiment, R₇ is Me.

In one embodiment, R₈ is C₁₋₃ alkyl; in another embodiment, R₈ is Me.

In one embodiment, G₇ is a chemical bond; in another embodiment, G₇ is C₁₋₁₂ alkylene; in another embodiment, G₇ is C₁₋₆ alkylene; in another embodiment, G₇ is C₁₋₅ alkylene; in another embodiment, G₇ is C₁₋₅ linear alkylene; in another embodiment, G₇ is -CH₂-; in another embodiment, G₇ is -(CH₂)₂-; in another embodiment, G₇ is -(CH₂)₄-; in another embodiment, G₇ is -(CH₂)₅-; in another embodiment, G₇ is optionally substituted with 1, 2, 3, 4, 5 or 6 R; in another embodiment, 1, 2 or 3 methylenes in G₇ are optionally and independently substituted with 1 R; in another embodiment, 1 or 2 methylenes in G₇ are optionally and independently substituted with 1 R; in another embodiment, the methylene of G₇ that is collected to M₁ is not substituted with R.

In one embodiment, G₈ is a chemical bond; in another embodiment, G₈ is C₁₋₁₂ alkylene; in another embodiment, G₈ is C₁₋₁₀ alkylene; in another embodiment, G₈ is C₁₋₈ alkylene; in another embodiment, G₈ is C₁₋₈ linear alkylene; in another embodiment, G₈ is -(CH₂)₂-; in another embodiment, G₈ is -(CH₂)₄-; in another embodiment, G₈ is -(CH₂)₆-; in another embodiment, G₈ is -(CH₂)₇-; in another embodiment, G₈ is -(CH₂)₈-; in another embodiment, G₈ is optionally substituted with 1, 2, 3, 4, 5 or 6 R; in another embodiment, 1, 2 or 3 methylenes in G₈ are optionally and independently substituted with 1 R; in another embodiment, 1 or 2 alkylene in G₈ are optionally and independently substituted with 1 R.

In one embodiment, G₇ and G₈ have a total length of 6 carbon atoms; in another embodiment, G₇ and G₈ have a total length of 7 carbon atoms; in another embodiment, G₇ and G₈ have a total length of 8 carbon atoms; in another embodiment, G₇ and G₈ have a total length of 9 carbon atoms; in another embodiment, G₇ and G₈ have a total length of 10 carbon atoms; in another embodiment, G₇ and G₈ have a total length of 11 carbon atoms; in another embodiment, G₇ and G₈ have a total length of 12 carbon atoms.

In a more specific embodiment, G₇ and G₈ have a total length of 6, 7, 8, 9 or 10 carbon atoms.

In a more specific embodiment, G₇ and G₈ have a total length of 6, 7 or 8 carbon atoms.

In one embodiment, L₁ is -(CRR')₂-; in another embodiment, L₁ is -CH=CH-; in another embodiment, L₁ is -C≡C-; in another embodiment, L₁ is -NR"-; in another embodiment, L₁ is -(CHR)₂-.

In one embodiment, G₉ is a chemical bond; in another embodiment, G₉ is C₁₋₁₂ alkylene; in another embodiment, G₉ is C₁₋₆ alkylene; in another embodiment, G₉ is C₁₋₅ alkylene; in another embodiment, G₉ is C₁₋₅ linear alkylene; in another embodiment, G₉ is -CH₂-; in another embodiment, G₉ is -(CH₂)₂-; in another embodiment, G₉ is -(CH₂)₄-; in another embodiment, G₉ is -(CH₂)₅-; in another embodiment, G₉ is optionally substituted with 1, 2, 3, 4, 5 or 6 R; in another embodiment, 1, 2 or 3 methylenes in G₉ are optionally and independently substituted with 1 R; in another embodiment, 1 or 2 methylenes in G₉ are optionally and independently substituted with 1 R; in another embodiment, the methylene of G₉ that is collected to M₂ is not substituted with R.

In one embodiment, G₁₀ is a chemical bond; in another embodiment, G₁₀ is C₁₋₁₂ alkylene; in another embodiment, G₁₀ is C₁₋₁₀ alkylene; in another embodiment, G₁₀ is C₁₋₈ alkylene; in another embodiment, G₁₀ is C₁₋₈ linear alkylene; in another embodiment, G₁₀ is -(CH₂)₂-; in another embodiment, G₁₀ is -(CH₂)₄-; in another embodiment, G₁₀ is -(CH₂)₆-; in another embodiment, G₁₀ is -(CH₂)₇-; in another embodiment, G₁₀ is -(CH₂)₈-; in another embodiment, G₁₀ is optionally substituted with 1, 2, 3, 4, 5 or 6 R; in another embodiment, 1, 2 or 3 methylenes in G₁₀ are optionally and independently substituted with 1 R; in another embodiment, 1 or 2 methylenes in G₁₀ are optionally and independently substituted with 1 R.

In one embodiment, G₉ and G₁₀ have a total length of 6 carbon atoms; in another embodiment, G₉ and G₁₀ have a total length of 7 carbon atoms; in another embodiment, G₉ and G₁₀ have a total length of 8 carbon atoms; in another embodiment, G₉ and G₁₀ have a total length of 9 carbon atoms; in another embodiment, G₉ and G₁₀ have a total length of 10 carbon atoms; in another embodiment, G₉ and G₁₀ have a total length of 11 carbon atoms; in another embodiment, G₉ and G₁₀ have a total length of 12 carbon atoms.

In a more specific embodiment, G₉ and G₁₀ have a total length of 6, 7, 8, 9 or 10 carbon atoms.

In a more specific embodiment, G₉ and G₁₀ have a total length of 6, 7 or 8 carbon atoms.

In one embodiment, L₂ is -(CRR')₂-; in another embodiment, L₂ is -CH=CH-; in another embodiment, L₂ is -C≡C-; in another embodiment, L₂ is -NR"-; in another embodiment, L₁ is -(CHR)₂-.

In a more specific embodiment, L₁ and L₂ are independently -(CRR')₂-, -CH=CH-, -C≡C- or -NR"-;
G₇, G₈, G₉ and G₁₀ are independently a chemical bond or C₁₋₁₂ alkylene, which is optionally substituted with 1, 2, 3, 4, 5 or 6 R;
G₇ and G₈ have a total length of 6, 7, 8, 9, 10, 11 or 12 carbon atoms;
G₉ and G₁₀ have a total length of 6, 7, 8, 9, 10, 11 or 12 carbon atoms;
1, 2 or 3 methylenes in G₇, G₈, G₉ or G₁₀ are optionally and independently substituted with 1 R;
R' is independently H, C₁₋₂₀ alkyl, -Lₐ-ORₐ or -Lₐ-NRₐR'ₐ.

In another more specific embodiment, L₁ and L₂ are independently -(CHR)₂-, -CH=CH-, -C≡C- or -NR"-;
G₇ and G₉ are independently a chemical bond or C₁₋₆ alkylene;
G₈ and G₁₀ are independently C₁₋₁₀ alkylene;
G₇ and G₈ have a total length of 6, 7, 8, 9 or 10 carbon atoms;
G₉ and G₁₀ have a total length of 6, 7, 8, 9 or 10 carbon atoms;
1, 2 or 3 methylenes in G₇, G₈, G₉ or G₁₀ are optionally and independently substituted with 1 R.

In another more specific embodiment, L₁ and L₂ are independently -(CHR)₂-, -CH=CH-, -C≡C- or -NR"-;
G₇ and G₉ are independently a chemical bond or C₁₋₅ alkylene, alternatively a chemical bond or C₁₋₅ linear alkylene;
G₈ and G₁₀ are independently C₁₋₈ alkylene, alternatively C₁₋₈ linear alkylene;
G₇ and G₈ have a total length of 6, 7, 8, 9, 10 carbon atoms, alternatively 6, 7, 8, 9, 10 carbon atoms;
G₉ and G₁₀ have a total length of 6, 7, 8, 9, 10 carbon atoms, alternatively 6, 7, 8, 9, 10 carbon atoms;
1 or 2 methylenes in G₇, G₈, G₉ or G₁₀ are optionally and independently substituted with 1 R.

Alternatively, the methylene collected to M₁ and M₂ is not substituted with R.

In another more specific embodiment, L₁ and L₂ are independently -(CHR)₂-, -CH=CH-, -C≡C- or -NR"-;
G₇ and G₉ are independently a chemical bond, -CH₂-, -(CH₂)₂-, -(CH₂)₄- or -(CH₂)₅-;
G₈ and G₁₀ are independently -(CH₂)₂-, -(CH₂)₄-, -(CH₂)₆-, -(CH₂)₇- or -(CH₂)₈-;
G₇ and G₈ have a total length of 6, 7, 8, 9, 10 carbon atoms, alternatively 6, 7, 8, 9, 10 carbon atoms;
G₉ and G₁₀ have a total length of 6, 7, 8, 9, 10 carbon atoms, alternatively 6, 7, 8, 9, 10 carbon atoms;
1 or 2 methylenes in G₇, G₈, G₉ or G₁₀ are optionally and independently substituted with 1 R.

Alternatively, the methylene collected to M₁ and M₂ is not substituted with R.

In another more specific embodiment, -G₇-L₁-G₈-H or -G₉-L₂-G₁₀-H is independently selected from: -(CH₂)₅CH₃, -(CH₂)₆CH₃, -(CH₂)₇CH₃, -(CH₂)₈CH₃, -(CH₂)₉CH₃, -(CH₂)₁₀CH₃, -(CH₂)₁₁CH₃, -CH₂-C≡C-(CH₂)₅CH₃, -CH₂-C≡C-(CH₂)₆CH₃, -(CH₂)₂-C≡C-(CH₂)₅CH₃, -(CH₂)₄-C≡C-(CH₂)₃CH₃, -CH₂-CH=CH-(CH₂)₅CH₃, -CH₂-CH=CH-(CH₂)₆CH₃, -(CH₂)₂-CH=CH-(CH₂)₅CH₃, -(CH₂)₄-CH=CH-(CH₂)₃CH₃, -(CH₂)₅-CH=CH-CH₂CH₃,

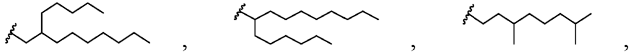

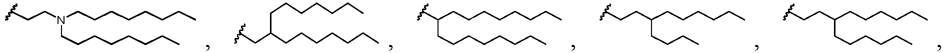

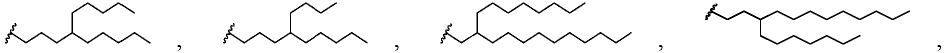

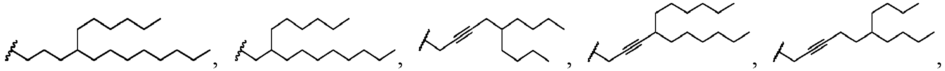

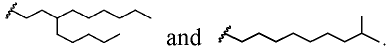

### R

In one embodiment, R is H; in another embodiment, R is C₁₋₈ alkyl; in another embodiment, R is C₁₋₈ linear alkyl.

In a more specific embodiment, R is H, Me, -(CH₂)₃CH₃, -(CH₂)₄CH₃, -(CH₂)₅CH₃, -(CH₂)₆CH₃ or -(CH₂)₇CH₃.

### R"

In one embodiment, R'' is H; in another embodiment, R" is C₁₋₁₀ alkyl; in another embodiment, R" is C₇₋₉ alkyl; in another embodiment, R" is -(CH₂)₇CH₃.

### Lₐ

In one embodiment, Lₐ is a chemical bond; in another embodiment, Lₐ is C₁₋₁₄ alkylene; in another embodiment, Lₐ is C₁₋₁₀ alkylene.

### L_{b}

In one embodiment, L_{b} is a chemical bond; in another embodiment, L_{b} is C₁₋₆ alkylene.

### Rₐ and R'ₐ

In one embodiment, Rₐ is H; in another embodiment, Rₐ is 3- to 10-membered cycloalkyl; in another embodiment, Rₐ is 3- to 10-membered heterocyclyl; in another embodiment, Rₐ is C₁₋₁₄ alkyl; in another embodiment, Rₐ is C₁₋₁₀ alkyl; in another embodiment, Rₐ is C₈₋₁₀ alkyl; in another embodiment, Rₐ is C₈₋₁₀ linear alkyl; in another embodiment, Rₐ is -(CH₂)₈CH₃.

In one embodiment, R'ₐ is H; in another embodiment, R'ₐ is 3- to 10-membered cycloalkyl; in another embodiment, R'ₐ is 3- to 10-membered heterocyclyl; in another embodiment, R'ₐ is C₁₋₁₄ alkyl; in another embodiment, R'ₐ is C₁₋₁₀ alkyl; in another embodiment, R'ₐ is C₈₋₁₀ alkyl; in another embodiment, R'ₐ is C₈₋₁₀ linear alkyl; in another embodiment, R'ₐ is -(CH₂)₈CH₃.

### R_{b} and R'_{b}

In one embodiment, R_{b} is H; in another embodiment, R_{b} is C₁₋₆ alkyl; in another embodiment, R_{b} is 3- to 10-membered cycloalkyl; in another embodiment, R_{b} is 3- to 10-membered heterocyclyl.

In one embodiment, R'_{b} is H; in another embodiment, R'_{b} is C₁₋₆ alkyl; in another embodiment, R'_{b} is 3- to 10-membered cycloalkyl; in another embodiment, R'_{b} is 3- to 10-membered heterocyclyl.

Any of the above technical solutions in any specific embodiment or any combination thereof may be combined with any technical solution or any combination thereof in other specific embodiments. For example, any technical solution of Q, or any combination thereof, may be combined with any technical solution of R₃, R₄, R₅, R₆, R₇, R₈, R, R", Lₐ, L_{b}, Rₐ, R'ₐ, R_{b}, and R'_{b}, , or any combination thereof. The present disclosure is intended to include all of these combinations of technical solutions and, for reasons of space, will not be listed.

In a more specific embodiment, the present disclosure provides a nanoparticle composition, comprising a lipid ingredient, and optionally comprising a load;
wherein the lipid ingredient comprises the following components in the molar percentages:
Ionizable cationic lipids 20 mol%-85 mol%:
Structure lipids 10 mol%-75 mol%;
Neutral lipids 1.0 mol%-30 mol%;
Polymer lipids 0.25 mol%-10 mol%;
   wherein the ionizable cationic lipid is a compound of formula (VI) or formula (VII) or a pharmaceutically acceptable salt, isotopic variant, tautomer or stereoisomer thereof: 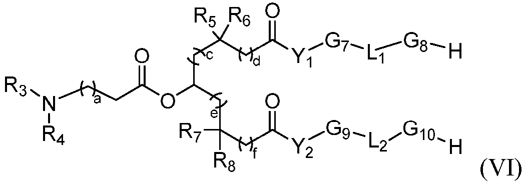 or 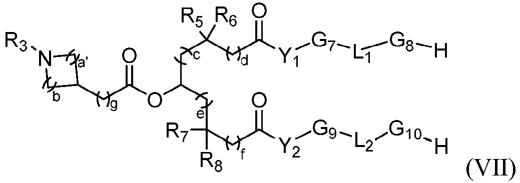 wherein,
   a, a', b and g are independently 0, 1, 2, 3, 4 or 5, a' and b are not 0 at the same time;
   a'+g=0, 1, 2, 3, 4 or 5;
   c and e are independently 3, 4, 5, 6, 7, 8 or 9;
   d and f are independently 0, 1, 2, 3 or 4;
   c+d=3, 4, 5, 6, 7, 8 or 9, e+f=3, 4, 5, 6, 7, 8 or 9;
   methylenes in 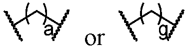 are optionally and independently substituted with 1, 2, 3, 4 or 5 C₁₋₆ alkyl.

The remaining groups are defined in any one of the above.

In a more specific embodiment, the present disclosure provides a nanoparticle composition as described above, wherein in the compound of formula (VI) or formula (VII),
a, a', b and g are independently 0, 1, 2, 3, 4 or 5, a' and b are not 0 at the same time;
a'+g=0, 1, 2, 3, 4 or 5;
c and e are independently 3, 4, 5, 6, 7, 8 or 9;
d and f are independently 0, 1, 2, 3 or 4;
c+d=3, 4, 5, 6, 7, 8 or 9, e+f=3, 4, 5, 6, 7, 8 or 9;
methylenes in 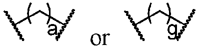 are optionally and independently substituted with 1, 2, 3, 4 or 5 C₁₋₆ alkyl;
R₃ and R₄ are independently H, C₁₋₆ alkyl, C₁₋₆ haloalkyl, 3- to 10-membered cycloalkyl or 3- to 10-membered heterocyclyl, which is optionally substituted with 1, 2, 3, 4 or 5 R*;
or, R₃ and R₄ are taken together with the N atom to which they are attached to form 3- to 10-membered heterocyclyl, which is optionally substituted with 1, 2, 3, 4 or 5 R*;
R* is independently H, halogen, cyano, C₁₋₆ alkyl, C₁₋₆ haloalkyl, -L_{b}-OR_{b} or -L_{b}-NR_{d}R'_{b};
R₅, R₆, R₇ and R₈ are independently C₁₋₃ alkyl;
Y₁ and Y₂ are independently O, S or NRₐ;
L₁ and L₂ are independently -(CRR')₂-, -CH=CH-, -C≡C- or -NR"-;
G₇, G₈, G₉ and G₁₀ are independently a chemical bond or C₁₋₁₂ alkylene, which is optionally substituted with 1, 2, 3, 4, 5 or 6 R;
G₇ and G₈ have a total length of 6, 7, 8, 9, 10, 11 or 12 carbon atoms;
G₉ and G₁₀ have a total length of 6, 7, 8, 9, 10, 11 or 12 carbon atoms;
1, 2 or 3 methylenes in G₇, G₈, G₉ or G₁₀ are optionally and independently substituted with 1 R;
R and R' are independently H, C₁₋₁₄ alkyl, -Lₐ-ORₐ or -Lₐ-NRₐR'ₐ;
Lₐ is independently a chemical bond or C₁₋₁₄ alkylene;
L_{b} is independently a chemical bond or C₁₋₆ alkylene;
Rₐ and R'ₐ are independently H, C₁₋₁₄ alkyl, 3- to 10-membered cycloalkyl or 3- to 10-membered heterocyclyl;
R_{b} and R'_{b} are independently H, C₁₋₆ alkyl, 3- to 10-membered cycloalkyl or 3- to 10-membered heterocyclyl;
R" is independently H or C₁₋₁₄ alkyl.

In a more specific embodiment, the present disclosure provides a nanoparticle composition as described above, wherein in the compound of formula (VI) or formula (VII),
a is 0, 1, 2, 3 or 4, alternatively 1, 2, 3 or 4, alternatively 2, 3 or 4;
a' and b are independently 0, 1, 2, 3 or 4, alternatively 2;
g is 0, 1 or 2, alternatively 0 or 1;
a'+g=0, 1, 2, 3, 4 or 5, alternatively a'+g=2 or 3;
c and e are independently 3, 4, 5 or 6;
d and f are independently 0, 1 or 2;
c+d=4, 5 or 6, e+f=4, 5 or 6;
methylenes in 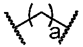 are optionally and independently substituted with 1, 2, 3, 4 or 5 C₁₋₆ alkyl;
methylenes in 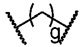 are optionally and independently substituted with 1 or 2 C₁₋₆ alkyl;
R₃ and R₄ are independently C₁₋₆ alkyl, which is optionally substituted with 1, 2 or 3 R*;
R* is independently H, C₁₋₆ alkyl, C₁₋₆ haloalkyl or -OR_{b};
or, R₃ and R₄ are taken together with the N atom to which they are attached to form 3- to 7-membered heterocyclyl, alternatively 5-membered heterocyclyl, which is optionally substituted with 1, 2 or 3 R*;
R₅, R₆, R₇ and R₈ are independently C₁₋₃ alkyl;
Y₁ and Y₂ are independently O, S or NRₐ, alternatively O or S;
L₁ and L₂ are independently -(CHR)₂-, -CH=CH-, -C≡C- or -NR" -, alternatively -(CHR)₂-, -CH=CH- or -C≡C-;
G₇ and G₉ are independently a chemical bond or C₁₋₆ alkylene;
G₈ and G₁₀ are independently C₁₋₁₀ alkylene;
G₇ and G₈ have a total length of 6, 7, 8, 9 or 10 carbon atoms;
G₉ and G₁₀ have a total length of 6, 7, 8, 9 or 10 carbon atoms;
1, 2 or 3 methylenes in G₇, G₈, G₉ or G₁₀ are optionally and independently substituted with 1 R, alternatively the methylene collected to Y₁ and Y₂ is not substituted with R;
R is independently H or C₁₋₈ alkyl;
R" is independently H or C₁₋₁₀ alkyl;
Rₐ is independently H or C₁₋₁₀ alkyl;
R_{b} is independently H or C₁₋₆ alkyl, alternatively H.

In a more specific embodiment, the present disclosure provides a nanoparticle composition as described above, wherein in the compound of formula (VI),
a is 0, 1, 2, 3 or 4, alternatively 1, 2, 3 or 4, alternatively 2, 3 or 4;
c and e are independently 3, 4, 5 or 6;
d and f are independently 0, 1 or 2;
c+d=4, 5 or 6, e+f=4, 5 or 6;
R₃ and R₄ are independently C₁₋₆ alkyl;
or, R₃ and R₄ are taken together with the N atom to which they are attached to form 4- to 6-membered heterocyclyl, alternatively 5-membered heterocyclyl, which is optionally substituted with 1, 2 or 3 R*;
R* is independently H, C₁₋₆ alkyl or C₁₋₆ haloalkyl;
R₅, R₆, R₇ and R₈ are independently C₁₋₃ alkyl;
Y₁ and Y₂ are independently O, S or NRₐ, alternatively O or S;
L₁ and L₂ are independently -(CHR)₂-, -CH=CH-, -C≡C- or -NR" -, alternatively -(CHR)₂-, -CH=CH- or -C≡C-;
G₇ and G₉ are independently a chemical bond or C₁₋₅ alkylene, alternatively a chemical bond or C₁₋₅ linear alkylene;
G₈ and G₁₀ are independently C₁₋₈ alkylene, alternatively C₁₋₈ linear alkylene;
G₇ and G₈ have a total length of 6, 7, 8, 9 or 10 carbon atoms;
G₉ and G₁₀ have a total length of 6, 7, 8, 9 or 10 carbon atoms;
1, 2 or 3 methylenes in G₇, G₈, G₉ or G₁₀ are optionally and independently substituted with 1 R, alternatively the methylene collected to Y₁ and Y₂ is not substituted with R;
R is independently H or C₁₋₈ alkyl, alternatively H or C₁₋₇ alkyl, alternatively H or C₁₋₆ alkyl;
R" is independently H or C₇₋₉ alkyl;
Rₐ is independently H or C₈₋₁₀ alkyl.

Alternatively, R is independently H or C₁₋₈ linear alkyl, alternatively H or C₁₋₇ linear alkyl, alternatively H or C₁₋₆ linear alkyl.

Alternatively, R" is independently H or C₇₋₉ linear alkyl.

Alternatively, Rₐ is independently H or C₈₋₁₀ linear alkyl.

In a more specific embodiment, the present disclosure provides a nanoparticle composition as described above, wherein in the compound of formula (VI),
a is 0, 1, 2, 3 or 4, alternatively 1, 2, 3 or 4, alternatively 2, 3 or 4;
c and e are independently 3, 4, 5 or 6;
d and f are independently 0, 1 or 2;
c+d=4, 5 or 6, e+f=4, 5 or 6; alternatively, 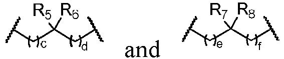 are independently -(CH₂)₄-C(CH₃)₂-, -(CH₂)₅-C(CH₃)₂-, -(CH₂)₆-C(CH₃)₂-, -(CH₂)₄-C(CH₃)₂-CH₂- or -(CH₂)₃-C(CH₃)₂-(CH₂)₂-;
R₃ and R₄ are independently C₁₋₆ alkyl, alternatively Me;
or, R₃ and R₄ are taken together with the N atom to which they are attached to form 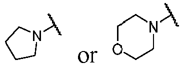 alternatively 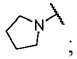
R₅, R₆, R₇ and R₈ are Me;
Y₁ and Y₂ are independently O, S or NRₐ, alternatively O or S;
L₁ and L₂ are independently -(CHR)₂-, -CH=CH-, -C≡C- or -NR" -, alternatively -(CHR)₂-, -CH=CH- or -C≡C-;
G₇ and G₉ are independently a chemical bond, -CH₂-, -(CH₂)₂-, -(CH₂)₄- or -(CH₂)₅-;
G₈ and G₁₀ are independently -(CH₂)₂-, -(CH₂)₄-, -(CH₂)₆-, -(CH₂)₇- or -(CH₂)₈-;
G₇ and G₈ have a total length of 6, 7, 8, 9, 10 carbon atoms;
G₉ and G₁₀ have a total length of 6, 7, 8, 9, 10 carbon atoms;
1, 2 or 3 methylenes in G₇, G₈, G₉ or G₁₀ are optionally and independently substituted with 1 R, alternatively the methylene collected to Y₁ and Y₂ is not substituted with R;
R is independently H, Me, -(CH₂)₃CH₃, -(CH₂)₄CH₃, -(CH₂)₅CH₃, -(CH₂)₆CH₃ or -(CH₂)₇CH₃, alternatively H, Me, -(CH₂)₃CH₃, -(CH₂)₄CH₃, -(CH₂)₅CH₃ or -(CH₂)₆CH₃, alternatively H, Me, -(CH₂)₃CH₃, -(CH₂)₄CH₃ or -(CH₂)₅CH₃;
Rₐ is independently H or -(CH₂)₈CH₃;
R" is -(CH₂)₇CH₃.

Alternatively, -G₇-L₁-G₈-H or -G₉-L₂-G₁₀-H is independently selected from the following groups:
-(CH₂)₇CH₃, -(CH₂)₈CH₃, -(CH₂)₉CH₃, -(CH₂)₁₁CH₃, -CH₂-C≡C-(CH₂)₅CH₃, -CH₂-C≡C-(CH₂)₆CH₃, -(CH₂)₂-C≡C-(CH₂)₅CH₃, -(CH₂)₄-C≡C-(CH₂)₃CH₃, -CH₂-CH=CH-(CH₂)₆CH₃, -(CH₂)₂-CH=CH-(CH₂)₅CH₃, -(CH₂)₄-CH=CH-(CH₂)₃CH₃, -(CH₂)₅-CH=CH-CH₂CH₃, 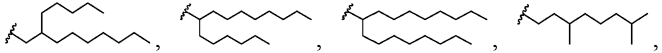 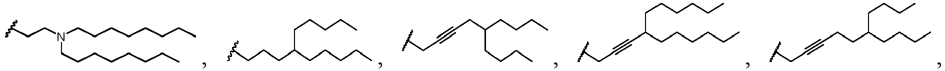 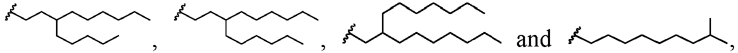 alternatively is not

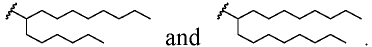

In a more specific embodiment, the present disclosure provides a nanoparticle composition as described above, wherein in the compound of formula (VI),
a is 2, 3 or 4;
c and e are independently 3, 4, 5 or 6;
d and f are independently 0, 1 or 2;
c+d=4, 5 or 6, e+f=4, 5 or 6;
R₃ and R₄ are independently C₁₋₆ alkyl, alternatively C₁₋₃ alkyl;
R₅, R₆, R₇ and R₈ are independently C₁₋₃ alkyl;
Y₁ and Y₂ are independently O or S;
L₁ and L₂ are independently -(CHR)₂-, -CH=CH- or -C≡C-;
G₇ and G₉ are independently C₁₋₄ alkylene, alternatively C₁₋₄ linear alkylene;
G₈ and G₁₀ are independently C₂₋₇ alkylene, alternatively C₂₋₇ linear alkylene;
G₇ and G₈ have a total length of 6, 7 or 8 carbon atoms;
G₉ and G₁₀ have a total length of 6, 7 or 8 carbon atoms;
1, 2 or 3 methylenes in G₇, G₈, G₉ or G₁₀ are optionally and independently substituted with 1 R;
R is independently H or C₁₋₇ alkyl, alternatively H or C₁₋₇ linear alkyl;
provided that, when L₁ is -C≡C-, then G₇ is C₁₋₂ alkylene, alternatively C₁₋₂ linear alkylene; and when L₂ is -C≡C-, then G₉ is C₁₋₂ alkylene, alternatively C₁₋₂ linear alkylene.

Alternatively, the methylene collected to Y₁ and Y₂ is not substituted with R.

In a more specific embodiment, the present disclosure provides a nanoparticle composition as described above, in the compound of formula (VI),
a is 2, 3 or 4;
c and e are independently 3, 4, 5 or 6;
d and f are independently 0, 1 or 2;
c+d=4, 5 or 6, e+f=4, 5 or 6; alternatively, 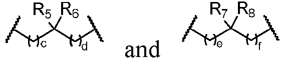 are independently -(CH₂)₄-C(CH₃)₂-, -(CH₂)₅-C(CH₃)₂-, -(CH₂)₆-C(CH₃)₂-, -(CH₂)₄-C(CH₃)₂-CH₂- or -(CH₂)₃-C(CH₃)₂-(CH₂)₂-;
R₃ and R₄ are independently C₁₋₆ alkyl, alternatively Me;
R₅, R₆, R₇ and R₈ are independently C₁₋₃ alkyl, alternatively Me;
Y₁ and Y₂ are independently O or S;
L₁ and L₂ are independently -(CHR)₂-, -CH=CH- or -C≡C-;
G₇ and G₉ are independently -CH₂-, -(CH₂)₂- or -(CH₂)₄-;
G₈ and G₁₀ are independently -(CH₂)₄-, -(CH₂)₆- or -(CH₂)₇-;
G₇ and G₈ have a total length of 6, 7 or 8 carbon atoms;
G₉ and G₁₀ have a total length of 6, 7 or 8 carbon atoms;
1, 2 or 3 methylenes in G₇, G₈, G₉ or G₁₀ are optionally and independently substituted with 1 R;
R is independently H, Me, -(CH₂)₃CH₃, -(CH₂)₄CH₃, -(CH₂)₅CH₃ or -(CH₂)₆CH₃;
provided that, when L₁ is -C≡C-, then G₇ is -CH₂- or -(CH₂)₂-, and when L₂ is -C≡C-, then G₉ is -CH₂- or -(CH₂)₂-;
alternatively, -G₇-L₁-G₈-H or -G₉-L₂-G₁₀-H is independently selected from the following groups:
   -(CH₂)₇CH₃, -(CH₂)₈CH₃, -(CH₂)₉CH₃, -CH₂-C≡C-(CH₂)₅CH₃, -CH₂-C≡C-(CH₂)₆CH₃, -(CH₂)₂-C≡C-(CH₂)₅CH₃, -CH₂-CH=CH-(CH₂)₆CH₃, -(CH₂)₂-CH=CH-(CH₂)₅CH₃, -(CH₂)₄-CH=CH-(CH₂)₃CH₃,

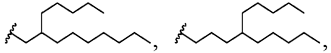

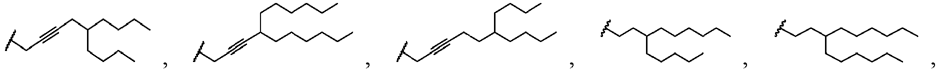

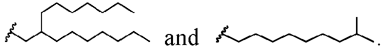

In a more specific embodiment, the present disclosure provides a nanoparticle composition as described above, wherein in the compound of formula (VI),
a is 2, 3 or 4, alternatively 2 or 3;
c and e are independently 4, 5 or 6;
d and f are 0;
R₃ and R₄ are independently C₁₋₆ alkyl, alternatively C₁₋₃ alkyl;
R₅, R₆, R₇ and R₈ are independently C₁₋₃ alkyl, alternatively Me;
Y₁ and Y₂ are O;
L₁ and L₂ are independently -(CHR)₂- or -CH=CH-;
G₇ and G₉ are independently -CH₂- or -CH₂CHR-;
G₈ and G₁₀ are independently -(CH₂)₆- or -(CH₂)₇-;
G₇ and G₈ have a total length of 7 or 8 carbon atoms;
G₉ and G₁₀ have a total length of 7 or 8 carbon atoms;
1, 2 or 3 methylenes in G₈ or G₁₀ are optionally and independently substituted with 1 R;
R is independently H or C₄₋₆ alkyl, alternatively H or C₅ alkyl;
alternatively -G₇-L₁-G₈-H and -G₉-L₂-G₁₀-H are not -(CH₂)₉CH₃ at the same time.
Alternatively, R is independently H or C₄₋₆ linear alkyl, alternatively H or C₅ linear alkyl.

In a more specific embodiment, the present disclosure provides a nanoparticle composition as described above, in the compound of formula (VI),
a is 2, 3 or 4, alternatively 2 or 3;
c and e are independently 4, 5 or 6;
d and f are 0;
R₃ and R₄ are Me;
R₅, R₆, R₇ and R₈ are Me;
Y₁ and Y₂ are O;
-G₇-L₁-G₈-H and -G₉-L₂-G₁₀-H are independently selected from the following groups:
-(CH₂)₈CH₃, -(CH₂)₉CH₃, -CH₂-CH=CH-(CH₂)₆CH₃, -(CH₂)₂-CH=CH-(CH₂)₅CH₃, 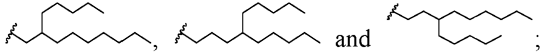 alternatively -(CH₂)₈CH₃, -(CH₂)₉CH₃, -CH₂-CH=CH-(CH₂)₆CH₃, -(CH₂)₂-CH=CH-(CH₂)₅CH₃ and 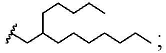 alternatively -G₇-L₁-G₈-H and -G₉-L₂-G₁₀-H are not -(CH₂)₉CH₃ at the same time.

In a more specific embodiment, the present disclosure provides a nanoparticle composition as described above, wherein in the compound of formula (VI),
a is 3;
c and e are independently 5 or 6, alternatively 6;
d and f are 0;
R₃ and R₄ are independently C₁₋₃ alkyl;
R₅, R₆, R₇ and R₈ are independently C₁₋₃ alkyl;
Y₁ and Y₂ are O;
L₁ and L₂ are independently -(CHR)₂- or -CH=CH, alternatively -(CHR)₂-;
G₇ and G₉ are independently -CH₂- or -CH₂CHR-;
G₈ and G₁₀ are independently -(CH₂)₆- or -(CH₂)₇-;
G₇ and G₈ have a total length of 7 or 8 carbon atoms, alternatively 7 carbon atoms;
G₉ and G₁₀ have a total length of 7 or 8 carbon atoms, alternatively 7 carbon atoms;
1, 2 or 3 methylenes in G₈ or G₁₀ are optionally and independently substituted with 1 R;
R is independently H or C₄₋₆ alkyl, alternatively H or C₅ alkyl.

Alternatively, R is independently H or C₄₋₆ linear alkyl, alternatively H or C₅ linear alkyl.

In a more specific embodiment, the present disclosure provides a nanoparticle composition as described above, wherein in the compound of formula (VI),
a is 3;
c and e are 6;
d and f are 0;
R₃ and R₄ are Me;
R₅, R₆, R₇ and R₈ are Me;
Y₁ and Y₂ are O;
-G₇-L₁-G₈-H and -G₉-L₂-G₁₀-H are independently selected from the following groups:
   -(CH₂)₈CH₃, 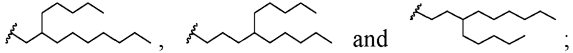 alternatively -(CH₂)₈CH₃ and

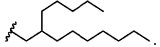

In a more specific embodiment, the present disclosure provides a nanoparticle composition as described above, wherein in the compound of formula (VI),
a is 2 or 3, alternatively 2;
c and e are independently 4, 5 or 6, alternatively 5;
d and f are 0;
R₃ and R₄ are independently C₁₋₃ alkyl;
R₅, R₆, R₇ and R₈ are independently C₁₋₃ alkyl;
one of Y₁ and Y₂ is O, and the other is S;
L₁ and L₂ are independently -(CHR)₂- or -CH=CH, alternatively -(CHR)₂-;
G₇ and G₉ are independently -CH₂- or -CH₂CHR-;
G₈ and G₁₀ are independently -(CH₂)₅- or -(CH₂)₆-;
G₇ and G₈ have a total length of 7 carbon atoms;
G₉ and G₁₀ have a total length of 7 carbon atoms;
1, 2 or 3 methylenes in G₈ or G₁₀ are optionally and independently substituted with 1 R;
R is independently H or C₄₋₆ alkyl, alternatively H or C₅ alkyl.

Alternatively, R is independently H or C₄₋₆ linear alkyl, alternatively H or C₅ linear alkyl.

In a more specific embodiment, the present disclosure provides a nanoparticle composition as described above, wherein in the compound of formula (VI),
a is 2;
c and e are 5;
d and f are 0;
R₃ and R₄ are Me;
R₅, R₆, R₇ and R₈ are Me;
one of Y₁ and Y₂ is O, and the other is S;
-G₇-L₁-G₈-H and -G₉-L₂-G₁₀-H are independently selected from the following groups:
   -(CH₂)₈CH₃, 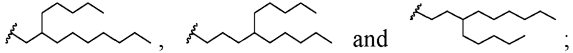 alternatively -(CH₂)₈CH₃.

In a more specific embodiment, the present disclosure provides a nanoparticle composition as described above, wherein in the compound of formula (VI),
a are 2;
c and e are independently 4, 5 or 6, alternatively 5;
d and f are 0;
R₃ and R₄ are independently C₁₋₆ alkyl, alternatively C₁₋₃ alkyl;
R₅, R₆, R₇ and R₈ are independently C₁₋₃ alkyl;
Y₁ and Y₂ are independently O or S;
one of L₁ and L₂ is -C≡C-, the other is -(CHR)₂-, or both of L₁ and L₂ are -C≡C-; alternatively one of L₁ and L₂ is -C≡C-, the other is -(CHR)₂-;
G₇ and G₉ are -CH₂-;
G₈ and G₁₀ are independently -(CH₂)₆- or -(CH₂)₇-;
1 methylene in G₈ or G₁₀ optionally and independently substituted with 1 R, alternatively G₈ and G₁₀ are independently -CHR-(CH₂)₅-, -CHR-(CH₂)₆-, -CH₂-CHR-(CH₂)₄- or -(CH₂)₂-CHR-(CH₂)₄-;
R is independently H or C₄₋₆ alkyl, alternatively H or C₅ alkyl;
provided that, only one of the -G₇-L₁-G₈-H and -G₉-L₂-G₁₀-H is substituted with one non-hydrogen R substituent and the other is unsubstituted.

Alternatively, R is independently H or C₄₋₆ linear alkyl, alternatively H or C₅ linear alkyl.

In a more specific embodiment, the present disclosure provides a nanoparticle composition as described above, wherein in the compound of formula (VI),
a is 2;
c and e are 5;
d and f are 0;
R₃ and R₄ are Me;
R₅, R₆, R₇ and R₈ are Me;
Y₁ and Y₂ are independently O or S;
-G₇-L₁-G₈-H and -G₉-L₂-G₁₀-H are independently selected from the following groups:
   -(CH₂)₈CH₃, -(CH₂)₉CH₃, -CH₂-C≡C-(CH₂)₆CH₃, 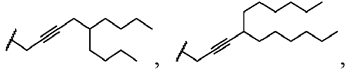 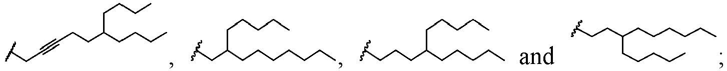
provided that, at least one of -G₇-L₁-G₈-H and -G₉-L₂-G₁₀-H comprises alkynyl, and one of the two has a substituent while the other one has no substituent.

In a more specific embodiment, the present disclosure provides a nanoparticle composition as described above, wherein in the compound of formula (VI),
a is 2;
c and e are independently 4, 5 or 6, alternatively 5;
d and f are 0;
R₃ and R₄ are independently C₁₋₃ alkyl, alternatively Me;
R₅, R₆, R₇ and R₈ are independently C₁₋₃ alkyl, alternatively Me;
Y₁ and Y₂ are independently O or S, alternatively O;
both of L₁ and L₂ are -C≡C-;
G₇ and G₉ are -CH₂-;
G₈ and G₁₀ are independently -(CH₂)₆- or -(CH₂)₇-, alternatively -(CH₂)₇-.

In a more specific embodiment, the present disclosure provides a nanoparticle composition as described above, wherein in the compound of formula (VI),
a is 2;
c and e are 3;
d and f are 2;
R₃ and R₄ are independently C₁₋₃ alkyl, alternatively Me;
R₅, R₆, R₇ and R₈ are independently C₁₋₃ alkyl;
Y₁ and Y₂ are independently O or S, alternatively O;
L₁ and L₂ are -(CHR)₂-;
G₇ and G₉ are independently -CH₂- or -CH₂CHR-;
G₈ and G₁₀ are independently -(CH₂)₅-, -(CH₂)₆- or -(CH₂)₇-;
G₇ and G₈ have a total length of 6, 7 or 8 carbon atoms, alternatively 7 carbon atoms;
G₉ and G₁₀ have a total length of 6, 7 or 8 carbon atoms, alternatively 7 carbon atoms;
1, 2 or 3 methylenes in G₈ or G₁₀ are optionally and independently substituted with 1 R;
R is independently H or C₁₋₇ alkyl, alternatively H or C₁₋₆ alkyl, alternatively Me.

Alternatively, R is independently H or C₁₋₇ linear alkyl, alternatively H or C₁₋₆ linear alkyl, alternatively Me.

In a more specific embodiment, the present disclosure provides a nanoparticle composition as described above, wherein in the compound of formula (VI),
a is 2;
c and e are 3;
d and f are 2;
R₃ and R₄ are Me;
R₅, R₆, R₇ and R₈ are Me;
Y₁ and Y₂ are independently O or S, alternatively O;
-G₇-L₁-G₈-H and -G₉-L₂-G₁₀-H are independently selected from the following groups: 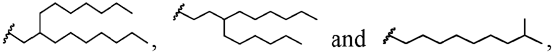 alternatively 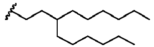 and 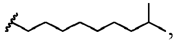 alternatively

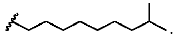

In a more specific embodiment, the present disclosure provides a nanoparticle composition as described above, wherein in the compound of formula (VI),
a is 2;
c and e are 4, 5, or 6, alternatively 5;
d and f are 0;
R₃ and R₄ are independently C₁₋₆ alkyl, alternatively C₁₋₃ alkyl;
R₅, R₆, R₇ and R₈ are independently C₁₋₃ alkyl;
Y₁ and Y₂ are S;
L₁ and L₂ are -(CHR)₂-;
G₇ and G₉ are independently -CH₂- or -CH₂CHR-;
G₈ and G₁₀ are independently -(CH₂)₅-, -(CH₂)₆- or -(CH₂)₇-;
G₇ and G₈ have a total length of 7 or 8 carbon atoms, alternatively 8 carbon atoms;
G₉ and G₁₀ have a total length of 7 or 8 carbon atoms, alternatively 8 carbon atoms;
1, 2 or 3 methylenes in G₈ or G₁₀ are optionally and independently substituted with 1 R;
R is independently H or C₄₋₆ alkyl, alternatively H or C₅ alkyl.

Alternatively, R is independently H or C₄₋₆ linear alkyl, alternatively H or C₅ linear alkyl.

In a more specific embodiment, the present disclosure provides a nanoparticle composition as described above, wherein in the compound of formula (VI),
a is 2;
c and e are 5;
d and f are 0;
R₃ and R₄ are independently Me;
R₅, R₆, R₇ and R₈ are independently Me;
Y₁ and Y₂ are S;
-G₇-L₁-G₈-H and -G₉-L₂-G₁₀-H are independently selected from the following:
   -(CH₂)₈CH₃, -(CH₂)₉CH₃, 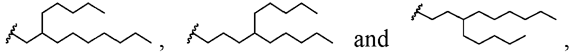 alternatively -(CH₂)₈CH₃ or -(CH₂)₉CH₃, alternatively -(CH₂)₉CH₃.

In a more specific embodiment, the present disclosure provides a nanoparticle composition as described above, wherein in the compound of formula (VII),
a' and b are 2;
g is 0 or 1;
c and e are 5;
d and f are 0;
R₃ is C₁₋₆ alkyl, which is optionally substituted with 1, 2 or 3 R*;
R* is independently H, C₁₋₆ alkyl, C₁₋₆ haloalkyl or -OR_{b}, alternatively H, C₁₋₆ alkyl or C₁₋₆ haloalkyl;
R₅, R₆, R₇ and R₈ are independently C₁₋₃ alkyl;
Y₁ and Y₂ are independently O or S;
L₁ and L₂ are -(CHR)₂-;
G₇ and G₉ are independently -CH₂- or -CH₂CHR-;
G₈ and G₁₀ are independently -(CH₂)₅-, -(CH₂)₆- or -(CH₂)₇-;
G₇ and G₈ have a total length of 6, 7 or 8 carbon atoms;
G₉ and G₁₀ have a total length of 6, 7 or 8 carbon atoms;
1, 2 or 3 methylenes in G₈ or G₁₀ are optionally and independently substituted with 1 R;
R is independently H or C₄₋₆ alkyl;
R_{b} is independently H or C₁₋₆ alkyl, alternatively H.

Alternatively, R is independently H or C₄₋₆ linear alkyl.

In a more specific embodiment, the present disclosure provides a nanoparticle composition as described above, wherein in the compound of formula (VII),
a' and b are 2;
g is 0 or 1;
c and e are 5;
d and f are 0;
R₃ is Me, -CH₂CH₃, -CH₂CH₂OH or -CH(CH₃)₂, alternatively Me, -CH₂CH₃ or -CH(CH₃)₂;
R₅, R₆, R₇ and R₈ are independently Me;
Y₁ and Y₂ are independently O or S;
-G₇-L₁-G₈-H or -G₉-L₂-G₁₀-H is independently selected from the following groups:
   -(CH₂)₇CH₃, -(CH₂)₈CH₃, -(CH₂)₉CH₃, 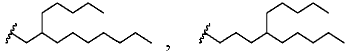 and 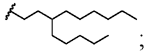 alternatively -(CH₂)₇CH₃, -(CH₂)₈CH₃, -(CH₂)₉CH₃ and

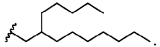

In a more specific embodiment, the present disclosure provides a nanoparticle composition as described above, wherein in the compound of formula (VII),
R₃ is Me or -CH₂CH₃, alternatively Me;
Both of Y₁ and Y₂ are O;
G₇ and G₈ have a total length of 6 or 7 carbon atoms, alternatively 7 carbon atoms;
G₉ and G₁₀ have a total length of 6 or 7 carbon atoms, alternatively 7 carbon atoms.

In a more specific embodiment, the present disclosure provides a nanoparticle composition as described above, wherein in the compound of formula (VII),
a' and b are 2;
g is 0 or 1;
c and e are 5;
d and f are 0;
R₃ is Me or -CH₂CH₃, alternatively Me;
R₅, R₆, R₇ and R₈ are independently Me;
both of Y₁ and Y₂ are O;
-G₇-L₁-G₈-H or -G₉-L₂-G₁₀-H is independently selected from the following groups:
   -(CH₂)₇CH₃, -(CH₂)₈CH₃, 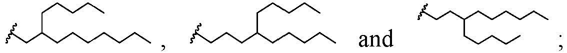 alternatively -(CH₂)₇CH₃, -(CH₂)₈CH₃ and 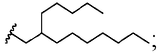 alternatively is not -(CH₂)₇CH₃.

In a more specific embodiment, the present disclosure provides a nanoparticle composition as described above, wherein in the compound of formula (VII),
R₃ is Me or -CH₂CH₃;
Y₁ and Y₂ are independently O or S, where Y₁ and Y₂ are not O at the same time;
G₇ and G₈ have a total length of 6, 7 or 8 carbon atoms;
G₉ and G₁₀ have a total length of 6, 7 or 8 carbon atoms.

In a more specific embodiment, the present disclosure provides a nanoparticle composition as described above, wherein in the compound of formula (VII),
g is 0 or 1, alternatively 1;
R₃ is Me or -CH₂CH₃, alternatively Me;
one of Y₁ and Y₂ is O, and the other is S;
G₇ and G₈ have a total length of 7 carbon atoms;
G₉ and G₁₀ have a total length of 7 carbon atoms.

In a more specific embodiment, the present disclosure provides a nanoparticle composition as described above, wherein in the above compound of formula (VII),
a' and b are 2;
g is 0 or 1, alternatively 1;
c and e are 5;
d and f are 0;
R₃ is Me or -CH₂CH₃, alternatively Me;
R₅, R₆, R₇ and R₈ are independently Me;
one of Y₁ and Y₂ is O, and the other is S;
-G₇-L₁-G₈-H or -G₉-L₂-G₁₀-H is independently selected from the following groups:
   -(CH₂)₈CH₃, 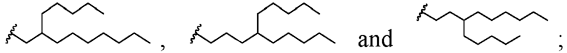 alternatively -(CH₂)₈CH₃ and

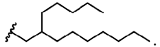

In a more specific embodiment, the present disclosure provides a nanoparticle composition as described above, wherein in the above compound of formula (VII),
g is 0 or 1, alternatively 0;
R₃ is Me or -CH₂CH₃;
both of Y₁ and Y₂ are S;
G₇ and G₈ have a total length of 7 or 8 carbon atoms;
G₉ and G₁₀ have a total length of 7 or 8 carbon atoms.

In a more specific embodiment, the present disclosure provides a nanoparticle composition as described above, wherein in the above compound of formula (VII),
a' and b are 2;
g is 0 or 1, alternatively 0;
c and e are 5;
d and f are 0;
R₃ is Me or -CH₂CH₃;
R₅, R₆, R₇ and R₈ are independently Me;
both of Y₁ and Y₂ are S;
-G₇-L₁-G₈-H or -G₉-L₂-G₁₀-H is independently selected from the following groups:
   -(CH₂)₈CH₃, -(CH₂)₉CH₃, 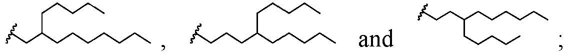 alternatively -(CH₂)₈CH₃, -(CH₂)₉CH₃ and

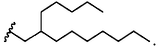

In a more specific embodiment, the present disclosure provides a nanoparticle composition as described above, wherein the compound of formula (IV) is selected from the following:

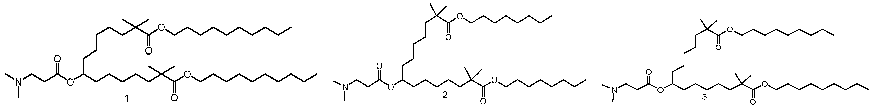

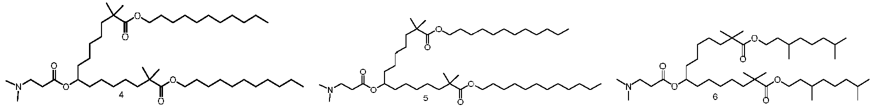

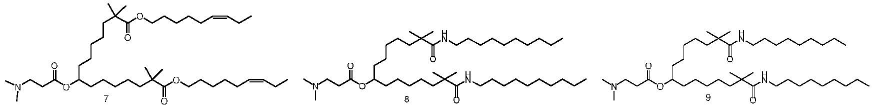

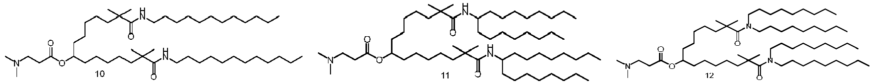

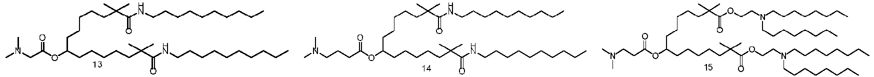

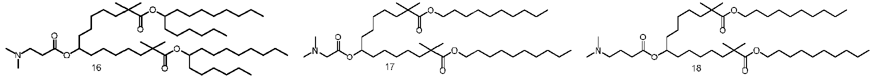

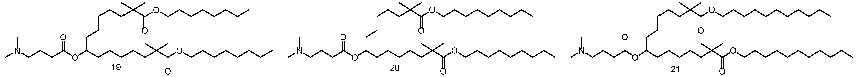

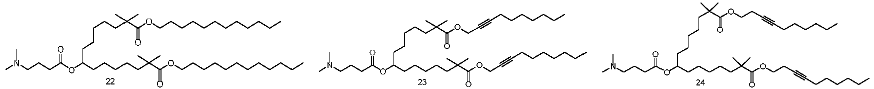

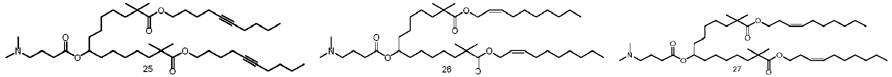

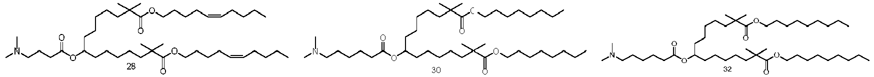

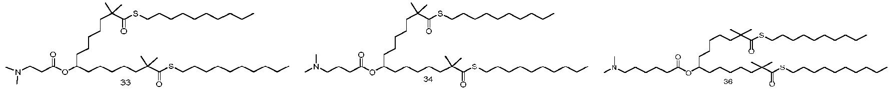

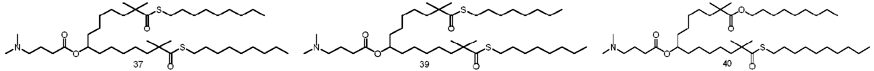

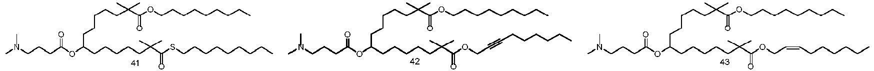

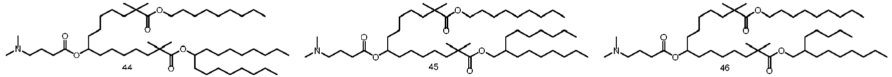

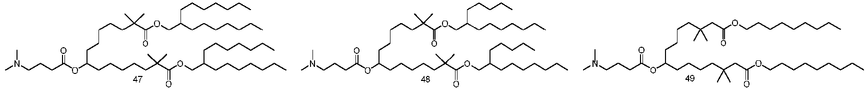

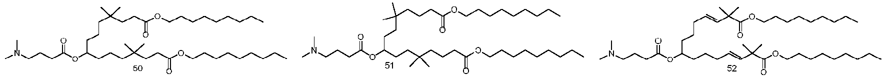

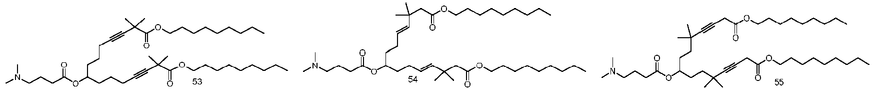

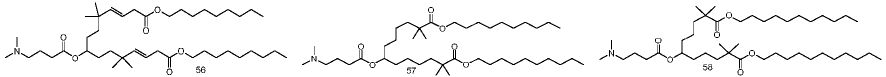

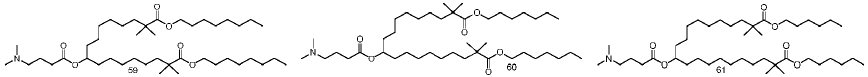

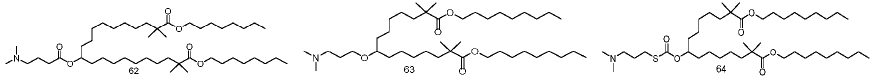

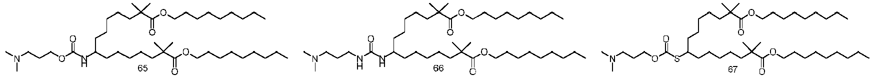

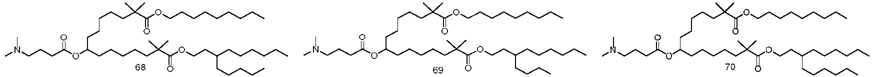

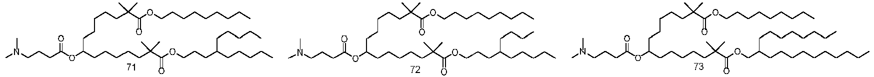

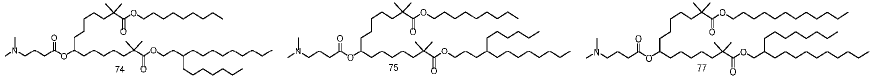

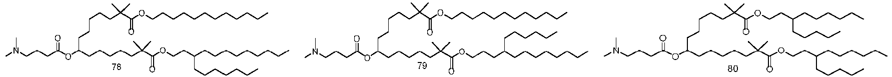

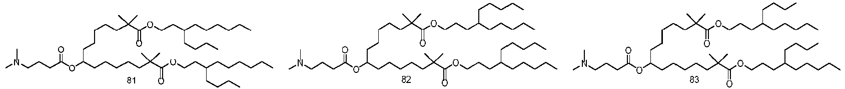

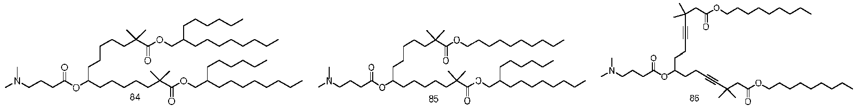

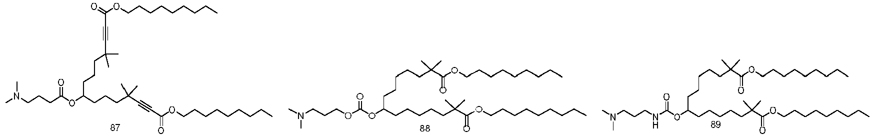

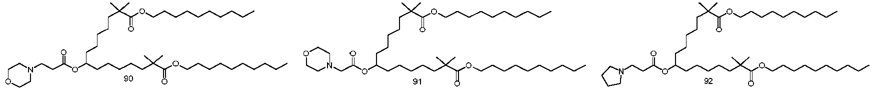

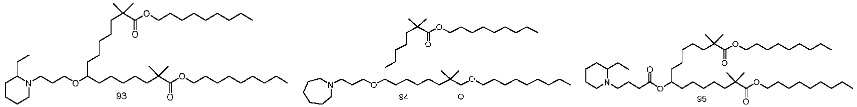

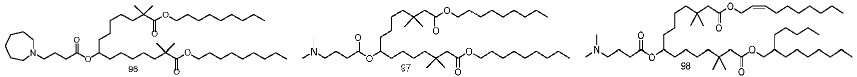

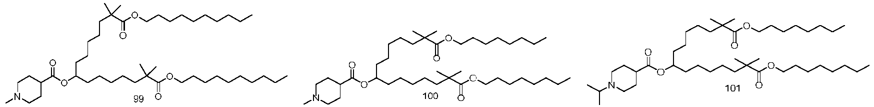

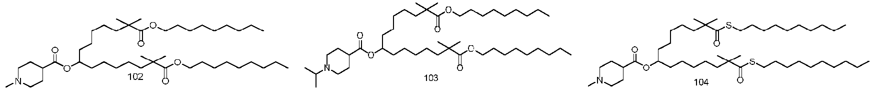

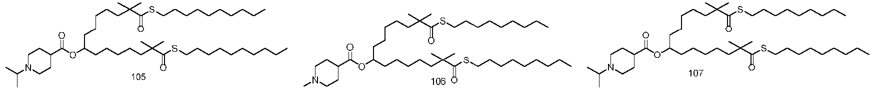

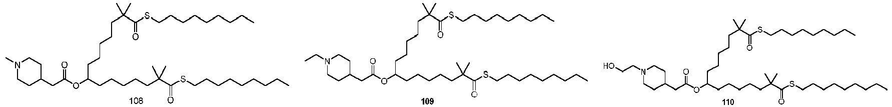

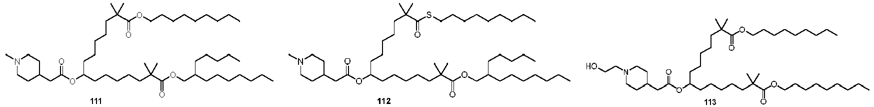

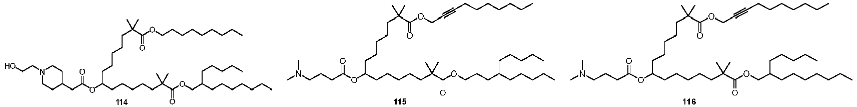

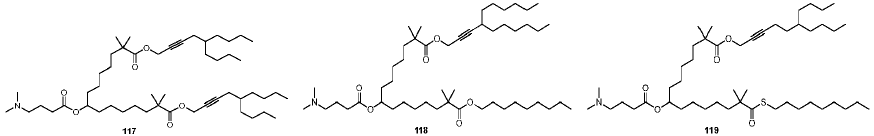

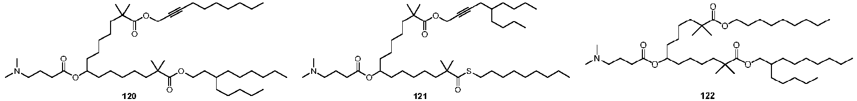

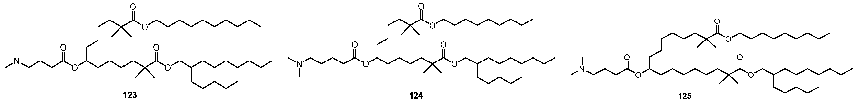

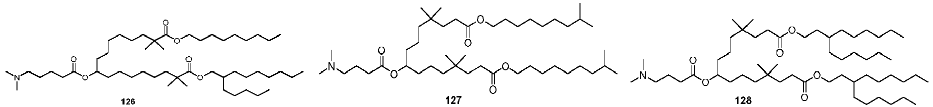

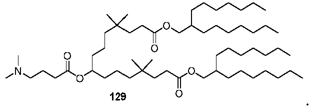

In a more specific embodiment, the present disclosure provides a nanoparticle composition as described above, wherein, the N:P molar ratio of N atoms in the ionizable cationic lipids to P atoms in the load molecules is 1-15:1, alternatively 3-12:1, alternatively 3-7:1, alternatively 6-12:1, more alternatively 3-6:1.

In some specific embodiments, the N:P molar ratio of N atoms in the ionizable cationic lipids to P atoms in the load molecules is 1-10:1, alternatively 3-6:1, more alternatively 3-5:1.

In some specific embodiments, the N:P molar ratio of N atoms in the ionizable cationic lipids to P atoms in the load molecules is 1-15:1, alternatively 3-12:1, more alternatively 6-12:1.

In some specific embodiments, the N:P molar ratio of N atoms in the ionizable cationic lipids to P atoms in the load molecules is 1-10:1, alternatively 3-7:1, more alternatively 5:1.

In a more specific embodiment, the present disclosure provides a nanoparticle composition as described above, wherein, the particle size of the particles is 65-200 nm, alternatively 65-180 nm, alternatively 70-170nm, more alternatively 70-130 nm.

In some specific embodiments, the particle size of the particles is 70-180 nm, alternatively 80-180 nm, alternatively 90-180 nm, more alternatively 100-135 nm.

In some specific embodiments, the particle size of the particles is 65-160 nm, alternatively 65-150 nm, alternatively 70-150 nm, alternatively 90-130 nm, more alternatively 90-115 nm.

In some specific embodiments, the particle size of the particles is 65-140 nm, alternatively 65-130 nm, alternatively 70-130 nm, more alternatively 65-75 nm.

In a more specific embodiment, the present disclosure provides a nanoparticle composition as described above, wherein the lipid ingredient comprises the following components in the molar percentages:
Ionizable cationic lipids 30 mol%-55 mol%, alternatively 32.5 mol%-50 mol%;
Structure lipids 28 mol%-64 mol%, alternatively 30.6 mol%-61 mol%;
Neutral lipids 5 mol%-20 mol%;
Polymer lipids 1 mol%-3 mol%, alternatively 1 mol%-2 mol%;
the ionizable cationic lipid is: 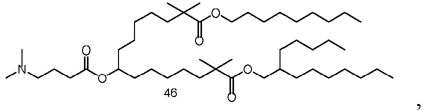 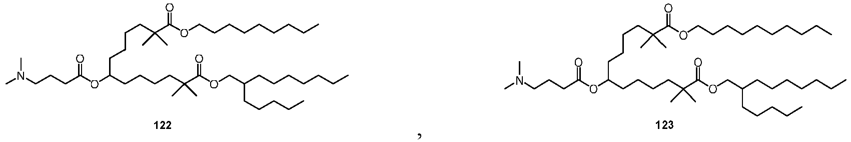 or 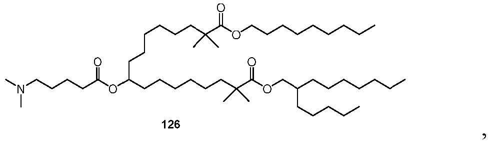 most alternatively is:

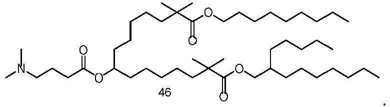

Alternatively, the lipid ingredient comprises the following components in the molar percentages:
Ionizable cationic lipids 40 mol%-52.5 mol%, alternatively 42.5 mol%-50 mol%;
Structure lipids 28 mol%-54 mol%, alternatively 30.6 mol%-51 mol%;
Neutral lipids 5 mol%-20 mol%;
Polymer lipids 1 mol%-3 mol%, alternatively 1 mol%-2 mol%;
the ionizable cationic lipid is: 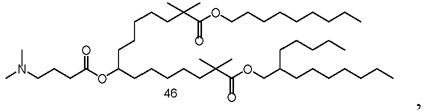 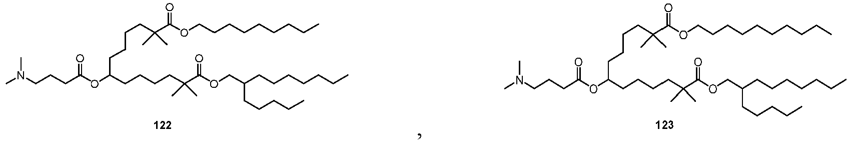 or 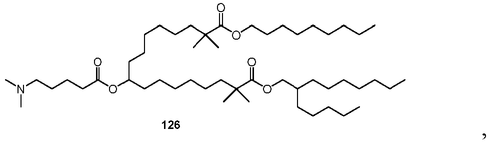 most alternatively is:

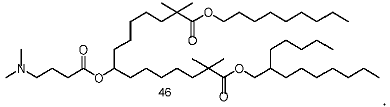

Alternatively, the lipid ingredient comprises the following components in the molar percentages:
Ionizable cationic lipids 45 mol%-52.5 mol%, alternatively 47.5 mol%-50 mol%;
Structure lipids 28 mol%-43 mol%, alternatively 30.6 mol%-40.5 mol%;
Neutral lipids 10 mol%-20 mol%;
Polymer lipids 1.4 mol%-2 mol%;
the ionizable cationic lipid is: 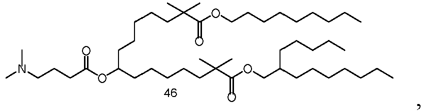 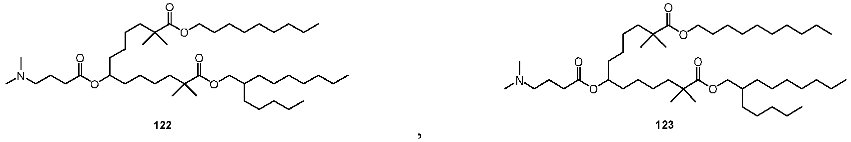 or 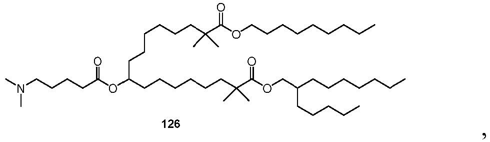 most alternatively is:

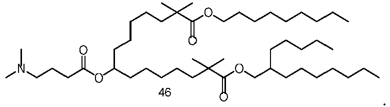

Alternatively, the lipid ingredient comprises the following components in the molar percentages:
Ionizable cationic lipids 45 mol%-50 mol%, alternatively 47 mol%-48 mol%, alternatively 47.6 mol%;
Structure lipids 28 mol%-40.5 mol%, alternatively 30.6 mol%-38.1 mol%;
Neutral lipids 12.5 mol%-20 mol%;
Polymer lipids 1.8 mol%-2 mol%;
the ionizable cationic lipid is: 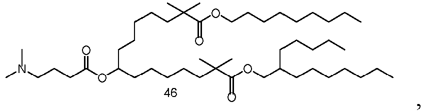 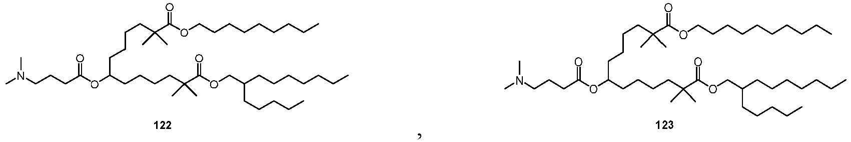 or 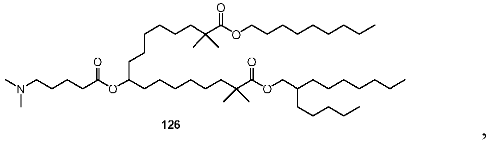 most alternatively is:

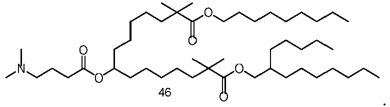

In a more specific embodiment, the present disclosure provides a nanoparticle composition as described above, wherein, the molar ratio of ionizable cationic lipids: structure lipids: neutral lipids: polymer lipids is: 32.5:61:5:1.5, 32.5:51.5:15:1, 42.5:49:7.5: 1, 42.5:51:5:1.5, 47.6:38.1:12.5:1.8, 47.6:30.6:20:1.8, 47.5:40.5:10:2, 47.6:32.9:17.5:2, 47.6:36:15:1.4 or 50:38.5:10:1.5; alternatively: 42.5:49:7.5:1, 42.5:51:5:1.5, 47.6:38.1:12.5:1.8, 47.6:30.6:20:1.8, 47.5:40.5:10:2, 47.6:32.9:17.5:2, 47.6:36:15:1.4 or 50:38.5:10:1.5; alternatively: 47.6:38.1:12.5:1.8, 47.6:30.6:20:1.8, 47.5:40.5:10:2, 47.6:32.9:17.5:2, 47.6:36:15:1.4 or 50:38.5:10:1.5; more alternatively: 47.6:38.1:12.5:1.8, 47.6:30.6:20:1.8 or 47.6:32.9:17.5:2.

In a more specific embodiment, the present disclosure provides a nanoparticle composition as described above, wherein, the N:P molar ratio of N atoms in the ionizable cationic lipids to P atoms in the load molecules is 1-10:1, alternatively 3-6:1, more alternatively 3-5:1.

In a more specific embodiment, the present disclosure provides a nanoparticle composition as described above, wherein, the particle size of the particles is 70-180 nm, alternatively 80-180 nm, alternatively 90-180 nm, more alternatively 100-135 nm.

In a more specific embodiment, the present disclosure provides a nanoparticle composition as described above, wherein the lipid ingredient comprises the following components in the molar percentages:
Ionizable cationic lipids 30 mol%-60 mol%;
Structure lipids 27.5 mol%-66 mol%;
Neutral lipids 1.5 mol%-20 mol%;
Polymer lipids 1.5 mol%-5 mol%;
the ionizable cationic lipid is 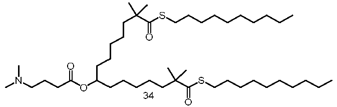 or 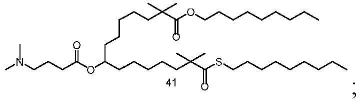 alternatively is: 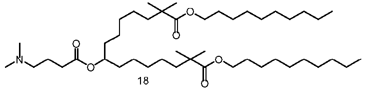 or 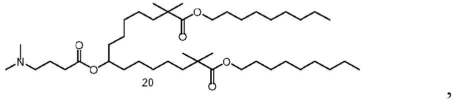 most alternatively is:

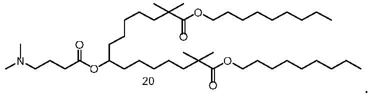

Alternatively, the lipid ingredient comprises the following components in the molar percentages:
Ionizable cationic lipids 30 mol%-50 mol%;
Structure lipids 35 mol%-66 mol%;
Neutral lipids 1.5 mol%-20 mol%;
Polymer lipids 1.5 mol%-5 mol%;
the ionizable cationic lipid is 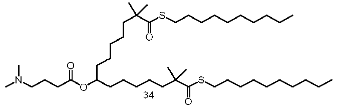 or 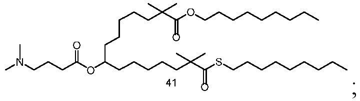 alternatively is: 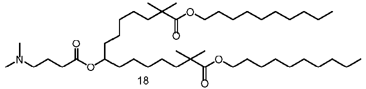 or 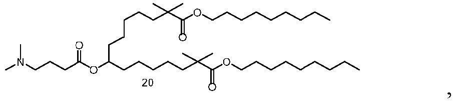 most alternatively is:

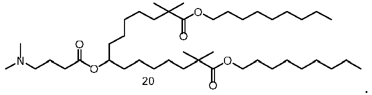

Alternatively, the lipid ingredient comprises the following components in the molar percentages:
Ionizable cationic lipids 30 mol%-50 mol%;
Structure lipids 38.5 mol%-63.5 mol%;
Neutral lipids 1.5 mol%-10 mol%;
Polymer lipids 1.5 mol%-4 mol%, alternatively 1.5 mol%-3.5 mol%;
the ionizable cationic lipid is 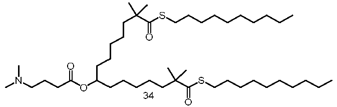 or 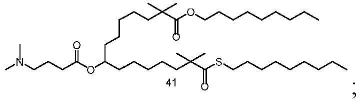 alternatively is: 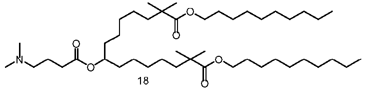 or 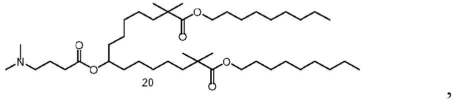 most alternatively is:

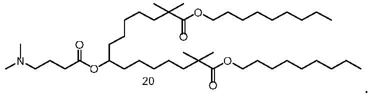

Alternatively, the lipid ingredient comprises the following components in the molar percentages:
Ionizable cationic lipids 30 mol%-50 mol%, alternatively 30 mol%-48.5 mol%;
Structure lipids 43 mol%-60 mol%, alternatively 45.5 mol%-57.5 mol%;
Neutral lipids 1.5 mol%-10 mol%;
Polymer lipids 1.5 mol%-4 mol%, alternatively 1.5 mol%-3.5 mol%;
the ionizable cationic lipid is 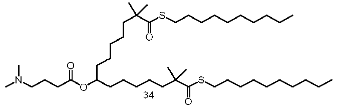 or 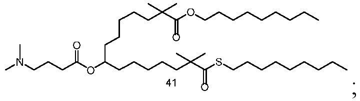 alternatively is: 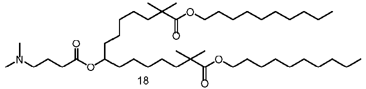 or 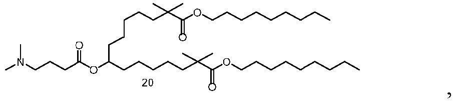 most alternatively is:

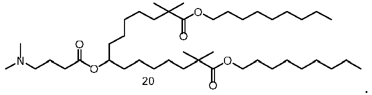

Alternatively, the lipid ingredient comprises the following components in the molar percentages:
Ionizable cationic lipids 46mol%-50 mol%, alternatively 48 mol%-49 mol%, alternatively 48.5 mol%;
Structure lipids 43 mol%-50 mol%, alternatively 45.5 mol%-47.5 mol%;
Neutral lipids 1.5 mol%-3.5 mol%, alternatively 1.5 mol%-2.5 mol%;
Polymer lipids 2 mol%-4 mol%, alternatively 2.5 mol%-3.5 mol%;
the ionizable cationic lipid is 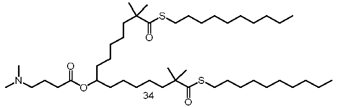 or 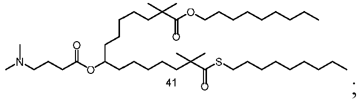 alternatively is: 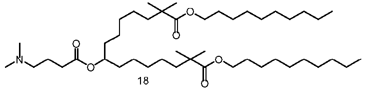 or 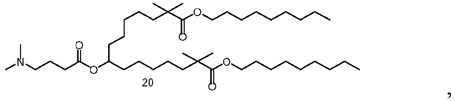 most alternatively is:

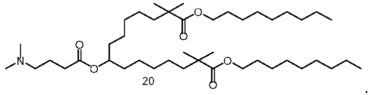

Alternatively, the lipid ingredient comprises the following components in the molar percentages:
Ionizable cationic lipids 46mol%-50 mol%, alternatively 48 mol%-49 mol%, alternatively 48.5 mol%;
Structure lipids 43 mol%-49 mol%, alternatively 45.5 mol%-46.5 mol%;
Neutral lipids 1.5 mol%-3.5 mol%, alternatively 1.5 mol%-2.5 mol%;
Polymer lipids 3 mol%-4 mol%, alternatively 3.5 mol%;
the ionizable cationic lipid is 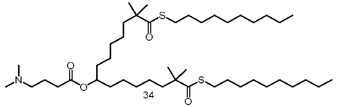 or 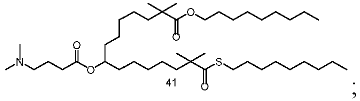 alternatively is: 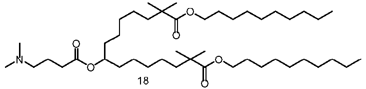 or 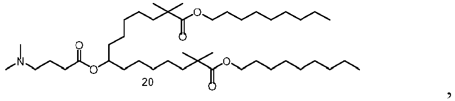 most alternatively is:

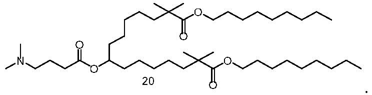

In a more specific embodiment, the present disclosure provides a nanoparticle composition as described above, wherein, the molar ratio of ionizable cationic lipids: structure lipids: neutral lipids: polymer lipids is: 40:35:20:5, 40:48.5:10:1.5, 30:66:2.5:1.5, 40:53.5:5:1.5, 30:63.5:5:1.5, 40:55:2.5:2.5, 30:62.5:5:2.5, 30:57.5:10:2.5, 48.5:47.5:1.5:2.5, 48.5:46.5:2.5:2.5, 48.5:45.5:2.5:3.5, 48.5:46.5:1.5:3.5, 50:42.5:5:2.5, 60:27.5:10:2.5 or 50:38.5:10:1.5;
alternatively: 40:35:20:5, 40:48.5:10:1.5, 30:66:2.5:1.5, 40:53.5:5:1.5, 30:63.5:5:1.5, 40:55:2.5:2.5, 30:62.5:5:2.5, 30:57.5:10:2.5, 48.5:47.5:1.5:2.5, 48.5:46.5:2.5:2.5, 48.5:45.5:2.5:3.5, 48.5:46.5:1.5:3.5, 50:42.5:5:2.5 or 50:38.5:10:1.5;
alternatively: 40:48.5:10:1.5, 40:53.5:5:1.5, 30:63.5:5:1.5, 40:55:2.5:2.5, 30:62.5:5:2.5, 30:57.5:10:2.5, 48.5:47.5:1.5:2.5, 48.5:46.5:2.5:2.5, 48.5:45.5:2.5:3.5, 48.5:46.5:1.5:3.5, 50:42.5:5:2.5 or 50:38.5:10:1.5;
alternatively: 40:48.5:10:1.5, 40:53.5:5:1.5, 40:55:2.5:2.5, 30:57.5:10:2.5, 48.5:47.5:1.5:2.5, 48.5:46.5:2.5:2.5, 48.5:45.5:2.5:3.5 or 48.5:46.5:1.5:3.5;
alternatively: 48.5:47.5:1.5:2.5, 48.5:46.5:2.5:2.5, 48.5:45.5:2.5:3.5 or 48.5:46.5:1.5:3.5; alternatively: 48.5:45.5:2.5:3.5 or 48.5:46.5:1.5:3.5.

In a more specific embodiment, the present disclosure provides a nanoparticle composition as described above, wherein, the N:P molar ratio of N atoms in the ionizable cationic lipids to P atoms in the load molecules is 1-15:1, alternatively 3-12:1, more alternatively 6-12:1.

In a more specific embodiment, the present disclosure provides a nanoparticle composition as described above, wherein, the particle size of the particles is 65-160 nm, alternatively 65-150 nm, alternatively 70-150 nm, alternatively 90-130 nm, more alternatively 90-115 nm.

In a more specific embodiment, the present disclosure provides a nanoparticle composition as described above, wherein the lipid ingredient comprises the following components in the molar percentages:
Ionizable cationic lipids 27.5 mol%-55 mol%, alternatively 30 mol%-50 mol%;
Structure lipids 35 mol%-68.5 mol%, alternatively 38.5 mol%-66 mol%;
Neutral lipids 1.5 mol%-20 mol%, alternatively 2.5 mol%-15 mol%;
Polymer lipids 1 mol%-5 mol%, alternatively 1.5 mol%-3.5 mol%;
the ionizable cationic lipid is: 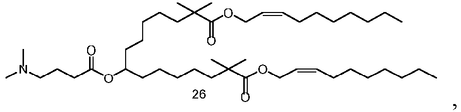 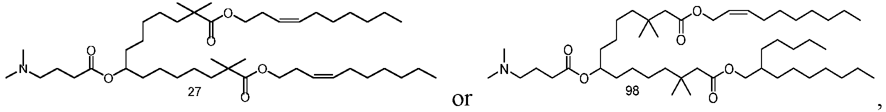 most alternatively is:

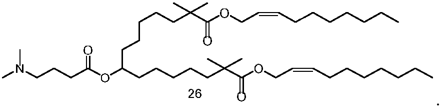

Alternatively, the lipid ingredient comprises the following components in the molar percentages:
Ionizable cationic lipids 27.5 mol%-42.5 mol%, alternatively 30 mol%-40 mol%;
Structure lipids 41 mol%-68.5 mol%, alternatively 43.5 mol%-66 mol%;
Neutral lipids 2 mol%-18 mol%, alternatively 2.5 mol%-15 mol%;
Polymer lipids 1 mol%-4 mol%, alternatively 1.5 mol%-3.5 mol%;
the ionizable cationic lipid is: 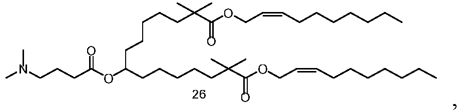 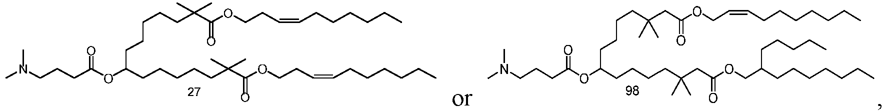 most alternatively is:

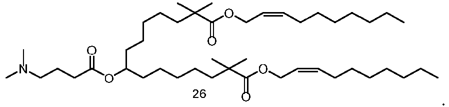

Alternatively, the lipid ingredient comprises the following components in the molar percentages:
Ionizable cationic lipids 37.5 mol%-42.5 mol%, alternatively 40 mol%;
Structure lipids 41 mol%-58.5 mol%, alternatively 43.5 mol%-56 mol%;
Neutral lipids 2 mol%-18 mol%, alternatively 2.5 mol%-15 mol%;
Polymer lipids 1 mol%-4 mol%, alternatively 1.5 mol%-3.5 mol%;
the ionizable cationic lipid is: 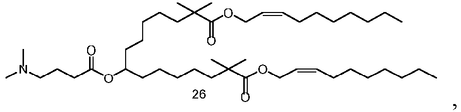 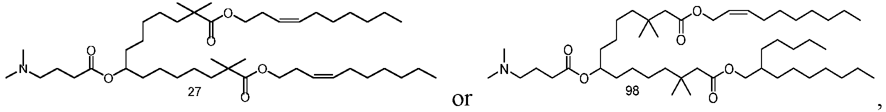 most alternatively is:

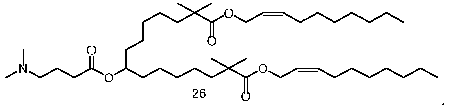

Alternatively, the lipid ingredient comprises the following components in the molar percentages:
Ionizable cationic lipids 27.5 mol%-32.5 mol%, alternatively 30 mol%;
Structure lipids 63.5 mol%-68.5 mol%, alternatively 66 mol%;
Neutral lipids 2 mol%-3 mol%, alternatively 2.5 mol%;
Polymer lipids 1 mol%-2 mol%, alternatively 1.5 mol%;
the ionizable cationic lipid is: 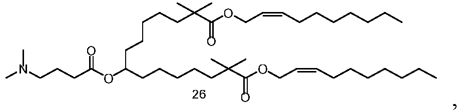 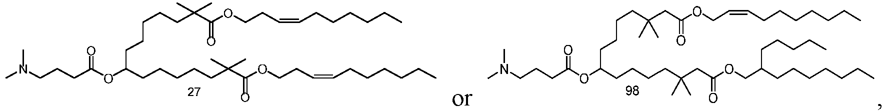 most alternatively is:

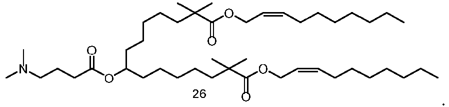

In a more specific embodiment, the present disclosure provides a nanoparticle composition as described above, the molar ratio of ionizable cationic lipids: structure lipids: neutral lipids: polymer lipids is: 40:44:12.5:3.5, 40:53.5:5:1.5, 40:43.5:15:1.5, 30:66:2.5:1.5, 40:56:2.5:1.5, 40:51:7.5:1.5 or 50:38.5:10:1.5; alternatively: 40:44:12.5:3.5, 40:53.5:5:1.5, 40:43.5:15:1.5, 30:66:2.5:1.5, 40:56:2.5:1.5 or 40:51:7.5:1.5; alternatively: 30:66:2.5:1.5.

In a more specific embodiment, the present disclosure provides a nanoparticle composition as described above, wherein, the N:P molar ratio of N atoms in the ionizable cationic lipids to P atoms in the load molecules is 1-10:1, alternatively 3-7:1, more alternatively 5:1.

In a more specific embodiment, the present disclosure provides a nanoparticle composition as described above, wherein, the particle size of the particles is 65-140 nm, alternatively 65-130 nm, alternatively 70-130 nm, more alternatively 65-75 nm.

In a more specific embodiment, the present disclosure provides the nanoparticle composition described above, wherein, the neutral lipids are selected from one or more of DSPC, DMPC, DOPC, DPPC, POPC, DOPE, DMPE, POPE and DPPE, alternatively DSPC and/or DOPE.

In a more specific embodiment, the present disclosure provides the nanoparticle composition described above, wherein, the structure lipids are selected from one or more of cholesterol, sitosterol, coprosterol, fucosterol, brassicasterol, ergosterol, tomatine, ursolic acid, α-tocopherol, stigmasterol, avenasterol, ergocalciferol and campestero, alternatively cholesterol and/or β-sitosterol, more alternatively cholesterol.

In a more specific embodiment, the present disclosure provides the nanoparticle composition described above, wherein, the polymer lipids are polyethylene glycolated lipids.

Optionally, the polyethylene glycolated lipids are selected from one or more of: PEG modified phosphatidylethanolamine, PEG modified phosphatidic acid, PEG modified ceramide, PEG modified dialkyl amine, PEG modified diacylglycerol, and PEG modified dialkylglycerol.

Alternatively, the polyethylene glycolated lipids contain a PEG moiety of about 1000 Da to about 20 kDa, alternatively a PEG moiety of about 1000 Da to about 5000 Da.

Alternatively, the polyethylene glycolated lipids are selected from one or more of DMPE-PEG1000, DPPE-PEG1000, DSPE-PEG1000, DOPE-PEG1000, DMG-PEG2000, Ceramide-PEG2000, DMPE-PEG2000, DPPE-PEG2000, DSPE-PEG2000, Azido-PEG2000, DSPE-PEG2000-Mannose, Ceramide-PEG5000, and DSPE-PEG5000, alternatively DMG-PEG2000.

In a more specific embodiment, the present disclosure provides the nanoparticle composition described above, wherein, the load is selected from one or more of therapeutic, prophylactic and diagnostic agents;
alternatively, the therapeutic, prophylactic or diagnostic agent is a nucleic acid;
alternatively, the nucleic acid is selected from one or more of ASO, RNA and DNA;
alternatively, the RNA is selected from one or more of interfering RNA (RNAi), small interfering RNA (siRNA), short hairpin RNA (shRNA), antisense RNA (aRNA), messenger RNA (mRNA), modified messenger RNA (mmRNA), long non-coding RNA (lncRNA), microRNA (miRNA), small activating RNA (saRNA), multimeric coding nucleic acid (MCNA), polymeric coding nucleic acid (PCNA), guide RNA (gRNA), CRISPRRNA (crRNA) and nucleases, alternatively mRNA, more alternatively, modified mRNA.

The present disclosure provides a lipid compound, a pharmaceutically acceptable salt or stereoisomer thereof, wherein the lipid compound is selected from the following compounds:

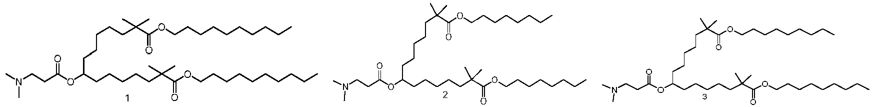

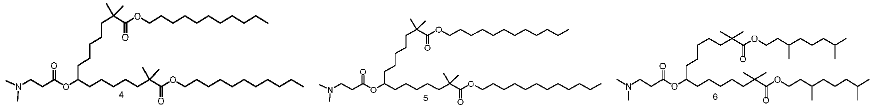

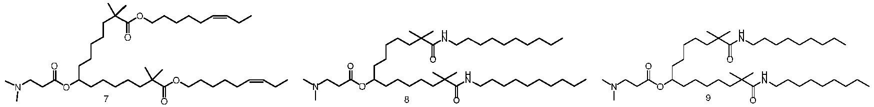

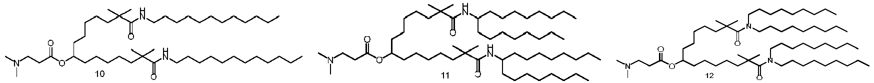

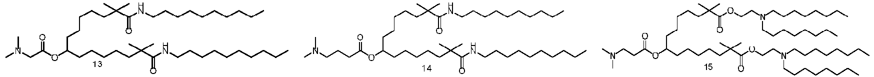

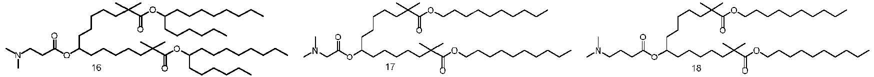

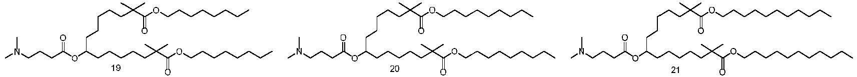

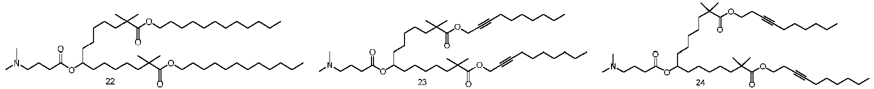

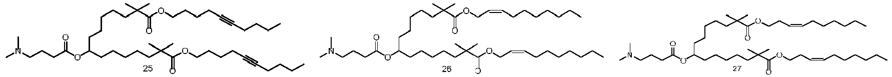

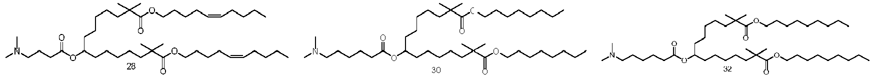

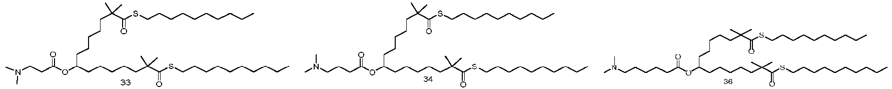

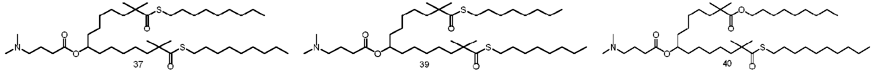

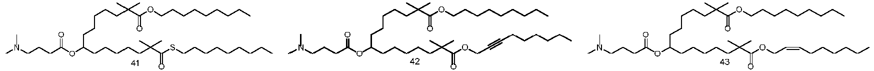

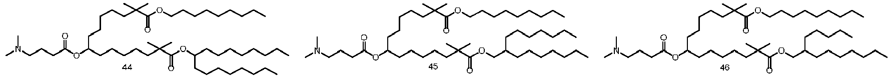

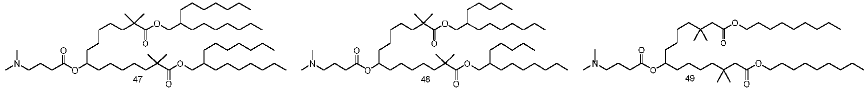

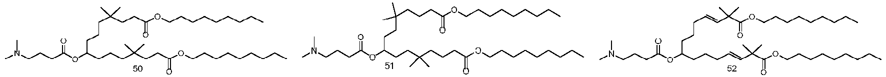

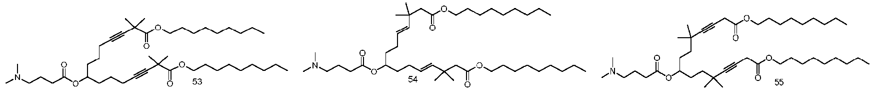

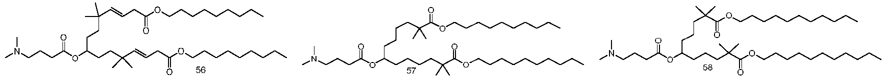

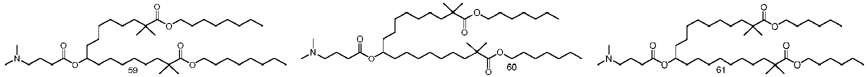

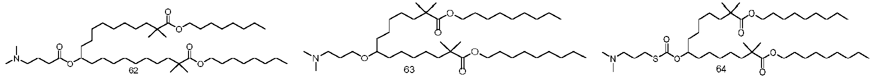

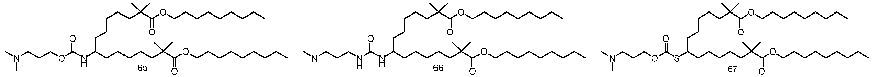

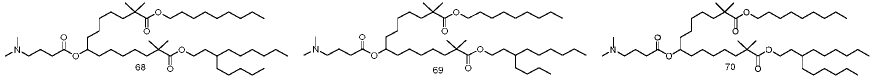

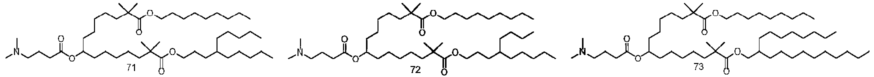

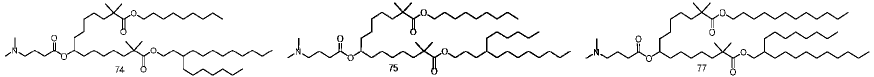

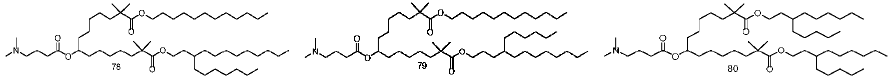

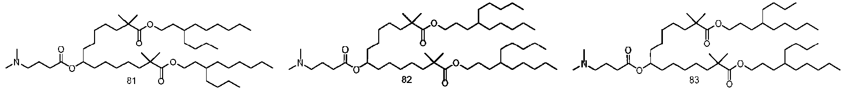

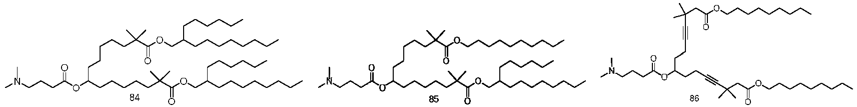

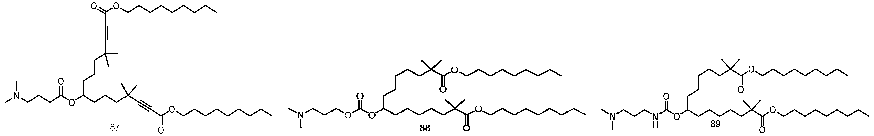

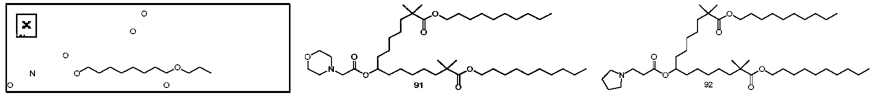

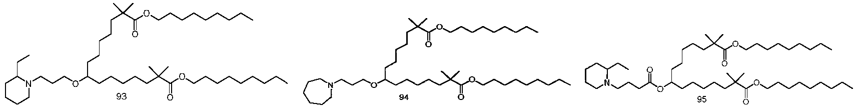

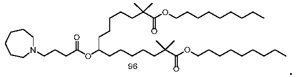

The present disclosure provides a composition, the composition comprises a biologically active substance and lipid compounds of the present disclosure.

Further, the biologically active substance is DNA or RNA.

Further, the composition further comprises neutral lipids, structure lipids and polymeric lipids.

Further, the neutral lipids are DSPC, DMPC, DOPC, DPPC, POPC, DOPE, DMPE, POPE or DPPE.

Further, the structure lipids are selected from one of, or a combination of cholesterol, sitosterol, coprosterol, fucosterol, brassicasterol, ergosterol, tomatine, ursolic acid, α-tocopherol, stigmasterol, avenasterol, ergocalciferol and campesterol.

Further, the polymeric lipids are selected from one of, or a combination of DMPE-PEG1000, DPPE-PEG1000, DSPE-PEG1000, DOPE-PEG1000, DMG-PEG2000, Ceramide-PEG2000, DMPE-PEG2000, DPPE-PEG2000, DSPE-PEG2000, Azido-PEG2000, DSPE-PEG2000-Mannose, Ceramide-PEG5000, and DSPE-PEG5000.

The present disclosure provides a lipid nanoparticle comprising the lipid compound of the present disclosure or the composition of the present disclosure.

The present disclosure provides a pharmaceutical composition comprising the lipid compound of the present disclosure, the composition of the present disclosure or the lipid nanoparticle of the present disclosure, and pharmaceutically acceptable excipient(s).

The present disclosure provides a pharmaceutical composition comprising the lipid compound of the present disclosure, the composition of the present disclosure or the lipid nanoparticle of the present disclosure, and pharmaceutically acceptable excipient(s).

The compounds of the present disclosure may include one or more asymmetric centers, and thus may exist in a variety of stereoisomeric forms, for example, enantiomers and/or diastereomers. For example, the compounds of the present disclosure may be in the form of an individual enantiomer, diastereomer or geometric isomer (e.g., cis- and trans-isomers), or may be in the form of a mixture of stereoisomers, including racemic mixture and a mixture enriched in one or more stereoisomers. The isomers can be separated from the mixture by the methods known to those skilled in the art, including chiral high pressure liquid chromatography (HPLC) and the formation and crystallization of chiral salts; or alternative isomers can be prepared by asymmetric synthesis.

The compounds of the present disclosure may exist in tautomer forms. The tautomer is a functional group isomer resulting from the rapid shift of an atom between two positions in a molecule. The tautomer is a special functional group isomer, wherein a pair of tautomers can convert between each other, but usually exist in a relatively stable isomer as its main form. The most important examples are the enol and keto tautomers.

The present disclosure also comprises compounds that are labeled with isotopes (isotope variants), which are equivalent to those described in formula (IV), but one or more atoms are replaced by atoms having an atom mass or mass number that are different from that of atoms that are common in nature. Examples of isotopes which may be introduced into the compounds of the disclosure include isotopes of hydrogen, carbon, nitrogen, oxygen, phosphorus, sulfur, fluorine and chlorine, such as 2H, ³H, ¹³C, ¹¹C, ¹⁴C, ¹⁵N, ¹⁸O, ¹⁷O, ³¹P, ³²P, ³⁵S, ¹⁸F and ³⁶Cl, respectively. Compounds of the present disclosure that comprise the above isotopes and/or other isotopes of other atoms, prodrugs thereof and pharmaceutically acceptable salts of said compounds or prodrugs all are within the scope of the present disclosure. Certain isotope-labeled compounds of the present disclosure, such as those incorporating radioactive isotopes (e.g., ³H and ¹⁴C), can be used for the measurement of the distribution of drug and/or substrate in tissue. Tritium, which is ³H and carbon-14, which is ¹⁴C isotope, are yet alternative, because they are easy to prepare and detect. Furthermore, replaced by heavier isotopes, such as deuterium, which is ²H, may provide therapeutic benefits due to the higher metabolic stability, such as prolonging the half-life *in vivo* or decreasing the dosage requirements, and thus may be alternative in some cases. Isotope-labeled compounds of formula (I) of the present disclosure and prodrugs thereof can be prepared generally by using readily available isotope-labeled reagents to replace non-isotope-labeled reagents in the following schemes and/or the procedures disclosed in the examples and preparation examples.

The present disclosure also provides a pharmaceutical formulation comprising a therapeutically effective amount of a compound of formula (VI), or therapeutically acceptable salts thereof, and pharmaceutically acceptable carriers, diluents or excipients thereof. All of these forms belong to the present disclosure.

### Pharmaceutical compositions and kits

In another aspect, the present disclosure provides a pharmaceutical composition comprising nanoparticle compositions of the present disclosure and pharmaceutically acceptable excipient(s), the nanoparticle composition comprises the compounds of the present disclosure.

A pharmaceutically acceptable excipient for use in the present disclosure refers to a non-toxic carrier, adjuvant or vehicle which does not destroy the pharmacological activity of the compound formulated together. Pharmaceutically acceptable carriers, adjuvants, or vehicles that may be used in the compositions of the present disclosure include, but are not limited to, ion exchangers, alumina, aluminum stearate, lecithin, serum proteins (e.g., human serum albumin), buffer substances (such as phosphate), glycine, sorbic acid, potassium sorbate, a mixture of partial glycerides of saturated plant fatty acids, water, salt or electrolyte (such as protamine sulfate), disodium hydrogen phosphate, potassium hydrogen phosphate, sodium chloride, zinc salt, silica gel, magnesium trisilicate, polyvinyl pyrrolidone, cellulose-based materials, polyethylene glycol, sodium carboxymethyl cellulose, polyacrylate, wax, polyethylene-polyoxypropylene block polymers, polyethylene glycol and lanolin.

### Example

In order to make the technical solutions of the present disclosure clearer and more explicit, the present disclosure is further elaborated through the following examples. The technical means or methods, etc. not specifically described in the specific embodiments of the present disclosure are conventional technical means or methods, etc. in the art. The materials, reagents, etc. used in examples are commercially available if not otherwise specified.

**Table 1**

| **Abbreviation** | **Full name** |
|---|---|
| THF | Tetrahydrofuran |
| DCM | dichloromethane |
| MeOH | methanol |
| DMF | N, N-Dimethylformamide |
| DMSO | Dimethyl sulfoxide |
| DCE | 1, 2-Dichloroethane |
| CDCl₃ | Deuterated chloroform |
| TBAI | Tetrabutylammonium iodide |
| TsCH₂CN | 4-Toluenesulfonylacetonitrile |
| TMSOK | Potassium trimethylsiloxide |
| TBDMSCl | tert-Butyldimethylsilyl chloride |
| LDA | Lithium diisopropylamide |
| DMAP | 4-Dimethylaminopyridine |
| (COCl)₂ | Oxalyl chloride |
| SOCl₂ | Thionyl dichloride |
| NaBH₄ | Sodium borohydride |
| NaH | Sodium hydride |
| K₂CO₃ | Potassium carbonate |
| EDCI | 1-(3-Dimethylaminopropyl)-3-ethylcarbodiimide |
| DIPEA | N,N-Diisopropylethylamine |
| Et₃N | Triethylamine |
| AcOH | Acetic acid |
| NaBH₃CN | Sodium cyanoborohydride |
| Imidazole | Imidazole |
| NMO | 4-Methylmorpholine N-oxide |
| BDMEP | 2,6-di-tert-Butylpyridine |

### Example 1: Synthesis of compound 1

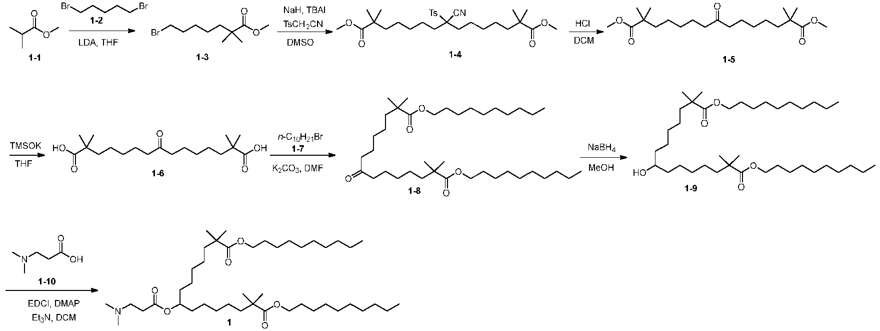

A solution of compound 1-1 (100 g, 979 mmol) in tetrahydrofuran (800 mL) was cooled to -40°C. LDA (2 M, 490 mL) was added slowly dropwise to the solution and the mixture was stirred for another 1 h after completion of the dropwise addition. A solution of 1-2 (315 g, 1.37 mol) in tetrahydrofuran (100 mL) was added dropwise to the reaction system at the same temperature and the reaction system was stirred overnight. The reaction system was quenched with saturated aqueous ammonium chloride, and extracted with ethyl acetate. The organic phases were combined, dried over anhydrous sodium sulfate, and filtered. The filtrate was concentrated to dryness to give the crude product. The crude product was purified by silica gel column to give compound 1-3 (115 g). ¹H NMR (400 MHz, CDCl₃): δ ppm 1.06-1.11 (m, 6 H), 1.13-1.22 (m, 2 H), 1.29-1.39 (m, 2 H), 1.42-1.49 (m, 2 H), 1.73-1.82 (m, 2 H), 3.28-3.40 (m, 2 H), 3.55-3.66 (m, 3 H).

A solution of compound 1-3 (100 g, 398 mmol), TsCH₂CN (38.9 g, 199 mmol) and TBAI (14.7 g, 39.8 mmol) in dimethyl sulfoxide (800 mL) was cooled to 0 °C, and sodium hydride (20.7 g, 517 mmol) was added slowly in batches. The mixture was reacted at room temperature overnight. The reaction system was quenched with saturated aqueous sodium chloride solution and extracted with ethyl acetate. The organic phases were combined, dried over anhydrous sodium sulfate, and filtered. The filtrate was concentrated to dryness to give 115 g of crude compound 1-4, which was used directly in the next reaction without isolation and purification.

To a solution of compound 1-4 crude (110 g, 205 mmol) in dichloromethane (880 mL) was added 330 mL of concentrated hydrochloric acid, and the mixture was reacted at room temperature for 2 h. The complete reaction of the substrate was monitored by TLC. The reaction system was quenched with saturated aqueous ammonium chloride solution and extracted with ethyl acetate. The organic phases were combined, dried over anhydrous sodium sulfate, and filtered. The filtrate was concentrated to dryness to give the crude product. The crude product was purified by silica gel column to give compound 1-5 (30.0 g, 80.9 mmol, yield 39.4%).

TMSOK (11.0 g, 86.4 mmol) was added to a solution of compound 1-5 (8.0 g, 21.6 mmol) in tetrahydrofuran (35.0 mL) at room temperature, and the reaction system was heated to 70°C with stirring. The complete consumption of reaction materials was monitored by TLC. The reaction solution was cooled to room temperature, and the organic solvent was removed by rotary evaporation. The crude product was added to 20 mL of water and extracted with dichloromethane. The aqueous layer was collected, and the solution was adjusted to a pH of <5 with 1 M hydrochloric acid. The solution was extracted with dichloromethane. The organic phases were combined, dried over anhydrous sodium sulfate, and filtered. The filtrate was collected and concentrated to give compound 1-6 (7.0 g). ¹H NMR (400 MHz, CDCl₃): δ ppm 1.03 (s, 12H), 1.08-1.17 (m, 8H), 1.34-1.45 (m, 8H), 2.21 (t, *J* = 7.2 Hz, 4H).

Potassium carbonate (482 mg, 3.48 mmol) was added to a solution of compound 1-6 (294 mg, 0.87 mmol) and 1-7 (771 mg, 3.48 mmol) in DMF, then the reaction was warmed up to 60 °C for 6 h. The complete disappearance of reactant 1-6 was monitored. The mixture was cooled to room temperature. The reaction system was quenched with saturated aqueous sodium chloride solution and extracted with ethyl acetate. The organic phases were combined, dried over anhydrous sodium sulfate, and filtered. The filtrate was concentrated to dryness to give the crude product. The crude was purified by silica gel column to give compound 1-8 (325 mg).

Compound 1-8 (325 mg) was dissolved in 4.0 mL of methanol and sodium borohydride (30 mg, 0.84 mmol) was added to the reaction system. The mixture was reacted at room temperature. The complete disappearance of the reactants was monitored by TLC. The reaction system was quenched with saturated aqueous sodium chloride solution and extracted with dichloromethane. The organic phases were combined, dried over anhydrous sodium sulfate, and filtered. The filtrate was concentrated to dryness to give crude compound 1-9 (260 mg), which was used directly in the next reaction without purification.

Crude compound 1-9 (260 mg, 0.42 mmol), 1-10 (73.1 mg, 0.63 mmol), EDCI (238 mg, 1.26 mmol), triethylamine (0.17 mL, 1.26 mmol) and DMAP (51 mg, 0.42 mmol) were dissolved in 5.0 mL of dichloromethane, and the reaction solution was stirred to react at room temperature for 12 h. The reaction solution was quenched with saturated aqueous sodium chloride and extracted with dichloromethane. The organic phases were combined, dried over anhydrous sodium sulfate, and filtered. The organic phase was collected and the organic solvent was removed using a rotary-evaporator to give the crude product, which was purified by preparative high performance liquid chromatography to give compound 1 (130 mg).

¹H NMR (400 MHz, CDCl₃): δ ppm 0.89 (t, *J =* 7.2 Hz, 6H), 1.15 (s, 12H), 1.27 (m, 40H), 1.49 (m, 8H), 1.61 (m, 4H), 2.26 (s, 6H), 2.44-2.52 (t, *J =* 7.2 Hz, 2H), 2.63 (t, *J =* 7.2 Hz, 2H), 4.04 (t, *J=* 6.8 Hz, 4H), 4.86 (m, 1H); ESI-MS m/z: 724.7 [M+H]⁺.

### Example 2: Synthesis of compound 2

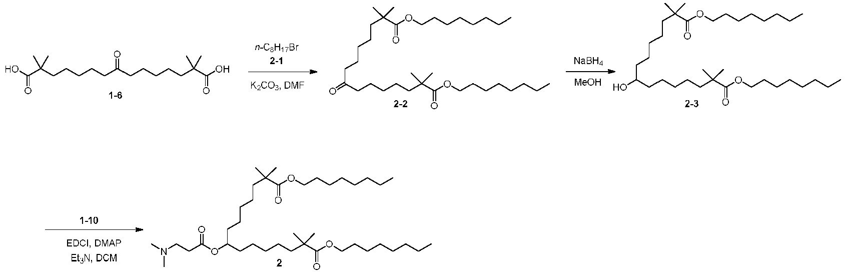

Referring to the method of Example 1, compound 2 was prepared as an oily product: 25.7 mg.

¹H NMR (400 MHz, CDCl₃): δ ppm 0.89 (t, *J =* 6.8 Hz, 6H), 1.15 (s, 12H), 1.29 (m, 32H), 1.49 (m, 8H), 1.60 (m, 4H), 2.24 (s, 6H), 2.46 (t, *J =* 7.2 Hz, 2H), 2.61(t, *J =* 7.2 Hz, 2H), 4.04 (t, *J =* 6.8 Hz, 4H), 4.86 (m, 1H); ESI-MS m/z: 668.6 [M+H]⁺.

### Example 3: Synthesis of compound 3

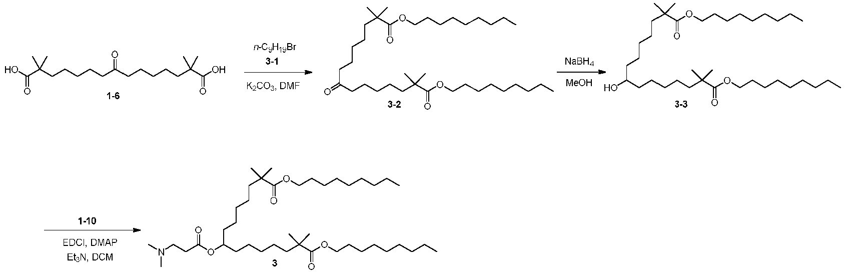

Referring to the method of Example 1, compound 3 was prepared as an oily product: 31.2 mg.

¹H NMR (400 MHz, CDCl₃): δ ppm 0.89 (t, *J =* 6.8 Hz, 6H), 1.16 (s, 12H), 1.28 (m, 36H), 1.49 (m, 8H), 1.62 (m, 4H), 2.25 (s, 6H), 2.47 (t, *J =* 7.2 Hz, 2H), 2.62(t, *J* = 7.2 Hz, 2H), 4.05 (t, *J =* 6.8 Hz, 4H), 4.88 (m, 1H); ESI-MS m/z: 696.6 [M+H]⁺.

### Example 4: Synthesis of compound 4

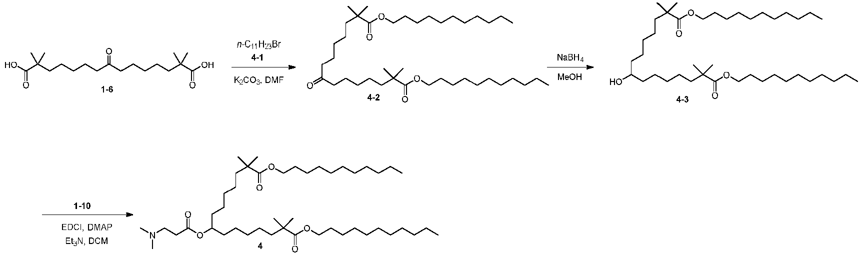

Referring to the method of Example 1, compound 4 was prepared as an oily product: 32 mg.

¹H NMR (400 MHz, CDCl₃): δ ppm 0.89 (t, *J* = 6.8 Hz, 6H), 1.16 (s, 12H), 1.28 (m, 44H), 1.49 (m, 8H), 1.52 (m, 4H), 2.51 (s, 6H), 2.53 (t, *J=* 7.2 Hz, 2H), 3.12 (t, *J=* 7.2 Hz, 2H), 3.91 (t, *J =* 6.8 Hz, 4H), 4.82 (m, 1H); ESI-MS m/z: 752.7 [M+H]⁺.

### Example 5: Synthesis of compound 5

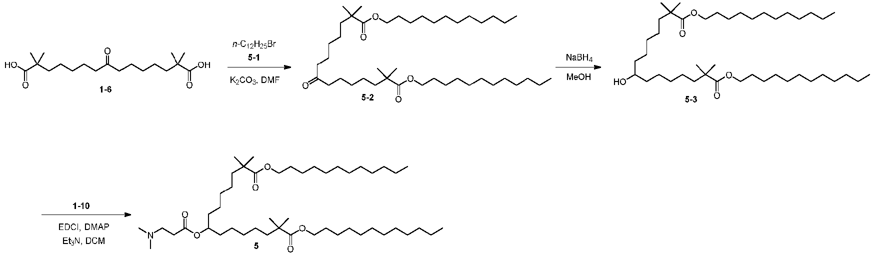

Referring to the method of Example 1, compound 5 was prepared as an oily product: 31.4 mg.

¹H NMR (400 MHz, CDCl₃): δ ppm 0.88 (t, *J* = 6.8 Hz, 6H), 1.15 (s, 12H), 1.25 (m, 48H), 1.49 (m, 8H), 1.52 (m, 4H), 2.46 (s, 6H), 2.63 (m, 2H), 2.86 (m, 2H), 4.03 (t, *J* = 6.8 Hz, 4H), 4.84 (m, 1H); ESI-MS m/z: 780.7 [M+H]⁺.

### Example 6: Synthesis of compound 6

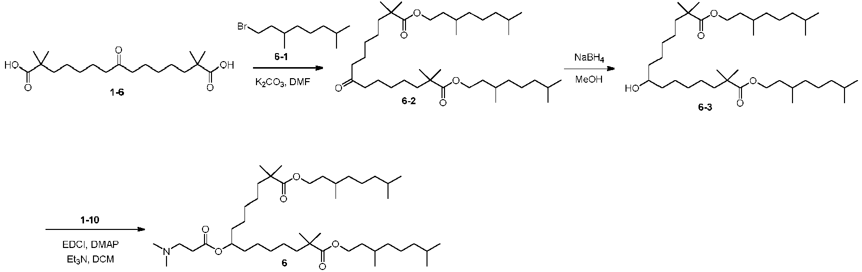

Referring to the method of Example 1, compound 6 was prepared as an oily product: 30.7 mg.

¹H NMR (400 MHz, CDCl₃): δ ppm 0.83 (t, *J* = 6.8 Hz, 18H), 1.00-1.28 (m, 34H), 1.31-1.62 (m, 18H), 2.21 (s, 6H), 2.36-2.46 (m, 2H), 2.51-2.62 (m, 2H), 4.02 (t, *J=* 6.8 Hz, 4H), 4.71-4.85 (m, 1H); ESI-MS m/z: 724.6 [M+H]⁺.

### Example 7: Synthesis of compound 7

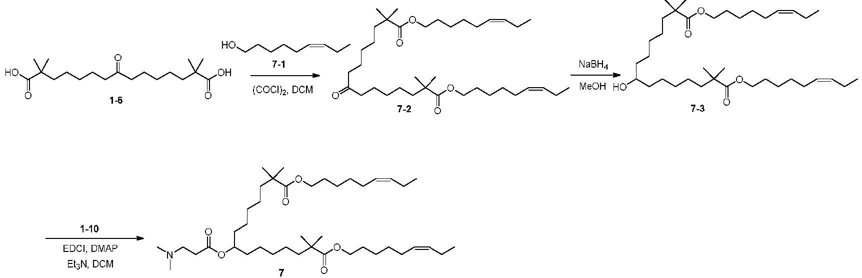

Compound 1-6 (548 mg, 1.5 mmol) was dissolved in 5.0 mL of dichloromethane, and the reaction system was cooled to 0 °C in an ice bath. DMF (12 µL, 0.15 mmol) was added and oxalyl chloride (0.47 mL, 6.0 mmol) was added dropwise to the reaction solution. The ice bath was removed and the mixture was stirred for 1 h at room temperature. The solvent was removed using a rotary-evaporator to give acyl chloride crude product (458 mg) as an oil, which was used directly in the next reaction step.

The above obtained acyl chloride crude product (458 mg) was dissolved in 3.0 mL of 1,2-dichloroethane, and then compound 7-1 (429 mg, 3.0 mmol) was added to the reaction solution. The mixture was stirred at room temperature until the substrate was reacted completely. The solvent was removed using a rotary-evaporator to give the crude product, which was purified by silica gel column to give compound 7-2 (540 mg).

Then referring to the method of Example 1, compound 7 was prepared as an oily product: 33.2 mg.

¹H NMR (400 MHz, CDCl₃): δ ppm 0.89 (t, *J* = 6.8 Hz, 6H), 1.23 (s, 12H), 1.29-1.51 (m, 32H), 1.95 (m, 8H), 2.18 (s, 6H), 2.41 (m, 2H), 2.53 (m, 2H), 3.91 (t, *J =* 6.8 Hz, 4H), 4.78 (m, 1H), 5.25 (m, 4H); ESI-MS m/z: 692.6 [M+H]⁺.

### Example 8: Synthesis of compound 8

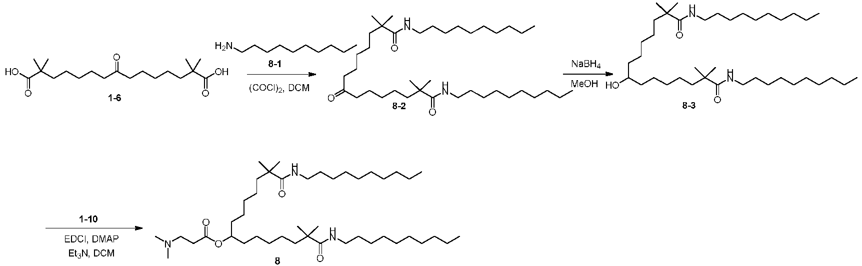

Compound 1-6 (548 mg, 1.5 mmol) was dissolved in 5.0 mL of dichloromethane, and the reaction system was cooled in an ice bath. DMF (12 µL, 0.15 mmol) was added and oxalyl chloride (0.47 mL, 6.0 mmol) was added dropwise to the reaction solution. The ice bath was removed and the mixture was stirred for 1 h at room temperature. The solvent was removed using a rotary-evaporator to give acyl chloride crude product (458 mg) as an oil, which was used directly in the next reaction step.

The above obtained 458 mg of acyl chloride crude product was dissolved in 3.0 mL of 1,2-dichloroethane, and then compound 8-1 (472 mg, 3.0 mmol) was added to the reaction solution. The mixture was stirred at room temperature until the substrate was reacted completely. The solvent was removed using a rotary-evaporator to give crude product, which was purified by silica gel column to give compound 8-2 (518 mg).

518 mg of compound 8-2 was dissolved in 5.0 mL of methanol, and sodium borohydride (48 mg, 1.25 mmol) was added to the reaction system. The mixture was reacted at room temperature. The complete disappearance of the reactants was monitored by TLC. The reaction system was quenched with saturated aqueous sodium chloride solution and extracted with dichloromethane. The organic phases were combined, dried over anhydrous sodium sulfate, and filtered. The filtrate was concentrated to dryness to give 473 mg of crude compound 8-3, which was used directly in the next reaction without purification.

Crude compound 8-3 (270 mg, 0.43 mmol), 1-10 (76.1 mg, 0.65 mmol), EDCI (248 mg, 1.3 mmol), triethylamine (0.18 mL, 1.3 mmol) and DMAP (53 mg, 0.43 mmol) were dissolved in 5.0 mL of dichloromethane, and the reaction solution was stirred to react at room temperature for 12 h. The reaction system was quenched with saturated aqueous sodium chloride and extracted with dichloromethane. The organic phases were combined, dried over anhydrous sodium sulfate, and filtered. The organic phase was collected and the organic solvent was removed using a rotary-evaporator to give the crude product, which was purified by preparative high performance liquid chromatography to give compound 8 (39 mg).

¹H NMR (400 MHz, CDCl₃): δ ppm 0.89 (t, *J* = 6.8 Hz, 6H), 1.14 (s, 12H), 1.15-1.31 (m, 40H), 1.40-1.52 (m, 12H), 2.25 (s, 6H), 2.45 (m, 2H), 2.60 (m, 2H), 3.15 (t, *J* = 6.8 Hz, 4H), 4.77-4.89 (m, 1H), 5.51-5.67 (m, 2H); ESI-MS m/z: 722.7 [M+H]⁺.

### Example 9: Synthesis of compound 9

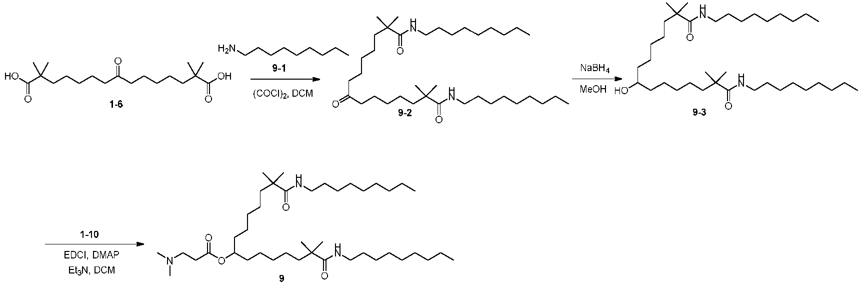

Referring to the method of Example 8, compound 9 (73 mg) was prepared.

¹H NMR (400 MHz, CDCl₃): δ ppm 0.89 (t, *J* = 6.8 Hz, 6H), 1.15 (s, 12H), 1.27-1.49 (m, 48H), 2.25 (s, 6H), 2.46 (t, *J =* 7.2 Hz, 2H), 2.62 (t, *J =* 7.2 Hz, 2H), 3.24 (m, 4H), 4.85 (m, 1H), 5.58 (m, 2H); ESI-MS m/z: 694.6 [M+H]⁺.

### Example 10: Synthesis of compound 10

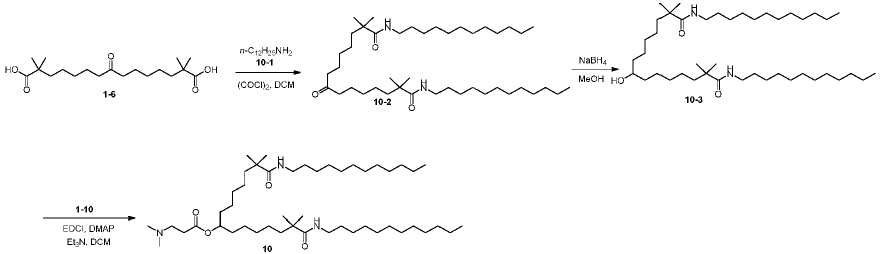

Referring to the method of Example 8, compound 10 (31.2 mg) was prepared.

¹H NMR (400 MHz, CDCl₃): δ ppm 0.79 (t, *J* = 7.2 Hz, 6H), 1.07 (s, 12H), 1.27-1.49 (m, 48H), 1.41 (m, 12H), 2.18 (s, 6H), 2.41 (t, *J =* 7.2 Hz, 2H), 2.55 (t, *J=* 7.2 Hz, 2H), 3.16 (m, 4H), 4.78 (m, 1H), 5.51 (m, 2H); ESI-MS m/z: 778.8 [M+H]⁺.

### Example 11: Synthesis of compound 11

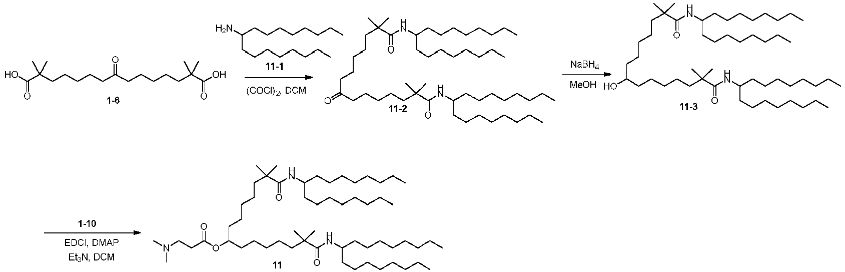

Referring to the method of Example 8, compound 11 (48.1 mg) was prepared.

¹H NMR (400 MHz, CDCl₃): δ ppm 0.78 (t, *J* = 7.2 Hz, 12H), 1.07 (s, 12H), 1.14-1.19 (m, 60H), 1.40 (m, 16H), 2.18 (s, 6H), 2.36-2.47 (m, 2H), 2.49-2.68 (m, 2H), 3.76-3.88 (m, 2H), 4.74-4.83 (m, 1H), 5.10-5.19 (m, 2H); ESI-MS m/z: 918.9 [M+H]⁺.

### Example 12: Synthesis of compound 12

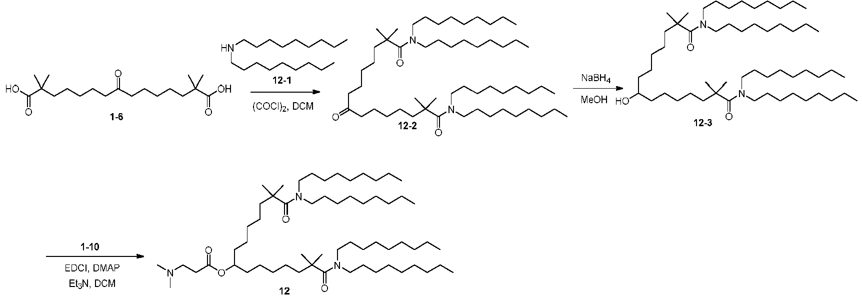

Referring to the method of Example 8, compound 12 (52 mg) was prepared.

¹H NMR (400 MHz, CDCl₃): δ ppm 0.82 (t, *J* = 6.8 Hz, 12H), 1.15-1.32 (m, 72H), 1.54 (m, 16H), 2.31 (s, 6H), 2.51 (t, *J =* 7.2 Hz, 2H), 2.60 (t, *J =* 7.2 Hz, 2H), 3.14-3.33 (m, 8H), 4.75-4.83 (m, 1H); ESI-MS m/z: 946.9 [M+H]⁺.

### Example 13: Synthesis of compound 13

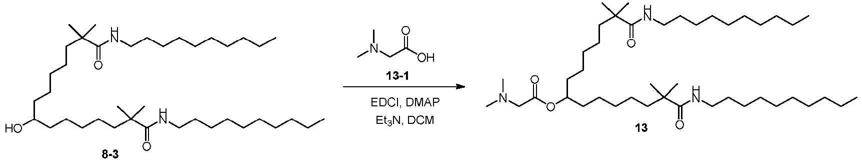

Referring to the method of Example 8, compound 13 (32 mg) was prepared.

¹H NMR (400 MHz, CDCl₃): δ ppm 0.81 (t, *J* = 7.2 Hz, 6H), 1.19 (m, 52H), 1.41 (m, 12H), 2.26 (s, 6H), 3.05 (s, 2H), 3.16 (m, 4H), 4.83 (m, 1H), 5.51 (m, 2H); ESI-MS m/z: 708.7 [M+H]⁺.

### Example 14: Synthesis of compound 14

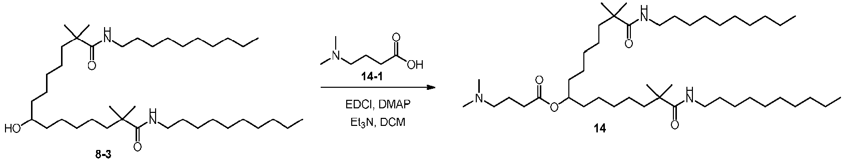

Referring to the method of Example 8, compound 14 (18 mg) was prepared.

¹H NMR (400 MHz, CDCl₃): δ ppm 0.81 (t, *J* = 7.2 Hz, 6H), 1.07 (s, 12H), 1.08-1.31 (m, 44H), 1.35-1.47 (m, 8H), 1.71-1.84 (m, 2H), 2.09-2.38 (m, 10H), 3.12-3.27 (m, 4H), 4.70-4.82 (m, 1H), 5.49-5.63 (m, 2H); ESI-MS m/z: 736.7 [M+H]⁺.

### Example 15: Synthesis of compound 15

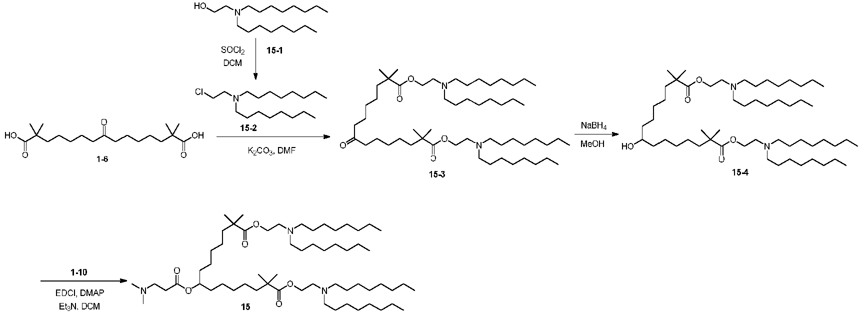

A solution of compound 15-1 (400 mg, 1.4 mmol) in dichloromethane (3.0 mL) was cooled to 0 °C, then a solution of SOCl₂ (0.12 mL, 1.68 mmol) in dichloromethane (2.0 mL) was added dropwise. After the dropwise addition was completed, the mixture was stirred at 0 °C for another 1 h. After the reaction was completed, the reaction was quenched by adding saturated sodium bicarbonate solution to the reaction system, and the reaction system was extracted with dichloromethane. The organic phases were combined and the organic solvent was removed to give crude compound 15-2, which was used directly in the next reaction without purification.

Compound 1-6 (223, 0.65 mmol), 15-2 (496 mg, 1.63 mmol) and potassium carbonate (361 mg, 2.6 mmol) were dissolved in 5.0 mL of DMF and the reaction solution was heated to 70°C to react for 6 hours. The reaction solution was cooled to room temperature, then the reaction was quenched by adding saturated sodium chloride solution to the reaction system, and the reaction system was extracted with dichloromethane. The organic phases were combined and the organic solvent was removed to give the crude product. The crude was purified by silica gel column to give compound 15-3.

Then referring to the method of Example 1, compound 15 (40 mg) was prepared.

¹H NMR (400 MHz, CDCl₃): δ ppm 0.89 (t, *J* = 7.2 Hz, 12H), 1.08 (s, 12H), 1.12-1.35 (m, 46H), 1.38-1.58 (m, 22H), 2.35 (s, 6H), 2.41-2.52 (m, 10H), 2.57-2.65 (m, 2H), 2.62 (m, 4H), 4.10 (t, *J =* 6.4 Hz, 4H), 4.86 (m, 1H); ESI-MS m/z: 978.9 [M+H]⁺.

### Example 16: Synthesis of compound 16

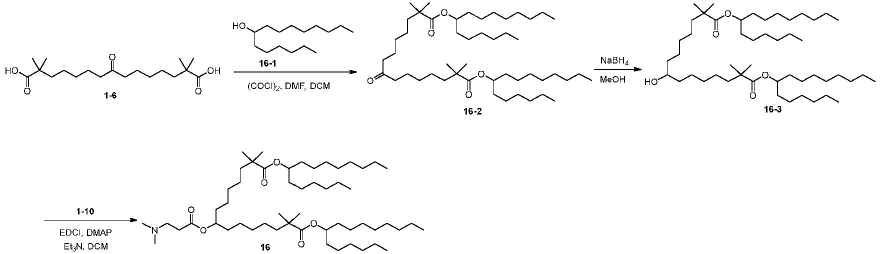

DMF (11 µL, 0.14 mmol) was added to a solution of compound 1-6 (460 mg, 1.34 mmol) in dichloromethane (5.0 mL) under ice bath conditions, and oxalyl chloride (0.47 mL, 5.37 mmol) was then added dropwise to the reaction solution. The ice bath was removed, and the mixture was stirred for 1 h at room temperature. The solvent was removed using a rotary-evaporator to give 255 mg of acyl chloride crude product as an oil, which was used directly in the next reaction step.

The above obtained acyl chloride crude product (255 mg, 0.67 mmol) was dissolved in 3.0 mL of 1,2-dichloroethane, and then compound 16-1 (384 mg, 1.68 mmol) was added to the reaction solution. The mixture was stirred at room temperature until the substrate was reacted completely. The solvent was removed using a rotary-evaporator to give the crude product, which was purified by silica gel column to give 300 mg of compound 16-2. ¹H NMR (400 MHz, CDCl₃): δ ppm 0.78-0.83 (m, 12H), 1.07 (s, 12H), 1.13-1.22 (m, 48H), 1.49 (br s, 16H), 2.29 (t, *J* = 7.50 Hz, 4H), 4.76 (m, 2H).

Compound 16-2 (300 mg, 0.39 mmol) was dissolved in 4.0 mL of methanol. Then NaBH₄ (45 mg, 1.17 mmol) was slowly added to the reaction solution and the mixture was stirred at room temperature for 2 h. The reaction solution was quenched with saturated ammonium chloride solution, extracted with ethyl acetate. The organic phases were combined and the organic solvent was removed to give 300 mg of crude compound 16-3, which was used directly in the next reaction without purification.

Crude compound 16-3 (300 mg, 0.39 mmol) was dissolved in 2.0 mL DMF, and then 1-10 (69 mg, 0.59 mmol), EDCI (225 mg, 1.17 mmol), triethylamine (119 mg, 1.17 mmol) and DMAP (48 mg, 0.39 mmol) were added. The mixture was stirred at room temperature until the reactants was reacted completely. The reaction solution was quenched with saturated sodium chloride solution and extracted with ethyl acetate. The organic phases were combined, dried over anhydrous sodium sulfate, and filtered. The filtrate was concentrated to dryness to give the crude product. The crude product was purified by preparative high performance liquid chromatography to give compound 16 (32.5 mg).

¹H NMR (400 MHz, CDCl₃): δ ppm 0.79 (t, *J* = 7.2 Hz, 12H), 1.07 (s, 12H), 1.19 (m, 52H), 1.40-1.46 (m, 16H), 2.15 (s, 6H), 2.34-2.58 (m, 4H), 4.74-4.81 (m, 3H); ESI-MS m/z: 864.8 [M+H]⁺.

### Example 17: Synthesis of compound 17

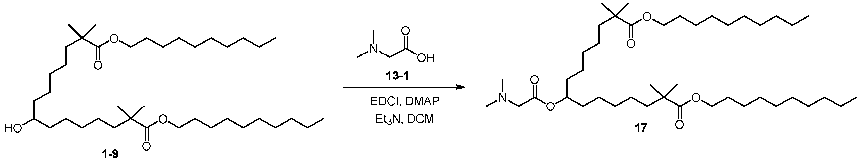

Referring to the method of Example 1, compound 17 was prepared as an oily product: 41.3 mg.

¹H NMR (400 MHz, CDCl₃): δ ppm 0.82 (t, *J* = 7.2 Hz, 6H), 1.08 (s, 12H), 1.14-1.20 (m, 36H), 1.40-1.64 (m, 16H), 2.32 (s, 6H), 3.08-3.21 (m, 2H), 3.97 (t, *J* = 7.2 Hz, 4H), 4.83-4.92 (m, 1H); ESI-MS m/z: 710.6 [M+H]⁺.

### Example 18: Synthesis of compound 18

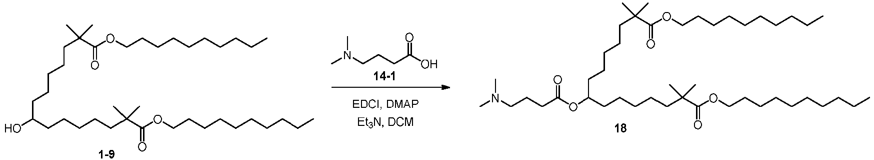

Referring to the method of Example 1, compound 18 was prepared as an oily product: 35.4 mg.

¹H NMR (400 MHz, CDCl₃): δ ppm 0.79 (t, *J* = 7.2 Hz, 6H), 1.08 (s, 12H), 1.13-1.25 (m, 36H), 1.28-1.47 (m, 10H), 1.47-1.62 (m, 6H), 1.68-1.79 (m, 2H), 2.15 (s, 6H), 2.21-2.31 (m, 4H), 3.97 (t, *J =* 7.2 Hz, 4H), 4.73-4.82 (m, 1H); ESI-MS m/z: 738.7 [M+H]⁺.

### Example 19: Synthesis of compound 19

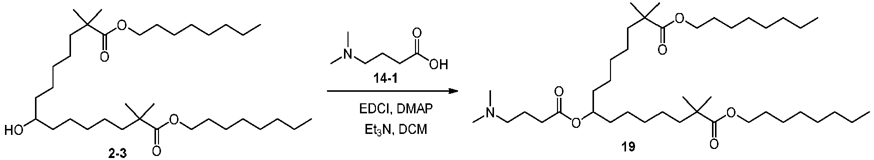

Referring to the method of Example 1, compound 19 was prepared as an oily product: 33.1 mg.

¹H NMR (400 MHz, CDCl₃): δ ppm 0.89 (t, *J* = 7.2 Hz, 6H), 1.15 (s, 12H), 1.29 (m, 30H), 1.50 (m, 8H), 1.60 (m, 6H), 1.64 (m, 2H), 2.23 (s, 6H), 2.33 (m, 4H), 4.05 (t, *J* = 6.8 Hz, 4H), 4.86 (m, 1H); ESI-MS m/z: 682.6 [M+H]⁺.

### Example 20: Synthesis of compound 20

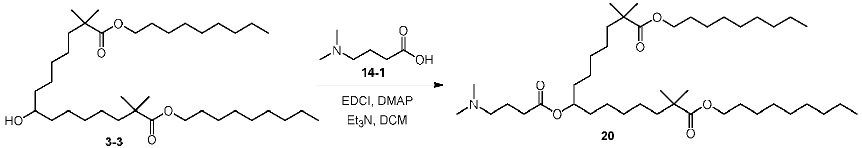

Referring to the method of Example 1, compound 20 was prepared as an oily product: 302 mg.

¹H NMR (400 MHz, CDCl₃): δ ppm 0.89 (t, *J* = 7.2 Hz, 6H), 1.15 (s, 12H), 1.27 (m, 34H), 1.47 (m, 8H), 1.51 (m, 6H), 1.79 (m, 2H), 2.23 (s, 6H), 2.33 (m, 4H), 4.04 (t, *J =* 6.8 Hz, 4H), 4.85 (m, 1H); ESI-MS m/z: 710.7 [M+H]⁺.

### Example 21: Synthesis of compound 21

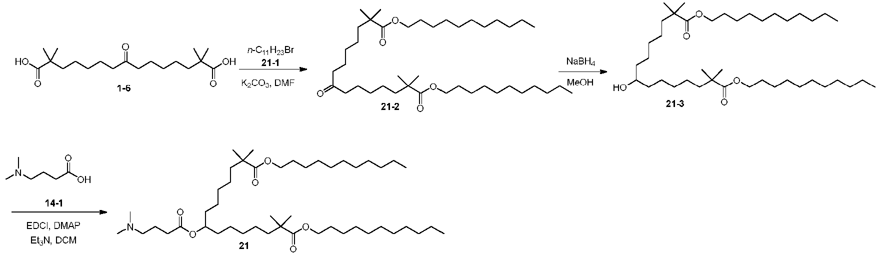

Referring to the method of Example 1, compound 21 was prepared as an oily product: 31.2 mg.

¹H NMR (400 MHz, CDCl₃): δ ppm 0.79 (t, *J* = 7.2 Hz, 6H), 1.08 (s, 12H), 1.25 (m, 44H), 1.39 (m, 8H), 1.51 (m, 4H), 1.82 (m, 2H), 2.25 (t, *J* = 7.2 Hz, 2H), 2.32 (s, 6H), 2.41 (m, 2H), 3.96 (t, *J =* 6.8 Hz, 4H), 4.75 (m, 1H); ESI-MS m/z: 766.7 [M+H]⁺.

### Example 22: Synthesis of compound 22

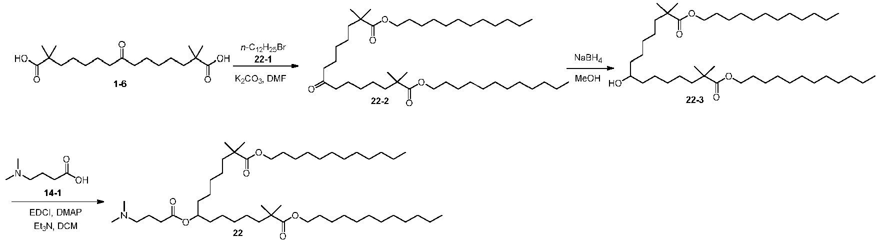

Referring to the method of Example 1, compound 22 was prepared as an oily product: 31.8 mg.

¹H NMR (400 MHz, CDCl₃): δ ppm 0.79 (t, *J* = 7.2 Hz, 6H), 1.07 (s, 12H), 1.28 (m, 48H), 1.40 (m, 8H), 1.53 (m, 4H), 1.84 (m, 2H), 2.26 (t, *J* = 7.2 Hz, 2H), 2.35 (s, 6H), 2.48 (m, 2H), 3.98 (t*, J =* 6.8 Hz, 4H), 4.75 (m, 1H); ESI-MS m/z: 794.7 [M+H]⁺.

### Example 23: Synthesis of compound 23

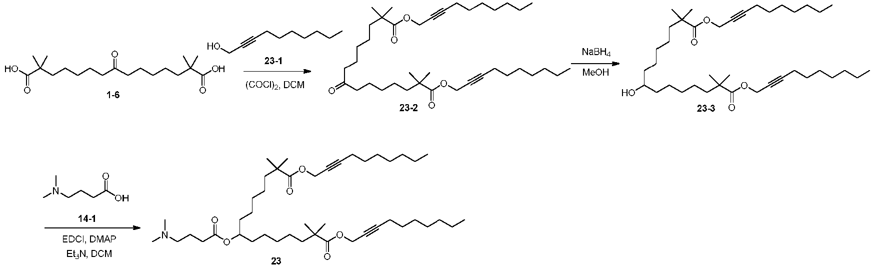

Referring to the method of Example 7, compound 23 was prepared as an oily product: 31.0 mg.

¹H NMR (400 MHz, CDCl₃): δ ppm 0.87 (t, *J* = 7.2 Hz, 6H), 1.16 (s, 12H), 1.20-1.39 (m, 28H), 1.45-1.54 (m, 12H), 1.74-1.82 (m, 2H), 2.12-2.35 (m, 14), 4.63 (t, *J =* 2.4 Hz, 4H), 4.79-4.88 (m, 1H); ESI-MS m/z: 730.6 [M+H]⁺.

### Example 24: Synthesis of compound 24

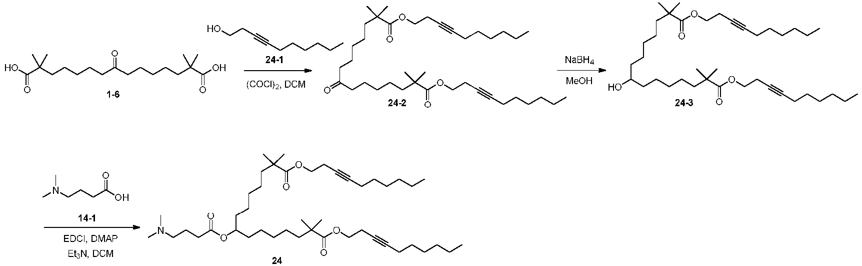

Referring to the method of Example 7, compound 24 was prepared as an oily product: 31.0 mg.

¹H NMR (400 MHz, CDCl₃): δ ppm 0.88 (t, *J* = 7.2 Hz, 6H), 1.15 (s, 12H), 1.20-1.38 (m, 24H), 1.43-1.52 (m, 12H), 1.76-1.84 (m, 2H), 2.09-2.14 (m, 4H), 2.23 (s, 6H), 2.28-2.36 (m, 4H), 2.43-2.49 (m, 4H), 4.10 (t, *J* = 7.2 Hz, 4H), 4.80-4.88 (m, 1H); ESI-MS m/z: 730.6 [M+H]⁺.

### Example 25: Synthesis of compound 25

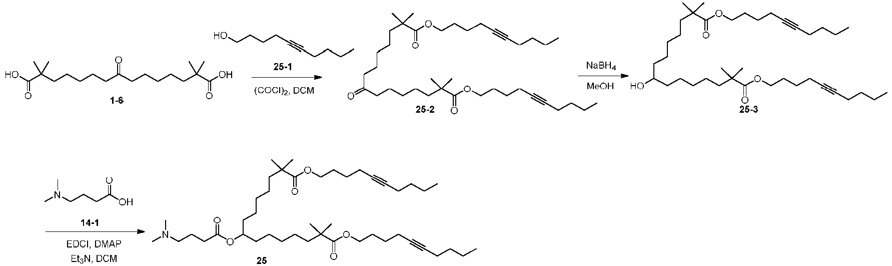

Referring to the method of Example 7, compound 25 was prepared as an oily product: 32.1 mg.

¹H NMR (400 MHz, CDCl₃): δ ppm 0.84 (t, *J* = 7.2 Hz, 6H), 1.08 (s, 12H), 1.02-1.21 (m, 12H), 1.38-1.47 (m, 22 H), 1.59-1.78 (m, 6H), 2.02-2.17 (m, 14 H), 2.19-2.30 (m, 4 H), 4.01 (t, *J* = 6.8 Hz, 4H), 4.71-4.83 (m, 1H); ESI-MS m/z: 730.6 [M+H]⁺.

### Example 26: Synthesis of compound 26

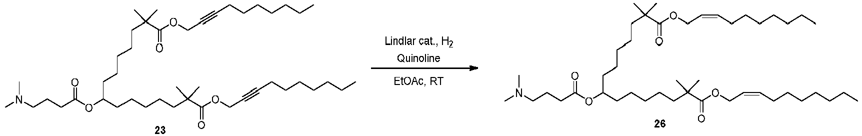

Compound 23 (300 mg, 0.41 mmol) and quinoline (106 mg, 0.82 mmol) were dissolved in 3.0 mL of ethyl acetate, and the air in the reaction system was replaced with nitrogen for 2~3 min at room temperature, then lindlar catalyst (16.9 mg) was added. Hydrogen gas was introduced to the reaction solution and the air was replaced with hydrogen for 2~3 min. The reaction system was kept under hydrogen atmosphere (15 psi) at room temperature for 30 min. The complete disappearance of the reactants was monitored by LC-MS. The reaction solution was filtered, and the filter cake was rinsed with ethyl acetate 3~4 times. The combined ethyl acetate was collected and the organic solvent was removed using a rotary-evaporator to give the crude product, which was purified by preparative high performance liquid chromatography to give compound 26 (31.3 mg).

¹H NMR (400 MHz, CDCl₃): δ ppm 0.81 (t, *J* = 7.2 Hz, 6H), 1.08 (s, 12H), 1.15-1.28 (m, 32H), 1.38-1.44 (m, 8H), 1.70-1.79 (m, 2H), 2.01 (m, 4H), 2.15 (s, 6H), 2.16-2.28 (m, 4H), 4.54 (d, *J =* 12.0 Hz, 4H), 4.75 (m, 1H), 5.39-5.59 (m, 4H); ESI-MS m/z: 734.6 [M+H]⁺.

### Example 27: Synthesis of compound 27

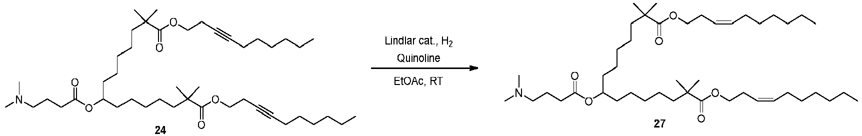

Referring to the method of Example 26, compound 27 was prepared as an oily product: 35.0 mg.

¹H NMR (400 MHz, CDCl₃): δ ppm 0.82 (m, 6H), 1.08 (s, 12H), 1.14-1.31 (m, 28H), 1.37-1.45 (m, 8H), 1.70-1.79 (m, 2H), 1.96 (m, 4H), 2.06-2.36 (m, 14H), 3.98 (t, *J=* 7.2 Hz, 4H), 4.74-4.82 (m, 1H), 5.22-5.31 (m, 2H), 5.37-5.48 (m, 2H); ESI-MS m/z: 734.7 [M+H]⁺.

### Example 28: Synthesis of compound 28

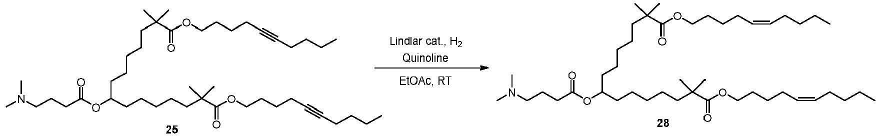

Referring to the method of Example 26, compound 28 was prepared as an oily product: 31.8 mg.

¹H NMR (400 MHz, CDCl₃): δ ppm 0.92 (t, *J* = 6.8 Hz, 6H), 1.18 (s, 12H), 1.21-1.39 (m, 22H), 1.40-1.59 (m, 12H), 1.60-1.72 (m, 4 H), 1.89-2.01 (m, 2 H), 2.02-2.15 (m, 8 H), 2.34-2.69 (m, 8 H), 4.08 (t, *J =* 6.4 Hz, 4 H), 4.82-4.92 (m, 1 H), 5.30-5.48 (m, 4 H); ESI-MS m/z: 734.6 [M+H]⁺.

### Example 30: Synthesis of compound 30

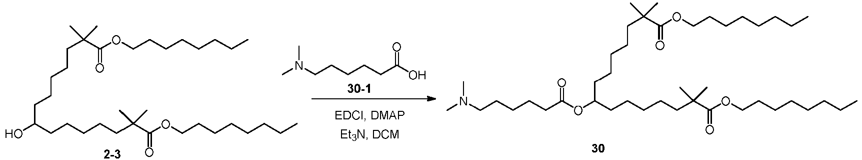

Referring to the method of Example 1, compound 30 was prepared as an oily product: 33.0 mg.

¹H NMR (400 MHz, CDCl₃): δ ppm 0.92 (t, *J* = 6.8 Hz, 6 H), 1.18 (s, 12 H), 1.19-1.37 (m, 36 H), 1.45-1.57 (m, 8 H), 1.58-1.74 (m, 8 H), 2.27-2.50 (m, 8 H), 4.07 (t, *J* = 6.8 Hz, 4 H), 4.83-4.90 (m, 1 H); ESI-MS m/z: 710.6 [M+H]⁺.

### Example 32: Synthesis of compound 32

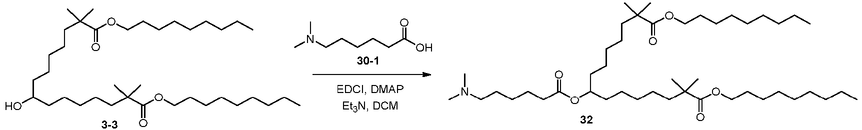

Referring to the method of Example 1, compound 32 was prepared as an oily product: 31.1 mg.

¹H NMR (400 MHz, CDCl₃): δ ppm 0.80 (t, *J* = 6.8 Hz, 6H), 1.08 (s, 12H), 1.20-1.27 (m, 34H), 1.34-1.47 (m, 12H), 1.48-1.62 (m, 8H), 2.15 (s, 6H), 2.19-2.24 (m, 4H), 3.97 (t, *J =* 6.8 Hz, 4H), 4.74-4.80 (m, 1H); ESI-MS m/z: 738.6 [M+H]⁺.

### Example 33: Synthesis of compound 33

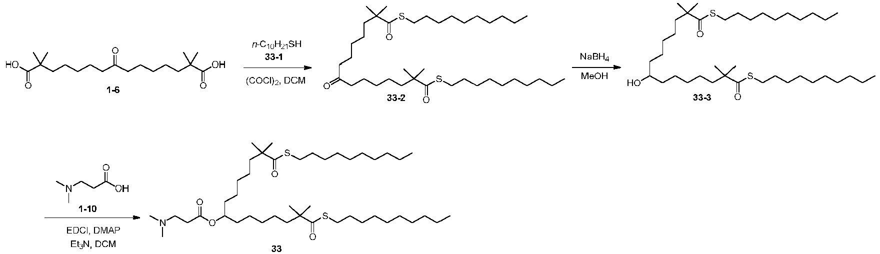

Compound 1-6 (448 mg, 1.3 mmol) was dissolved in 5.0 mL of dichloromethane, and the reaction system was cooled to 0 °C in an ice bath. DMF (10 µL, 0.13 mmol) was added, and oxalyl chloride (0.44 mL, 5.2 mmol) was then added dropwise to the reaction solution. The ice bath was removed after the dropwise addition was completed and the mixture was stirred for 1 h at room temperature. The solvent was removed using a rotary-evaporator to give acyl chloride crude product (330 mg) as an oil, which was used directly in the next reaction step.

1-Decanethiol 33-1 (455 mg, 2.61 mmol) was added to a solution of crude acyl chloride (330 mg, 0.87 mmol) in DCE (3.0 mL), and the reaction was heated to 70 °C to react overnight. The reaction solution was cooled to room temperature and the solvent was removed using a rotary-evaporator to give the crude product, which was purified by silica gel column to give compound 33-2 (400 mg). ¹H NMR (400 MHz, CDCl₃): δ ppm 0.84-0.87 (m, 6H), 1.14-1.18 (m, 12H), 1.20-1.28 (m, 36H), 1.48-1.55 (m, 12H), 2.33 (t, *J* = 7.2 Hz, 4H), 2.79 (t, *J =* 7.2 Hz, 4H).

Compound 33-2 (300 mg, 0.46 mmol) was dissolved in 3.0 mL of methanol and NaBH₄ (52.5 mg, 1.38 mmol) was added in batches. The reaction solution was stirred under nitrogen atmosphere at room temperature for 2 h. The complete disappearance of the reaction material was monitored by TLC. The reaction solution was quenched by adding saturated ammonium chloride solution, and extracted with ethyl acetate. The organic phases were combined, dried over anhydrous sodium sulfate, and filtered. The filtrate was collected and concentrated to give 300 mg of crude compound 33-3, which was directly used in the next reaction step without further purification.

Crude compound 33-3 (150 mg, 0.23 mmol) was dissolved in 3.0 mL of dichloromethane, and 1-10 (80.2 mg, 0.69 mmol), EDCI (131 mg, 0.69 mmol), triethylamine (0.1 mL, 0.69 mmol) and DMAP (28 mg, 0.23 mmol) were added to the reaction system. The reaction solution was stirred at room temperature for 12 h. The reaction solution was then quenched by adding saturated ammonium chloride solution, and extracted with dichloromethane. The organic phases were combined, dried over anhydrous sodium sulfate, and filtered. The filtrate was collected and concentrated to give the crude product, which was passed through preparative high performance liquid chromatography to give compound 33 (28.6 mg).

¹H NMR (400 MHz, CDCl₃): δ ppm 0.81 (t, *J =* 7.2 Hz, 6H), 1.15 (s, 12H), 1.31 (m, 40H), 1.48 (m, 12H), 2.23 (s, 6H), 2.42 (m, 4H), 2.80 (t, *J* = 7.2 Hz, 4H), 4.82 (m, 1H); ESI-MS m/z: 756.6 [M+H]⁺.

### Example 34: Synthesis of compound 34

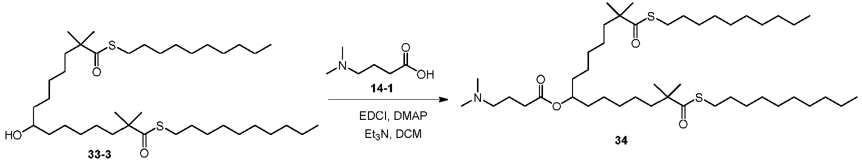

Referring to the method of Example 33, compound 34 was prepared as an oily product: 105.2 mg.

¹H NMR (400 MHz, CDCl₃): δ ppm 0.85 (t, *J* = 7.2 Hz, 6H), 1.15 (s, 12H), 1.6-1.32 (m, 40H), 1.37-1.53 (m, 14H), 1.75 (m, 2H), 2.24-2.34 (m, 8H), 2.80 (t, *J* = 7.2 Hz, 4H), 4.72-4.82 (m, 1H); ESI-MS m/z: 770.6 [M+H]⁺.

### Example 36: Synthesis of compound 36

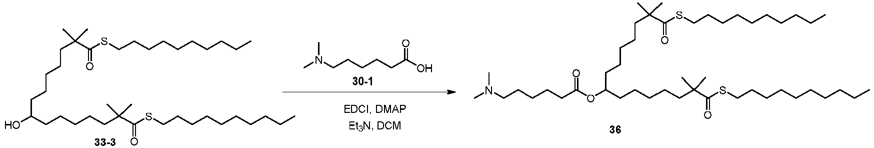

Referring to the method of Example 33, compound 36 was prepared as an oily product: 33.4 mg.

¹H NMR (400 MHz, CDCl₃): δ ppm 0.86 (t, *J* = 6.8 Hz, 6H), 1.16 (s, 12H), 1.18-1.38 (m, 40H), 1.41-1.59 (m, 16H), 1.61-1.67 (m, 2H), 2.19-2.33 (m, 10H), 2.82 (t, *J=* 7.2 Hz, 4H), 4.83 (m, H); ESI-MS m/z: 798.6 [M+H]⁺.

### Example 37: Synthesis of compound 37

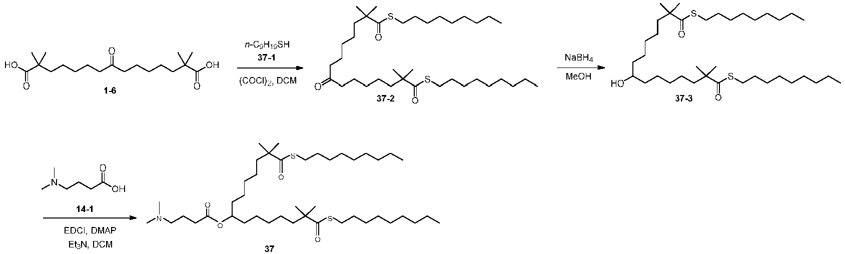

Referring to the method of Example 33, compound 37 was prepared as an oily product: 33.2 mg.

¹H NMR (400 MHz, CDCl₃): δ ppm 0.87 (t, *J* = 6.8 Hz, 6H), 1.20 (s, 12H), 1.19-1.37 (m, 36H), 1.39-1.56 (m, 12H), 1.75-1.84 (m, 2H), 2.24 (s, 6H), 2.28-2.34 (m, 4H), 2.81 (t, *J=* 7.2 Hz, 4H), 4.79-4.87 (m, 1H); ESI-MS m/z: 742.6 [M+H]⁺.

### Example 39: Synthesis of compound 39

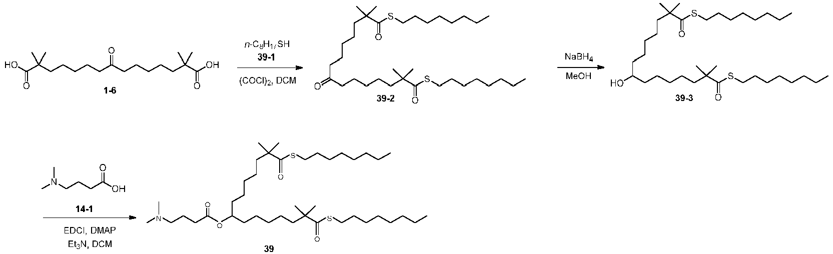

Referring to the method of Example 33, compound 39 was prepared as an oily product: 30.7 mg.

¹H NMR (400 MHz, CDCl₃): δ ppm 0.91 (t, *J* = 7.2 Hz, 6H), 1.22 (s, 12H), 1.17-1.38 (m, 32H), 1.47-1.58 (m, 12H), 1.78-1.87 (m, 2H), 2.28 (s, 6H), 2.34-2.37 (m, 4H), 2.85 (t, *J=* 7.2 Hz, 4H), 4.81-4.90 (m, 1H); ESI-MS m/z: 714.6 [M+H]⁺.

### Example 40: Synthesis of compound 40

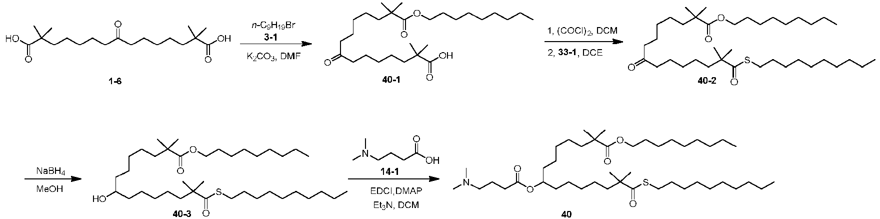

Potassium carbonate (1.55 g, 11.2 mmol, 4.0 eq.) was added to a solution of compound 1-6 (959 mg, 2.8 mmol, 1.0 eq.) and 3-1 (638 mg, 3.08 mmol, 1.1 eq.) in DMF. Then the reaction was warmed up to 60 °C for 4 h. The reaction was cooled to room temperature. The reaction system was quenched with saturated aqueous sodium chloride solution and extracted with ethyl acetate. The organic phases were combined, dried over anhydrous sodium sulfate, and filtered. The filtrate was concentrated to dryness to give the crude product, which was purified by silica gel column to give compound 40-1 (682 mg).

Compound 40-1 (324 mg, 0.69 mmol, 1.0 eq.) was dissolved in 5.0 mL of dichloromethane, and the reaction system was cooled to 0 °C in an ice bath. 2 drops of DMF were added and oxalyl chloride (0.24 mL, 2.8 mmol, 4.0 eq.) was then added dropwise to the reaction solution. The ice bath was removed after the dropwise addition was completed and the mixture was stirred for 1 h at room temperature. The solvent was removed using a rotary-evaporator to give acyl chloride crude product (309 mg) as an oil, which was used directly in the next reaction step.

1-Decanethiol 33-1 (331 mg, 1.9 mmol, 3.0 eq) was added to a solution of crude acyl chloride (309 mg) in DCE (3.0 mL), and the reaction was heated to 70 °C to react overnight. The reaction solution was cooled to room temperature and the solvent was removed using a rotary-evaporator to give the crude product, which was purified by silica gel column to give compound 40-2 (274 mg).

Then referring to the method of Example 1, compound 40 was prepared as an oily product: 34.2 mg.

¹H NMR (400 MHz, CDCl₃): δ ppm 0.81 (t, *J* = 7.2 Hz, 6H), 1.05 (s, 6H), 1.12 (s, 6H), 1.08-1.28 (m, 36H), 1.37-1.57 (m, 14H), 1.71-1.76 (m, 2H), 2.22 (s, 6H), 2.25-2.31 (m, 4H), 2.75 (t, *J =* 7.2 Hz, 2H), 3.97 (t, *J =* 7.2 Hz, 2H), 4.75-4.84 (m, 1H); ESI-MS m/z: 740.6 [M+H]⁺.

### Example 41: Synthesis of compound 41

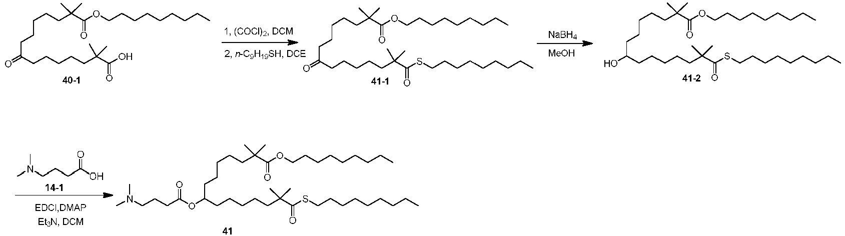

Referring to the method of Example 40, compound 41 was prepared as an oily product: 31.1 mg.

¹H NMR (400 MHz, CDCl₃): δ ppm 0.86-0.89 (m, 6H), 1.10 (s, 6H), 1.15 (s, 6H), 1.08-1.31 (m, 34H), 1.41-1.61 (m, 14H), 1.74-1.82 (m, 2H), 2.17-2.35 (m, 10H), 2.85 (t, *J=* 7.2 Hz, 2H), 4.03 (t, *J =* 7.2 Hz, 2H), 4.82-4.87 (m, 1H); ESI-MS m/z: 726.6 [M+H]⁺.

### Example 42: Synthesis of compound 42

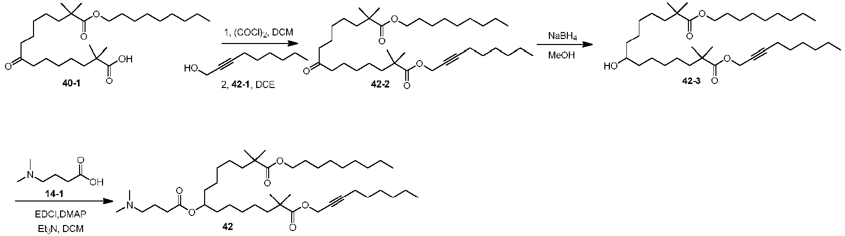

Referring to the method of Example 40, compound 42 was prepared as an oily product: 30.9 mg.

¹H NMR (400 MHz, CDCl₃): δ ppm 0.77-0.82 (m, 6H), 1.05 (s, 6H), 1.10 (s, 6H), 1.11-1.28 (m, 31H), 1.33-1.42 (m, 9H), 1.47-1.59 (m, 2H), 1.73-1.81 (m, 2H), 2.08-2.14 (m, 2H), 2.21-2.33 (m, 10H), 3.97 (t, *J =* 7.2 Hz, 2H), 4.55 (m, 2H), 4.72-4.81 (m, 1H); ESI-MS m/z: 706.6 [M+H]⁺.

### Example 43: Synthesis of compound 43

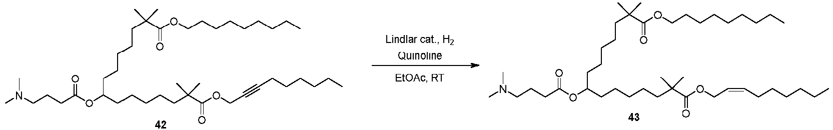

Referring to the method of Example 26, compound 43 was prepared as an oily product: 31.3 mg.

¹H NMR (400 MHz, CDCl₃): δ ppm 0.80 (m, 6H), 1.05 (s, 12H), 1.08-1.28 (m, 34H), 1.36-1.47 (m, 8H), 1.49-1.58 (m, 2H), 1.73-1.82 (m, 2H), 1.98-2.07 (m, 2H), 2.21-2.38 (m, 8H), 3.97 (t, *J* = 7.2 Hz, 2H), 4.53 (d, *J* = 7.2 Hz, 2H), 4.72-4.78 (m, 1H), 5.41-5.59 (m, 2H); ESI-MS m/z: 708.6 [M+H]⁺.

### Example 44: Synthesis of compound 44

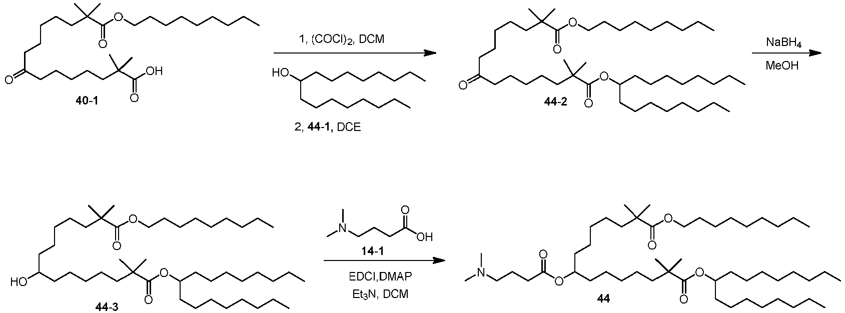

Referring to the method of Example 40, compound 44 was prepared as an oily product: 33.1 mg.

¹H NMR (400 MHz, CDCl₃): δ ppm 0.85 (m, 9H), 1.13 (s, 12H), 1.14-1.33 (m, 46H), 1.37-1.59 (m, 16H), 1.78-1.87 (m, 2H), 2.17-2.35 (m, 10H), 4.03 (t, *J* = 6.8 Hz, 2H), 4.79-4.88 (m, 2H); ESI-MS m/z: 822.8 [M+H]⁺.

### Example 45: Synthesis of compound 45

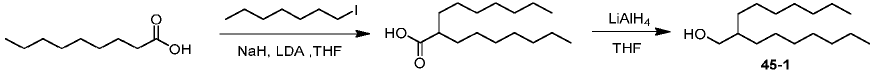

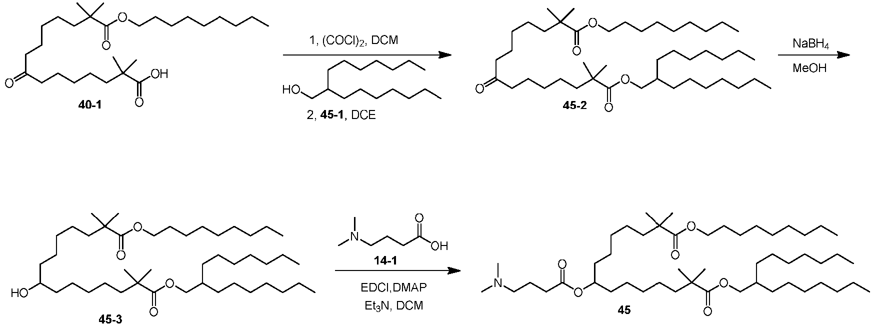

n-Nonanoic acid (3.0 g, 19 mmol) was added to 50 mL of anhydrous tetrahydrofuran and the reaction solution was cooled to 0°C in an ice bath. Sodium hydride (836 mg, 20.9 mmol) and LDA (49.4 mL, 24.7 mmol) were added to the reaction solution, and the reaction solution was stirred at 0°C for 1 hour. Then 1-iodoheptane was added dropwise to the reaction system. The ice bath was removed, then the mixture was reacted at room temperature for 12 h. The reaction solution was quenched by pouring the reaction solution into saturated ammonium chloride solution, and extracted with ethyl acetate. The organic phase was collected, dried over anhydrous sodium sulfate, and filtered. The filtrate was collected, and concentrated to remove the solvent to give the crude product, which was purified by silica gel column to give 2.0 g of compound 2-heptylnonanoic acid.

The 2-heptylnonanoic acid (2.0 g, 7.8 mmo) obtained in the previous step was dissolved in 30 mL of anhydrous tetrahydrofuran, and lithium tetrahydroaluminum (593 mg, 15.6 mmol) was added to the reaction solution. The reaction system was heated to 80°C to react for 2 hours. The reaction solution was cooled to room temperature, quenched by pouring the reaction solution into saturated ammonium chloride solution, and extracted with ethyl acetate. The organic phase was collected, dried over anhydrous sodium sulfate, and filtered. The filtrate was collected, and concentrated to remove the solvent to give the crude product, which was purified by silica gel column to give 1.3 g of compound 45-1.

Then referring to the method of Example 40, compound 45 was prepared as an oily product: 31.6 mg.

¹H NMR (400 MHz, CDCl₃): δ ppm 0.88 (t, *J* = 6.8 Hz, 9H), 1.14 (s, 12H), 1.15-1.26 (m, 47H), 1.47-1.50 (m, 8H), 1.57-1.62 (m, 4H), 1.79-1.81 (m, 2H), 2.25 (s, 6H), 2.32 (t, *J =* 7.2 Hz, 4H), 3.93 (d, *J* = 5.6 Hz, 2H), 4.03 (t, *J* = 7.2 Hz, 2H), 4.81-4.87 (m, 1H); ESI-MS m/z: 808.7 [M+H]⁺.

### Example 46: Synthesis of compound 46

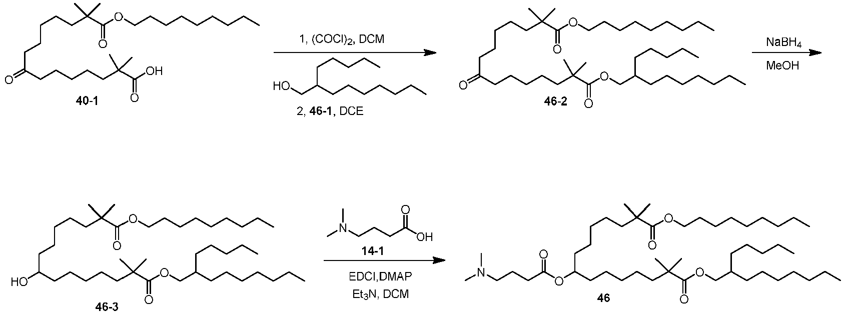

Referring to the method of Example 40, compound 46 was prepared as an oily product: 32.6 mg.

¹H NMR (400 MHz, CDCl₃): δ ppm 0.88 (t, *J* = 7.2 Hz, 9H), 1.14 (s, 12H), 1.15-1.28 (m, 37H), 1.47-1.59 (m, 18H), 1.75-1.84 (m, 2H), 2.24-2.35 (m, 10H), 3.95 (d, *J=* 5.6 Hz, 2H), 4.03 (t, *J =* 6.8 Hz, 2H), 4.80-4.87 (m, 1H); ESI-MS m/z: 780.7 [M+H]⁺.

### Example 47: Synthesis of compound 47

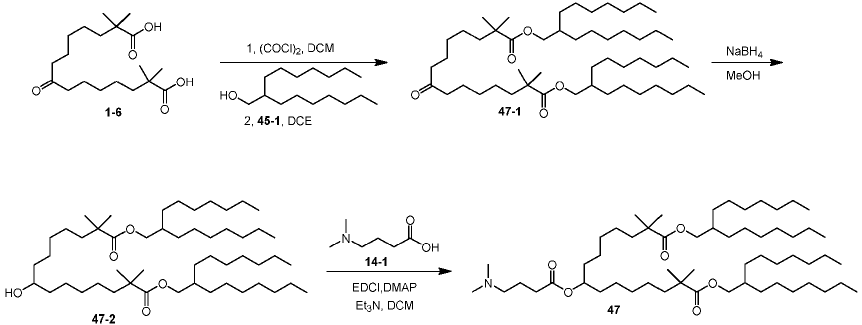

Referring to the method of Example 7, compound 47 was prepared as an oily product: 33.1 mg.

¹H NMR (400 MHz, CDCl₃): δ ppm 0.81 (t, *J* = 6.8 Hz, 12H), 1.08 (s, 12H), 1.09-1.24 (m, 56H), 1.40-1.61 (m, 14H), 1.67-1.72 (m, 2H), 2.17 (s, 6H), 2.19-2.28 (m, 4H), 3.88 (d, *J =* 5.6 Hz, 4H), 4.74-4.80 (m, 1H); ESI-MS m/z: 906.8 [M+H]⁺.

### Example 48: Synthesis of compound 48

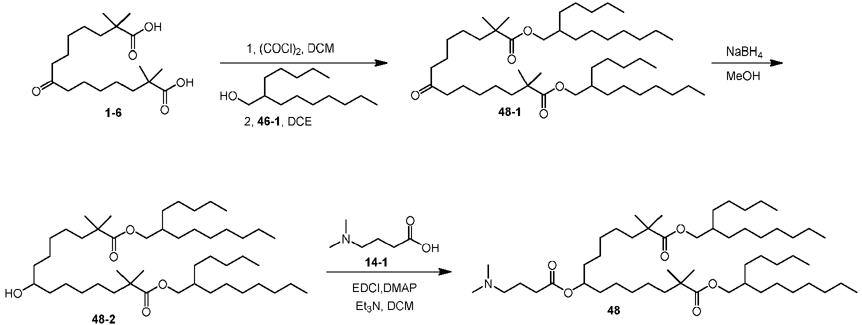

Referring to the method of Example 7, compound 48 was prepared as an oily product: 34.8 mg.

¹H NMR (400 MHz, CDCl₃): δ ppm 0.81 (t, *J* = 7.2 Hz, 12H), 1.08 (s, 12H), 1.09-1.23(m, 48H), 1.37-1.64 (m, 14H), 1.67-1.73 (m, 2H), 2.15 (s, 6H), 2.20-2.37 (m, 4H), 3.88 (d, *J =* 5.6 Hz, 4H), 4.74-4.89 (m, 1H); ESI-MS m/z: 850.8 [M+H]⁺.

The compounds of Table 2 were synthesized using the methods of the above examples, or similar methods using the corresponding intermediates.

**Table 2**

| | |
|---|---|
| 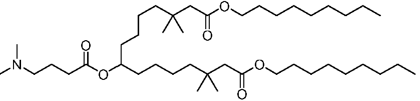 Example 49: compound 49 | 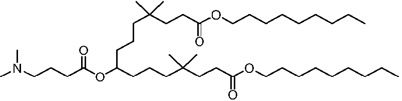 Example 50: compound 50 |
| [M+H]⁺: 710.6 | [M+H]⁺: 710.6 |
| 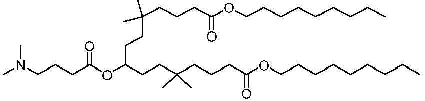 Example 51: compound 51 | 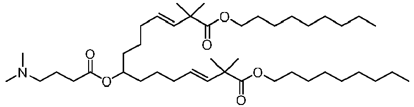 Example 52: compound 52 |
| [M+H]⁺: 710.6 | [M+H]⁺: 706.6 |
| 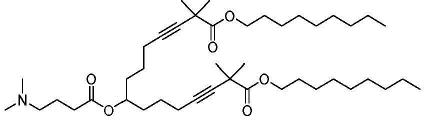 Example 53: compound 53 | 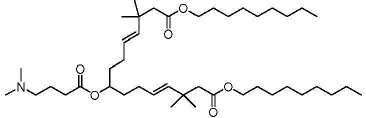 Example 54: compound 54 |
| [M+H]⁺: 702.6 | [M+H]⁺: 706.6 |
| 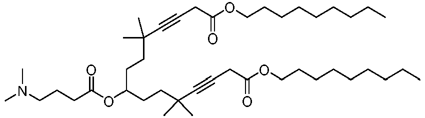 Example 55: compound 55 | 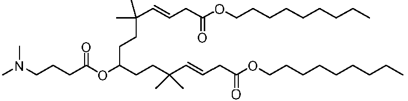 Example 56: compound 56 |
| [M+H]⁺: 702.6 | [M+H]⁺: 706.6 |
| 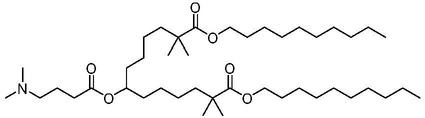 Example 57: compound 57 | 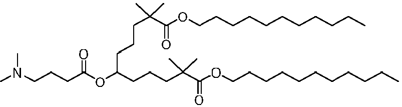 Example 58: compound 58 710.6 |
| [M+H]⁺: 710.6 | [M+H]⁺: |
| 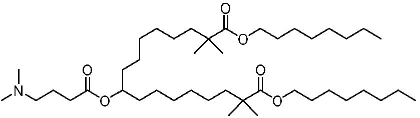 Example 59: compound 59 | 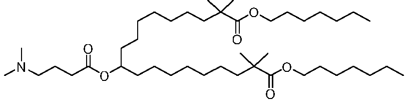 Example 60: compound 60 |
| [M+H]⁺: 710.6 | [M+H]⁺: 710.6 |
| 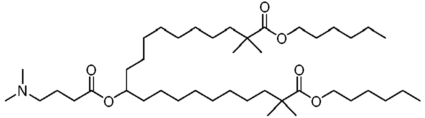 Example 61: compound 61 | 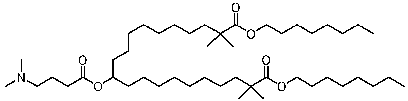 Example 62: compound 62 |
| [M+H]⁺: 710.6 | [M+H]⁺: 766.7 |
| 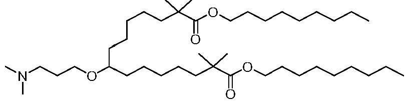 Example 63: compound 63 | 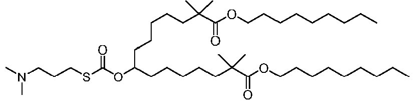 Example 64: compound 64 |
| [M+H]⁺: 682.6 | [M+H]⁺: 742.6 |
| 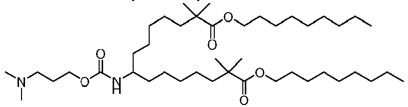 Example 65: compound 65 | 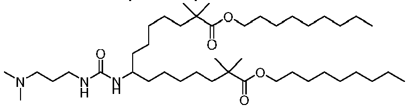 Example 66: compound 66 |
| [M+H]⁺: 725.6 | [M+H]⁺: 724.6 |
| 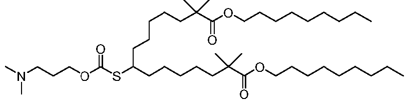 Example 67: compound 67 | 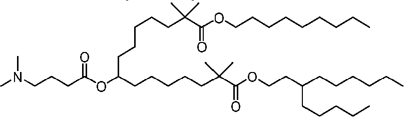 Example 68: compound 68 |
| [M+H]⁺: 742.6 | [M+H]⁺: 780.7 |
| 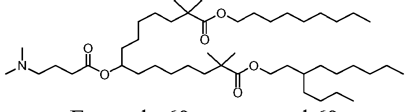 Example 69: compound 69 | 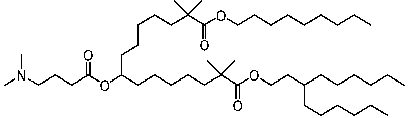 Example 70: compound 70 |
| [M+H]⁺: 766.7 | [M+H]⁺: 794.7 |
| 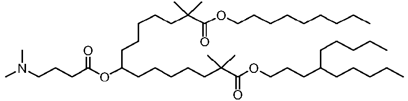 Example 71: compound 71 | 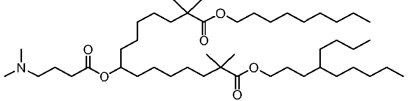 Example 72: compound 72 |
| [M+H]⁺: 780.7 | [M+H]⁺: 766.7 |
| 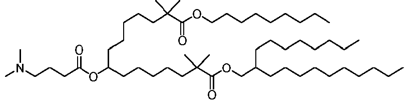 Example 73: compound 73 | 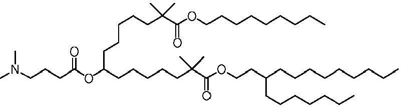 Example 74: compound 74 |
| [M+H]⁺: 864.8 | [M+H]⁺: 850.8 |
| 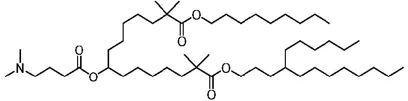 Example 75: compound 75 | |
| [M+H]⁺: 836.8 | |
| 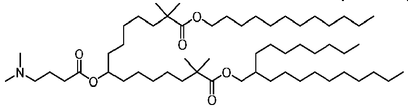 Example 77: compound 77 | 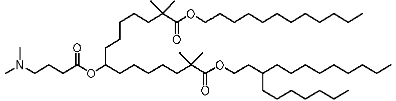 Example 78: compound 78 |
| [M+H]⁺: 906.8 | [M+H]⁺: 892.8 |
| 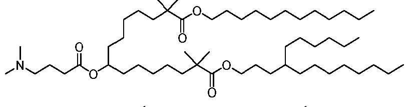 Example 79: compound 79 | 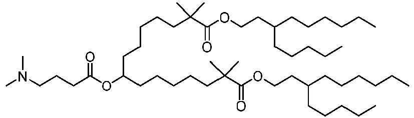 Example 80: compound 80 |
| [M+H]⁺: 878.8 | [M+H]⁺: 850.8 |
| 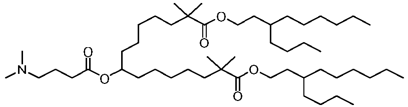 Example 81: compound 81 | 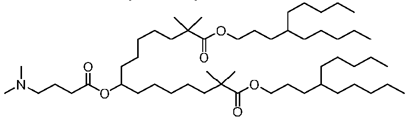 Example 82: compound 82 |
| [M+H]⁺: 822.8 | [M+H]⁺: 850.8 |
| 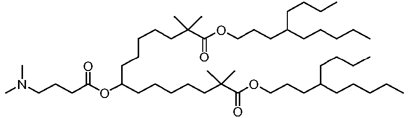 Example 83: compound 83 | 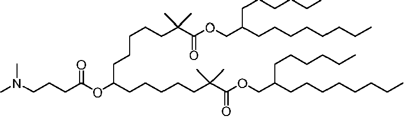 Example 84: compound 84 |
| [M+H]⁺: 822.8 | [M+H]⁺: 906.8 |
| 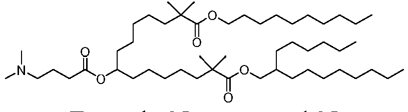 Example 85: compound 85 | 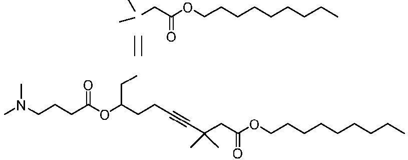 Example 86: compound 86 |
| [M+H]⁺: 822.8 | [M+H]⁺: 702.6 |
| 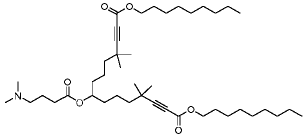 Example 87: compound 87 | 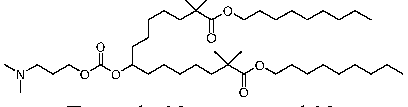 Example 88: compound 88 |
| [M+H]⁺: 702.6 | [M+H]⁺: 726.6 |
| 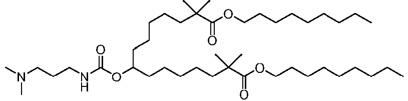 Example 89: compound 89 | |
| [M+H]⁺: 725.6 | |

### Example 90: Synthesis of compound 90

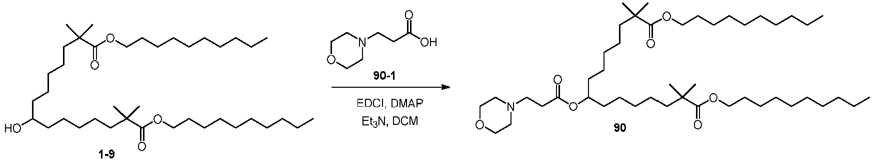

Referring to the method of Example 1, compound 90 was prepared as an oily product: 40.5 mg.

¹H NMR (400 MHz, CDCl₃): δ ppm 0.81 (t, *J* = 6.8 Hz, 6H), 1.08 (s, 12H), 1.10-1.28 (m, 36H), 1.38-1.47 (m, 12H), 1.50-1.58 (m, 4H), 2.40 (m, 6H), 2.58 (t, *J=* 6.8 Hz, 2H), 3.59-3.65 (m, 4H), 3.97 (t, *J =* 6.8 Hz, 4H), 4.75-4.83 (m, 1H); ESI-MS m/z: 766.7 [M+H]⁺.

### Example 91: Synthesis of compound 91

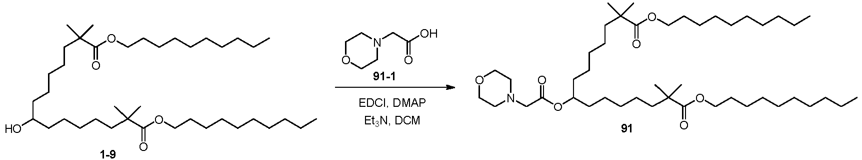

Referring to the method of Example 1, compound 91 was prepared as an oily product: 32.2 mg.

¹H NMR (400 MHz, CDCl₃): δ ppm 0.88 (t, *J* = 6.8 Hz, 6H), 1.15 (s, 12H), 1.16-1.38 (m, 40H), 1.46 (m, 8H), 1.60 (m, 4H), 2.59 (m, 4H), 3.19 (s, 2H), 3.76 (t, *J* = 4.8 Hz, 4H), 4.04 (t, *J =* 6.8 Hz, 4H), 4.91 (m, 1H); ESI-MS m/z: 752.7 [M+H]⁺.

### Example 92: Synthesis of compound 92

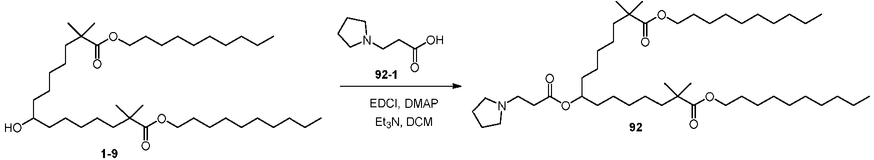

Referring to the method of Example 1, compound 92 was prepared as an oily product: 32 mg.

¹H NMR (400 MHz, CDCl₃): δ ppm 0.87 (t, *J* = 6.8 Hz, 6H), 1.13 (s, 12H), 1.28 (m, 42H), 1.46 (m, 8H), 1.45 (m, 4H), 1.76 (m, 4H), 2.50 (m, 4H), 2.76 (m, 2H), 4.01 (t, *J =* 6.8 Hz, 4H), 4.84 **(m, 1H);** ESI-MS m/z: 750.9 [M+H]⁺.

### Example 93: Synthesis of compound 93

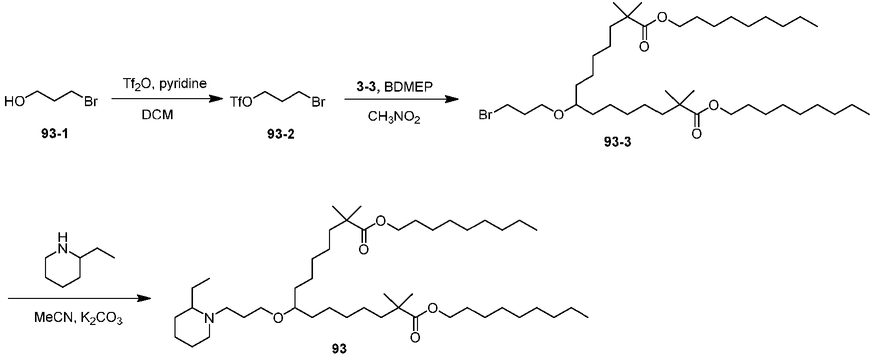

3-Bromopropanol (20 g, 144 mmol), trifluoromethanesulfonic anhydride (26.6 mL, 158 mmol) and pyridine (14.0 mL, 173 mmol) were added to a round bottom flask containing 500 mL of dichloromethane. The mixture was stirred at room temperature until the reaction materials were completely consumed by TLC monitoring. The reaction solution was quenched with 1 M hydrochloric acid solution, and extracted with dichloromethane. The organic phase were combined, dried over anhydrous sodium sulfate, and filtered to remove the sodium sulfate. The filtrate was collected. The solvent was removed using a rotary-evaporator to give 25 g of crude compound 93-2, which was used directly for subsequent reactions without further purification.

3-3 (6.0 g, 10 mmol) and crude compound 93-2 (3.0 g, 11 mmol) were added to a round bottom flask containing 50 mL of nitromethane, then 2,6-di-tert-butylpyridine (3.37 mL, 15 mmol) was added to the reaction solution. The reaction solution was warmed up to 95°C to react overnight. The reaction solution was cooled to room temperature. The solvent was removed using a rotary-evaporator to give the crude product. The crude product was then dissolved in dichloromethane, extracted after adding saturated aqueous ammonium chloride. The organic phase were collected and combined, dried over anhydrous sodium sulfate, and filtered to remove the sodium sulfate. The filtrate was collected. The solvent was removed using a rotary-evaporator and then purified by silica gel column to give compound 93-3 (2.3 g).

Compound 93-3 (251 mg, 0.35 mmol) and 2-ethylpiperidine (71 µL, 0.53 mmol) were dissolved in 3.0 mL of anhydrous acetonitrile and anhydrous potassium carbonate (73 mg, 0.53 mmol) was added to the reaction solution. The mixture was warmed up to 80 °C to react for 6 hours. The reaction solution was cooled to room temperature, quenched by adding saturated aqueous ammonium chloride, and extracted with dichloromethane. The organic phase were collected and combined, dried over anhydrous sodium sulfate, and filtered to remove the sodium sulfate. The filtrate was collected. The solvent was removed using a rotary-evaporator and then purified by preparative high performance liquid chromatography to give compound 93 (82 mg).

¹H NMR (400 MHz, CDCl₃): δ ppm 0.72-0.91 (m, 9H), 1.08 (s, 12H), 1.11-1.75 (m, 60H), 1.87-2.25 (m, 3H), 2.57-2.93 (m, 4H), 3.04-3.15 (m, 2H), 3.32-3.45 (m, 2H), 3.98 (d, *J* = 6.8 Hz, 4H); ESI-MS m/z: 750.6 [M+H]⁺.

### Example 94: Synthesis of compound 94

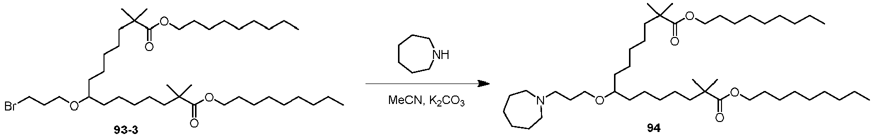

Referring to the method of Example 93, compound 94 was prepared as an oily product: 79.2 mg.

¹H NMR (400 MHz, CDCl₃): δ ppm 0.82 (t, *J* = 7.2 Hz, 6H), 1.09 (s, 12H), 1.11-1.34 (m, 44H), 1.52 (m, 14H), 2.45-2.74 (m, 6H), 3.07 (m, 1H), 3.38 (m, 2H), 3.94 (t, *J* = 6.8 Hz, 4H); ESI-MS m/z: 736.6 [M+H]⁺.

The compounds of Table 3 were synthesized using the methods of the above examples, or similar methods using the corresponding intermediates.

**Table 3**

| | |
|---|---|
| 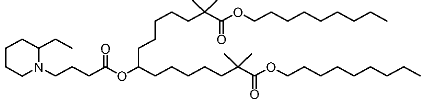 Example 95: compound 95 | 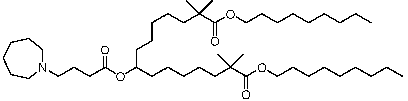 Example 96: compound 96 |
| [M+H]⁺: 778.7 | [M+H]⁺: 764.7 |

### Example 97: Synthesis of compound 97

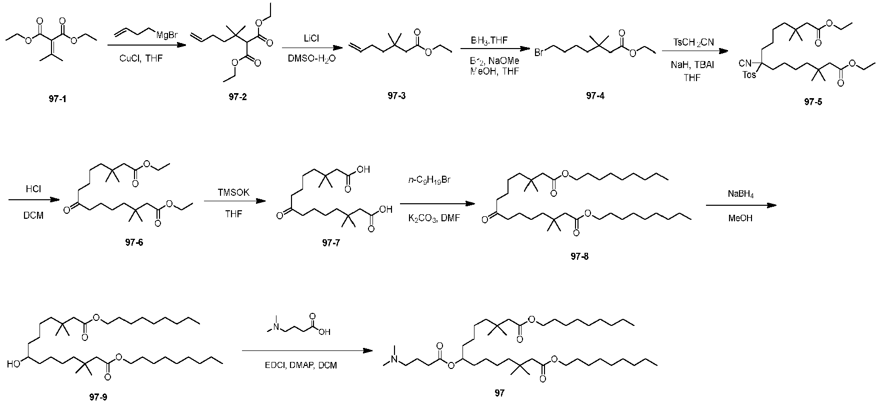

To a round bottom flask were added CuCl (989 mg, 9.99 mmol) and 160 mL THF, and the reaction system was cooled to -30°C. Then 3-butenylmagnesium bromide (1 M, 299 mL) was added. 160 mL of solution of compound 97-1 (40.0 g, 199 mmol) in tetrahydrofuran was slowly added to the reaction system. After the dropwise addition was completed, the reaction system was warmed up to room temperature and stirred to react for another 2 hours. After the reaction material 97-1 was reacted completely by TLC monitoring, the reaction solution was quenched with 300 mL of saturated aqueous ammonium chloride, and extracted with ethyl acetate. The organic phases were combined, dried over anhydrous sodium sulfate, and filtered. The filtrate was concentrated to dryness to give the crude product. The crude was purified by silica gel column to give compound 97-2 (45.0 g).

Compound 97-2 (42.0 g, 164 mmol) was dissolved in 400 mL of DMSO, and 4 mL of water and LiCl (27.8 g, 655 mmol) were added to the reaction solution. Then the reaction system was heated to 180 °C and stirred until the reactant 97-2 was reacted completely by TLC monitoring. The reaction system was cooled to room temperature, then poured into water and extracted with ethyl acetate. The organic phases were combined, dried over anhydrous sodium sulfate, and filtered. The filtrate was concentrated to dryness to give the crude product 97-3 (31.0 g), which was used directly in the next reaction without further purification.

Crude product 97-3 (30.0 g, 163 mmol) was dissolved in 240 mL of tetrahydrofuran and BH₃·THF (1 M, 244 mL) was added dropwise to the reaction solution in an ice bath. Then the mixture was warmed up to room temperature and stirred for 2 h. The reaction system was then cooled to 0 °C in an ice bath and methanol (13.2 mL, 325 mmol), Br₂ (8.39 mL, 163 mmol) and sodium methoxide (43.9 g, 244 mmol) were added sequentially. The mixture was warmed up to room temperature and stirred for another 1h. The reaction solution was quenched with cold saturated aqueous ammonium chloride solution and extracted with ethyl acetate. The organic phases were combined, dried over anhydrous sodium sulfate, and filtered. The filtrate was concentrated to dryness to give the crude product, which was purified by silica gel column to give compound 97-4 (14.0 g).

Then referring to the method of Example 1, compound 97 was prepared as an oily product: 31.6 mg.

¹H NMR (400 MHz, CDCl₃): δ ppm 0.84-0.90 (m, 6H), 0.93-1.01 (m, 12H), 1.20-1.31 (m, 32H), 1.45-1.62 (m, 16H), 2.17 (s, 4H), 2.19 - 2.44 (m, 8H), 3.99-4.08 (m, 4H), 4.81-4.91 (m, 1H); ESI-MS m/z: 710.7 [M+H]⁺.

### Example 98: Synthesis of compound 98

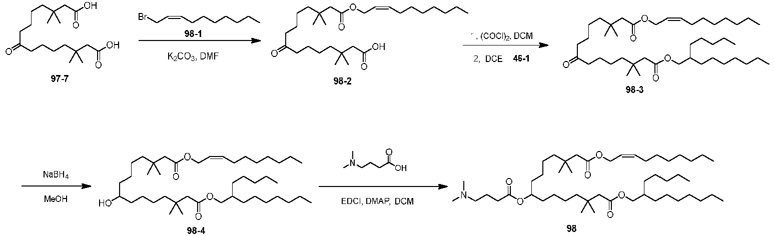

Referring to the method of Example 97, compound 98 was prepared as an oily product: 31.0 mg.

¹H NMR (400 MHz, CDCl₃): δ ppm 0.88 (t, *J* = 6.8 Hz, 9H), 0.97 (s, 12H), 1.25-1.39 (m, 38H), 1.45-1.59 (m, 10H), 1.79 (m, 2H), 2.06-2.13 (m, 2H), 2.18 (m, 4H), 2.20-2.39 (m, 9H), 3.94 (d, *J =* 5.6 Hz, 2H), 4.59 (d, *J =* 6.8 Hz, 2H), 4.82-4.87 (m, 1H), 5.48-5.53 (m, 1H), 5.60-5.64 (m, 1H); ESI-MS m/z: 792.7 [M+H]⁺.

### Example 99: Synthesis of compound 99

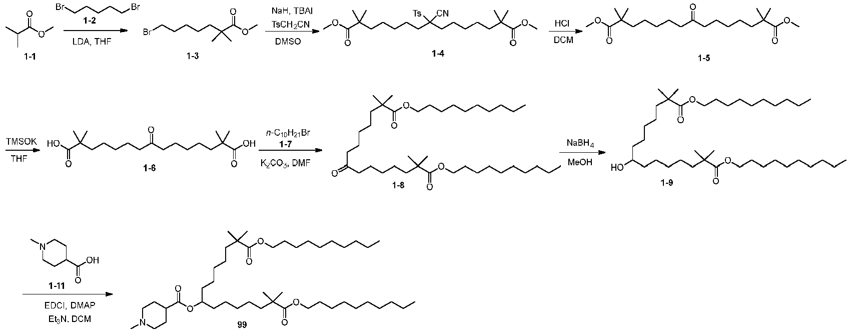

A solution of compound 1-1 (100 g, 979 mmol) in tetrahydrofuran (800 mL) was cooled to -40°C. LDA (2 M, 490 mL) was added slowly dropwise to the solution and the mixture was stirred for another 1 h after completion of the dropwise addition. A solution of 1-2 (315 g, 1.37 mol) in tetrahydrofuran (100 mL) was added dropwise to the reaction system at the same temperature and the reaction system was stirred overnight. The reaction system was quenched with saturated aqueous ammonium chloride, and extracted with ethyl acetate. The organic phases were combined, dried over anhydrous sodium sulfate, and filtered. The filtrate was concentrated to dryness to give the crude product. The crude product was purified by silica gel column to give compound 1-3 (115 g). ¹H NMR (400 MHz, CDCl₃): δ ppm 1.06-1.11 (m, 6 H), 1.13-1.22 (m, 2 H), 1.29-1.39 (m, 2 H), 1.42-1.49 (m, 2 H), 1.73-1.82 (m, 2 H), 3.28-3.40 (m, 2 H), 3.55-3.66 (m, 3 H).

A solution of compound 1-3 (100 g, 398 mmol), TsCH₂CN (38.9 g, 199 mmol) and TBAI (14.7 g, 39.8 mmol) in dimethyl sulfoxide (800 mL) was cooled to 0 °C, and sodium hydride (20.7 g, 517 mmol, 60% purity) was added slowly in batches. The mixture was reacted at room temperature overnight. The reaction system was quenched with saturated aqueous sodium chloride solution and extracted with ethyl acetate. The organic phases were combined, dried over anhydrous sodium sulfate, and filtered. The filtrate was concentrated to dryness to give 115 g of crude compound 1-4, which was used directly in the next reaction without isolation and purification.

To a solution of compound 1-4 crude (110 g, 205 mmol) in dichloromethane (880 mL) was added 330 mL of concentrated hydrochloric acid, and the mixture was reacted at room temperature for 2 h. The complete reaction of the substrate was monitored by TLC. The reaction system was quenched with saturated aqueous ammonium chloride solution and extracted with ethyl acetate. The organic phases were combined, dried over anhydrous sodium sulfate, and filtered. The filtrate was concentrated to dryness to give the crude product. The crude product was purified by silica gel column to give compound 1-5 (30.0 g, 80.9 mmol, 39.4%).

TMSOK (11.0 g, 86.4 mmol) was added to a solution of compound 1-5 (8.0 g, 21.6 mmol) in tetrahydrofuran (35.0 mL) at room temperature, and the reaction system was heated to 70°C with stirring. The complete consumption of reaction materials was monitored by TLC. The reaction solution was cooled to room temperature, and the organic solvent was removed by rotary evaporation. The crude product was added to 20 mL of water and extracted with dichloromethane. The aqueous layer was collected, and the solution was adjusted to a pH of <5 with 1 M hydrochloric acid. The solution was extracted with dichloromethane. The organic phases were combined, dried over anhydrous sodium sulfate, and filtered. The filtrate was collected and concentrated to give compound 1-6 (7.0 g). ¹H NMR (400 MHz, CDCl₃): δ ppm 1.03 (s, 12H), 1.08-1.17 (m, 8H), 1.34-1.45 (m, 8H), 2.21 (t, *J =* 7.2 Hz, 4H).

Potassium carbonate (482 mg, 3.48 mmol) was added to a solution of compound 1-6 (294 mg, 0.87 mmol) and 1-7 (771 mg, 3.48 mmol) in DMF, then the reaction was warmed up to 60 °C for 6 h. The complete disappearance of reactant 1-6 was monitored. The mixture was cooled to room temperature. The reaction system was quenched with saturated aqueous sodium chloride solution and extracted with ethyl acetate. The organic phases were combined, dried over anhydrous sodium sulfate, and filtered. The filtrate was concentrated to dryness to give the crude product. The crude was purified by silica gel column to give compound 1-8 (325 mg).

Compound 1-8 (325 mg) was dissolved in 4.0 mL of methanol and sodium borohydride (30 mg, 0.84 mmol) was added to the reaction system. The mixture was reacted at room temperature. The complete disappearance of the reactants was monitored by TLC. The reaction system was quenched with saturated aqueous sodium chloride solution and extracted with dichloromethane. The organic phases were combined, dried over anhydrous sodium sulfate, and filtered. The filtrate was concentrated to dryness to give crude compound 1-9 (260 mg), which was used directly in the next reaction without purification.

Crude compound 1-9 (250 mg, 0.40 mmol), 1-11 (35.9 mg, 0.60 mmol), EDCI (230 mg, 1.20 mmol), triethylamine (0.17 mL, 1.20 mmol) and DMAP (49 mg, 0.40 mmol) were dissolved in 5.0 mL of dichloromethane, and the reaction solution was stirred to react at room temperature for 12 h. The reaction solution was quenched with saturated aqueous sodium chloride and extracted with dichloromethane. The organic phases were combined, dried over anhydrous sodium sulfate, and filtered. The organic phase was collected and the organic solvent was removed using a rotary-evaporator to give the crude product, which was purified by preparative high performance liquid chromatography to give compound 99 (31.6 mg)

¹H NMR (400 MHz, CDCl₃): δ ppm 0.86 (t, *J* = 6.8 Hz, 6H), 1.13 (s, 12H), 1.25 (m, 43H), 1.46 (m, 8H), 1.57 (m, 4H), 1.84 (m, 4H), 2.33 (s, 3H), 2.86 (m, 2H), 4.01 (m, 4H), 4.81 (m, 1H); ESI-MS m/z: 751.0 [M+H]⁺.

### Example 100: Synthesis of compound 100

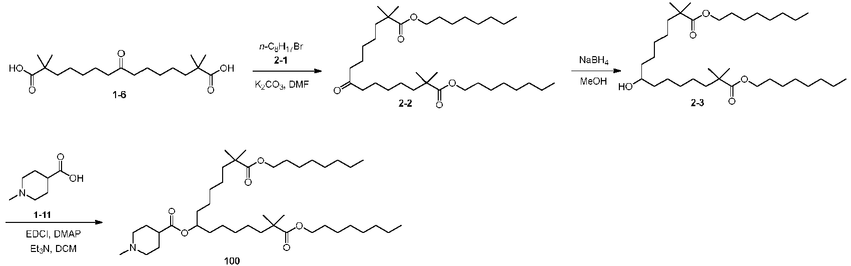

Referring to the method of Example 99, compound 100 was prepared as an oily product: 33.5 mg.

¹H NMR (400 MHz, CDCl₃): δ ppm 0.81 (t, *J* = 6.8 Hz, 6 H), 1.08 (s, 12 H), 1.11-1.31 (m, 30 H), 1.41 (m, 9 H), 1.54 (m, 5 H), 1.65-1.77 (m, 2 H), 1.78-1.98 (m, 4H), 2.20 (m, 4H), 2.74 (m, 2H), 3.97 (t, *J =* 6.8 Hz, 4 H), 4.71-4.85 (m, 1 H); ESI-MS m/z: 694.6 [M+H]⁺.

### Example 101: Synthesis of compound 101

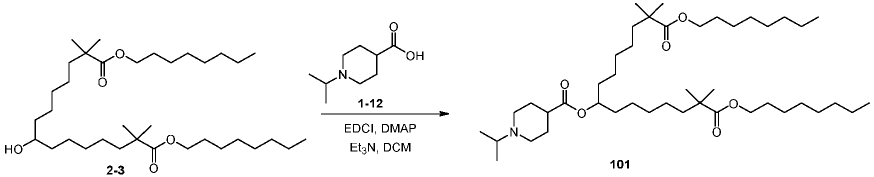

Referring to the method of Example 99, compound 101 was prepared as an oily product: 30.8 mg.

¹H NMR (400 MHz, CDCl₃): δ ppm 0.81 (t, *J* = 6.8 Hz, 6 H), 0.96 (d, *J =* 6.8 Hz, 6H), 1.08 (s, 12 H), 1.11-1.31 (m, 32 H), 1.35-1.46 (m, 8 H), 1.54 (m, 4 H), 1.59-1.74 (m, 4 H), 2.01-2.13 (m, 3H), 2.62 (m, 1H), 2.77 (m, 2H), 3.97 (t, *J* = 6.8 Hz, 4 H), 4.71-4.83 (m, 1 H); ESI-MS m/z: 722.6 [M+H]⁺.

### Example 102: Synthesis of compound 102

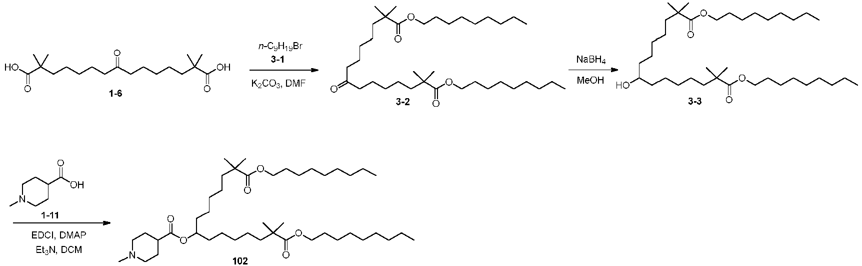

Referring to the method of Example 99, compound 102 was prepared as an oily product: 32.4 mg.

¹H NMR (400 MHz, CDCl₃): δ ppm 0.90 (t, *J* = 6.8 Hz, 6H), 1.17 (s, 12H), 1.20-1.41 (m, 34H), 1.44-1.55 (m, 8H), 1.57-1.69 (m, 5H), 1.73-1.86 (m, 3H), 1.92 (m, 2H), 2.02 (m, 2H), 2.21-2.33 (m, 4H), 2.82-2.85 (m, 2H), 4.06 (t, *J =* 6.8 Hz, 4H), 4.84-4.90 (m, 1H); ESI-MS m/z: 722.6 [M+H]⁺.

### Example 103: Synthesis of compound 103

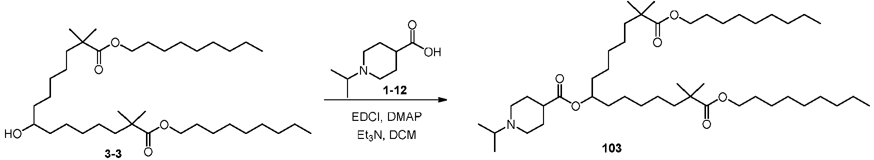

Referring to the method of Example 99, compound 103 was prepared as an oily product: 32.8 mg.

¹H NMR (400 MHz, CDCl₃): δ ppm 0.81 (t, *J =* 6.8 Hz, 6H), 0.96 (d, *J =* 6.8 Hz, 6H), 1.08 (s, 12H), 1.11-1.33 (m, 34H), 1.34-1.47 (m, 8H), 1.54 (m, 5H), 1.60-1.75 (m, 3H), 1.83 (m, 2H), 2.03-2.24 (m, 3H), 2.56-2.79 (m, 3H), 3.97 (t, *J* = 6.8 Hz, 4H), 4.74-4.81 (m, 1H); ESI-MS m/z: 750.6 [M+H]⁺.

### Example 104: Synthesis of compound 104

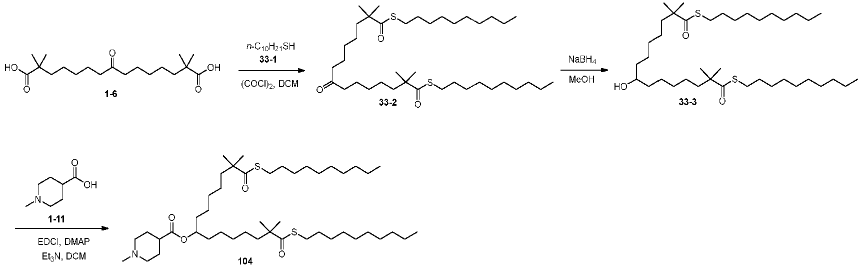

Compound **1-6** (448 mg, 1.3 mmol) was dissolved in 5.0 mL of dichloromethane, and the reaction system was cooled to 0 °C in an ice bath. DMF (10 µL, 0.13 mmol) was added and oxalyl chloride (0.44 mL, 5.2 mmol) was then added dropwise to the reaction solution. The ice bath was removed after the dropwise addition was completed and the mixture was stirred for 1 h at room temperature. The solvent was removed using a rotary-evaporator to give acyl chloride crude product (330 mg) as an oil, which was used directly in the next reaction step.

1-Decanethiol **33-1** (455 mg, 2.61 mmol) was added to a solution of crude acyl chloride (330 mg, 0.87 mmol) in DCE (3.0 mL), and the reaction was heated to 70 °C to react overnight. The reaction solution was cooled to room temperature and the solvent was removed using a rotary-evaporator to give the crude product, which was purified by silica gel column to give compound **33-2** (400 mg). ¹H NMR (400 MHz, CDCl₃): δ ppm 0.84-0.87 (m, 6H), 1.14-1.18 (m, 12H), 1.20-1.28 (m, 36H), 1.48-1.55 (m, 12H), 2.33 (t, *J* = 7.2 Hz, 4H), 2.79 (t, *J =* 7.2 Hz, 4H).

Compound **33-2** (300 mg, 0.46 mmol) was dissolved in 3.0 mL of methanol and NaBH₄ (52.5 mg, 1.38 mmol) was added in batches. The reaction solution was stirred under nitrogen atmosphere at room temperature for 2 h. The complete disappearance of the reaction material was monitored by TLC. The reaction solution was quenched by adding saturated ammonium chloride solution, and extracted with ethyl acetate. The organic phases were combined, dried over anhydrous sodium sulfate, and filtered. The filtrate was collected and concentrated to give 300 mg of crude compound **33-3,** which was directly used in the next reaction step without further purification.

Crude compound **33-3** (300 mg, 0.46 mmol), **1-11** (98.8 mg, 0.69 mmol), EDCI (264.5 mg, 1.38 mmol), triethylamine (0.19 mL, 1.38 mmol) and DMAP (56.2 mg, 0.46 mmol) were dissolved in 8.0 mL of dichloromethane, and the reaction solution was stirred at room temperature until the reaction material **33-3** was completely consumed. The reaction solution was quenched with saturated aqueous sodium chloride and extracted with dichloromethane. The organic phases were combined, dried over anhydrous sodium sulfate, and filtered. The organic phase was collected, and the organic solvent was removed using a rotary-evaporator. The crude product was purified by preparative high performance liquid chromatography to give the compound **104** (67.3 mg).

¹H NMR (400 MHz, CDCl₃): δ ppm 0.81 (t, *J* = 6.8 Hz, 6H), 1.08 (s, 12H), 1.09-1.31 (m, 42H), 1.35-1.51 (m, 14H), 1.61-2.25 (m, 8H), 2.73 (t, *J =* 7.2 Hz, 4H), 4.77 (m, 1H); ESI-MS m/z: 782.7 [M+H]⁺.

### Example 105: Synthesis of compound 105

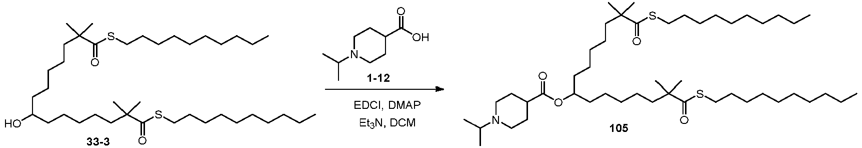

Referring to the method of Example 104, compound **105** was prepared as an oily product: 27.1 mg.

¹H NMR (400 MHz, CDCl₃): δ ppm 0.85-0.89 (m, 6H), 1.02 (br d, *J* = 6.4 Hz, 6H), 1.18 (s, 12H), 1.20-1.40 (m, 40H), 1.42-1.59 (m, 12H), 1.64-1.83 (m, 3H), 1.87-1.93 (m, 2H), 2.11 - 2.23 (m, 3H), 2.66-2.94 (m, 6H), 4.72-4.94 (m, 1H); ESI-MS m/z: 810.6 [M+H]⁺.

### Example 106: Synthesis of compound 106

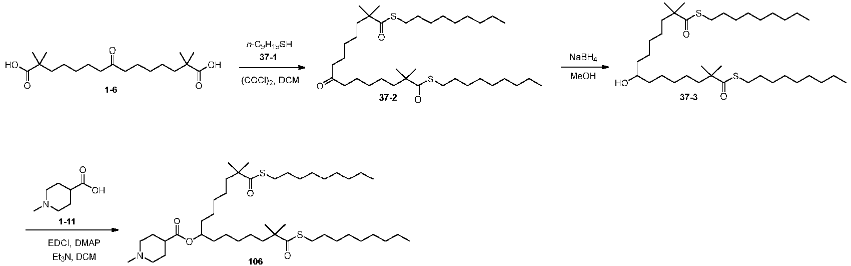

Referring to the method of Example 104, compound **106** was prepared as an oily product: 38.4 mg.

¹H NMR (400 MHz, CDCl₃): δ ppm 0.88 (t, *J* = 7.2 Hz, 6H), 1.17 (s, 12H), 1.15-1.34 (m, 36H), 1.43-1.57 (m, 15H), 1.69-2.09 (m, 5H), 2.27-2.34 (m, 3H), 2.77-2.86 (m, 5H), 4.78-4.85 (m, 1H); ESI-MS m/z: 754.6 [M+H]⁺.

### Example 107: Synthesis of compound 107

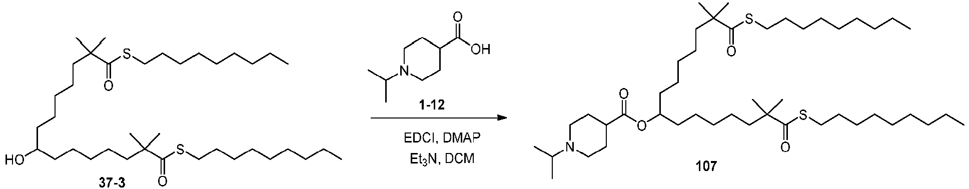

Referring to the method of Example 104, compound **107** was prepared as an oily product: 39 mg.

¹H NMR (400 MHz, CDCl₃): δ ppm 0.88 (t, *J =* 6.8 Hz, 6H), 1.05 (d, *J =* 6.8 Hz, 6H), 1.16 (s, 12H), 1.12-1.35 (m, 34H), 1.37-1.55 (m, 15H), 1.62-1.92 (m, 4H), 2.15-2.19 (m, 3H), 2.71-2.93 (m, 6H), 4.78-4.85 (m, 1H); ESI-MS m/z: 782.6 [M+H]⁺.

### Example 108: Synthesis of compound 108

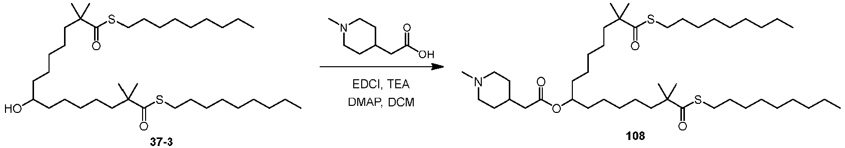

Referring to the method of Example 104, compound 108 was prepared as an oily product: 43.8 mg.

¹H NMR (400 MHz, CDCl₃): δ ppm 0.88 (t, *J* = 6.80 Hz, 6H), 1.18 (s, 12 H), 1.20-1.39 (m, 38H), 1.40-1.62 (m, 14H), 1.66-1.86 (m, 3H), 1.89-2.10 (m, 2H), 2.19-2.27 (m, 3H), 2.28 (br s, 2H), 2.79-2.83 (m, 4H), 4.79-4.88 (m, 1H); ESI-MS m/z: 768.5 [M+H]⁺.

### Example 109: Synthesis of compound 109

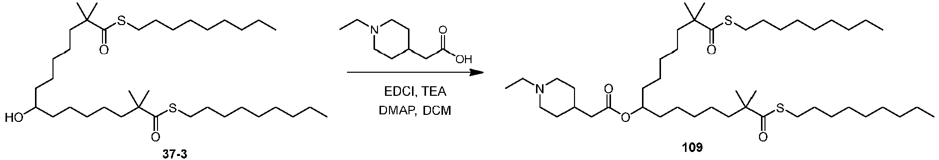

Referring to the method of Example **104,** compound **109** was prepared as an oily product: 44.8 mg.

¹H NMR (400 MHz, CDCl₃): δ ppm 0.86-0.89 (m, 6H), 1.18 (s, 15H), 1.23-1.37 (m, 36H), 1.46-1.54 (m, 14H), 1.76-1.93 (m, 4H), 2.11-2.20 (m, 1H), 2.24-2.28 (m, 2H), 2.54-2.71 (m, 2H), 2.81 (d, *J =* 7.2 Hz, 4 H), 3.08-3.24 (m, 2H), 4.79-4.88 (m, 1H); ESI-MS m/z: 782.6 [M+H]⁺.

### Example 110: Synthesis of compound 110

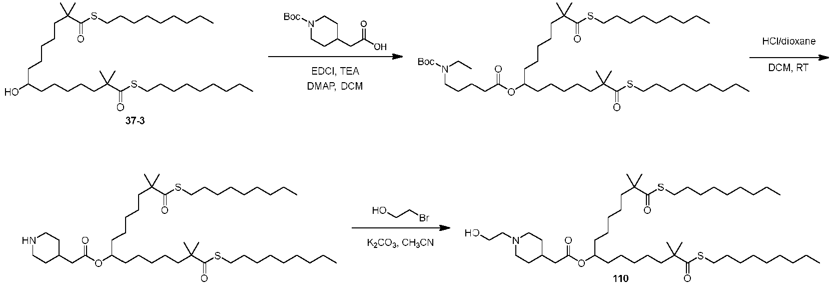

Referring to the method of Example **104,** compound **110** was prepared as an oily product: 34.4 mg.

¹H NMR (400 MHz, CDCl₃): δ ppm 0.81 (t, *J* = 7.2 Hz, 6H), 1.12 (s, 12H), 1.14-1.27 (m, 34H), 1.44-1.48 (m, 12H), 1.66-1.77 (m, 7H), 2.05-2.24 (m, 4H), 2.53 (m, 2H), 2.75 (t, J = 7.2 Hz, 4H), 2.90-2.92 (m, 2H), 3.57 (t, *J* = 5.2 Hz, 2H), 4.74-4.80 (m, 1H); ESI-MS m/z: 798.6 [M+H]⁺.

### Example 111: Synthesis of compound 111

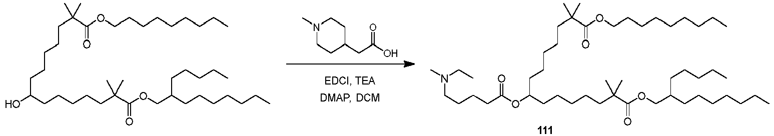

Referring to the method of Example **104,** compound **111** was prepared as an oily product: 31.4 mg.

¹H NMR (400 MHz, CDCl₃): δ ppm 0.92 (t, *J* = 6.8 Hz, 9H), 1.19 (s, 12H), 1.20-1.35 (m, 43H), 1.47-1.55 (m, 9H), 1.54-1.82 (m, 12H), 2.07-2.37 (m, 7H), 2.94-3.01 (m, 2H), 3.98 (d, *J =* 6.8 Hz, 2H), 4.07 (t, *J =* 6.8 Hz, 2H), 4.84-4.91 (m, 1H); ESI-MS m/z: 806.7 [M+H]⁺.

### Example 112: Synthesis of compound 112

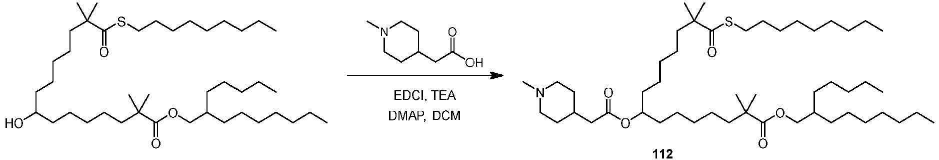

Referring to the method of Example 104, compound 112 was prepared as an oily product: 24.4 mg.

¹H NMR (400 MHz, CDCl₃): δ ppm 0.80-0.83 (m, 9H), 1.08 (s, 12H), 1.10-1.35 (m, 28H), 1.41-1.57 (m, 28H), 1.65-1.75 (m, 4H), 1.95-2.10 (m, 2H), 2.16 (d, *J* = 6.4 Hz, 2H), 2.30 (s, 3H), 2.73-2.91 (m, 4H), 3.87 (d, *J =* 5.6 Hz, 2H), 4.75-4.79 (m, 1H); ESI-MS m/z: 822.7 [M+H]⁺.

### Example 113: Synthesis of compound 113

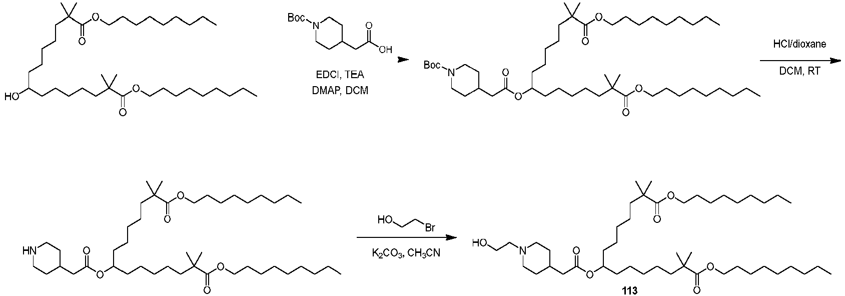

Referring to the method of Example 110, compound 113 was prepared as an oily product: 31.1 mg.

¹H NMR (400 MHz, CDCl₃): δ ppm 0.81 (t, *J* = 7.2 Hz, 6H), 1.08 (s, 12H), 1.10-1.24 (m, 36H), 1.36-1.43 (m, 8H), 1.48-1.54 (m, 6H), 1.64-1.72 (m, 6H), 2.05 (t, *J =* 6.8 Hz, 1H), 2.15 (d, *J* = 6.8 Hz, 2H), 2.47 (t, *J* = 5.6 Hz, 2H), 2.82-2.89 (m, 2H), 3.54 (t, *J* = 5.6 Hz, 2H), 3.97 (t, *J =* 6.8 Hz, 4H), 4.73-4.79 (m, 1H); ESI-MS m/z: 766.6 [M+H]⁺.

### Example 114: Synthesis of compound 114

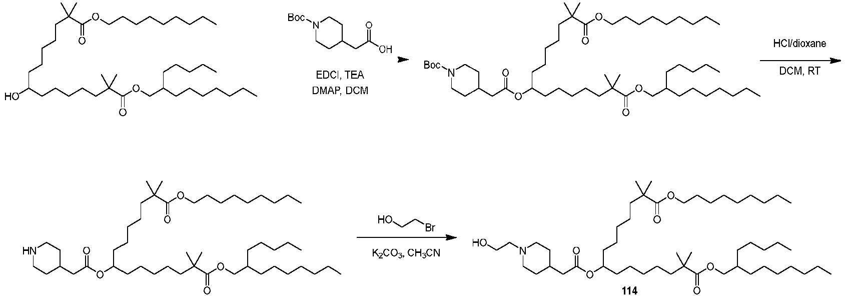

Referring to the method of Example **110,** compound **114** was prepared as an oily product: 32.7 mg.

¹H NMR (400 MHz, CDCl₃): δ ppm 0.85-0.88 (m, 9H), 1.07 (s, 12H), 1.09-1.35 (m, 46H), 1.41-1.58 (m, 13H), 1.97-2.25 (m, 3H), 2.32 (d, *J* = 5.6 Hz, 2H), 2.83-2.86 (m, 2H), 3.17-3.19 (m, 2H), 3.78-3.81 (d, *J =* 7.2 Hz, 2H), 3.92 (d, *J =* 5.6 Hz, 2H), 4.01 (t, *J =* 6.4 Hz, 2H), 4.10 (m, 1H), 4.81-4.86 (m, 1H); ESI-MS m/z: 836.7 [M+H]⁺.

### Example 115: Synthesis of compound 115

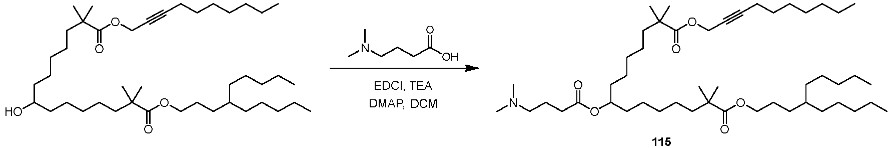

Referring to the method of Example **104,** compound **115** was prepared as an oily product: 31.0 mg.

¹H NMR (400 MHz, CDCl₃): δ ppm 0.87-0.91 (m, 9H), 1.14-1.37 (m, 51H), 1.49-1.60 (m, 12H), 1.75-1.81 (m, 2H), 2.21-2.26 (m, 2H), 2.28 (s, 6H), 2.32-2.36 (m, 4H), 4.03 (t*, J* = 6.4 Hz, 2H), 4.65 (s, 2H), 4.82-4.88 (m, 1H); ESI-MS m/z: 790.6 [M+H]⁺.

### Example 116: Synthesis of compound 116

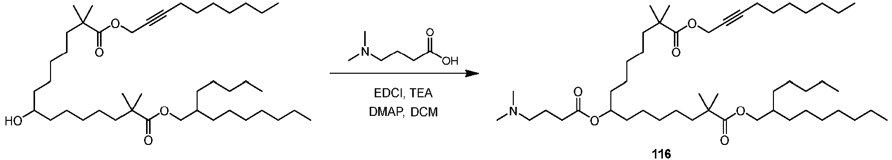

Referring to the method of Example **104,** compound **116** was prepared as an oily product: 31.3 mg.

¹H NMR (400 MHz, CDCl₃): δ ppm 0.80-0.83 (m, 9H), 1.07-1.27 (m, 51H), 1.40-1.45 (m, 12H), 1.70-1.81 (m, 2H), 2.13-2.36 (m, 12H), 3.87 (d, *J =* 5.6 Hz, 2H), 4.57 (s, 2H), 4.74-4.80 (m, 1H); ESI-MS m/z: 790.6 [M+H]⁺.

### Example 117: Synthesis of compound 117

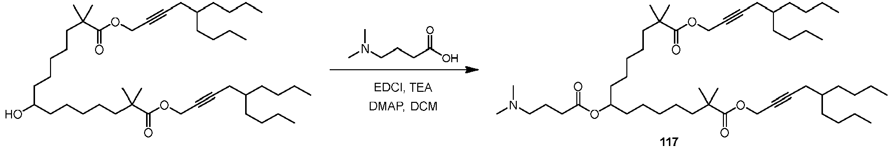

Referring to the method of Example **104,** compound **117** was prepared as an oily product: 35.9 mg.

¹H NMR (400 MHz, CDCl₃): δ ppm 0.90 (t, *J =* 6.8 Hz, 12H), 1.15 (s, 12H), 1.16-1.33 (m, 38H), 1.48-1.53 (m, 8H), 1.82-1.86 (m, 2H), 2.18-2.20 (m, 4H), 2.30-2.42 (m, 10 H), 4.65 (m, 4H), 4.82-4.88 (m, 1H); ESI-MS m/z: 814.6 [M+H]⁺.

### Example 118: Synthesis of compound 118

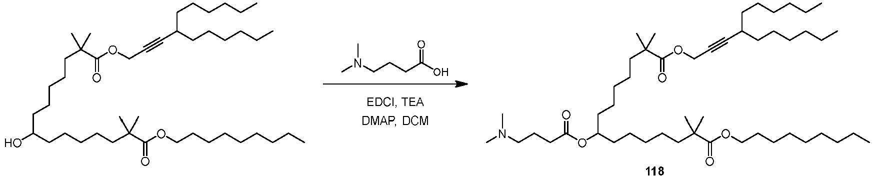

Referring to the method of Example **104,** compound **118** was prepared as an oily product: 32.0 mg.

¹H NMR (400 MHz, CDCl₃): δ ppm 0.87-0.90 (m, 9H), 1.15-1.32 (m, 50H), 1.40-1.61 (m, 16H), 1.76-1.84 (m, 2H), 2.23 (s, 6H), 2.29-2.34 (m, 6H), 4.04 (t, *J =* 6.8 Hz, 2H), 4.66 (d, *J =* 2.0 Hz, 1H), 4.83-4.87 (m, 1H); ESI-MS m/z: 804.6 [M+H]⁺.

### Example 119: Synthesis of compound 119

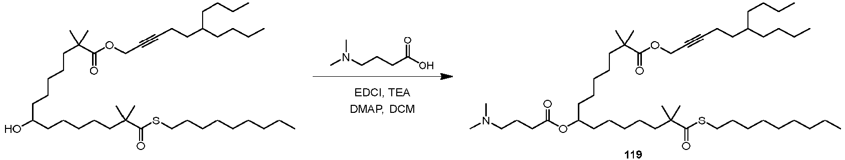

Referring to the method of Example **104,** compound **119** was prepared as an oily product: 34.8 mg.

¹H NMR (400 MHz, CDCl₃): δ ppm 0.91-0.94 (m, 9H), 1.21-1.40 (m, 48H), 1.51-1.57 (m, 12H), 1.61-1.86 (m, 5H), 2.23 (m, 2H), 2.31 (s, 6H), 2.35-2.39 (m, 2H), 2.85 (t, *J =* 7.2 Hz, 2H), 4.67 (t, *J =* 2.0 Hz, 1H), 4.84-4.90 (m, 1H); ESI-MS m/z: 792.6 [M+H]⁺.

### Example 120: Synthesis of compound 120

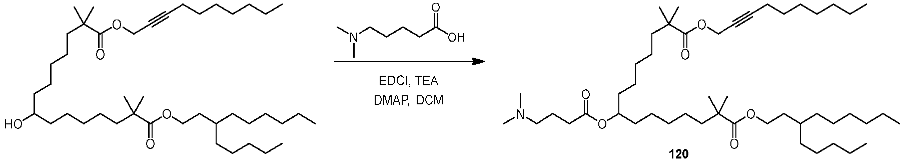

Referring to the method of Example **104,** compound **120** was prepared as an oily product: 34.5 mg.

¹H NMR (400 MHz, CDCl₃): δ ppm 0.89 (t, *J =* 6.8 Hz, 9H), 1.15-1.31 (m, 48H), 1.47-1.57 (m, 16H), 1.75-1.85 (m, 4H), 2.19-2.41 (m, 11H), 4.07 (t, *J* = 6.8 Hz, 2H), 4.65 (t, *J* = 2.0 Hz, 2H), 4.27-4.88 (m, 1H); ESI-MS m/z: 804.6 [M+H]⁺.

### Example 121: Synthesis of compound 121

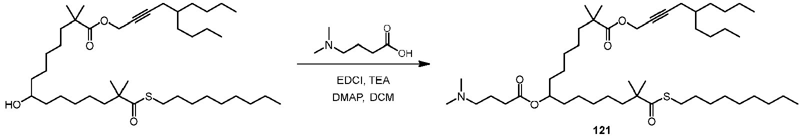

Referring to the method of Example **104,** compound **121** was prepared as an oily product: 33.8 mg.

¹H NMR (400 MHz, CDCl₃): δ ppm 0.91-0.95 (m, 9H), 1.20-1.43 (m, 46H), 1.47-1.57 (m, 10H), 1.63-1.85 (m, 6H), 2.29-2.40 (m, 12H), 2.85 (t, *J=* 7.2 Hz, 2H), 4.68 (s, 2H), 4.84-4.90 (m, 1H); ESI-MS m/z: 764.6 [M+H]⁺.

### Example 122: Synthesis of compound 122

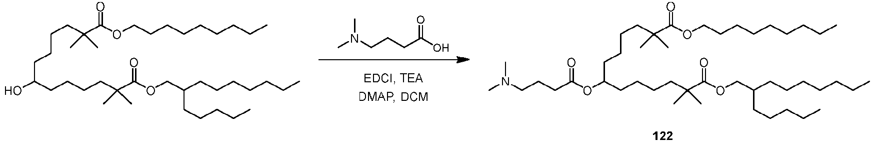

Referring to the method of Example **104,** compound **122** was prepared as an oily product: 33.7 mg.

¹H NMR (400 MHz, CDCl₃): δ ppm 0.81 (t, *J =* 6.8 Hz, 9H), 1.08-1.31 (m, 46H), 1.40-1.58 (m, 17H), 1.68-1.73 (m, 2H), 2.17 (s, 6H), 2.25 (t, *J =* 7.2 Hz, 4H), 3.87 (d, *J =* 5.6 Hz, 2H), 3.97 (t, *J =* 6.8 Hz, 2H), 4.73-4.80 (m, 1H); ESI-MS m/z: 752.7 [M+H]⁺.

### Example 123: Synthesis of compound 123

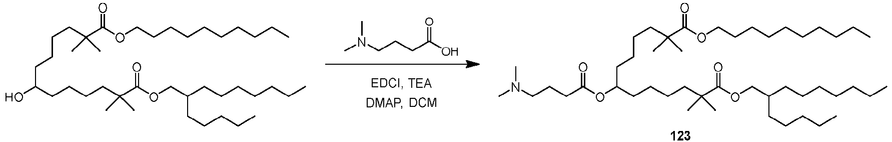

Referring to the method of Example **104,** compound **123** was prepared as an oily product: 35.2 mg.

¹H NMR (400 MHz, CDCl₃): δ ppm 0.81 (t, *J* = 6.8 Hz, 9H), 1.08 (s, 12H), 1.10-1.33 (m, 36H), 1.40-1.57 (m, 17H), 1.68-1.73 (m, 2H), 2.17 (s, 6H), 2.25 (t, *J* = 7.2 Hz, 4H), 3.87 (d, *J =* 5.6 Hz, 2H), 3.97 (t, *J =* 6.8 Hz, 2H), 4.73-4.79 (m, 1H); ESI-MS m/z: 766.7 [M+H]⁺.

### Example 124: Synthesis of compound 124

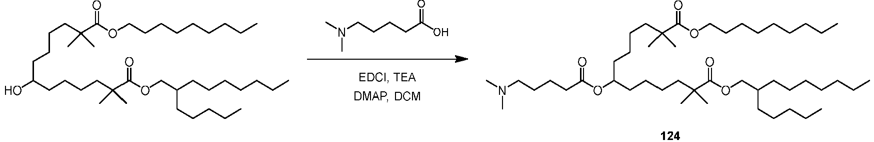

Referring to the method of Example **104,** compound **124** was prepared as an oily product: 33.4 mg.

¹H NMR (400 MHz, CDCl₃): δ ppm 0.89 (t, *J* = 6.8 Hz, 9H), 1.15 (s, 12H), 1.18-1.37 (m, 39H), 1.47-1.65 (m, 16H), 2.28 (s, 6H), 2.29-2.35 (m, 4H), 3.95 (d, *J=* 5.6 Hz, 2H), 4.04 (t, *J =* 6.8 Hz, 2H), 4.79-4.86 (m, 1H); ESI-MS m/z: 766.6 [M+H]⁺.

### Example 125: Synthesis of compound 125

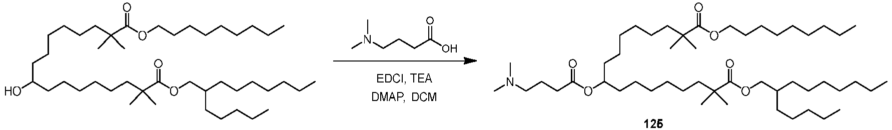

Referring to the method of Example **104,** compound **125** was prepared as an oily product: 31.1 mg.

¹H NMR (400 MHz, CDCl₃): δ ppm 0.89 (t, *J* = 6.8 Hz, 9H), 1.15 (s, 12H), 1.18-1.37 (m, 48H), 1.48-1.51 (m, 8H), 1.60-1.63 (m, 3H), 1.80-1.88 (m, 2H), 2.32-2.41 (m, 10H), 3.95 (d, *J =* 5.6 Hz, 2H), 4.04 (t, *J =* 6.8 Hz, 2H), 4.81-4.88 (m, 1H); ESI-MS m/z: 808.7 [M+H]⁺.

### Example 126: Synthesis of compound 126

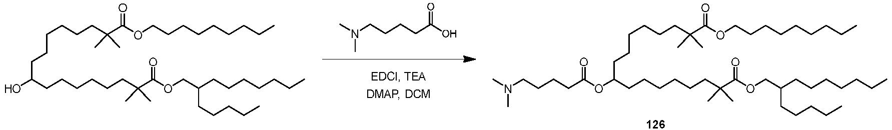

Referring to the method of Example **104,** compound **126** was prepared as an oily product: 35.1 mg.

¹H NMR (400 MHz, CDCl₃): δ ppm 0.89 (t, *J* = 6.8 Hz, 9H), 1.16 (s, 12H), 1.18-1.37 (m, 48H), 1.48-1.65 (m, 15H), 2.32-2.43 (m, 10H), 3.95 (d, *J =* 5.6 Hz, 2H), 4.05 (t, J = 6.8 Hz, 2H), 4.81-4.89 (m, 1H); ESI-MS m/z: 822.7 [M+H]⁺.

### Example 127: Synthesis of compound 127

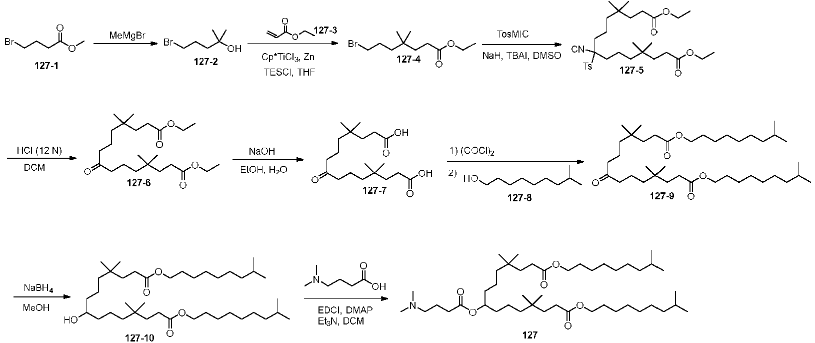

A solution of compound 127-1 (100 g, 552.4 mmol) in anhydrous ether (800 mL) was cooled to 0 °C in an ice bath, and methylmagnesium bromide (3 M in ether, 737 mL) was slowly added dropwise to the solution. After the dropwise addition was completed, the ice bath was removed and the mixture was stirred to react for 4 h at room temperature. The reaction system was quenched with saturated ammonium chloride aqueous solution, and extracted with ether. The organic phases were combined, dried over anhydrous sodium sulfate, and filtered. The filtrate was concentrated to dryness to give the crude product. The crude product was purified by silica gel column to give compound 127-2 (100 g).

Compound 127-2 (42 g, 232 mmol), compound 127-3 (30.3 mL, 278 mmol), Cp*TiCl₃ (5.09 g, 23.2 mmol), zinc powder (45.5 g, 696 mmol), and triethylchlorosilane (116.8 mL, 696 mmol) were added to a round bottom flask. Then anhydrous tetrahydrofuran (1200 mL) was added to the reaction system and the reaction was carried out under the protection of argon gas. The reaction system was heated to 60 °C and stirred to react for 1 hour. The reaction system was quenched with saturated aqueous sodium chloride solution and extracted with ethyl acetate. The organic phases were combined, dried over anhydrous sodium sulfate, and filtered. The filtrate was concentrated to dryness to give crude product 1-4, which was purified by silica gel column to give compound 127-4 (21 g).

Compound TosMIC (7.03 g, 36 mmol) was dissolved in DMSO (200 mL), and NaH (4.32 g, 60%, 108 mmol) was added to the reaction system in batches under ice bath conditions. After the addition was completed, the ice bath was removed and the mixture was reacted at room temperature for another 1 h. Compound 127-4 (21 g, 79 mmol) and TBAI (1.33 g, 3.6 mmol) were added to the reaction system, and the mixture was stirred at room temperature overnight. The reaction system was quenched with saturated aqueous sodium chloride solution and extracted with ethyl acetate. The organic phases were combined, dried over anhydrous sodium sulfate, and filtered. The filtrate was concentrated to dryness to give crude compound 127-5 (21.9 g), which was used directly in the next reaction step without purification.

To a solution of crude compound 127-5 (21.9 g, 38.8 mmol) in dichloromethane (350 mL) was added 200 mL of concentrated hydrochloric acid, and the mixture was reacted at room temperature for 2 h. The complete reaction of the substrate was monitored by TLC. The reaction system was quenched with saturated aqueous ammonium chloride solution and extracted with ethyl acetate. The organic phases were combined, dried over anhydrous sodium sulfate, and filtered. The filtrate was concentrated to dryness to give the crude product, which was purified by silica gel column to give compound 127-6 (12.5 g).

Compound 127-6 (12.5 g, 31.4 mmol) was dissolved in ethanol (20 mL)-water (40 mL), and NaOH (3.77 g, 94.2 mmol) was added to the mixed solution in batches under ice bath conditions. After the addition was completed, the ice bath was removed and the mixture was stirred at room temperature. The complete consumption of the reaction materials was monitored by TLC. The organic solvent was removed by rotary evaporation, and the residue was extracted with dichloromethane. The aqueous layer was collected, and the solution was adjusted to a pH of <5 with 1 M hydrochloric acid. The solution was extracted with dichloromethane. The organic phases were combined, dried over anhydrous sodium sulfate, and filtered. The filtrate was collected and concentrated to give compound 127-7 (9.7 g).

DMF (17 µL, 0.22 mmol) was added to a solution of compound 127-7 (750 mg, 2.19 mmol) in dichloromethane (10.0 mL) under ice bath conditions, and oxalyl chloride (0.77 mL, 8.76 mmol) was then added dropwise to the reaction solution. The ice bath was removed, and the mixture was stirred for 1 h at room temperature. The solvent was removed using a rotary-evaporator to give acyl chloride crude product, which was used directly in the next reaction step.

The above obtained acyl chloride crude product was dissolved in 10.0 mL of 1,2-dichloroethane, and then compound 127-8 (693 mg, 4.38 mmol) was added to the reaction solution. The mixture was stirred at room temperature until the substrate was reacted completely. The solvent was removed using a rotary-evaporator. The crude was purified by silica gel column to give compound 127-9 (800 mg).

Compound 127-9 (800 mg, 1.29 mmol) was dissolved in 5.0 mL of methanol and sodium borohydride (146 mg, 3.87 mmol) was added to the reaction system. The mixture was reacted at room temperature. The complete disappearance of the reactants was monitored by TLC. The reaction system was quenched with saturated aqueous sodium chloride solution and extracted with dichloromethane. The organic phases were combined, dried over anhydrous sodium sulfate, and filtered. The filtrate was concentrated to dryness to give crude compound 127-10 (800 mg), which was used directly in the next reaction without purification.

Crude compound 127-10 (300 mg, 0.48 mmol), 4-dimethylaminobutyric acid (94.4 mg, 0.72 mmol), EDCI (276 mg, 1.44 mmol), triethylamine (0.21 mL, 1.44 mmol) and DMAP (59 mg, 0.48 mmol) were dissolved in 5.0 mL of dichloromethane, and the reaction solution was stirred to react at room temperature for 12 h. The reaction solution was quenched with saturated aqueous sodium chloride and extracted with dichloromethane. The organic phases were combined, dried over anhydrous sodium sulfate, and filtered. The organic phase was collected, and the organic solvent was removed using a rotary-evaporator. The crude product was purified by preparative high performance liquid chromatography to give the compound 127 (43.6 mg)

¹H NMR (400 MHz, CD₃OD): δ ppm 0.77-0.93 (m, 28H), 1.08-1.74 (m, 38H), 1.76-1.84 (m, 2H), 1.22-2.27 (m, 10H), 2.33-2.37 (m, 4H), 4.03-4.12 (m, 4H), 4.92-4.97 (m, 1H); ESI-MS m/z: 738.6 [M+H]⁺.

### Example 128: Synthesis of compound 128

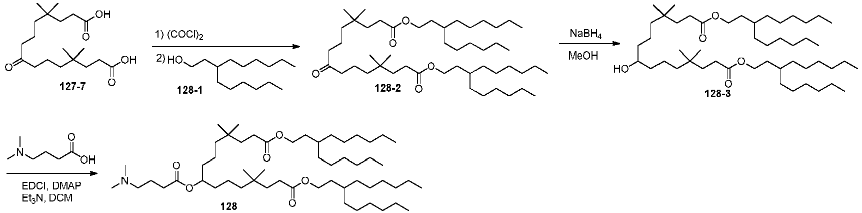

Referring to the method of Example 127, compound **128** was prepared as an oily product: 64.2 mg.

¹H NMR (400 MHz, CD₃OD): δ ppm 0.86 (s, 12 H), 0.91 (t, J = 6.8 Hz, 12H), 1.22-1.37 (m, 48 H), 1.51-1.61 (m, 14 H), 1.78-1.86 (m, 2 H), 2.24 (t, J = 8.0 Hz, 4H), 2.30 (s, 6H), 2.37 (t, J = 7.2 Hz, 2H), 2.43 (t, *J* = 8.0 Hz, 2 H), 4.10 (t, J = 6.8 Hz, 4H), 4.92-4.97 (m, 1 H); ESI-MS m/z: 878.7 [M+H]⁺.

### Example 129: Synthesis of compound 129

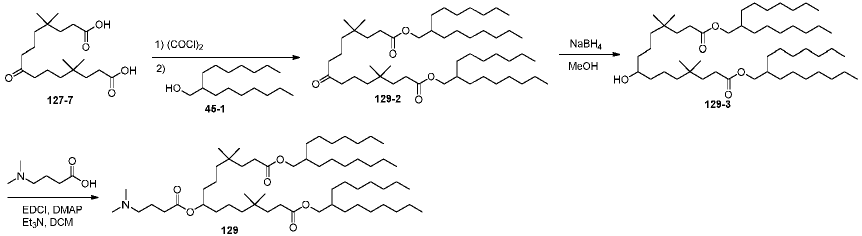

Referring to the method of Example 127, compound **129** was prepared as an oily product: 67.0 mg.

¹H NMR (400 MHz, CD₃OD): δ ppm 0.86 (s, 12 H), 0.88-0.93 (m, 12H), 1.12-1.40 (m, 52 H), 1.49-1.56 (m, 8 H), 1.59-1.66 (m, 4 H), 1.77-1.84 (m, 4 H), 2.22-2.27 (m, 10H), 2.34-2.38 (m, 4H), 4.01 (t, *J* = 6.8 Hz, 4 H), 4.91-4.97 (m, 1 H); ESI-MS m/z: 906.8 [M+H]⁺.

### Comparative Examples

### Synthesis of comparative compound 1 (D1)

D1 was prepared according to the method of Example 20, [M+H]⁺: 654.6. ¹H NMR (400 MHz, CDCl₃): δ ppm 0.77-0.86 (t, *J* = 7.2 Hz, 6H), 1.15-1.30 (m, 38H), 1.40-1.59 (m, 12H), 1.87-1.96 (m, 2H), 2.21 (t, *J* = 7.2 Hz, 4H), 2.28-2.37 (m, 2H), 2.40-2.50 (m, 5H), 2.56-2.67 (m, 2H), 3.92-4.07 (m, 4H), 4.72-4.90 (m, 1H).

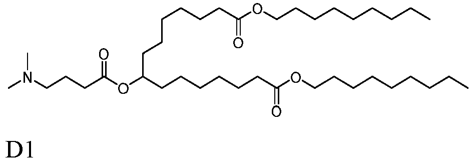

### Synthesis of comparative compound 2 (D2)

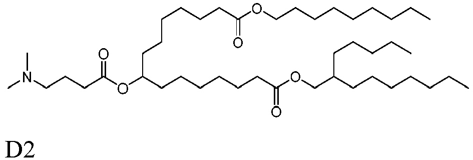

D2 was prepared according to the method of Example 46, [M+H]⁺: 724.6. ¹H NMR (400 MHz, CDCl₃): δ ppm 0.89 (t, *J =* 7.2 Hz, 9H), 1.21-1.30 (m, 44H), 1.50-1.63 (m, 11H), 1.77-1.92 (m, 2H), 2.27-2.36 (m, 14H), 3.97 (d, *J =* 5.6 Hz, 2H), 4.06 (t, *J =* 6.8 Hz, 2H), 4.85-4.92 (m, 1H).

### Pharmacological assay

### Assay example 1: Preparation of nanoparticles

Materials used for lipid nanoparticle assembly include: (1) ionizable lipid compounds: e.g., ionizable lipids designed and synthesized in the present disclosure or DLin-MC3-DMA (purchased from AVT) as a control; (2) structure lipids: e.g., Cholesterol (purchased from Sigma-Aldrich); (3) phospholipids: e.g., DSPC i.e., 1,2-distearoyl-SN-glycero-3-phosphocholine (Distearoylphosphatidylcholine, purchased from AVT); (4) polyethylene glycolated lipids: e.g. DMG-PEG2000 i.e., dimyristoylglycero-polyethylene glycol 2000 (1,2-dimyristoyl-rac-glycero-3-methoxypolyethylene glycol-2000, purchased from AVT); (5) active ingredients of nucleic acid fragments: e.g. Luciferase mRNA, siRNA, CRISPR Cas 9 mRNA, etc. (manufactured in-house). The names of materials of the lipid nanoparticle assembly and their structural formulae are detailed in Table 4.

**Table 4**

| No. | Name | Structural formula |
|---|---|---|
| 1 | DLin-MC3-DMA | 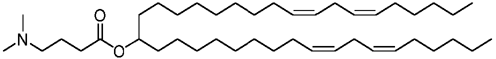 |
| 2 | Cholesterol | 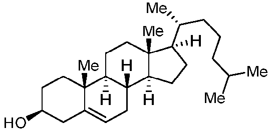 |
| 3 | DSPC | 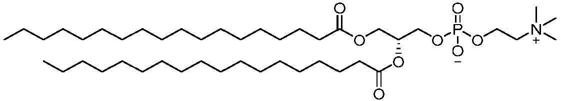 |
| 4 | DMG-PEG2000 | 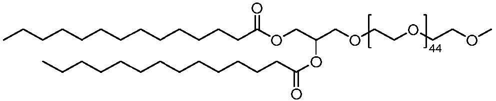 |
| 5 | SM-102 | 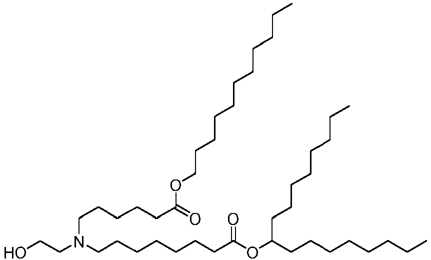 |

Lipid nanoparticles were prepared by (1) dissolving and mixing ionizable lipid compounds, cholesterol, phospholipids and polyethylene glycolated lipids in ethanol at (molar percentages) 50%, 38.5%, 10% and 1.5%, respectively; (2) dissolving the mRNA active ingredient in 25 mM sodium acetate solution (pH = 4.5); (3) using an automated high-throughput microfluidic system to mix the organic phase containing the lipid mixture and the aqueous phase containing the mRNA component in the flow ratio range of 1:1 to 1:4 at a mixing speed of 10 mL/min to 18 mL/min; (4) the prepared lipid nanoparticles (N/P ratio of 6) were diluted with phosphate buffer solution and the nanoparticle solutions were ultrafiltered to the original preparation volume using ultrafiltration tubes (purchased from Millipore) with a cut-off molecular weight of 30 kDa; and (5) the obtained nanoparticles were filtered through a sterile 0.2 µm filter membrane and then stored in a sealed glass vial at low temperature.

### Assay example 2: Characterization of physical properties of lipid nanoparticles

The particle size and particle size dispersity index (PDI) of the prepared lipid nanoparticles were measured using a Zetasizer Pro (purchased from Malvern Instruments Ltd) and a DynaPro NanoStar (purchased from Wyatt) dynamic light scattering instrument. The degree of RNA encapsulation by lipid nanoparticles was characterized by the Encapsulation Efficiency%, which reflects the degree of binding of lipid nanoparticles to RNA fragments. This parameter was measured by the method of Quant-it^{™} RiboGreen RNA Assay (purchased from Invitrogen). Lipid nanoparticle samples were diluted in TE buffer (10 mM Tris-HCl, 1 mM EDTA, pH = 7.5). A portion of the sample solution was removed, to which 0.5% Triton (Triton X-100) was added, and then allowed to stand at 37° C for 30 minutes. Immediately after the addition of RIBOGREEN^{®} reaction solution, the fluorescence values were read on a Varioskan LUX multifunctional microplate reader (purchased from Thermofisher) at 485 nm for absorption and 528 nm for emission to give the encapsulation efficiency values.

### Assay example 3: Animal experiment

The delivery effect and safety of nanoparticles encapsulated with luciferase mRNA (Trilink, L-7202) in mice were evaluated. The test mice were SPF-grade C57BL/6 mice, female, 6-8 weeks old, weighing 18-22 g, and were purchased from SPF (Beijing) Biotechnology Co., Ltd. All animals were acclimatized for more than 7 days prior to the experiment, and had free access to food and water during the experiment. The conditions include alternating light and dark for 12/12 h, the indoor temperature of 20-26°C and the humidity of 40-70%. The mice were randomly grouped. The lipid nanoparticles encapsulated with luciferase mRNA prepared above were injected into mice by intravenous administration at a single dose of 0.5 mg/kg mRNA, and the mice were subjected to in vivo bioluminescence assay using a Small Animal In Vivo Imaging System (IVIS LUMINA III, purchased from PerkinElmer) at 6 h after administration. The assay was performed as follows: D-luciferin solution was prepared in saline at a concentration of 15 mg/mL, and each mouse was given the substrate by intraperitoneal injection. At ten minutes after administration of the substrate, the mice were anesthetized in an anesthesia chamber with isoflurane at a concentration of 2.5%. The anesthetized mice were placed in IVIS for fluorescence imaging, and data acquisition and analysis were performed on the concentrated distribution area of fluorescence.

The in vivo delivery efficiency of lipid nanoparticle carriers was expressed as the mean values of fluorescence intensity and total photon count in different animals within the same subject group, as shown in Table 5. Higher values of fluorescence intensity and total photon count indicate higher in vivo delivery efficiency of this mRNA fragment by lipid nanoparticles. The lipid nanoparticles containing the cationic lipids of the present disclosure have good in vivo delivery efficiency. Unexpectedly, compared with the molecule without tetramethyl, the corresponding lipid nanoparticles of the present disclosure have significantly increased in vivo delivery efficiency, e.g., compound 20 vs. D1, compound 46 vs. D2.

**Table 5**

| **Cationic lipid compound** | **Particle size (nm)** | **Particle size dispersity (PDI)** | **Encaps ulation efficiency (%)** | **Total photon count in vivo at 6 hours after administration (Total Flux)** |
|---|---|---|---|---|
| 1 | 80.07 | 0.06 | 91.54 | 6.43E+08 |
| 2 | 229.35 | 0.03 | 79.14 | 4.23E+08 |
| 3 | 133.80 | 0.04 | 86.88 | 1.75E+09 |
| 6 | 106.18 | 0.05 | 90.26 | 2.25E+09 |
| 7 | 203.67 | 0.04 | 69.51 | 1.16E+08 |
| 8 | 127.82 | 0.07 | 60.36 | 3.26E+07 |
| 9 | 352.82 | 0.07 | 19.41 | 6.05E+06 |
| 10 | 118.59 | 0.07 | 53.83 | 1.26E+09 |
| 11 | 46.49 | 0.03 | 95.61 | 4.42E+06 |
| 12 | 89.34 | 0.07 | 85.02 | 1.10E+09 |
| 13 | 157.10 | 0.08 | 56.65 | 1.08E+06 |
| 14 | 306.07 | 0.08 | 45.66 | 6.34E+07 |
| 15 | 91.68 | 0.06 | 84.78 | 4.89E+08 |
| 16 | 55.39 | 0.05 | 96.52 | 3.97E+07 |
| 17 | 81.43 | 0.12 | 30.95 | 6.77E+05 |
| 18 | 161.29 | 0.05 | 87.86 | 1.14E+10 |
| 19 | 135.15 | 0.19 | 67.96 | 3.20E+09 |
| 20 | 99.61 | 0.10 | 71.93 | 2.40E+10 |
| 23 | 151.35 | 0.07 | 70.22 | 1.43E+10 |
| 24 | 133.72 | 0.04 | 60.98 | 5.56E+09 |
| 25 | 236.90 | 0.06 | 32.43 | 1.89E+08 |
| 26 | 96.69 | 0.05 | 81.29 | >4.00E+10 |
| 27 | 105.70 | 0.05 | 88.99 | 2.20E+ 10 |
| 28 | 138.16 | 0.06 | 80.36 | 3.21E+09 |
| 32 | 116.61 | 0.07 | 99.02 | 7.30E+09 |
| 33 | 105.84 | 0.04 | 88.67 | 2.12E+08 |
| 34 | 104.71 | 0.04 | 90.29 | 1.26E+10 |
| 36 | 88.33 | 0.07 | 97.52 | 4.84E+09 |
| 37 | 92.18 | 0.03 | 89.07 | 7.88E+09 |
| 40 | 83.90 | 0.05 | 97.06 | 4.22E+09 |
| 41 | 104.27 | 0.05 | 93.91 | 2.19E+ 10 |
| 42 | 152.04 | 0.07 | 71.95 | 5.32E+09 |
| 46 | 97.30 | 0.09 | 95.68 | >4.00E+10 |
| 90 | 18.17 | 0.05 | 83.21 | 1.04E+07 |
| 91 | 32.61 | 0.05 | 102.99 | 1.29E+06 |
| 92 | 107.56 | 0.05 | 91.66 | 2.13E+09 |
| 97 | 157.00 | 0.25 | 93.72 | 3.37E+09 |
| 98 | 107.87 | 0.13 | 93.28 | 2.12E+ 10 |
| 99 | 139.99 | 0.05 | 90.69 | 5.30E+09 |
| 100 | 118.19 | 0.07 | 83.29 | 1.18E+10 |
| 101 | 152.45 | 0.06 | 76.09 | 5.19E+09 |
| 102 | 102.18 | 0.03 | 90.03 | 2.03E+10 |
| 103 | 148.67 | 0.04 | 90.97 | 5.69E+09 |
| 104 | 71.14 | 0.06 | 85.54 | 2.61E+10 |
| 105 | 78.80 | 0.05 | 94.67 | 3.33E+09 |
| 106 | 83.09 | 0.05 | 85.65 | 1.85E+10 |
| 107 | 99.54 | 0.05 | 83.74 | 1.36E+10 |
| 108 | 121.41 | 0.05 | 92.63 | 1.21E+10 |
| 109 | 101.04 | 0.06 | 87.11 | 1.32E+10 |
| 110 | 97.59 | 0.01 | 92.65 | 2.06E+08 |
| 111 | 141.30 | 0.06 | 95.12 | 1.79E+10 |
| 112 | 121.05 | 0.08 | 94.41 | 3.53E+10 |
| 113 | 112.65 | 0.03 | 94.77 | 3.59E+08 |
| 114 | 72.32 | 0.04 | 93.41 | 1.06E+08 |
| 115 | 77.12 | 0.04 | 91.76 | 2.56E+10 |
| 116 | 88.62 | 0.05 | 89.36 | >4.00E+10 |
| 117 | 127.31 | 0.06 | 95.29 | 4.48E+09 |
| 118 | 88.33 | 0.07 | 93.87 | 3.14E+ 10 |
| 119 | 80.98 | 0.05 | 86.92 | 3.25E+10 |
| 120 | 98.99 | 0.07 | 95.55 | 3.15E+10 |
| 121 | 103.57 | 0.06 | 93.62 | >4.00E+10 |
| 122 | 96.72 | 0.07 | 91.76 | >4.00E+10 |
| 123 | 84.98 | 0.05 | 92.27 | >4.00E+10 |
| 125 | 95.69 | 0.07 | 89.45 | 1.99E+10 |
| 126 | 133.76 | 0.06 | 94.35 | 1.77E+10 |
| 127 | 83.01 | 0.05 | 62.35 | 3.59E+10 |
| 128 | 83.10 | 0.05 | 79.88 | 1.00E+10 |
| 129 | 112.08 | 0.06 | 57.87 | 7.82E+09 |
| DLin-MC3-D MA | 96.83 | 0.04 | 95.62 | 8.04E+09 |
| D1 | 131.2 | 0.18 | 97.08 | 4.45E+07 |
| D2 | 245.33 | 0.20 | 79.63 | 1.57E+09 |

### Assay example 4: Evaluation of delivery efficiency and safety in vitro

The delivery effect and safety of nanoparticles encapsulated with luciferase mRNA were evaluated at the cellular level in vitro. The cells used in the assay were human embryonic kidney cells 293 (HEK293T cells) cultured in DMEM (Dulbecco's Modified Eagle Medium) (purchased from Thermo Fisher) containing 10% fetal bovine serum and 5% penicillin-streptomycin double antibiotics at a indoor temperature of 37°C and a CO₂ concentration of 5%. The cells were uniformly dispersed and spread in 48-well plates, and incubated in the incubator for 24 h. Then a solution of the lipid nanoparticles encapsulated with luciferase mRNA were added. After 24 h, the cells were lysed, and the intracellular expression intensity and relative light units (RLU) of luciferase in each type of lipid nanoparticles were measured with a luciferase detection reagent (purchased from Promega). The higher the intensity of expression, the higher the delivery efficiency of the lipid material at the cellular level. Meanwhile, CCK-8 reagent (purchased from DOJINDO) was used in cytotoxicity testing for the parallel lipid nanoparticle-treated cell groups after 24 hours. In the test, the group of cells to which only PBS was added was used as a negative control. The procedure was as follows: after the addition of CCK-8 solution, the cells were left to stand in an incubator at 37°C for 4 h. The absorbance values were read on a multifunctional microplate reader at an absorbance band of 450 nm. The ratio of the absorbance value of the nanoparticle-treated cells to that of the negative control was used as a characterization parameter for cell viability.

The delivery effects and the toxicity data of nanoparticles at the cellular level in vitro are shown in Table 6.

**Table 6**

| **Cationic lipids** | **Cell fluorescence intensity (RLU)** | **Cell viability (%)** |
|---|---|---|
| 32 | 6.99E+06 | 96.25 |
| 37 | 1.04E+07 | 97.48 |
| 41 | 1.23E+07 | 104.91 |
| 100 | 1.98E+06 | 104.67 |
| DLin-MC3-DMA | 2.95E+06 | 97.84 |

### Assay Example 5: Characterization of physical properties of lipid nanoparticles of different formulations

Lipid nanoparticles of formulations 1-39 were prepared using the same method as used in the assay examples, wherein the ionizable cationic lipid in formulations 1-9 was compound 46, the ionizable cationic lipid in formulations 10-31 was compound 20, and the ionizable cationic lipid in formulations 32-39 was compound 26. Specific formulation information can be found in Tables 10-12. The formulations for DLin-MC3-DMA and SM-102 used as controls are as follows:

### MC3 formulation:

MC3: cholesterol: DSPC: DMG-PEG=50:38.5:10:1.5, with an N/P ratio of 6;

### SM102 formulation:

SM102: cholesterol: DSPC: DMG-PEG=50:38.5:10:1.5, with an N/P ratio of 6.

The particle size and particle size dispersity index (PDI) of the prepared lipid nanoparticles were measured using a Zetasizer Pro (purchased from Malvern Instruments Ltd) and a DynaPro NanoStar (purchased from Wyatt) dynamic light scattering instrument. The degree of RNA encapsulation by lipid nanoparticles was characterized by the Encapsulation Efficiency%, which reflects the degree of binding of lipid nanoparticles to RNA fragments. This parameter was measured by the method of Quant-it^{™} RiboGreen RNA Assay (purchased from Invitrogen). Lipid nanoparticle samples were diluted in TE buffer (10 mM Tris-HCl, 1 mM EDTA, pH = 7.5). A portion of the sample solution was removed, to which 0.5% Triton (Triton X-100) was added, and then allowed to stand at 37° C for 30 minutes. Immediately after the addition of RIBOGREEN^{®} reaction solution, the fluorescence values were read on a Varioskan LUX multifunctional microplate reader (purchased from Thermofisher) at 485 nm for absorption and 528 nm for emission to obtain the encapsulation efficiency values.

**Table 7**

| **Formulation No.** | **Particle size (nm)** | **Particle size dispersity (PDI)** | **Encapsulation efficiency (%)** |
|---|---|---|---|
| 1 | 80 | 0.30 | 98.93 |
| 2 | 106 | 0.10 | 92.77 |
| 3 | 104 | 0.10 | 90.90 |
| 4 | 129 | 0.30 | 91.77 |
| 5 | 125 | 0.33 | 90.87 |
| 6 | 112 | 0.23 | 95.82 |
| 7 | 168 | 0.37 | 92.06 |
| 8 | 111 | 0.20 | 90.32 |
| 9 | 102 | 0.10 | 96.34 |
| MC3 | 88.9 | 0.10 | 95.62 |
| SM102 | 92.83 | 0.10 | 98.18 |

**Table 8**

| **Formulation No.** | **Particle size (nm)** | **Particle size dispersity (PDI)** | **Encapsulation efficiency (%)** |
|---|---|---|---|
| 10 | 81 | 0.37 | 71.82 |
| 11 | 129 | 0.17 | 96.34 |
| 12 | 79 | 0.10 | 87.95 |
| 13 | 93 | 0.13 | 82.10 |
| 14 | 69 | 0.10 | 90.65 |
| 15 | 115 | 0.23 | 77.97 |
| 16 | 83 | 0.13 | 77.24 |
| 17 | 141 | 0.10 | 92.25 |
| 18 | 72 | 0.07 | 83.44 |
| 19 | 77 | 0.10 | 87.68 |
| 20 | 122 | 0.23 | 94.56 |
| 21 | 106 | 0.23 | 83.93 |
| 22 | 104 | 0.17 | 86.44 |
| 23 | 117 | 0.20 | 83.48 |
| 24 | 102 | 0.17 | 84.03 |
| 25 | 121 | 0.17 | 86.10 |
| 26 | 107 | 0.27 | 83.80 |
| 27 | 120 | 0.20 | 85.83 |
| 28 | 105 | 0.23 | 83.78 |
| 29 | 116 | 0.13 | 87.34 |
| 30 | 118.53 | 0.1 | 90.32 |
| 31 | 131.66 | 0.1 | 57.37 |

**Table 9**

| **Formulation No.** | **Particle size (nm)** | **Particle size dispersity (PDI)** | **Encapsulation efficiency (%)** |
|---|---|---|---|
| 32 | 112 | 0.43 | 89.37 |
| 33 | 103 | 0.37 | 91.76 |
| 34 | 120 | 0.20 | 89.15 |
| 35 | 69 | 0.13 | 96.21 |
| 36 | 107 | 0.27 | 94.35 |
| 37 | 77 | 0.17 | 89.42 |
| 38 | 103 | 0.17 | 97.70 |
| 39 | 92 | 0.17 | 90.97 |

### Assay example 6: Animal experiment with different formulations

The in vivo delivery effect and safety of nanoparticles encapsulated with luciferase mRNA (Trilink, L-7202) in mice were evaluated. The test mice were SPF-grade C57BL/6 mice, female, 6-8 weeks old, weighing 18-22 g, and were purchased from SPF (Beijing) Biotechnology Co., Ltd. All animals were acclimatized for more than 7 days prior to the experiment, and had free access to food and water during the experiment. The conditions include alternating light and dark for 12/12 h, the indoor temperature of 20-26°C and the humidity of 40-70%. The mice were randomly grouped. The lipid nanoparticles encapsulated with luciferase mRNA prepared above were injected into mice by intravenous administration at a single dose of 0.5 mg/kg mRNA, and the mice were subjected to in vivo bioluminescence assay using a Small Animal In Vivo Imaging System (IVIS LUMINA III, purchased from PerkinElmer) at 6 h after administration. The assay was performed as follows: D-luciferin solution was prepared in saline at a concentration of 15 mg/mL, and each mouse was given the substrate by intraperitoneal injection. At ten minutes after administration of the substrate, the mice were anesthetized in an anesthesia chamber with isoflurane at a concentration of 2.5%. The anesthetized mice were placed in IVIS for fluorescence imaging, and data acquisition and analysis were performed on the concentrated distribution area of fluorescence.

The in vivo delivery efficiency of lipid nanoparticle carriers was expressed as the mean values of fluorescence intensity and total photon count in different animals within the same subject group. Higher values of fluorescence intensity and total photon count indicate higher in vivo delivery efficiency of this mRNA fragment by lipid nanoparticles. The MC3 fold and SM102 fold in Tables 10-12 indicate the fold of improvement of the present disclosure LNP relative to the MC3 LNP or SM102 LNP, respectively.

The LNPs of the present disclosure have good in vivo delivery efficiency (Tables 10-12) and are more efficiently expressed in vivo than MC3 or SM102 when delivering luciferase mRNA, up to more than 35-fold.

**Teble 10**

| **Formulation No.** | **N/P** | **LNP Formulation** | | | | **MC3 fold** | **SM102 fold** |
|---|---|---|---|---|---|---|---|
| | | **Compound 46 (%)** | **Cholesterol (%)** | **DSPC (%)** | **DMG-PEG(%)** | | |
| 1 | 4 | 32.5 | 61 | 5 | 1.5 | 2.8 | 2.7 |
| 2 | 5 | 32.5 | 51.5 | 15 | 1 | 2.6 | 2.5 |
| 3 | 5 | 42.5 | 49 | 7.5 | 1 | 8.4 | 8.3 |
| 4 | 5 | 42.5 | 51 | 5 | 1.5 | 9.1 | 8.9 |
| 5 | 3 | 47.6 | 38.1 | 12.5 | 1.8 | 21.3 | 20.9 |
| 6 | 4 | 47.6 | 30.6 | 20 | 1.8 | 20.5 | 20.1 |
| 7 | 4 | 47.5 | 40.5 | 10 | 2 | 11.2 | 11 |
| 8 | 5 | 47.6 | 32.9 | 17.5 | 2 | 19.7 | 19.3 |
| 9 | 6 | 47.6 | 36 | 15 | 1.4 | 13.7 | 13.4 |

**Table 11**

| **Formulation No.** | **N/P** | **LNP Formulation** | | | | **MC3 fold** | **SM102 fold** |
|---|---|---|---|---|---|---|---|
| | | **Compound 20 (%)** | **Cholesterol (%)** | **DSPC (%)** | **DMG-PEG(%)** | | |
| 10 | 9 | 40 | 35 | 20 | 5 | 2.95 | 2.89 |
| 11 | 9 | 40 | 48.5 | 10 | 1.5 | 12.18 | 11.93 |
| 12 | 3 | 30 | 66 | 2.5 | 1.5 | 3.56 | 3.49 |
| 13 | 3 | 40 | 53.5 | 5 | 1.5 | 9.13 | 8.95 |
| 14 | 6 | 30 | 66 | 2.5 | 1.5 | 4.55 | 4.46 |
| 15 | 6 | 40 | 53.5 | 5 | 1.5 | 10.52 | 10.30 |
| 16 | 9 | 30 | 63.5 | 5 | 1.5 | 8.08 | 7.92 |
| 17 | 9 | 40 | 48.5 | 10 | 1.5 | 10.51 | 10.30 |
| 18 | 3 | 40 | 55 | 2.5 | 2.5 | 11.33 | 11.10 |
| 19 | 6 | 30 | 62.5 | 5 | 2.5 | 4.93 | 4.83 |
| 20 | 9 | 30 | 57.5 | 10 | 2.5 | 12.21 | 11.96 |
| 21 | 9 | 40 | 55 | 2.5 | 2.5 | 11.15 | 10.93 |
| 22 | 6 | 48.5 | 47.5 | 1.5 | 2.5 | 17.45 | 17.10 |
| 23 | 6 | 48.5 | 46.5 | 2.5 | 2.5 | 18.04 | 17.67 |
| 24 | 6 | 48.5 | 45.5 | 2.5 | 3.5 | 30.69 | 30.07 |
| 25 | 9 | 48.5 | 47.5 | 1.5 | 2.5 | 22.87 | 22.40 |
| 26 | 9 | 48.5 | 45.5 | 2.5 | 3.5 | 26.20 | 25.67 |
| 27 | 12 | 48.5 | 47.5 | 1.5 | 2.5 | 21.21 | 20.78 |
| 28 | 12 | 48.5 | 46.5 | 1.5 | 3.5 | 35.00 | 34.29 |
| 29 | 12 | 48.5 | 46.5 | 2.5 | 2.5 | 15.56 | 15.25 |
| 30 | 6 | 50 | 42.5 | 5 | 2.5 | 6.46 | / |
| 31 | 3 | 60 | 27.5 | 10 | 2.5 | 1.07 | / |

**Table 12**

| **Formulation No.** | **N/P** | **LNP Formulation** | | | | **Phospholipid** | **MC3 fold** | **SM102 fold** |
|---|---|---|---|---|---|---|---|---|
| | | **Compound 26 (%)** | **Cholesterol (%)** | **Phospholipid (%)** | **DMG-PEG(%)** | | | |
| 32 | 7 | 40 | 44 | 12.5 | 3.5 | DSPC | 8.19 | 8.02 |
| 33 | 5 | 40 | 53.5 | 5 | 1.5 | DOPE | 6.33 | 6.20 |
| 34 | 7 | 40 | 43.5 | 15 | 1.5 | DSPC | 8.26 | 8.09 |
| 35 | 5 | 30 | 66 | 2.5 | 1.5 | DOPE | 12.40 | 12.15 |
| 36 | 5 | 40 | 56 | 2.5 | 1.5 | DOPE | 5.92 | 5.80 |
| 37 | 5 | 40 | 51 | 7.5 | 1.5 | DOPE | 7.08 | 6.93 |
| 38 | 3 | 40 | 56 | 2.5 | 1.5 | DOPE | 7.13 | 6.99 |
| 39 | 3 | 40 | 56 | 2.5 | 1.5 | DSPC | 9.15 | 8.97 |

## Claims

1. A nanoparticle composition, comprising a lipid ingredient, and optionally comprising a load;
wherein the lipid ingredient comprises the following components in the molar percentages:
Ionizable cationic lipids 20 mol%-85 mol%:
Structure lipids 10 mol%-75 mol%;
Neutral lipids 1.0 mol%-30 mol%;
Polymer lipids 0.25 mol%-10 mol%;
preferably, the lipid ingredient comprises the following components in the molar percentages:
Ionizable cationic lipids 25 mol%-65 mol%;
Structure lipids 25 mol%-70 mol%;
Neutral lipids 1 mol%-25 mol%;
Polymer lipids 0.5 mol%-8 mol%;
preferably, the lipid ingredient comprises the following components in the molar percentages:
Ionizable cationic lipids 30 mol%-60 mol%;
Structure lipids 27.5 mol%-66 mol%;
Neutral lipids 1.5 mol%-20 mol%;
Polymer lipids 1 mol%-5 mol%;
preferably, the lipid ingredient comprises the following components in the molar percentages:
Ionizable cationic lipids 30 mol%-50 mol%;
Structure lipids 30.5 mol%-66 mol%;
Neutral lipids 1.5 mol%-20 mol%;
Polymer lipids 1 mol%-5 mol%;
wherein,
the structure lipids are steroids;
the polymer lipids are polyethylene glycolated lipids;
the ionizable cationic lipid is the compound of formula (VI) or formula (VII), or a pharmaceutically acceptable salt, isotopic variant, tautomer or stereoisomer thereof, 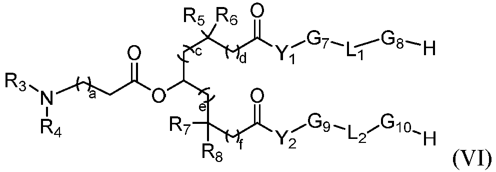 or 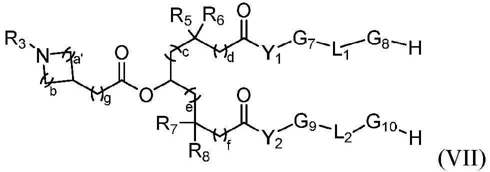 wherein,
a, a', b and g are independently 0, 1, 2, 3, 4 or 5, a' and b are not 0 at the same time;
a'+g=0, 1, 2, 3, 4 or 5;
c and e are independently 3, 4, 5, 6, 7, 8 or 9;
d and f are independently 0, 1, 2, 3 or 4;
c+d=3, 4, 5, 6, 7, 8 or 9, e+f=3, 4, 5, 6, 7, 8 or 9;
methylenes in 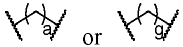 are optionally and independently substituted with 1, 2, 3, 4 or 5 C₁₋₆ alkyl;
R₃ and R₄ are independently H, C₁₋₆ alkyl, C₁₋₆ haloalkyl, 3- to 10-membered cycloalkyl or 3- to 10-membered heterocyclyl, which is optionally substituted with 1, 2, 3, 4 or 5 R*;
or, R₃ and R₄ are taken together with the N atom to which they are attached to form 3- to 10-membered heterocyclyl, which is optionally substituted with 1, 2, 3, 4 or 5 R*;
R* is independently H, halogen, cyano, C₁₋₆ alkyl, C₁₋₆ haloalkyl, -L_{b}-OR_{b} or -L_{b}-NR_{b}R'_{b};
R₅, R₆, R₇ and R₈ are independently C₁₋₃ alkyl;
Y₁ and Y₂ are independently O, S or NRₐ;
L₁ and L₂ are independently -(CRR')₂-, -CH=CH-, -C=C- or -NR"-;
G₇, G₈, G₉ and G₁₀ are independently a chemical bond or C₁₋₁₂ alkylene, which is optionally substituted with 1, 2, 3, 4, 5 or 6 R;
G₇ and G₈ have a total length of 6, 7, 8, 9, 10, 11 or 12 carbon atoms;
G₉ and G₁₀ have a total length of 6, 7, 8, 9, 10, 11 or 12 carbon atoms;
R and R' are independently H, C₁₋₁₄ alkyl, -Lₐ-ORₐ or -Lₐ-NRₐR'ₐ;
Lₐ is independently a chemical bond or C₁₋₁₄ alkylene;
L_{b} is independently a chemical bond or C₁₋₆ alkylene;
Rₐ and R'ₐ are independently H, C₁₋₁₄ alkyl, 3- to 10-membered cycloalkyl or 3- to 10-membered heterocyclyl;
R_{b} and R'_{b} are independently H, C₁₋₆ alkyl, 3- to 10-membered cycloalkyl or 3- to 10-membered heterocyclyl;
R" is independently H or C₁₋₁₄ alkyl;
alternatively, wherein in the compound of formula (VI) or formula (VII),
a is 0, 1, 2, 3 or 4, alternatively 1, 2, 3 or 4, alternatively 2, 3 or 4;
a' and b are independently 0, 1, 2, 3 or 4, alternatively 2;
g is 0, 1 or 2, alternatively 0 or 1;
a'+g=0, 1, 2, 3, 4 or 5, alternatively a'+g=2 or 3;
c and e are independently 3, 4, 5 or 6;
d and f are independently 0, 1 or 2;
c+d=4, 5 or 6, e+f=4, 5 or 6;
methylenes in 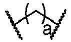 are optionally and independently substituted with 1, 2, 3, 4 or 5 C₁₋₆ alkyl;
methylenes in 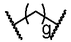 are optionally and independently substituted with 1 or 2 C₁₋₆ alkyl;
R₃ and R₄ are independently C₁₋₆ alkyl, which is optionally substituted with 1, 2 or 3 R*;
R* is independently H, C₁₋₆ alkyl, C₁₋₆ haloalkyl or -OR_{b};
or, R₃ and R₄ are taken together with the N atom to which they are attached to form 3- to 7-membered heterocyclyl, alternatively 5-membered heterocyclyl, which is optionally substituted with 1, 2 or 3 R*;
R₅, R₆, R₇ and R₈ are independently C₁₋₃ alkyl;
Y₁ and Y₂ are independently O, S or NRₐ, alternatively O or S;
L₁ and L₂ are independently -(CHR)₂-, -CH=CH-, -C=C- or -NR"-, alternatively -(CHR)₂-, -CH=CH- or -C=C-;
G₇ and G₉ are independently a chemical bond or C₁₋₆ alkylene;
G₈ and G₁₀ are independently C₁₋₁₀ alkylene;
G₇ and G₈ have a total length of 6, 7, 8, 9 or 10 carbon atoms;
G₉ and G₁₀ have a total length of 6, 7, 8, 9 or 10 carbon atoms;
1, 2 or 3 methylenes in G₇, G₈, G₉ or G₁₀ are optionally and independently substituted with 1 R;
R is independently H or C₁₋₈ alkyl;
R" is independently H or C₁₋₁₀ alkyl;
Rₐ is independently H or C₁₋₁₀ alkyl;
R_{b} is independently H or C₁₋₆ alkyl, alternatively H.

2. The nanoparticle composition of claim 1, wherein in the compound of formula (VI),
a is 0, 1, 2, 3 or 4, alternatively 1, 2, 3 or 4, alternatively 2, 3 or 4;
c and e are independently 3, 4, 5 or 6;
d and f are independently 0, 1 or 2;
c+d=4, 5 or 6, e+f=4, 5 or 6;
R₃ and R₄ are independently C₁₋₆ alkyl;
or, R₃ and R₄ are taken together with the N atom to which they are attached to form 4- to 6-membered heterocyclyl, alternatively 5-membered heterocyclyl, which is optionally substituted with 1, 2 or 3 R*;
R* is independently H, C₁₋₆ alkyl or C₁₋₆ haloalkyl;
R₅, R₆, R₇ and R₈ are independently C₁₋₃ alkyl;
Y₁ and Y₂ are independently O, S or NRₐ, alternatively O or S;
L₁ and L₂ are independently -(CHR)₂-, -CH=CH-, -C=C- or -NR"-, alternatively -(CHR)₂-, -CH=CH- or -C=C-;
G₇ and G₉ are independently a chemical bond or C₁₋₅ alkylene;
G₈ and G₁₀ are independently C₁₋₈ alkylene;
G₇ and G₈ have a total length of 6, 7, 8, 9 or 10 carbon atoms;
G₉ and G₁₀ have a total length of 6, 7, 8, 9 or 10 carbon atoms;
1, 2 or 3 methylenes in G₇, G₈, G₉ or G₁₀ are optionally and independently substituted with 1 R;
R is independently H or C₁₋₈ alkyl, alternatively H or C₁₋₇ alkyl, alternatively H or C₁₋₆ alkyl;
R" is independently H or C₇₋₉ alkyl;
Rₐ is independently H or C₈₋₁₀ alkyl;
alternatively, wherein in the compound of formula (VI),
a is 2, 3 or 4;
c and e are independently 3, 4, 5 or 6;
d and f are independently 0, 1 or 2;
c+d=4, 5 or 6, e+f=4, 5 or 6;
R₃ and R₄ are independently C₁₋₆ alkyl;
R₅, R₆, R₇ and R₈ are independently C₁₋₃ alkyl;
Y₁ and Y₂ are independently O or S;
L₁ and L₂ are independently -(CHR)₂-, -CH=CH- or -C=C-;
G₇ and G₉ are independently C₁₋₄ alkylene;
G₈ and G₁₀ are independently C₂₋₇ alkylene;
G₇ and G₈ have a total length of 6, 7 or 8 carbon atoms;
G₉ and G₁₀ have a total length of 6, 7 or 8 carbon atoms;
1, 2 or 3 methylenes in G₇, G₈, G₉ or G₁₀ are optionally and independently substituted with 1 R;
R is independently H or C₁₋₇ alkyl;
provided that, when L₁ is -C≡C-, then G₇ is C₁₋₂ alkylene, or when L₂ is -C≡C-, then G₉ is C₁₋₂ alkylene;
alternatively, wherein in the compound of formula (VI),
a is 2, 3 or 4, alternatively 2 or 3;
c and e are independently 4, 5 or 6;
d and f are 0;
R₃ and R₄ are independently C₁₋₆ alkyl;
R₅, R₆, R₇ and R₈ are independently C₁₋₃ alkyl, alternatively Me;
Y₁ and Y₂ are O;
L₁ and L₂ are independently -(CHR)₂- or -CH=CH-;
G₇ and G₉ are independently -CH₂- or -CH₂CHR-;
G₈ and G₁₀ are independently -(CH₂)₆- or -(CH₂)₇-;
G₇ and G₈ have a total length of 7 or 8 carbon atoms;
G₉ and G₁₀ have a total length of 7 or 8 carbon atoms;
1, 2 or 3 methylenes in G₈ or G₁₀ are optionally and independently substituted with 1 R;
R is independently H or C₄₋₆ alkyl, alternatively H or C₅ alkyl;
alternatively -G₇-L₁-G₈-H and -G₉-L₂-G₁₀-H are not -(CH₂)₉CH₃ at the same time;
alternatively, wherein in the compound of formula (VI),
a is 2;
c and e are independently 4, 5 or 6, alternatively 5;
d and f are 0;
R₃ and R₄ are independently C₁₋₆ alkyl;
R₅, R₆, R₇ and R₈ are independently C₁₋₃ alkyl, alternatively Me;
Y₁ and Y₂ are independently O or S;
one of L₁ and L₂ is -C=C-, the other is -(CHR)₂-, or both of L₁ and L₂ are -C≡C-; alternatively one of L₁ and L₂ is -C=C-, the other is -(CHR)₂-;
G₇ and G₉ are -CH₂-;
G₈ and G₁₀ are independently -(CH₂)₆- or -(CH₂)₇-;
1 methylene in G₈ or G₁₀ is optionally and independently substituted with 1 R, alternatively G₈ and G₁₀ are independently -CHR-(CH₂)₅-, -CHR-(CH₂)₆-, -CH₂-CHR-(CH₂)₄- or -(CH₂)₂-CHR-(CH₂)₄-;
R is independently H or C₄₋₆ alkyl, alternatively H or C₅ alkyl;
provided that, only one of the -G₇-L₁-G₈-H and -G₉-L₂-G₁₀-H is substituted with one non-hydrogen R substituent and the other is unsubstituted;
alternatively, wherein in the compound of formula (VI),
a is 2;
c and e are independently 4, 5 or 6, alternatively 5;
d and f are 0;
R₃ and R₄ are independently C₁₋₃ alkyl, alternatively Me;
R₅, R₆, R₇ and R₈ are independently C₁₋₃ alkyl, alternatively Me;
Y₁ and Y₂ are independently O or S, alternatively O;
both of L₁ and L₂ are -C=C-;
G₇ and G₉ are -CH₂-;
G₈ and G₁₀ are independently -(CH₂)₆- or -(CH₂)₇-, alternatively -(CH₂)₇-;
alternatively, wherein in the compound of formula (VI),
a is 2;
c and e are 3;
d and f are 2;
R₃ and R₄ are independently C₁₋₃ alkyl, alternatively Me;
R₅, R₆, R₇ and R₈ are independently C₁₋₃ alkyl, alternatively Me;
Y₁ and Y₂ are independently O or S, alternatively O;
L₁ and L₂ are -(CHR)₂-;
G₇ and G₉ are independently -CH₂- or -CH₂CHR-;
G₈ and G₁₀ are independently -(CH₂)₅-, -(CH₂)₆- or -(CH₂)₇-;
G₇ and G₈ have a total length of 6, 7 or 8 carbon atoms, alternatively 7 carbon atoms;
G₉ and G₁₀ have a total length of 6, 7 or 8 carbon atoms, alternatively 7 carbon atoms;
1, 2 or 3 methylenes in G₈ or G₁₀ are optionally and independently substituted with 1 R;
R is independently H or C₁₋₇ alkyl, alternatively H or C₁₋₆ alkyl, alternatively Me; alternatively, wherein in the compound of formula (VI),
a is 2;
c and e are 4, 5, or 6, alternatively 5;
d and f are 0;
R₃ and R₄ are independently C₁₋₆ alkyl;
R₅, R₆, R₇ and R₈ are independently C₁₋₃ alkyl, alternatively Me;
Y₁ and Y₂ are S;
L₁ and L₂ are -(CHR)₂-;
G₇ and G₉ are independently -CH₂- or -CH₂CHR-;
G₈ and G₁₀ are independently -(CH₂)₅-, -(CH₂)₆- or -(CH₂)₇-;
G₇ and G₈ have a total length of 7 or 8 carbon atoms, alternatively 8 carbon atoms;
G₉ and G₁₀ have a total length of 7 or 8 carbon atoms, alternatively 8 carbon atoms;
1, 2 or 3 methylenes in G₈ or G₁₀ are optionally and independently substituted with 1 R;
R is independently H or C₄₋₆ alkyl, alternatively H or C₅ alkyl.

3. The nanoparticle composition of claim 1, wherein in the compound of formula (VII),
a' and b are 2;
g is 0 or 1;
c and e are 5;
d and f are 0;
R₃ is C₁₋₆ alkyl, which is optionally substituted with 1, 2 or 3 R*;
R* is independently H, C₁₋₆ alkyl, C₁₋₆ haloalkyl or -OR_{b}, alternatively H, C₁₋₆ alkyl or C₁₋₆ haloalkyl;
R₅, R₆, R₇ and R₈ are independently C₁₋₃ alkyl, alternatively Me;
Y₁ and Y₂ are independently O or S;
L₁ and L₂ are -(CHR)₂-;
G₇ and G₉ are independently -CH₂- or -CH₂CHR-;
G₈ and G₁₀ are independently -(CH₂)₅-, -(CH₂)₆- or -(CH₂)₇-;
G₇ and G₈ have a total length of 6, 7 or 8 carbon atoms;
G₉ and G₁₀ have a total length of 6, 7 or 8 carbon atoms;
1, 2 or 3 methylenes in G₈ or G₁₀ are optionally and independently substituted with 1 R;
R is independently H or C₄₋₆ alkyl;
R_{b} is independently H or C₁₋₆ alkyl, alternatively H;
alternatively, wherein in the compound of formula (VII),
R₃ is Me or -CH₂CH₃, alternatively Me;
both of Y₁ and Y₂ are O;
G₇ and G₈ have a total length of 6 or 7 carbon atoms, alternatively 7 carbon atoms;
G₉ and G₁₀ have a total length of 6 or 7 carbon atoms, alternatively 7 carbon atoms; alternatively, wherein in the compound of formula (VII),
R₃ is Me or -CH₂CH₃;
Y₁ and Y₂ are independently O or S, where Y₁ and Y₂ are not O at the same time;
G₇ and G₈ have a total length of 6, 7 or 8 carbon atoms;
G₉ and G₁₀ have a total length of 6, 7 or 8 carbon atoms; alternatively, wherein in the compound of formula (VII),
g is 0 or 1, alternatively 1;
R₃ is Me or -CH₂CH₃, alternatively Me;
one of Y₁ and Y₂ is O, and the other is S;
G₇ and G₈ have a total length of 7 carbon atoms;
G₉ and G₁₀ have a total length of 7 carbon atoms; alternatively, wherein in the compound of formula (VII),
g is 0 or 1, alternatively 0;
R₃ is Me or -CH₂CH₃;
both of Y₁ and Y₂ are S;
G₇ and G₈ have a total length of 7 or 8 carbon atoms;
G₉ and G₁₀ have a total length of 7 or 8 carbon atoms.

4. The nanoparticle composition of claim 1, wherein the ionizable cationic lipid is selected from the following:

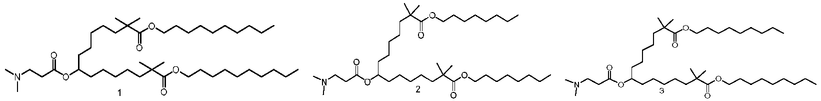

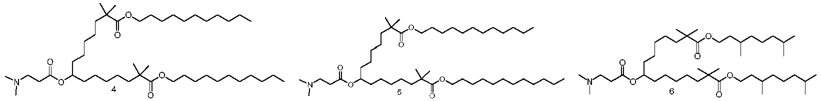

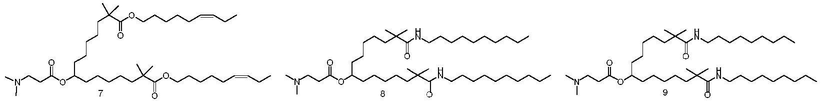

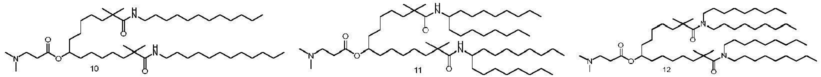

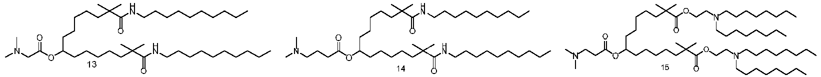

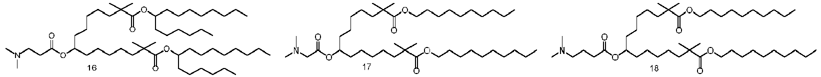

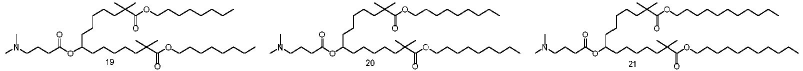

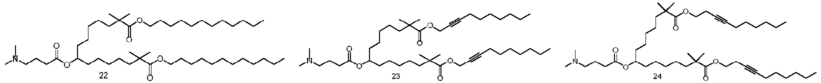

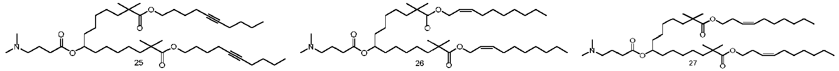

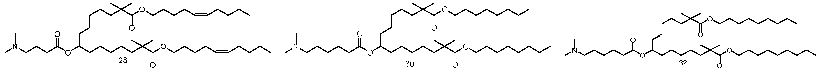

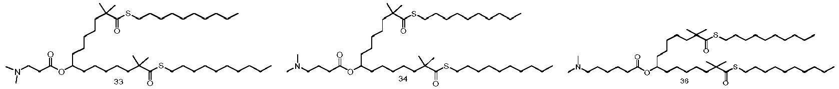

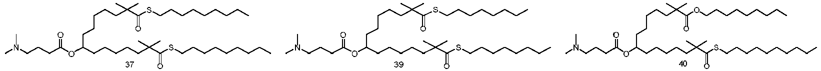

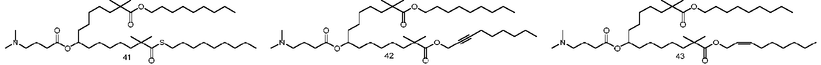

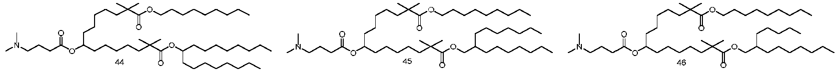

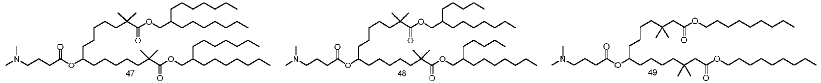

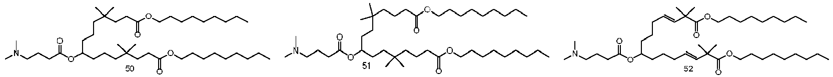

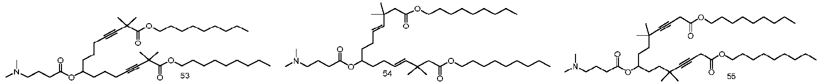

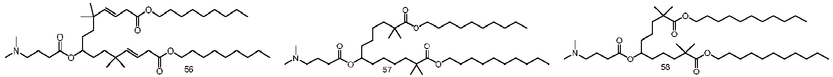

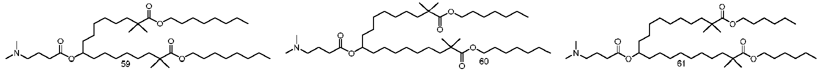

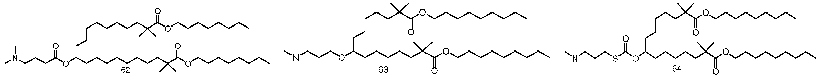

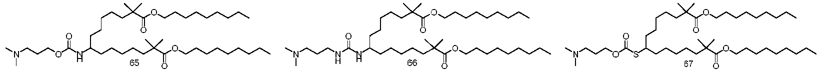

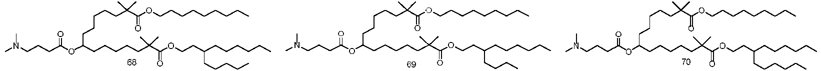

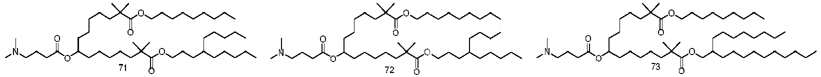

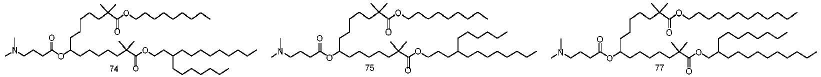

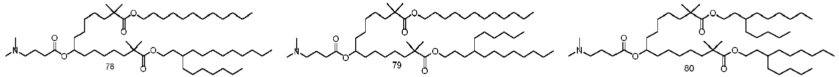

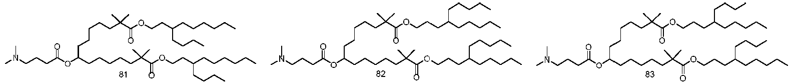

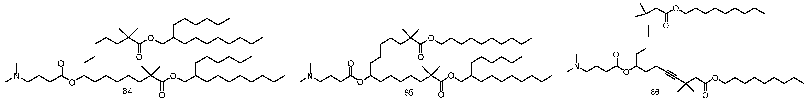

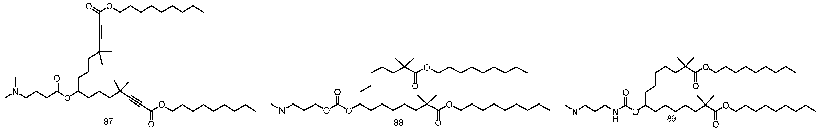

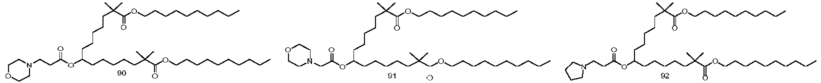

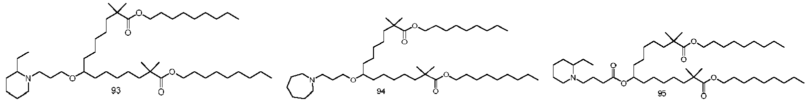

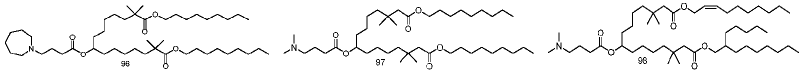

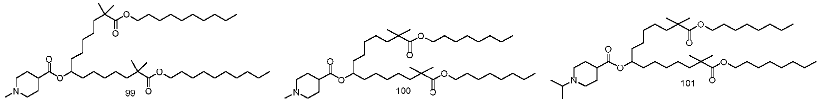

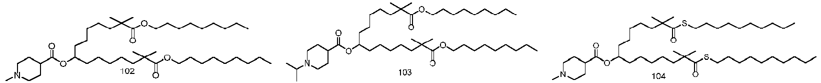

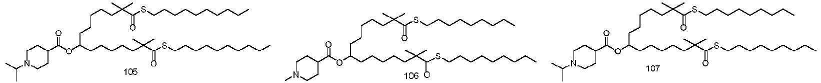

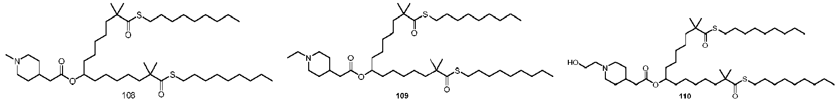

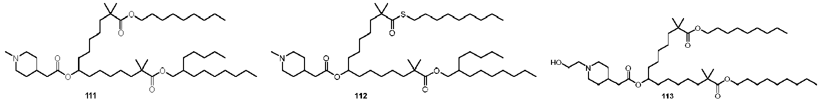

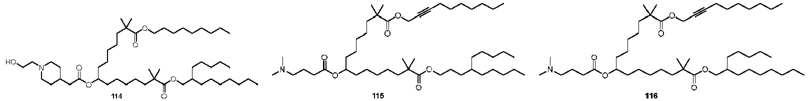

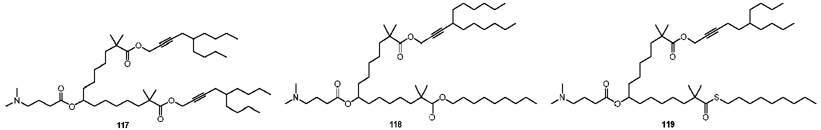

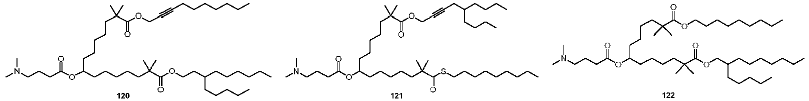

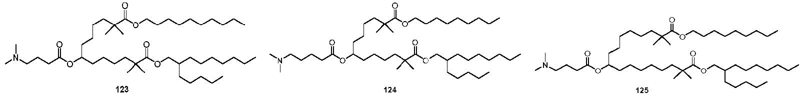

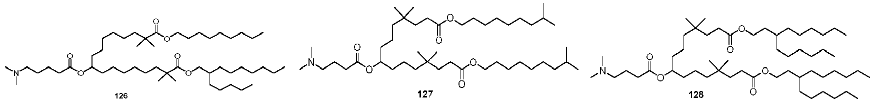

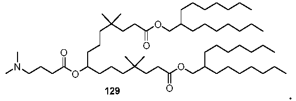

5. The nanoparticle composition of any one of claims 1-4, wherein, the load is a nucleic acid; the N:P molar ratio of N atoms in the ionizable cationic lipids to P atoms in the load molecules is 1-15:1, alternatively 3-12:1, alternatively 3-7:1, alternatively 6-12:1, more alternatively 3-6: 1;
alternatively, the N:P molar ratio of N atoms in the ionizable cationic lipids to P atoms in the load molecules is 1-10:1, alternatively 3-6:1, more alternatively 3-5:1;
alternatively, the N:P molar ratio of N atoms in the ionizable cationic lipids to P atoms in the load molecules is 1-15:1, alternatively 3-12:1, more alternatively 6-12:1;
alternatively, the N:P molar ratio of N atoms in the ionizable cationic lipids to P atoms in the load molecules is 1-10:1, alternatively 3-7:1, more alternatively 5:1.

6. The nanoparticle composition of any one of claims 1-5, wherein, the particle size of the particles is 65-200 nm, alternatively 65-180 nm, alternatively 70-170nm, more alternatively 70-130 nm;
alternatively, the particle size of the particles is 70-180 nm, alternatively 80-180 nm, alternatively 90-180 nm, more alternatively 100-135 nm;
alternatively, the particle size of the particles is 65-160 nm, alternatively 65-150 nm, alternatively 70-150 nm, alternatively 90-130 nm, more alternatively 90-115 nm;
alternatively, the particle size of the particles is 65-140 nm, alternatively 65-130 nm, alternatively 70-130 nm, more alternatively 65-75 nm.

7. The nanoparticle composition of any one of claims 1-6, wherein the lipid ingredient comprises the following components in the molar percentages:
Ionizable cationic lipids 30 mol%-55 mol%;
Structure lipids 28 mol%-64 mol%;
Neutral lipids 5 mol%-20 mol%;
Polymer lipids 1 mol%-3 mol%;
preferably, the lipid ingredient comprises the following components in the molar percentages:
Ionizable cationic lipids 40 mol%-52.5 mol%, alternatively 42.5 mol%-50 mol%;
Structure lipids 28 mol%-54 mol%, alternatively 30.6 mol%-51 mol%;
Neutral lipids 5 mol%-20 mol%;
Polymer lipids 1 mol%-3 mol%, alternatively 1 mol%-2 mol%;
wherein,
the structure lipids are steroids;
the polymer lipids are polyethylene glycolated lipids;
the ionizable cationic lipid is as defined in claim 4, alternatively is: 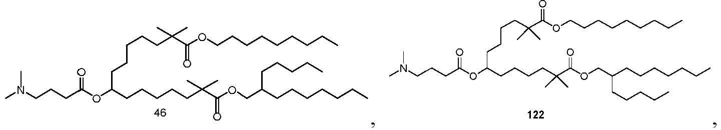 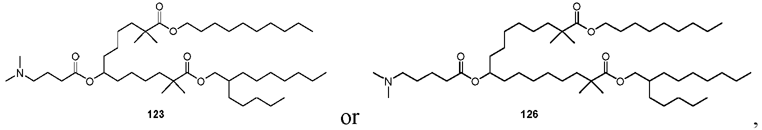 most alternatively is:

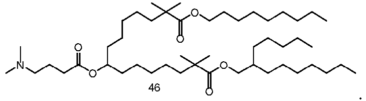

8. The nanoparticle composition of any one of claims 1-6, wherein the lipid ingredient comprises the following components in the molar percentages:
Ionizable cationic lipids 30 mol%-60 mol%;
Structure lipids 27.5 mol%-66 mol%;
Neutral lipids 1.5 mol%-20 mol%;
Polymer lipids 1.5 mol%-5 mol%;
preferably, the lipid ingredient comprises the following components in the molar percentages:
Ionizable cationic lipids 30 mol%-50 mol%;
Structure lipids 35 mol%-66 mol%;
Neutral lipids 1.5 mol%-20 mol%;
Polymer lipids 1.5 mol%-5 mol%;
preferably, the lipid ingredient comprises the following components in the molar percentages:
Ionizable cationic lipids 30 mol%-50 mol%;
Structure lipids 38.5 mol%-63.5 mol%;
Neutral lipids 1.5 mol%-10 mol%;
Polymer lipids 1.5 mol%-4 mol%;
preferably, the lipid ingredient comprises the following components in the molar percentages:
Ionizable cationic lipids 30 mol%-50 mol%, alternatively 30 mol%-48.5 mol%;
Structure lipids 43 mol%-60 mol%, alternatively 45.5 mol%-57.5 mol%;
Neutral lipids 1.5 mol%-10 mol%;
Polymer lipids 1.5 mol%-4 mol%, alternatively 1.5 mol%-3.5 mol%;
wherein,
the structure lipids are steroids;
the polymer lipids are polyethylene glycolated lipids;
the ionizable cationic lipid is as defined in claim 4, or is 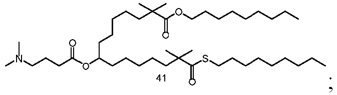 alternatively is: 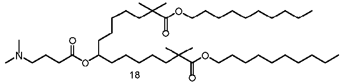 or 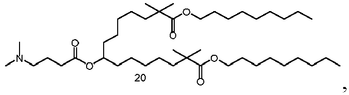 most alternatively is:

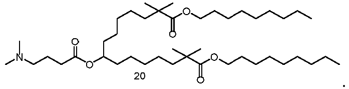

9. The nanoparticle composition of any one of claims 1-6, wherein the lipid ingredient comprises the following components in the molar percentages:
Ionizable cationic lipids 27.5 mol%-55 mol%;
Structure lipids 35 mol%-68.5 mol%;
Neutral lipids 1.5 mol%-20 mol%;
Polymer lipids 1 mol%-5 mol%;
preferably, the lipid ingredient comprises the following components in the molar percentages:
Ionizable cationic lipids 27.5 mol%-42.5 mol%, alternatively 30 mol%-40 mol%;
Structure lipids 41 mol%-68.5 mol%, alternatively 43.5 mol%-66 mol%;
Neutral lipids 2 mol%-18 mol%, alternatively 2.5 mol%-15 mol%;
Polymer lipids 1 mol%-4 mol%, alternatively 1.5 mol%-3.5 mol%;
wherein,
the structure lipids are steroids;
the polymer lipids are polyethylene glycolated lipids;
the ionizable cationic lipid is as defined in claim 4, alternatively is: 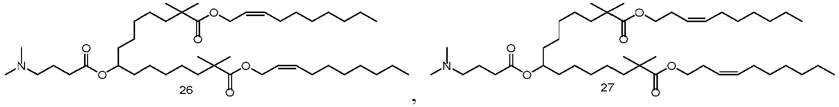 or 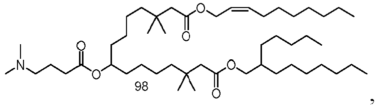
most alternatively is:

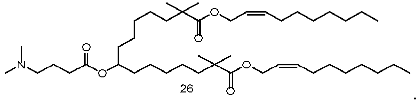

10. The nanoparticle composition of any one of claims 1-9, wherein, the neutral lipids are selected from one or more of DSPC, DMPC, DOPC, DPPC, POPC, DOPE, DMPE, POPE and DPPE, alternatively DSPC and/or DOPE;
alternatively, the structure lipids are selected from one or more of cholesterol, sitosterol, coprosterol, fucosterol, brassicasterol, ergosterol, tomatine, ursolic acid, α-tocopherol, stigmasterol, avenasterol, ergocalciferol and campesterol, alternatively cholesterol and/or β-sitosterol, more preferably cholesterol;
and/or, wherein, the polyethylene glycolated lipids are selected from one or more of PEG modified phosphatidylethanolamine, PEG modified phosphatidic acid, PEG modified ceramide, PEG modified dialkyl amine, PEG modified diacylglycerol and PEG modified dialkylglycerol;
alternatively, the polyethylene glycolated lipids contain a PEG moiety of about 1000 Da to about 20 kDa, alternatively contain a PEG moiety of about 1000 Da to about 5000 Da;
alternatively, the polyethylene glycolated lipids are selected from one or more of DMPE-PEG1000, DPPE-PEG1000, DSPE-PEG1000, DOPE-PEG1000, DMG-PEG2000, Ceramide-PEG2000, DMPE-PEG2000, DPPE-PEG2000, DSPE-PEG2000, Azido-PEG2000, DSPE-PEG2000-Mannose, Ceramide-PEG5000, and DSPE-PEG5000, alternatively DMG-PEG2000.

11. The nanoparticle composition of any one of claims 1-4, wherein, the load is selected from one or more of therapeutic, prophylactic and diagnostic agents;
alternatively, the therapeutic, prophylactic or diagnostic agent is a nucleic acid;
alternatively, the nucleic acid is one or more of ASO, RNA or DNA;
alternatively, the RNA is selected from one or more of small interfering RNA (siRNA), short hairpin RNA (shRNA), antisense RNA (aRNA), messenger RNA (mRNA), modified messenger RNA (mmRNA), long non-coding RNA (lncRNA), microRNA (miRNA), small activating RNA (saRNA), multimeric coding nucleic acid (MCNA), polymeric coding nucleic acid (PCNA), guide RNA (gRNA), CRISPRRNA (crRNA) and nucleases, alternatively mRNA, more alternatively, modified mRNA.

12. A pharmaceutical composition, comprising the nanoparticle composition of any one of claims 1-11, and pharmaceutically acceptable excipient(s).

13. The nanoparticle composition of any one of claims 1-11, or the pharmaceutical composition of claim 12, for use in treating, diagnosing, or preventing a disease, or delivering a load;
wherein, the load is one or more of therapeutic, prophylactic or diagnostic agents.

## Patentansprüche

1. Nanopartikelzusammensetzung, umfassend einen Lipidbestandteil und optional umfassend eine Ladung;
wobei der Lipidbestandteil die folgenden Komponenten in den Molprozenten umfasst:
ionisierbare kationische Lipide 20 Mol-% bis 85 Mol-%;
Strukturlipide 10 Mol-% bis 75 Mol-%;
neutrale Lipide 1,0 Mol-% bis 30 Mol-%;
Polymerlipide 0,25 Mol-% bis 10 Mol-%;
vorzugsweise wobei der Lipidbestandteil die folgenden Komponenten in den Molprozenten umfasst:
ionisierbare kationische Lipide 25 Mol-% bis 65 Mol-%;
Strukturlipide 25 Mol-% bis 70 Mol-%;
neutrale Lipide 1 Mol-% bis 25 Mol-%;
Polymerlipide 0,5 Mol-% bis 8 Mol-%;
vorzugsweise wobei der Lipidbestandteil die folgenden Komponenten in den Molprozenten umfasst:
ionisierbare kationische Lipide 30 Mol-% bis 60 Mol-%;
Strukturlipide 27,5 Mol-% bis 66 Mol %;
neutrale Lipide 1,5 Mol-% bis 20 Mol-%;
Polymerlipide 1 Mol-% bis 5 Mol-%;
vorzugsweise wobei der Lipidbestandteil die folgenden Komponenten in den Molprozenten umfasst:
ionisierbare kationische Lipide 30 Mol-% bis 50 Mol-%;
Strukturlipide 30,5 Mol-% bis 66 Mol-%;
neutrale Lipide 1,5 Mol-% bis 20 Mol-%;
Polymerlipide 1 Mol-% bis 5 Mol-%;
wobei
die Strukturlipide Steroide sind;
die Polymerlipide Polyethylenglykol-Lipide sind;
das ionisierbare kationische Lipid die Verbindung von Formel (VI) oder Formel (VII) oder ein pharmazeutisch annehmbares Salz, eine Isotopenvariante, ein Tautomer oder ein Stereoisomer davon ist, 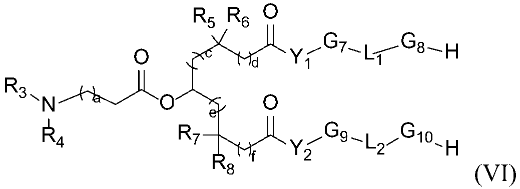 oder 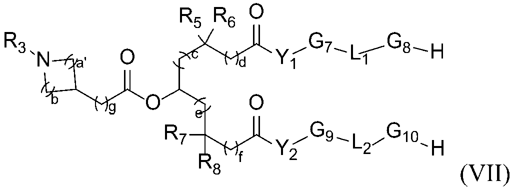 wobei
a, a', b und g unabhängig 0, 1, 2, 3, 4 oder 5 sind, a' und b nicht gleichzeitig 0 sind;
a' + g = 0, 1, 2, 3, 4 oder 5;
c und e unabhängig 3, 4, 5, 6, 7, 8 oder 9 sind;
d und f unabhängig 0, 1, 2, 3 oder 4 sind;
c + d = 3, 4, 5, 6, 7, 8 oder 9, e + f = 3, 4, 5, 6, 7, 8 oder 9;
Methylene in 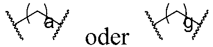 optional und unabhängig mit 1, 2, 3, 4 oder 5 C₁₋₆-Alkyl substituiert sind;
R₃ und R₄ unabhängig H, C₁₋₆-Alkyl, C₁₋₆-Halogenalkyl, 3- bis 10-gliedriges Cycloalkyl oder 3- bis 10-gliedriges Heterocyclyl, das optional mit 1, 2, 3, 4 oder 5 R* substituiert ist, sind;
oder R₃ und R₄ mit dem N-Atom zusammengenommen werden, an das sie angelagert sind, um ein 3- bis 10-gliedriges Heterocyclyl zu bilden, das optional mit 1, 2, 3, 4 oder 5 R* substituiert ist;
R* unabhängig H, Halogen, Cyano, C₁₋₆-Alkyl, C₁₋₆-Halogenalkyl, -L_{b}-OR_{b} oder -L_{b}-NR_{b}R'_{b} ist;
R₅, R₆, R₇ und R₈ unabhängig C₁₋₃-Alkyl sind;
Y₁ und Y₂ unabhängig O, S oder NRₐ sind;
L₁ und L₂ unabhängig -(CRR')₂-, -CH=CH-, -C≡C- oder -NR"- sind;
G₇, G₈, G₉ und G₁₀ unabhängig eine chemische Bindung oder C₁₋₁₂-Alkylen, das optional mit 1, 2, 3, 4, 5 oder 6 R substituiert ist, sind;
G₇ und G₈ eine Gesamtlänge von 6, 7, 8, 9, 10, 11 oder 12 Kohlenstoffatomen aufweisen;
G₉ und G₁₀ eine Gesamtlänge von 6, 7, 8, 9, 10, 11 oder 12 Kohlenstoffatomen aufweisen;
R und R' unabhängig H, C₁₋₁₄-Alkyl, -Lₐ-ORₐ oder -Lₐ-NRₐR'ₐ sind;
Lₐ unabhängig eine chemische Bindung oder C₁₋₁₄-Alkylen ist;
L_{b} unabhängig eine chemische Bindung oder C₁₋₆-Alkylen ist;
Rₐ und R'ₐ unabhängig H, C₁₋₁₄-Alkyl, 3- bis 10-gliedriges Cycloalkyl oder 3- bis 10-gliedriges Heterocyclyl sind;
R_{b} und R'_{b} unabhängig H, C₁₋₆-Alkyl, 3- bis 10-gliedriges Cycloalkyl oder 3- bis 10-gliedriges Heterocyclyl sind;
R" unabhängig H oder C₁₋₁₄-Alkyl ist;
alternativ wobei in der Verbindung von Formel (VI) oder Formel (VII)
a 0, 1, 2, 3 oder 4, alternativ 1, 2, 3 oder 4, alternativ 2, 3 oder 4 ist;
a' und b unabhängig 0, 1, 2, 3 oder 4, alternativ 2 sind;
g 0, 1 oder 2, alternativ 0 oder 1 ist;
a' + g = 0, 1, 2, 3, 4 oder 5, alternativ a' + g = 2 oder 3;
c und e unabhängig 3, 4, 5 oder 6 sind;
d und f unabhängig 0, 1 oder 2 sind;
c + d = 4, 5 oder 6, e + f = 4, 5 oder 6;
Methylene in 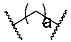 optional und unabhängig mit 1, 2, 3, 4 oder 5 C₁₋₆-Alkyl substituiert sind;
Methylene in 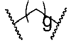 optional und unabhängig mit 1 oder 2 C₁₋₆-Alkyl substituiert sind;
R₃ und R₄ unabhängig C₁₋₆-Alkyl sind, das optional mit 1, 2 oder 3 R* substituiert ist;
R* unabhängig H, C₁₋₆-Alkyl, C₁₋₆-Halogenalkyl oder -OR_{b} ist;
oder R₃ und R₄ mit dem N-Atom zusammengenommen werden, an das sie angelagert sind, um ein 3- bis 7-gliedriges Heterocyclyl, alternativ 5-gliedriges Heterocyclyl zu bilden, das optional mit 1, 2 oder 3 R* substituiert ist;
R₅, R₆, R₇ und R₈ unabhängig C₁₋₃-Alkyl sind;
Y₁ und Y₂ unabhängig O, S oder NRₐ, alternativ O oder S sind;
L₁ und L₂ unabhängig -(CHR)₂-, -CH=CH-, -C≡C- oder -NR"-, alternativ -(CHR)₂-, -CH=CH- oder -C≡C- sind;
G₇ und G₉ unabhängig eine chemische Bindung oder C₁₋₆-Alkylen sind;
G₈ und G₁₀ unabhängig C₁₋₁₀-Alkylen sind;
G₇ und G₈ eine Gesamtlänge von 6, 7, 8, 9 oder 10 Kohlenstoffatomen aufweisen;
G₉ und G₁₀ eine Gesamtlänge von 6, 7, 8, 9 oder 10 Kohlenstoffatomen aufweisen;
1, 2 oder 3 Methylene in G₇, G₈, G₉ oder G₁₀ optional und unabhängig mit 1 R substituiert sind;
R unabhängig H oder C₁₋₈-Alkyl ist;
R" unabhängig H oder C₁₋₁₀-Alkyl ist;
Rₐ unabhängig H oder C₁₋₁₀-Alkyl ist;
R_{b} unabhängig H oder C₁₋₆-Alkyl, alternativ H ist.

2. Nanopartikelzusammensetzung nach Anspruch 1, wobei in der Verbindung von Formel (VI)
a 0, 1, 2, 3 oder 4, alternativ 1, 2, 3 oder 4, alternativ 2, 3 oder 4 ist;
c und e unabhängig 3, 4, 5 oder 6 sind;
d und f unabhängig 0, 1 oder 2 sind;
c + d = 4, 5 oder 6, e + f = 4, 5 oder 6;
R₃ und R₄ unabhängig C₁₋₆-Alkyl sind;
oder R₃ und R₄ mit dem N-Atom zusammengenommen werden, an das sie angelagert sind, um ein 4- bis 6-gliedriges Heterocyclyl, alternativ 5-gliedriges Heterocyclyl zu bilden, das optional mit 1, 2 oder 3 R* substituiert ist;
R* unabhängig H, C₁₋₆-Alkyl oder C₁₋₆-Halogenalkyl ist;
R₅, R₆, R₇ und R₈ unabhängig C₁₋₃-Alkyl sind;
Y₁ und Y₂ unabhängig O, S oder NRₐ, alternativ O oder S sind;
L₁ und L₂ unabhängig -(CHR)₂-, -CH=CH-, -C≡C- oder -NR"-, alternativ -(CHR)₂-, -CH=CH- oder -C≡C- sind;
G₇ und G₉ unabhängig eine chemische Bindung oder C₁₋₅-Alkylen sind;
G₈ und G₁₀ unabhängig C₁₋₈-Alkylen sind;
G₇ und G₈ eine Gesamtlänge von 6, 7, 8, 9 oder 10 Kohlenstoffatomen aufweisen;
G₉ und G₁₀ eine Gesamtlänge von 6, 7, 8, 9 oder 10 Kohlenstoffatomen aufweisen;
1, 2 oder 3 Methylene in G₇, G₈, G₉ oder G₁₀ optional und unabhängig mit 1 R substituiert sind;
R unabhängig H oder C₁₋₈-Alkyl, alternativ H oder C₁₋₇-Alkyl, alternativ H oder C₁₋₆-Alkyl ist;
R" unabhängig H oder C₇₋₉-Alkyl ist;
Rₐ unabhängig H oder C₈₋₁₀-Alkyl ist;
alternativ wobei in der Verbindung von Formel (VI)
a 2, 3 oder 4 ist;
c und e unabhängig 3, 4, 5 oder 6 sind;
d und f unabhängig 0, 1 oder 2 sind;
c + d = 4, 5 oder 6, e + f = 4, 5 oder 6;
R₃ und R₄ unabhängig C₁₋₆-Alkyl sind;
R₅, R₆, R₇ und R₈ unabhängig C₁₋₃-Alkyl sind;
Y₁ und Y₂ unabhängig O oder S sind;
L₁ und L₂ unabhängig -(CHR)₂-, -CH=CH- oder -C≡C- sind;
G₇ und G₉ unabhängig C₁₋₄-Alkylen sind;
G₈ und G₁₀ unabhängig C₂₋₇-Alkylen sind;
G₇ und G₈ eine Gesamtlänge von 6, 7 oder 8 Kohlenstoffatomen aufweisen;
G₉ und G₁₀ eine Gesamtlänge von 6, 7 oder 8 Kohlenstoffatomen aufweisen;
1, 2 oder 3 Methylene in G₇, G₈, G₉ oder G₁₀ optional und unabhängig mit 1 R substituiert sind;
R unabhängig H oder C₁₋₇-Alkyl ist;
vorausgesetzt, dass wenn L₁ -C≡C- ist, dann G₇ C₁₋₂-Alkylen ist, oder wenn L₂-C≡C- ist, dann G₉ C₁₋₂-Alkylen ist;
alternativ wobei in der Verbindung von Formel (VI)
a 2, 3 oder 4, alternativ 2 oder 3 ist;
c und e unabhängig 4, 5 oder 6 sind;
d und f 0 sind;
R₃ und R₄ unabhängig C₁₋₆-Alkyl sind;
R₅, R₆, R₇ und R₈ unabhängig C₁₋₃-Alkyl, alternativ Me sind;
Y₁ und Y₂ O sind;
L₁ und L₂ unabhängig -(CHR)₂- oder -CH=CH- sind;
G₇ und G₉ unabhängig -CH₂- oder -CH₂CHR- sind;
G₈ und G₁₀ unabhängig -(CH₂)₆- oder -(CH₂)₇- sind;
G₇ und G₈ eine Gesamtlänge von 7 oder 8 Kohlenstoffatomen aufweisen;
G₉ und G₁₀ eine Gesamtlänge von 7 oder 8 Kohlenstoffatomen aufweisen;
1, 2 oder 3 Methylene in G₈ oder G₁₀ optional und unabhängig mit 1 R substituiert sind;
R unabhängig H oder C₄₋₆-Alkyl, alternativ H oder C₅-Alkyl ist;
alternativ -G₇-L₁-G₈-H und -G₉-L₂-G₁₀-H nicht gleichzeitig -(CH₂)₉CH₃ sind;
alternativ wobei in der Verbindung von Formel (VI)
a 2 ist;
c und e unabhängig 4, 5 oder 6, alternativ 5 sind;
d und f 0 sind;
R₃ und R₄ unabhängig C₁₋₆-Alkyl sind;
R₅, R₆, R₇ und R₈ unabhängig C₁₋₃-Alkyl, alternativ Me sind;
Y₁ und Y₂ unabhängig O oder S sind;
eines von L₁ und L₂ -C≡C- ist, das andere -(CHR)₂- ist, oder beide von L₁ und L₂ -C≡C- sind;
alternativ eines von L₁ und L₂ -C≡C- ist, das andere -(CHR)₂- ist;
G₇ und G₉ -CH₂- sind;
G₈ und G₁₀ unabhängig -(CH₂)₆- oder -(CH₂)₇- sind;
1 Methylen in G₈ oder G₁₀ optional und unabhängig mit 1 R substituiert ist, alternativ G₈ und G₁₀ unabhängig -CHR-(CH₂)₅-, -CHR-(CH₂)₆-, -CH₂-CHR-(CH₂)₄- oder -(CH₂)₂-CHR-(CH₂)₄- substituiert sind;
R unabhängig H oder C₄₋₆-Alkyl, alternativ H oder C₅-Alkyl ist;
vorausgesetzt, dass nur eines von -G₇-L₁-G₈-H und -G₉-L₂-G₁₀-H mit einem Nicht-Wasserstoff-R-Substituenten substituiert ist und das andere unsubstituiert ist;
alternativ wobei in der Verbindung von Formel (VI)
a 2 ist;
c und e unabhängig 4, 5 oder 6, alternativ 5 sind;
d und f 0 sind;
R₃ und R₄ unabhängig C₁₋₃-Alkyl, alternativ Me sind;
R₅, R₆, R₇ und R₈ unabhängig C₁₋₃-Alkyl, alternativ Me sind;
Y₁ und Y₂ unabhängig O oder S, alternativ O sind;
beide von L₁ und L₂ -C≡C- sind;
G₇ und G₉ -CH₂- sind;
G₈ und G₁₀ unabhängig -(CH₂)₆- oder -(CH₂)₇-, alternativ -(CH₂)₇- sind;
alternativ wobei in der Verbindung von Formel (VI)
a 2 ist;
c und e 3 sind;
d und f 2 sind;
R₃ und R₄ unabhängig C₁₋₃-Alkyl, alternativ Me sind;
R₅, R₆, R₇ und R₈ unabhängig C₁₋₃-Alkyl, alternativ Me sind;
Y₁ und Y₂ unabhängig O oder S, alternativ O sind;
L₁ und L₂ (CHR)₂- sind;
G₇ und G₉ unabhängig -CH₂- oder -CH₂CHR- sind;
G₈ und G₁₀ unabhängig -(CH₂)₅-, -(CH₂)₆- oder -(CH₂)₇- sind;
G₇ und G₈ eine Gesamtlänge von 6, 7 oder 8 Kohlenstoffatomen, alternativ 7 Kohlenstoffatomen aufweisen;
G₉ und G₁₀ eine Gesamtlänge von 6, 7 oder 8 Kohlenstoffatomen, alternativ 7 Kohlenstoffatomen aufweisen;
1, 2 oder 3 Methylene in G₈ oder G₁₀ optional und unabhängig mit 1 R substituiert sind;
R unabhängig H oder C₁₋₇-Alkyl, alternativ H oder C₁₋₆-Alkyl, alternativ Me ist;
alternativ wobei in der Verbindung von Formel (VI)
a 2 ist;
c und e 4, 5, oder 6, alternativ 5 sind;
d und f 0 sind;
R₃ und R₄ unabhängig C₁₋₆-Alkyl sind;
R₅, R₆, R₇ und R₈ unabhängig C₁₋₃-Alkyl, alternativ Me sind;
Y₁ und Y₂ S sind;
L₁ und L₂ (CHR)₂- sind;
G₇ und G₉ unabhängig -CH₂- oder -CH₂CHR- sind;
G₈ und G₁₀ unabhängig -(CH₂)₅-, -(CH₂)₆- oder -(CH₂)₇- sind;
G₇ und G₈ eine Gesamtlänge von 7 oder 8 Kohlenstoffatomen, alternativ 8 Kohlenstoffatomen aufweisen;
G₉ und G₁₀ eine Gesamtlänge von 7 oder 8 Kohlenstoffatomen, alternativ 8 Kohlenstoffatomen aufweisen;
1, 2 oder 3 Methylene in G₈ oder G₁₀ optional und unabhängig mit 1 R substituiert sind;
R unabhängig H oder C₄₋₆-Alkyl, alternativ H oder C₅-Alkyl ist.

3. Nanopartikelzusammensetzung nach Anspruch 1, wobei in der Verbindung von Formel (VII)
a' und b 2 sind;
g 0 oder 1 ist;
c und e 5 sind;
d und f 0 sind;
R₃ C₁₋₆-Alkyl ist, das optional mit 1, 2 oder 3 R* substituiert ist;
R* unabhängig H, C₁₋₆-Alkyl, C₁₋₆-Halogenalkyl oder -OR_{b}, alternativ H, C₁₋₆-Alkyl oder C₁₋₆-Halogenalkyl ist;
R₅, R₆, R₇ und R₈ unabhängig C₁₋₃-Alkyl, alternativ Me sind;
Y₁ und Y₂ unabhängig O oder S sind;
L₁ und L₂ (CHR)₂- sind;
G₇ und G₉ unabhängig -CH₂- oder -CH₂CHR- sind;
G₈ und G₁₀ unabhängig -(CH₂)₅-, -(CH₂)₆- oder -(CH₂)₇- sind;
G₇ und G₈ eine Gesamtlänge von 6, 7 oder 8 Kohlenstoffatomen aufweisen;
G₉ und G₁₀ eine Gesamtlänge von 6, 7 oder 8 Kohlenstoffatomen aufweisen;
1, 2 oder 3 Methylene in G₈ oder G₁₀ optional und unabhängig mit 1 R substituiert sind;
R unabhängig H oder C₄₋₆-Alkyl ist;
R_{b} unabhängig H oder C₁₋₆-Alkyl, alternativ H ist;
alternativ wobei in der Verbindung von Formel (VII)
R₃ Me oder -CH₂CH₃, alternativ Me ist;
beide von Y₁ und Y₂ O sind;
G₇ und G₈ eine Gesamtlänge von 6 oder 7 Kohlenstoffatomen, alternativ 7 Kohlenstoffatomen aufweisen;
G₉ und G₁₀ eine Gesamtlänge von 6 oder 7 Kohlenstoffatomen, alternativ 7 Kohlenstoffatomen aufweisen;
alternativ wobei in der Verbindung von Formel (VII)
R₃ Me oder -CH₂CH₃ ist;
Y₁ und Y₂ unabhängig O oder S sind, wobei Y₁ und Y₂ nicht gleichzeitig O sind;
G₇ und G₈ eine Gesamtlänge von 6, 7 oder 8 Kohlenstoffatomen aufweisen;
G₉ und G₁₀ eine Gesamtlänge von 6, 7 oder 8 Kohlenstoffatomen aufweisen;
alternativ wobei in der Verbindung von Formel (VII)
g 0 oder 1, alternativ 1 ist;
R₃ Me oder -CH₂CH₃, alternativ Me ist;
eines von Y₁ und Y₂ O ist, und das andere S ist;
G₇ und G₈ eine Gesamtlänge von 7 Kohlenstoffatomen aufweisen;
G₉ und G₁₀ eine Gesamtlänge von 7 Kohlenstoffatomen aufweisen;
alternativ wobei in der Verbindung von Formel (VII)
g 0 oder 1, alternativ 0 ist;
R₃ Me oder -CH₂CH₃ ist;
beide von Y₁ und Y₂ S sind;
G₇ und G₈ eine Gesamtlänge von 7 oder 8 Kohlenstoffatomen aufweisen;
G₉ und G₁₀ eine Gesamtlänge von 7 oder 8 Kohlenstoffatomen aufweisen.

4. Nanopartikelzusammensetzung nach Anspruch 1, wobei das ionisierbare kationische Lipid ausgewählt ist aus den Folgenden:

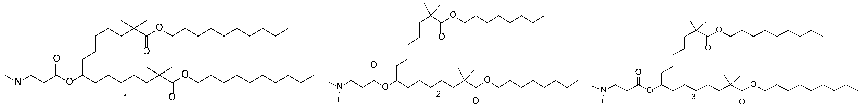

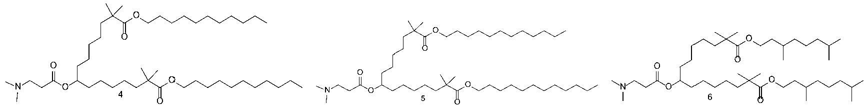

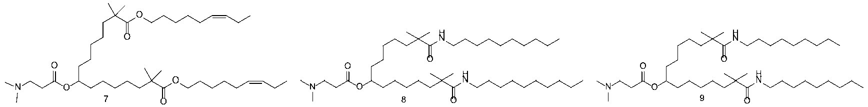

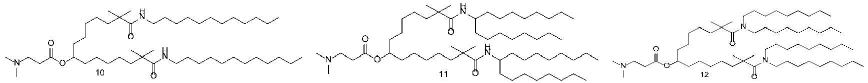

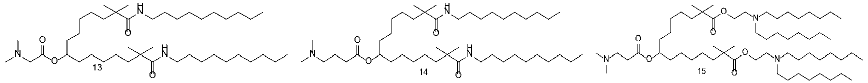

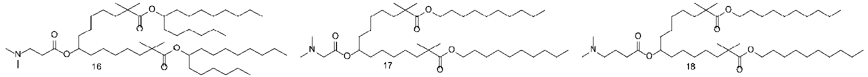

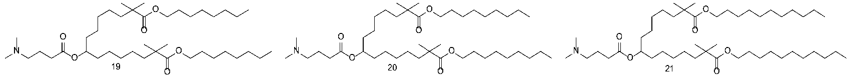

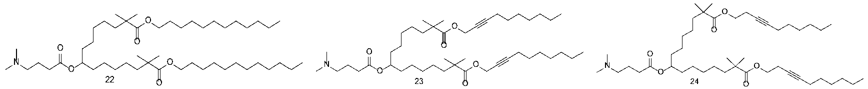

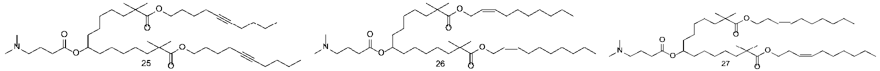

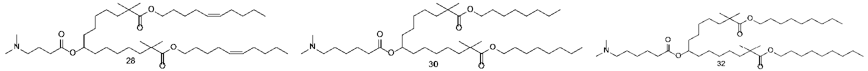

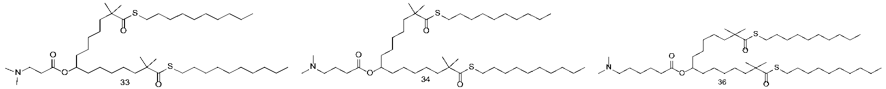

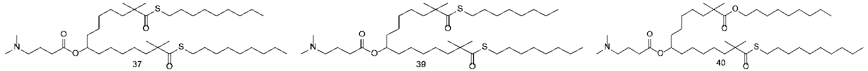

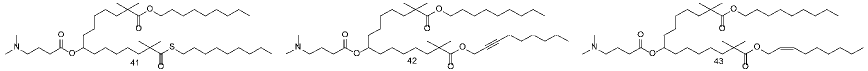

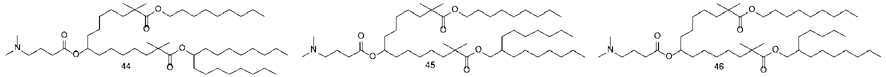

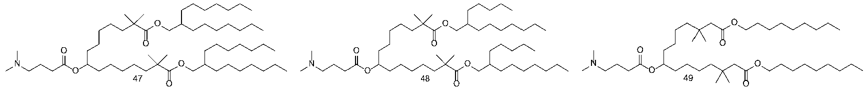

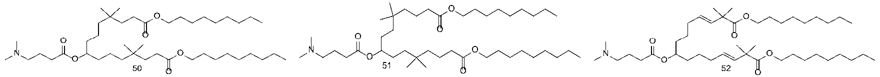

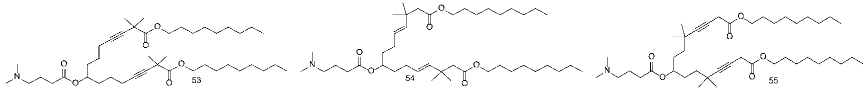

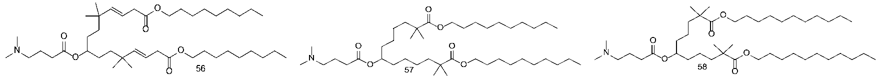

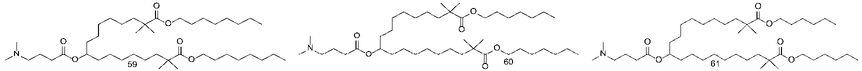

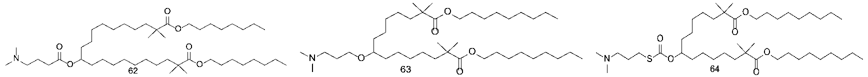

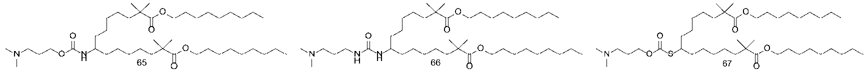

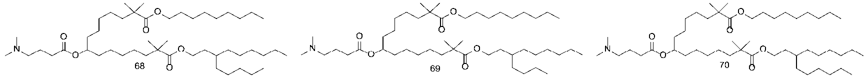

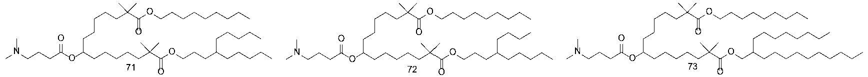

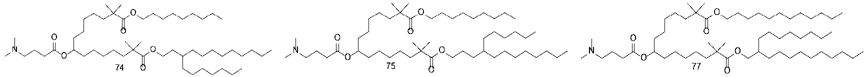

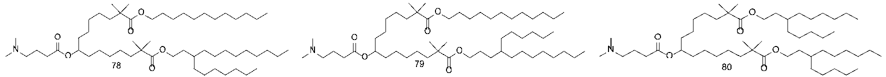

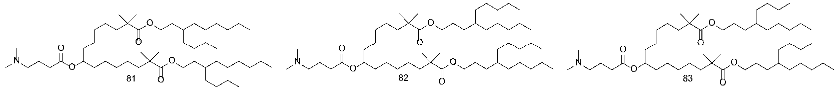

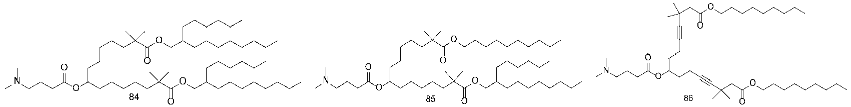

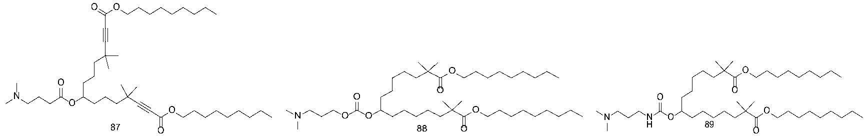

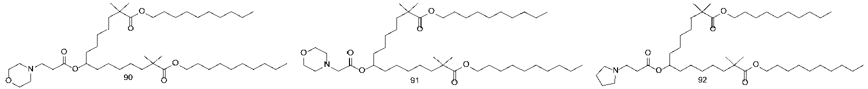

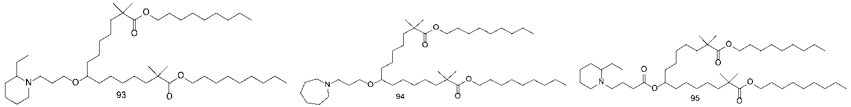

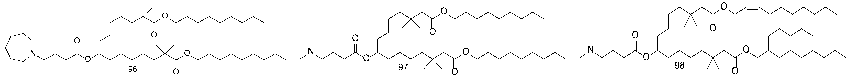

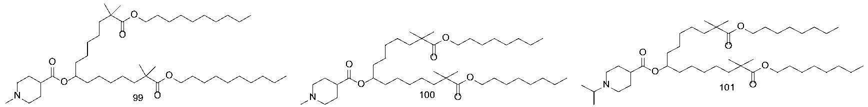

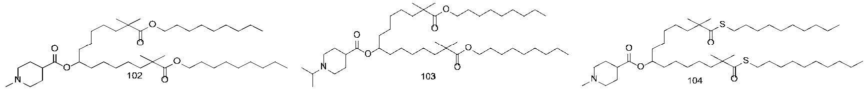

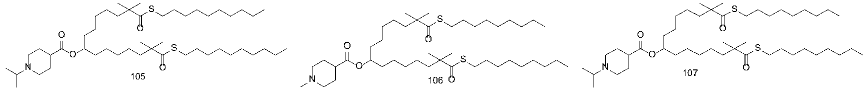

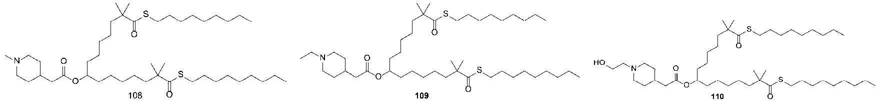

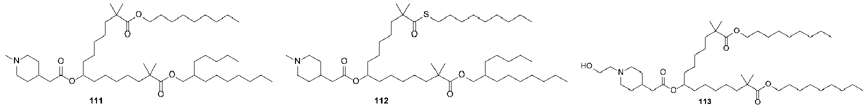

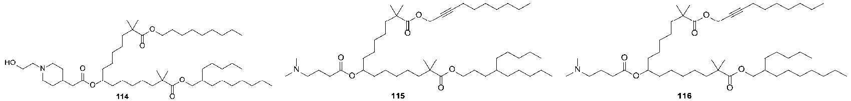

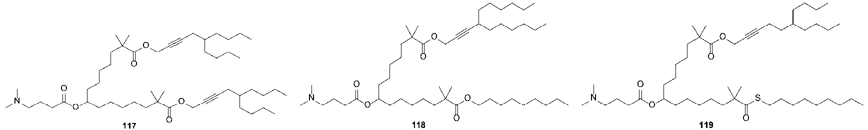

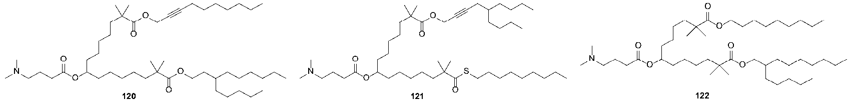

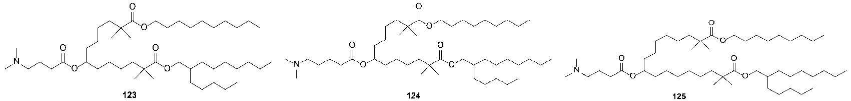

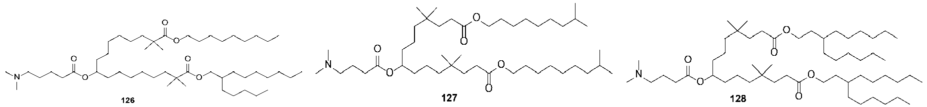

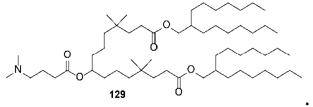

5. Nanopartikelzusammensetzung nach einem der Ansprüche 1 bis 4, wobei die Ladung eine Nukleinsäure ist; das N:P-Molverhältnis von N-Atomen in den ionisierbaren kationischen Lipiden zu P-Atomen in den Ladungsmolekülen 1-15:1, alternativ 3-12:1, alternativ 3-7:1, alternativ 6-12:1, alternativer 3-6:1 ist;
alternativ das N:P-Molverhältnis von N-Atomen in den ionisierbaren kationischen Lipiden zu P-Atomen in den Ladungsmolekülen 1-10:1, alternativ 3-6:1, alternativer 3-5:1 ist;
alternativ das N:P-Molverhältnis von N-Atomen in den ionisierbaren kationischen Lipiden zu P-Atomen in den Ladungsmolekülen 1-15:1, alternativ 3-12:1, alternativer 6-12:1 ist;
alternativ das N:P-Molverhältnis von N-Atomen in den ionisierbaren kationischen Lipiden zu P-Atomen in den Ladungsmolekülen 1-10:1, alternativ 3-7:1, alternativer 5:1 ist.

6. Nanopartikelzusammensetzung nach einem der Ansprüche 1 bis 5, wobei die Partikelgröße der Partikel 65 bis 200 nm, alternativ 65 bis 180 nm, alternativ 70 bis 170 nm, alternativer 70 bis 130 nm ist;
alternativ die Partikelgröße der Partikel 70 bis 180 nm, alternativ 80 bis 180 nm, alternativ 90 bis 180 nm, alternativer 100 bis 135 nm ist;
alternativ die Partikelgröße der Partikel 65 bis 160 nm, alternativ 65 bis 150 nm, alternativ 70 bis 150 nm, alternativ 90 bis 130 nm, alternativer 90 bis 115 nm ist;
alternativ die Partikelgröße der Partikel 65 bis 140 nm, alternativ 65 bis 130 nm, alternativ 70 bis 130 nm, alternativer 65 bis 75 nm ist.

7. Nanopartikelzusammensetzung nach einem der Ansprüche 1 bis 6, wobei der Lipidbestandteil die folgenden Komponenten in den Molprozenten umfasst:
ionisierbare kationische Lipide 30 Mol-% bis 55 Mol-%;
Strukturlipide 28 Mol-% bis 64 Mol-%;
neutrale Lipide 5 Mol-% bis 20 Mol-%;
Polymerlipide 1 Mol-% bis 3 Mol-%;
vorzugsweise wobei der Lipidbestandteil die folgenden Komponenten in den Molprozenten umfasst:
ionisierbare kationische Lipide 40 Mol-% bis 52,5 Mol-%, alternativ 42,5 Mol-% bis 50 Mol-%;
Strukturlipide 28 Mol-% bis 54 Mol-%, alternativ 30,6 Mol-% bis 51 Mol-%;
neutrale Lipide 5 Mol-% bis 20 Mol-%;
Polymerlipide 1 Mol-% bis 3 Mol-%, alternativ 1 Mol-% bis 2 Mol-%;
wobei
die Strukturlipide Steroide sind;
die Polymerlipide Polyethylenglykol-Lipide sind;
das ionisierbare kationische Lipid wie definiert in Anspruch 4 ist, alternativ wie folgt ist: 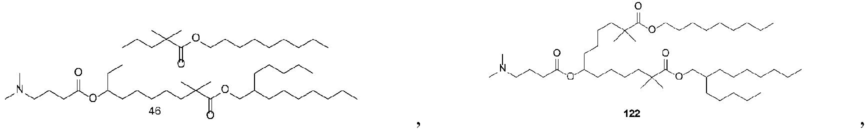 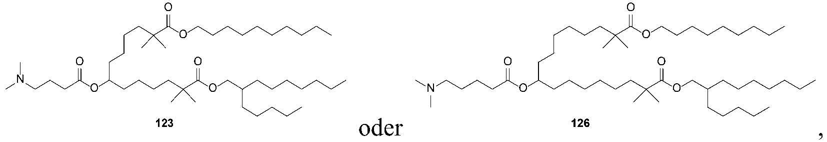 am meisten alternativ wie folgt ist:

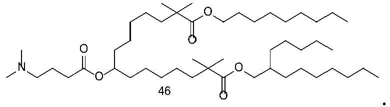

8. Nanopartikelzusammensetzung nach einem der Ansprüche 1 bis 6, wobei der Lipidbestandteil die folgenden Komponenten in den Molprozenten umfasst:
ionisierbare kationische Lipide 30 Mol-% bis 60 Mol-%;
Strukturlipide 27,5 Mol-% bis 66 Mol %;
neutrale Lipide 1,5 Mol-% bis 20 Mol-%;
Polymerlipide 1,5 Mol-% bis 5 Mol-%;
vorzugsweise wobei der Lipidbestandteil die folgenden Komponenten in den Molprozenten umfasst:
ionisierbare kationische Lipide 30 Mol-% bis 50 Mol-%;
Strukturlipide 35 Mol-% bis 66 Mol-%;
neutrale Lipide 1,5 Mol-% bis 20 Mol-%;
Polymerlipide 1,5 Mol-% bis 5 Mol-%;
vorzugsweise wobei der Lipidbestandteil die folgenden Komponenten in den Molprozenten umfasst:
ionisierbare kationische Lipide 30 Mol-% bis 50 Mol-%;
Strukturlipide 38,5 Mol-% bis 63,5 Mol-%;
neutrale Lipide 1,5 Mol-% bis 10 Mol-%;
Polymerlipide 1,5 Mol-% bis 4 Mol-%;
vorzugsweise wobei der Lipidbestandteil die folgenden Komponenten in den Molprozenten umfasst:
ionisierbare kationische Lipide 30 Mol-% bis 50 Mol-%, alternativ 30 Mol-% bis 48,5 Mol-%;
Strukturlipide 43 Mol-% bis 60 Mol-%, alternativ 45,5 Mol-% bis 57,5 Mol-%;
neutrale Lipide 1,5 Mol-% bis 10 Mol-%;
Polymerlipide 1,5 Mol-% bis 4 Mol-%, alternativ 1,5 Mol-% bis 3,5 Mol-%;
wobei
die Strukturlipide Steroide sind;
die Polymerlipide Polyethylenglykol-Lipide sind;
das ionisierbare kationische Lipid wie definiert in Anspruch 4 ist, oder 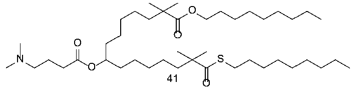 ist; alternativ wie folgt ist: 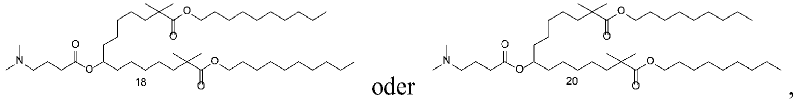 am meisten alternativ wie folgt ist:

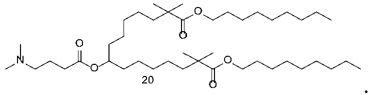

9. Nanopartikelzusammensetzung nach einem der Ansprüche 1 bis 6, wobei der Lipidbestandteil die folgenden Komponenten in den Molprozenten umfasst:
ionisierbare kationische Lipide 27,5 Mol-% bis 55 Mol-%;
Strukturlipide 35 Mol-% bis 68,5 Mol-%;
neutrale Lipide 1,5 Mol-% bis 20 Mol-%;
Polymerlipide 1 Mol-% bis 5 Mol-%;
vorzugsweise wobei der Lipidbestandteil die folgenden Komponenten in den Molprozenten umfasst:
ionisierbare kationische Lipide 27,5 Mol-% bis 42,5 Mol-%, alternativ 30 Mol-% bis 40 Mol-%;
Strukturlipide 41 Mol-% bis 68,5 Mol-%, alternativ 43,5 Mol-% bis 66 Mol-%;
neutrale Lipide 2 Mol-% bis 18 Mol-%, alternativ 2,5 Mol-% bis 15 Mol-%;
Polymerlipide 1 Mol-% bis 4 Mol-%, alternativ 1,5 Mol-% bis 3,5 Mol-%;
wobei
die Strukturlipide Steroide sind;
die Polymerlipide Polyethylenglykol-Lipide sind;
das ionisierbare kationische Lipid wie definiert in Anspruch 4 ist, alternativ wie folgt ist: 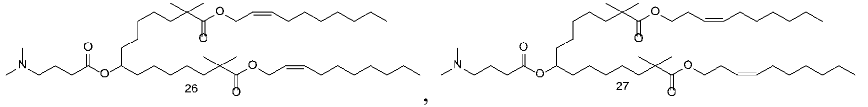 oder 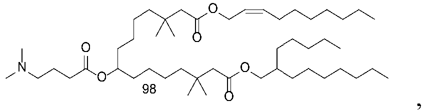 am meisten alternativ wie folgt ist:

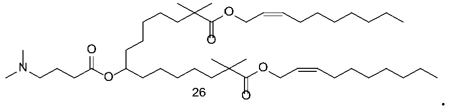

10. Nanopartikelzusammensetzung nach einem der Ansprüche 1 bis 9, wobei die neutralen Lipide ausgewählt sind aus einem oder mehreren von DSPC, DMPC, DOPC, DPPC, POPC, DOPE, DMPE, POPE und DPPE, alternativ DSPC und/oder DOPE;
alternativ die Strukturlipide ausgewählt sind aus einem oder mehreren von Cholesterin, Sitosterol, Coprosterol, Fucosterol, Brassicasterol, Ergosterol, Tomatin, Ursolsäure, α-Tocopherol, Stigmasterol, Avenasterol, Ergocalciferol und Campesterol, alternativ Cholesterin und/oder β-Sitosterol, vorzugsweise Cholesterin;
und/oder, wobei die Polyethylenglykol-Lipide ausgewählt sind aus einem oder mehreren von PEG-modifiziertem Phosphatidylethanolamin, PEG-modifizierter Phosphatidsäure, PEG-modifiziertem Ceramid, PEG-modifiziertem Dialkylamin, PEG-modifiziertem Diacylglycerin und PEG-modifiziertem Dialkylglycerin;
alternativ wobei die Polyethylenglykol-Lipide eine PEG-Gruppierung von etwa 1000 Da bis etwa 20 kDa enthalten, alternativ eine PEG-Gruppierung von etwa 1000 Da bis etwa 5000 Da enthalten;
alternativ die Polyethylenglykol-Lipide ausgewählt sind aus einem oder mehreren von DMPE-PEG1000, DPPE-PEG1000, DSPE-PEG1000, DOPE-PEG1000, DMG-PEG2000, Ceramid-PEG2000, DMPE-PEG2000, DPPE-PEG2000, DSPE-PEG2000, Azido-PEG2000, DSPE-PEG2000-Mannose, Ceramid-PEG5000 und DSPE-PEG5000, alternativ DMG-PEG2000.

11. Nanopartikelzusammensetzung nach einem der Ansprüche 1 bis 4, wobei die Ladung ausgewählt ist aus einem oder mehreren therapeutischen, prophylaktischen und diagnostischen Mitteln;
alternativ das therapeutische, prophylaktische oder diagnostische Mittel eine Nukleinsäure ist; alternativ die Nukleinsäure eines oder mehrere von ASO, RNA oder DNA ist;
alternativ die RNA ausgewählt ist aus einer oder mehreren von kleiner interferierender RNA (siRNA), kurzer Haarnadel-RNA (shRNA), Antisense-RNA (aRNA), Messenger-RNA (mRNA), modifizierter Messenger-RNA (mmRNA), langer nichtkodierender RNA (IncRNA), MicroRNA (miRNA), kleiner aktivierender RNA (saRNA), multimerer kodierender Nukleinsäure (MCNA), polymerer kodierender Nukleinsäure (PCNA), Guide RNA (gRNA), CRISPRRNA (crRNA) oder Nukleasen, alternativ mRNA, noch alternativer modifizierter mRNA.

12. Pharmazeutische Zusammensetzung, umfassend die Nanopartikelzusammensetzung nach einem der Ansprüche 1 bis 11 und pharmazeutisch annehmbare(n) Hilfsstoff(e).

13. Nanopartikelzusammensetzung nach einem der Ansprüche 1 bis 11 oder pharmazeutische Zusammensetzung nach Anspruch 12 zur Verwendung beim Behandeln, Diagnostizieren oder Vorbeugen einer Krankheit oder Zufühen einer Ladung;
wobei die Ladung eines oder mehrere von therapeutischen, prophylaktischen oder diagnostischen Mitteln ist.

## Revendications

1. Une composition de nanoparticules, comprenant un ingrédient lipidique et comprenant éventuellement une charge ;
dans laquelle l'ingrédient lipidique comprend les composants suivants dans les pourcentages molaires suivants :
lipides cationiques ionisables 20 mol% à 85 mol% :
lipides de structure 10 mol% à 75 mol% ;
lipides neutres 1,0 mol% à 30 mol% ;
lipides polymères 0,25 mol% à 10 mol% ;
de préférence, l'ingrédient lipidique comprend les composants suivants dans les pourcentages molaires suivants :
lipides cationiques ionisables 25 mol% à 65 mol% ;
lipides de structure 25 mol% à 70 mol% ;
lipides neutres 1 mol% à 25 mol% ;
lipides polymères 0,5 mol% à 8 mol% ;
de préférence, l'ingrédient lipidique comprend les composants suivants dans les pourcentages molaires suivants :
lipides cationiques ionisables 30 mol% à 60 mol% ;
lipides de structure 27,5 mol% à 66 mol% ;
lipides neutres 1,5 mol% à 20 mol% ;
lipides polymères 1 mol% à 5 mol% ;
de préférence, l'ingrédient lipidique comprend les composants suivants dans les pourcentages molaires suivants :
lipides cationiques ionisables 30 mol% à 50 mol% ;
lipides de structure 30,5 mol% à 66 mol% ;
lipides neutres 1,5 mol% à 20 mol% ;
lipides polymères 1 mol% à 5 mol% ;
dans laquelle,
les lipides de structure sont des stéroïdes ;
les lipides polymères sont des lipides polyéthylène glycolés ;
le lipide cationique ionisable est le composé de formule (VI) ou de formule (VII), ou un sel pharmaceutiquement acceptable, une variante isotopique, un tautomère ou un stéréoisomère de celui-ci, 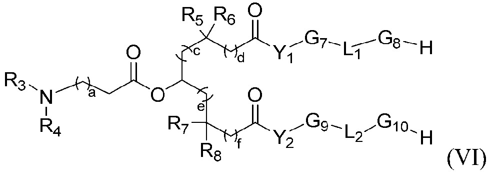 ou 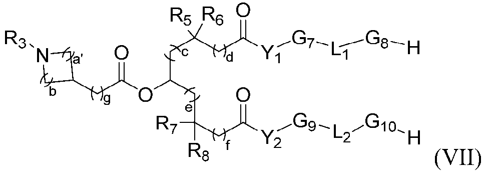 dans laquelle,
a, a', b et g sont indépendamment 0, 1, 2, 3, 4 ou 5, a' et b ne sont pas égaux à 0 en même temps ;
a'+g=0, 1, 2, 3, 4 ou 5 ;
c et e sont indépendamment 3, 4, 5, 6, 7, 8 ou 9 ;
d et f sont indépendamment 0, 1, 2, 3 ou 4 ;
c+d=3, 4, 5, 6, 7, 8 ou 9, e+f=3, 4, 5, 6, 7, 8 ou 9 ;
les groupes méthylènes dans 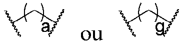 sont éventuellement et indépendamment substitués par 1, 2, 3, 4 ou 5 alkyles C₁₋₆ ;
R₃ et R₄ sont indépendamment H, un alkyle C₁₋₆, un haloalkyle C₁₋₆, un cycloalkyle de 3 à 10 chaînons ou un hétérocyclyle de 3 à 10 chaînons, qui est éventuellement substitué par 1, 2, 3, 4 ou 5 R* ;
ou, R₃ et R₄ sont pris ensemble avec l'atome d'azote auquel ils sont attachés pour former un hétérocyclyle de 3 à 10 chaînons, qui est éventuellement substitué par 1, 2, 3, 4 ou 5 R* ;
R* est indépendamment H, un halogène, un cyano, un alkyle C₁₋₆, un haloalkyle C₁₋₆, -L_{b}-OR_{b} ou -L_{b}-NR_{b}R'_{b} ;
R₅, R₆, R₇ et R₈ sont indépendamment des alkyles C₁₋₃ ;
Y₁ et Y₂ sont indépendamment O, S ou NRₐ ;
L₁ et L₂ sont indépendamment -(CRR')₂-, -CH=CH-, -C≡C- ou -NR"- ;
G₇, G₈, G₉ et G₁₀ sont indépendamment une liaison chimique ou un alkylène C₁₋₁₂, qui est éventuellement substitué par 1, 2, 3, 4, 5 ou 6 R ;
G₇ et G₈ ont une longueur totale de 6, 7, 8, 9, 10, 11 ou 12 atomes de carbone ;
G₉ et G₁₀ ont une longueur totale de 6, 7, 8, 9, 10, 11 ou 12 atomes de carbone ;
R et R' sont indépendamment H, un alkyle C₁₋₁₄, -Lₐ-ORₐ ou -Lₐ-NRₐR'ₐ ;
Lₐ est indépendamment une liaison chimique ou un alkylène C₁₋₁₄ ;
L_{b} est indépendamment une liaison chimique ou un alkylène C₁₋₆ ;
Rₐ et R'ₐ sont indépendamment H, un alkyle C₁₋₁₄, un cycloalkyle de 3 à 10 chaînons ou un hétérocyclyle de 3 à 10 chaînons ;
R_{b} et R'_{b} sont indépendamment H, un alkyle C₁₋₆, un cycloalkyle de 3 à 10 chaînons ou un hétérocyclyle de 3 à 10 chaînons ;
R" est indépendamment H ou un alkyle C₁₋₁₄ ;
alternativement, dans laquelle dans le composé de formule (VI) ou de formule (VII),
a est 0, 1, 2, 3 ou 4, alternativement 1, 2, 3 ou 4, alternativement 2, 3 ou 4 ;
a' et b sont indépendamment 0, 1, 2, 3 ou 4, alternativement 2 ;
g est 0, 1 ou 2, alternativement 0 ou 1 ;
a'+g=0, 1, 2, 3, 4 ou 5, alternativement a'+g=2 ou 3 ;
c et e sont indépendamment 3, 4, 5 ou 6 ;
d et f sont indépendamment 0, 1 ou 2 ;
c+d=4, 5 ou 6, e+f=4, 5 ou 6 ;
les groupes méthylènes dans 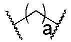 sont éventuellement et indépendamment substitués par 1, 2, 3, 4 ou 5 alkyles C₁₋₆ ;
les groupes méthylènes dans 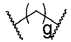 sont éventuellement et indépendamment substitués par 1 ou 2 alkyles C₁₋₆ ;
R₃ et R₄ sont indépendamment des alkyles C₁₋₆, qui est éventuellement substitués par 1, 2 ou 3 R* ;
R* est indépendamment H, un alkyle C₁₋₆, un haloalkyle C₁₋₆ ou -OR_{b} ;
ou, R₃ et R₄ sont pris ensemble avec l'atome d'azote auquel ils sont attachés pour former un hétérocyclyle de 3 à 7 chaînons, alternativement un hétérocyclyle à 5 chaînons, qui est éventuellement substitué par 1, 2 ou 3 R* ;
R₅, R₆, R₇ et R₈ sont indépendamment des alkyles C₁₋₃ ;
Y₁ et Y₂ sont indépendamment O, S ou NRₐ, alternativement O ou S ;
L₁ et L₂ sont indépendamment -(CHR)₂-, -CH=CH-, -C≡C- ou -NR"-, alternativement - (CHR)₂-, -CH=CH- ou -C≡C- ;
G₇ et G₉ sont indépendamment une liaison chimique ou un alkylène C₁₋₆ ;
G₈ et G₁₀ sont indépendamment des alkylènes C₁₋₁₀ ;
G₇ et G₈ ont une longueur totale de 6, 7, 8, 9 ou 10 atomes de carbone ;
G₉ et G₁₀ ont une longueur totale de 6, 7, 8, 9 ou 10 atomes de carbone ;
1, 2 ou 3 méthylènes dans G₇, G₈, G₉ ou G₁₀ sont éventuellement et indépendamment substitués par 1 R ;
R est indépendamment H ou un alkyle C₁₋₈ ;
R" est indépendamment H ou un alkyle C₁₋₁₀ ;
Rₐ est indépendamment H ou un alkyle C₁₋₁₀ ;
R_{b} est indépendamment H ou un alkyle C₁₋₆, alternativement H.

2. La composition de nanoparticules selon la revendication 1, dans laquelle le composé de formule (VI),
a est 0, 1, 2, 3 ou 4, alternativement 1, 2, 3 ou 4, alternativement 2, 3 ou 4 ;
c et e sont indépendamment 3, 4, 5 ou 6 ;
d et f sont indépendamment 0, 1 ou 2 ;
c+d=4, 5 ou 6, e+f=4, 5 ou 6 ;
R₃ et R₄ sont indépendamment des alkyles C₁₋₆ ;
ou, R₃ et R₄ sont pris ensemble avec l'atome d'azote auquel ils sont attachés pour former un hétérocyclyle de 4 à 6 chaînons, alternativement un hétérocyclyle à 5 chaînons, qui est éventuellement substitué par 1, 2 ou 3 R* ;
R* est indépendamment H, un alkyle C₁₋₆ ou un haloalkyle C₁₋₆ ;
R₅, R₆, R₇ et R₈ sont indépendamment des alkyles C₁₋₃ ;
Y₁ et Y₂ sont indépendamment O, S ou NRₐ, alternativement O ou S ;
L₁ et L₂ sont indépendamment -(CHR)₂-, -CH=CH-, -C≡C- ou -NR"-, alternativement - (CHR)₂-, -CH=CH- ou -C≡C- ;
G₇ et G₉ sont indépendamment une liaison chimique ou un alkylène C₁₋₅ ;
G₈ et G₁₀ sont indépendamment des alkylènes C₁₋₈ ;
G₇ et G₈ ont une longueur totale de 6, 7, 8, 9 ou 10 atomes de carbone ;
G₉ et G₁₀ ont une longueur totale de 6, 7, 8, 9 ou 10 atomes de carbone ;
1, 2 ou 3 méthylènes dans G₇, G₈, G₉ ou G₁₀ sont éventuellement et indépendamment substitués par 1 R ;
R est indépendamment H ou un alkyle C₁₋₈, alternativement H ou un alkyle C₁₋₇, alternativement H ou un alkyle C₁₋₆ ;
R" est indépendamment H ou un alkyle C₇₋₉ ;
Rₐ est indépendamment H ou un alkyle C₈₋₁₀ ;
alternativement, dans laquelle dans le composé de formule (VI),
a est 2, 3 ou 4 ;
c et e sont indépendamment 3, 4, 5 ou 6 ;
d et f sont indépendamment 0, 1 ou 2 ;
c+d=4, 5 ou 6, e+f=4, 5 ou 6 ;
R₃ et R₄ sont indépendamment des alkyles C₁₋₆ ;
R₅, R₆, R₇ et R₈ sont indépendamment des alkyles C₁₋₃ ;
Y₁ et Y₂ sont indépendamment O ou S ;
L₁ et L₂ sont indépendamment -(CHR)₂-, -CH=CH- ou -C≡C- ;
G₇ et G₉ sont indépendamment des alkylènes C₁₋₄ ;
G₈ et G₁₀ sont indépendamment des alkylènes C₂₋₇ ;
G₇ et G₈ ont une longueur totale de 6, 7 ou 8 atomes de carbone ;
G₉ et G₁₀ ont une longueur totale de 6, 7 ou 8 atomes de carbone ;
1, 2 ou 3 méthylènes dans G₇, G₈, G₉ ou G₁₀ sont éventuellement et indépendamment substitués par 1 R ;
R est indépendamment H ou un alkyle C₁₋₇ ;
à condition que, lorsque L₁ est -C≡C-, alors G₇ est un alkylène C₁₋₂, ou lorsque L₂ est -C≡C-, alors G₉ est un alkylène C₁₋₂ ;
alternativement, dans laquelle dans le composé de formule (VI),
a est 2, 3 ou 4, alternativement 2 ou 3 ;
c et e sont indépendamment 4, 5 ou 6 ;
d et f sont 0 ;
R₃ et R₄ sont indépendamment des alkyles C₁₋₆ ;
R₅, R₆, R₇ et R₈ sont indépendamment des alkyles C₁₋₃, alternativement Me ;
Y₁ et Y₂ sont O ;
L₁ et L₂ sont indépendamment -(CHR)₂- ou -CH=CH- ;
G₇ et G₉ sont indépendamment -CH₂- ou -CH₂CHR- ;
G₈ et G₁₀ sont indépendamment -(CH₂)₆- ou -(CH₂)₇- ;
G₇ et G₈ ont une longueur totale de 7 ou 8 atomes de carbone ;
G₉ et G₁₀ ont une longueur totale de 7 ou 8 atomes de carbone ;
1, 2 ou 3 groupes méthylènes dans G₈ ou G₁₀ sont éventuellement et indépendamment substitués par 1 R ;
R est indépendamment H ou un alkyle C₄₋₆, alternativement H ou un alkyle C₅ ;
alternativement -G₇-L₁-G₈-H et -G₉-L₂-G₁₀-H ne sont pas -(CH₂)₉CH₃ en même temps ;
alternativement, dans laquelle dans le composé de formule (VI),
a est 2 ;
c et e sont indépendamment 4, 5 ou 6, alternativement 5 ;
d et f sont 0 ;
R₃ et R₄ sont indépendamment des alkyles C₁₋₆ ;
R₅, R₆, R₇ et R₈ sont indépendamment des alkyles C₁₋₃, alternativement Me ;
Y₁ et Y₂ sont indépendamment O ou S ;
l'un de L₁ et L₂ est -C≡C-, l'autre est -(CHR)₂-, ou L₁ et L₂ sont tous deux -C≡C- ;
alternativement l'un des L₁ et L₂ est -C≡C-, l'autre est -(CHR)₂- ;
G₇ et G₉ sont -CH₂- ;
G₈ et G₁₀ sont indépendamment -(CH₂)₆- ou -(CH₂)₇- ;
1 groupement méthylène dans G₈ ou G₁₀ est éventuellement et indépendamment substitué par 1 R ; alternativement, G₈ et G₁₀ sont indépendamment -CHR-(CH₂)₅-, -CHR-(CH₂)₆-, -CH₂-CHR-(CH₂)₄- ou -(CH₂)₂-CHR-(CH₂)₄- ;
R est indépendamment H ou un alkyle C₄₋₆, alternativement H ou un alkyle C₅ ;
à condition qu'un seul parmi -G₇-L₁-G₈-H et -G₉-L₂-G₁₀-H soit substitué par un substituant R non hydrogène et que l'autre ne soit pas substitué ;
alternativement, dans laquelle dans le composé de formule (VI),
a est 2 ;
c et e sont indépendamment 4, 5 ou 6, alternativement 5 ;
d et f sont 0 ;
R₃ et R₄ sont indépendamment des alkyles C₁₋₃, alternativement Me ;
R₅, R₆, R₇ et R₈ sont indépendamment des alkyles C₁₋₃, alternativement Me ;
Y₁ et Y₂ sont indépendamment O ou S, alternativement O ;
L₁ et L₂ sont tous deux -C≡C- ;
G₇ et G₉ sont -CH₂- ;
G₈ et G₁₀ sont indépendamment -(CH₂)₆- ou -(CH₂)₇-, alternativement -(CH₂)₇- ;
alternativement, dans laquelle dans le composé de formule (VI),
a est 2 ;
c et e sont 3 ;
d et f sont 2 ;
R₃ et R₄ sont indépendamment des alkyles C₁₋₃, alternativement Me ;
R₅, R₆, R₇ et R₈ sont indépendamment des alkyles C₁₋₃, alternativement Me ;
Y₁ et Y₂ sont indépendamment O ou S, alternativement O ;
L₁ et L₂ sont -(CHR)₂- ;
G₇ et G₉ sont indépendamment -CH₂- ou -CH₂CHR- ;
G₈ et G₁₀ sont indépendamment -(CH₂)₅-, -(CH₂)₆- ou -(CH₂)₇- ;
G₇ et G₈ ont une longueur totale de 6, 7 ou 8 atomes de carbone, alternativement 7 atomes de carbone ;
G₉ et G₁₀ ont une longueur totale de 6, 7 ou 8 atomes de carbone, alternativement 7 atomes de carbone ;
1, 2 ou 3 groupes méthylènes dans G₈ ou G₁₀ sont éventuellement et indépendamment substitués par 1 R ;
R est indépendamment H ou un alkyle C₁₋₇, alternativement H ou un alkyle C₁₋₆, alternativement Me ;
alternativement, dans laquelle dans le composé de formule (VI),
a est 2 ;
c et e sont 4, 5 ou 6, alternativement 5 ;
d et f sont 0 ;
R₃ et R₄ sont indépendamment des alkyles C₁₋₆ ;
R₅, R₆, R₇ et R₈ sont indépendamment des alkyles C₁₋₃, alternativement Me ;
Y₁ et Y₂ sont S ;
L₁ et L₂ sont -(CHR)₂- ;
G₇ et G₉ sont indépendamment -CH₂- ou -CH₂CHR- ;
G₈ et G₁₀ sont indépendamment -(CH₂)₅-, -(CH₂)₆- ou -(CH₂)₇- ;
G₇ et G₈ ont une longueur totale de 7 ou 8 atomes de carbone, alternativement 8 atomes de carbone ;
G₉ et G₁₀ ont une longueur totale de 7 ou 8 atomes de carbone, alternativement 8 atomes de carbone ;
1, 2 ou 3 groupes méthylènes dans G₈ ou G₁₀ sont éventuellement et indépendamment substitués par 1 R ;
R est indépendamment H ou un alkyle C₄₋₆, alternativement H ou un alkyle C₅.

3. La composition de nanoparticules selon la revendication 1, dans laquelle le composé de formule (VII),
a' et b sont 2 ;
g est 0 ou 1 ;
c et e sont 5 ;
d et f sont 0 ;
R₃ est un alkyle C₁₋₆, qui est éventuellement substitué par 1, 2 ou 3 R* ;
R* est indépendamment H, un alkyle C₁₋₆, un haloalkyle C₁₋₆ ou -OR_{b}, alternativement H, un alkyle C₁₋₆ ou un haloalkyle C₁₋₆ ;
R₅, R₆, R₇ et R₈ sont indépendamment des alkyles C₁₋₃, alternativement Me ;
Y₁ et Y₂ sont indépendamment O ou S ;
L₁ et L₂ sont -(CHR)₂- ;
G₇ et G₉ sont indépendamment -CH₂- ou -CH₂CHR- ;
G₈ et G₁₀ sont indépendamment -(CH₂)₅-, -(CH₂)₆- ou -(CH₂)₇- ;
G₇ et G₈ ont une longueur totale de 6, 7 ou 8 atomes de carbone ;
G₉ et G₁₀ ont une longueur totale de 6, 7 ou 8 atomes de carbone ;
1, 2 ou 3 groupes méthylènes dans G₈ ou G₁₀ sont éventuellement et indépendamment substitués par 1 R ;
R est indépendamment H ou un alkyle C₄₋₆ ;
R_{b} est indépendamment H ou un alkyle C₁₋₆, alternativement H ;
alternativement, dans laquelle dans le composé de formule (VII),
R₃ est Me ou -CH₂CH₃, alternativement Me ;
Y₁ et Y₂ sont tous deux O ;
G₇ et G₈ ont une longueur totale de 6 ou 7 atomes de carbone, alternativement 7 atomes de carbone ;
G₉ et G₁₀ ont une longueur totale de 6 ou 7 atomes de carbone, alternativement 7 atomes de carbone ;
alternativement, dans laquelle dans le composé de formule (VII),
R₃ est Me ou -CH₂CH₃ ;
Y₁ et Y₂ sont indépendamment O ou S, Y₁ et Y₂ n'étant pas simultanément O ;
G₇ et G₈ ont une longueur totale de 6, 7 ou 8 atomes de carbone ;
G₉ et G₁₀ ont une longueur totale de 6, 7 ou 8 atomes de carbone ;
alternativement, dans laquelle dans le composé de formule (VII),
g est 0 ou 1, alternativement 1 ;
R₃ est Me ou -CH₂CH₃, alternativement Me ;
l'un des Y₁ et Y₂ est O, et l'autre est S ;
G₇ et G₈ ont une longueur totale de 7 atomes de carbone ;
G₉ et G₁₀ ont une longueur totale de 7 atomes de carbone ;
alternativement, dans laquelle dans le composé de formule (VII),
g est 0 ou 1, alternativement 0 ;
R₃ est Me ou -CH₂CH₃ ;
Y₁ et Y₂ sont tous deux S ;
G₇ et G₈ ont une longueur totale de 7 ou 8 atomes de carbone ;
G₉ et G₁₀ ont une longueur totale de 7 ou 8 atomes de carbone.

4. La composition de nanoparticules selon la revendication 1, dans laquelle le lipide cationique ionisable est sélectionné parmi les suivants :

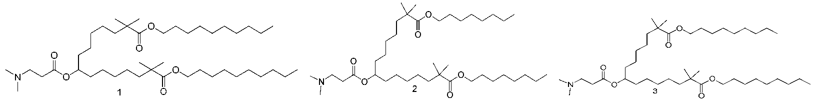

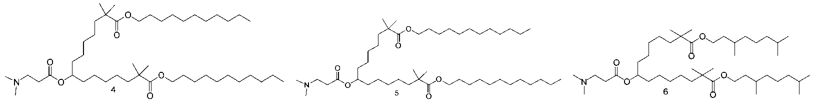

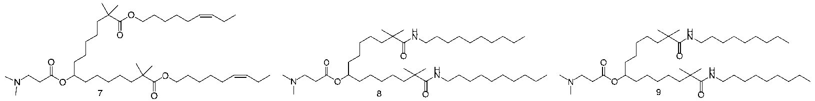

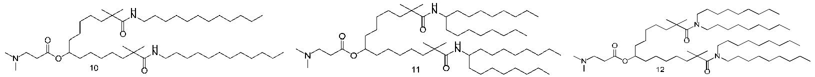

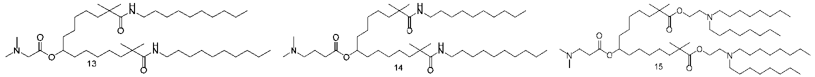

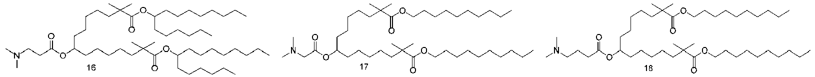

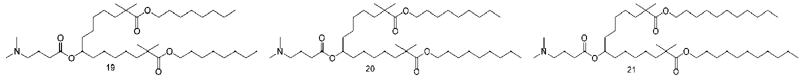

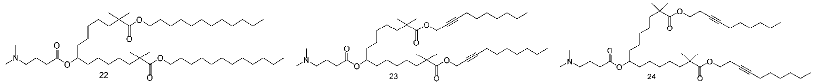

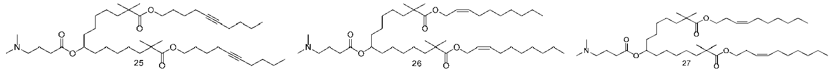

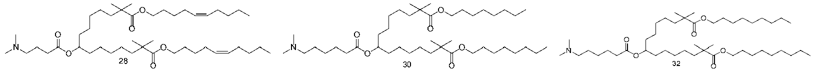

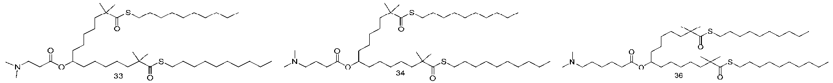

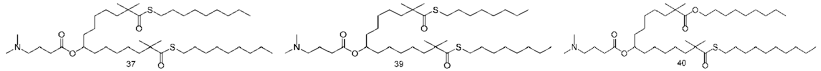

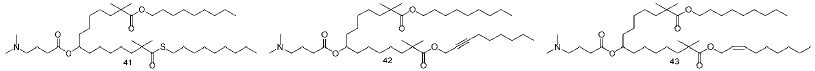

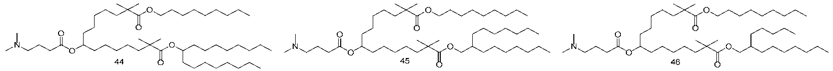

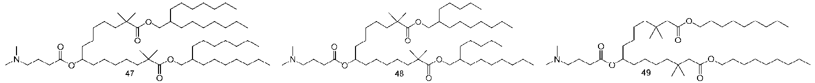

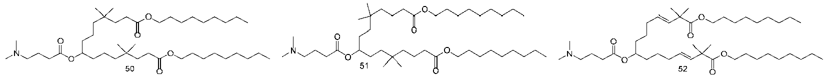

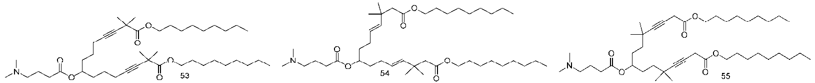

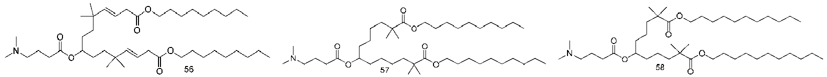

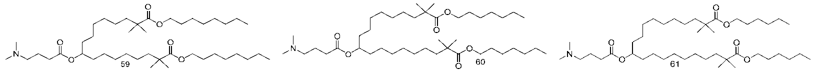

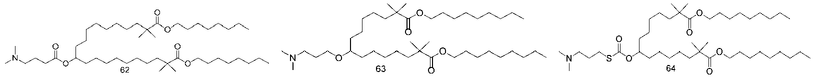

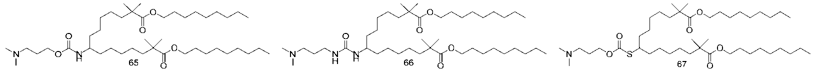

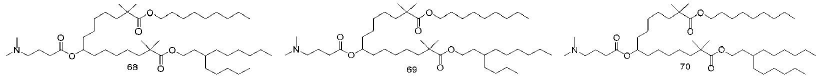

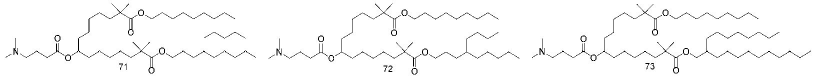

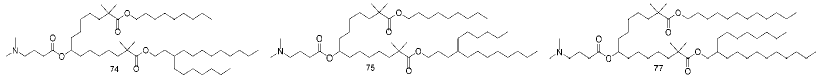

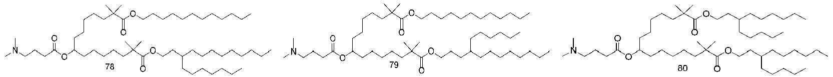

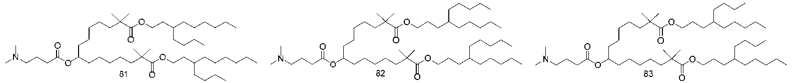

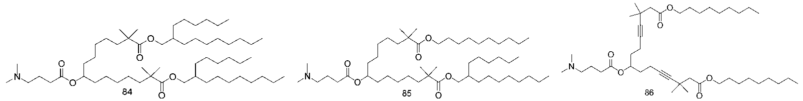

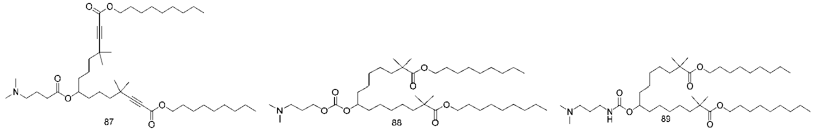

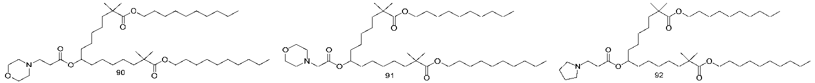

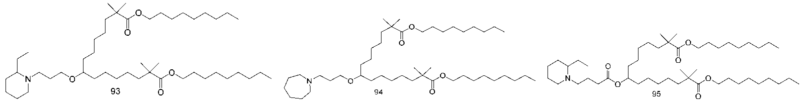

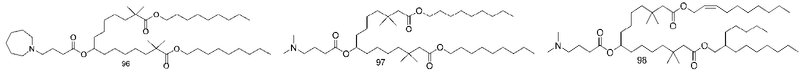

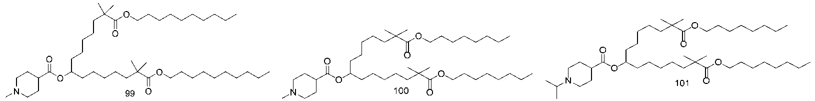

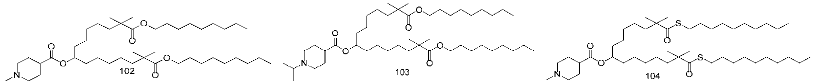

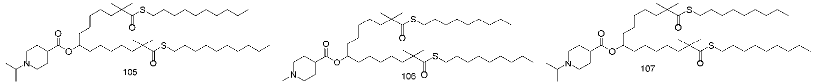

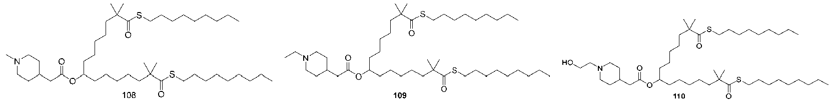

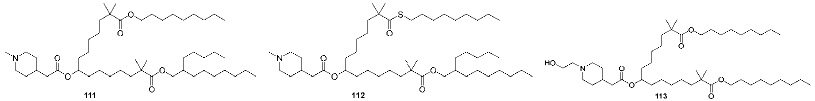

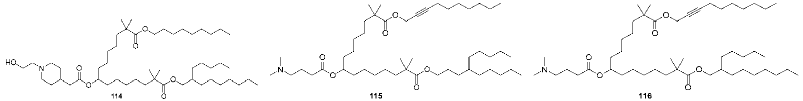

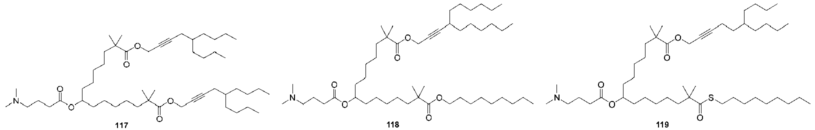

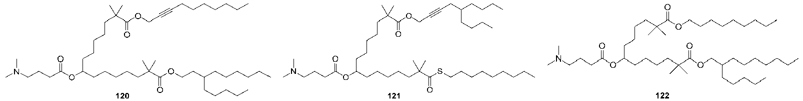

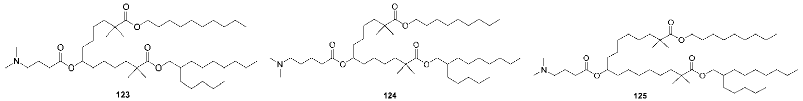

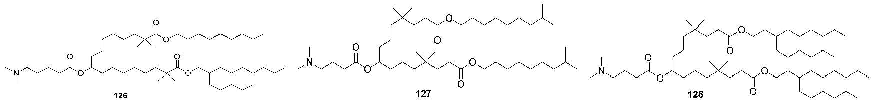

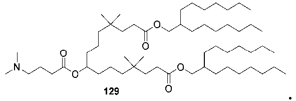

5. La composition de nanoparticules selon l'une quelconque des revendications 1 à 4, dans laquelle, la charge est un acide nucléique ; le rapport molaire N:P des atomes N dans les lipides cationiques ionisables aux atomes P dans les molécules chargées est de 1-15:1, alternativement 3-12:1, alternativement 3-7:1, alternativement 6-12:1, plus alternativement 3-6:1 ;
alternativement, le rapport molaire N:P des atomes N dans les lipides cationiques ionisables aux atomes P dans les molécules chargées est de 1-10:1, alternativement de 3-6:1, plus alternativement de 3-5:1 ;
alternativement, le rapport molaire N:P des atomes N dans les lipides cationiques ionisables aux atomes P dans les molécules chargées est de 1-15:1, alternativement de 3-12:1, plus alternativement de 6-12:1 ;
alternativement, le rapport molaire N:P des atomes N dans les lipides cationiques ionisables aux atomes P dans les molécules chargées est de 1-10:1, alternativement de 3-7:1, ou encore de 5:1.

6. La composition de nanoparticules selon l'une quelconque des revendications 1 à 5, dans laquelle, la taille des particules est de 65 à 200 nm, alternativement de 65 à 180 nm, alternativement de 70 à 170 nm, plus alternativement de 70 à 130 nm ;
alternativement, la taille des particules est de 70 à 180 nm, alternativement de 80 à 180 nm, alternativement de 90 à 180 nm, plus alternativement de 100 à 135 nm ;
alternativement, la taille des particules est de 65 à 160 nm, alternativement de 65 à 150 nm, alternativement de 70 à 150 nm, alternativement de 90 à 130 nm, plus alternativement de 90 à 115 nm ;
alternativement, la taille des particules est de 65 à 140 nm, alternativement de 65 à 130 nm, alternativement de 70 à 130 nm, plus alternativement de 65 à 75 nm.

7. La composition de nanoparticules selon l'une quelconque des revendications 1 à 6, dans laquelle l'ingrédient lipidique comprend les composants suivants dans les pourcentages molaires suivants :
lipides cationiques ionisables 30 mol% à 55 mol% ;
lipides de structure 28 mol% à 64 mol% ;
lipides neutres 5 mol% à 20 mol% ;
lipides polymères 1 mol% à 3 mol% ;
de préférence, l'ingrédient lipidique comprend les composants suivants dans les pourcentages molaires suivants :
lipides cationiques ionisables 40 mol% à 52,5 mol%, alternativement 42,5 mol% à 50 mol% ;
lipides de structure 28 mol% à 54 mol%, alternativement 30,6 mol% à 51 mol% ;
lipides neutres 5 mol% à 20 mol% ;
lipides polymères 1 mol% à 3 mol%, alternativement 1 mol% à 2 mol% ;
dans laquelle,
les lipides de structure sont des stéroïdes ;
les lipides polymères sont des lipides polyéthylène glycolés ;
le lipide cationique ionisable est tel que défini dans la revendication 4, alternativement est : 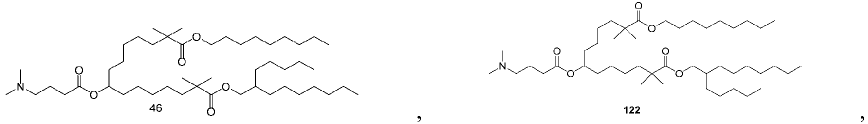 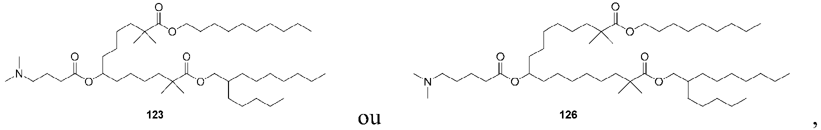 plus alternativement est :

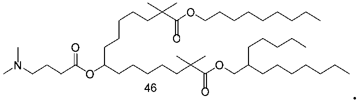

8. La composition de nanoparticules selon l'une quelconque des revendications 1 à 6, dans laquelle l'ingrédient lipidique comprend les composants suivants dans les pourcentages molaires suivants :
lipides cationiques ionisables 30 mol% à 60 mol% ;
lipides de structure 27,5 mol% à 66 mol% ;
lipides neutres 1,5 mol% à 20 mol% ;
lipides polymères 1,5 mol% à 5 mol% ;
de préférence, l'ingrédient lipidique comprend les composants suivants dans les pourcentages molaires suivants :
lipides cationiques ionisables 30 mol% à 50 mol% ;
lipides de structure 35 mol% à 66 mol% ;
lipides neutres 1,5 mol% à 20 mol% ;
lipides polymères 1,5 mol% à 5 mol% ;
de préférence, l'ingrédient lipidique comprend les composants suivants dans les pourcentages molaires suivants :
lipides cationiques ionisables 30 mol% à 50 mol% ;
lipides de structure 38,5 mol% à 63.5 mol% ;
lipides neutres 1,5 mol% à 10 mol% ;
lipides polymères 1,5 mol% à 4 mol% ;
de préférence, l'ingrédient lipidique comprend les composants suivants dans les pourcentages molaires suivants :
lipides cationiques ionisables 30 mol% à 50 mol%, alternativement 30 mol% à 48,5 mol% ;
lipides de structure 43 mol% à 60 mol%, alternativement 45,5 mol% à 57,5 mol% ;
lipides neutres 1,5 mol% à 10 mol% ;
lipides polymères 1,5 mol% à 4 mol%, alternativement 1,5 mol% à 3,5 mol% ;
dans laquelle,
les lipides de structure sont des stéroïdes ;
les lipides polymères sont des lipides polyéthylène glycolés ;
le lipide cationique ionisable est tel que défini dans la revendication 4, ou est 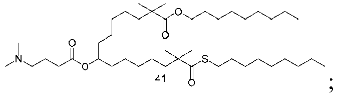 alternativement est : 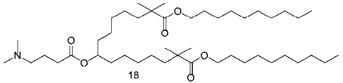 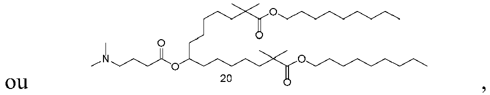 plus alternativement est :

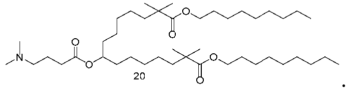

9. La composition de nanoparticules selon l'une quelconque des revendications 1 à 6, dans laquelle l'ingrédient lipidique comprend les composants suivants dans les pourcentages molaires suivants :
lipides cationiques ionisables 27,5 mol% à 55 mol% ;
lipides de structure 35 mol% à 68,5 mol% ;
lipides neutres 1,5 mol% à 20 mol% ;
lipides polymères 1 mol% à 5 mol% ;
de préférence, l'ingrédient lipidique comprend les composants suivants dans les pourcentages molaires suivants :
lipides cationiques ionisables 27,5 mol% à 42,5 mol%, alternativement 30 mol% à 40 mol% ;
lipides de structure 41 mol% à 68,5 mol%, alternativement 43,5 mol% à 66 mol% ;
lipides neutres 2 mol% à 18 mol%, alternativement 2,5 mol% à 15 mol% ;
lipides polymères 1 mol% à 4 mol%, alternativement 1,5 mol% à 3,5 mol% ;
dans laquelle,
les lipides de structure sont des stéroïdes ;
les lipides polymères sont des lipides polyéthylène glycolés ;
le lipide cationique ionisable est tel que défini dans la revendication 4, alternativement est : 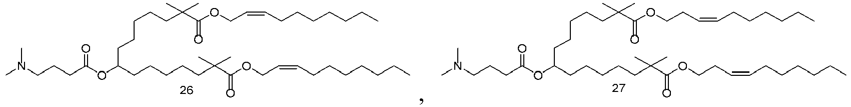 ou 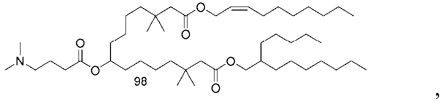 plus alternativement est :

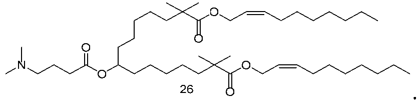

10. La composition de nanoparticules selon l'une quelconque des revendications 1 à 9, dans laquelle, les lipides neutres sont sélectionnés parmi un ou plusieurs des DSPC, DMPC, DOPC, DPPC, POPC, DOPE, DMPE, POPE et DPPE, alternativement DSPC et/ou DOPE ;
alternativement, les lipides structuraux sont sélectionnés parmi un ou plusieurs des suivants :
cholestérol, sitostérol, coprostérol, fucostérol, brassicastérol, ergostérol, tomatine, acide ursolique, α-tocophérol, stigmastérol, avénastérol, ergocalciférol et campestérol, alternativement cholestérol et/ou β-sitostérol, de préférence cholestérol ;
et/ou, dans laquelle, les lipides polyéthylène glycolés sont sélectionnés parmi un ou plusieurs des suivants : phosphatidyléthanolamine modifiée par PEG, acide phosphatidique modifié par PEG, céramide modifié par PEG, dialkylamine modifiée par PEG, diacylglycérol modifié par PEG et dialkylglycérol modifié par PEG ;
alternativement, les lipides polyéthylène glycolés contiennent une fraction PEG d'environ 1000 Da à environ 20 kDa, contiennent alternativement une fraction PEG d'environ 1000 Da à environ 5000 Da ;
alternativement, les lipides polyéthylène glycolés sont sélectionnés parmi un ou plusieurs des suivants : DMPE-PEG1000, DPPE-PEG1000, DSPE-PEG1000, DOPE-PEG1000, DMG-PEG2000, Ceramide-PEG2000, DMPE-PEG2000, DPPE-PEG2000, DSPE-PEG2000, Azido-PEG2000, DSPE-PEG2000-Mannose, Ceramide-PEG5000 et DSPE-PEG5000, ou encore DMG-PEG2000.

11. La composition de nanoparticules selon l'une quelconque des revendications 1 à 4, dans laquelle, la charge est choisie parmi un ou plusieurs agents thérapeutiques, prophylactiques et diagnostiques ;
alternativement, l'agent thérapeutique, prophylactique ou diagnostique est un acide nucléique ; alternativement, l'acide nucléique est un ou plusieurs parmi l'ASO, l'ARN ou l'ADN ;
alternativement, l'ARN est sélectionné parmi un ou plusieurs ARN interférents (siARN), ARN en épingle à cheveux courts (shARN), ARN antisens (aARN), ARN messager (ARNm), ARN messager modifié (mmARN), ARN non codant long (ARNnc long), microARN (miARN), petit ARN activateur (saARN), acide nucléique codant multimérique (MCNA), acide nucléique codant polymérique (PCNA), ARN guide (gARN), ARN CRISPR (crARN) et nucléases, ou encore ARNm modifié.

12. Une composition pharmaceutique, comprenant la composition de nanoparticules selon l'une quelconque des revendications 1 à 11, et un ou plusieurs excipients pharmaceutiquement acceptables.

13. La composition de nanoparticules selon l'une quelconque des revendications 1 à 11, ou la composition pharmaceutique selon la revendication 12, pour être utilisée dans le traitement, le diagnostic ou la prévention d'une maladie, ou pour administrer une charge ;
dans laquelle, la charge est un ou plusieurs agents thérapeutiques, prophylactiques ou diagnostiques.
